# EUROPEAN PATENT APPLICATION

(11) **EP 2 298 880 A1**
(43) Date of publication of application: **23.03.2011**
(21) Application number: 09722714.4
(22) Date of filing: 18.03.2009
(51) Int. Cl.: C12N 15/09, C12N 1/21, C12P 7/64, C12P 13/04, C12P 19/00, C12P 19/34, C12P 21/02

(54) **INDUSTRIALLY USEFUL MICROORGANISM**

(30) Priority: 18.03.2008 JP 2008068793
(71) Applicant: Kyowa Hakko Kirin Co., Ltd., Tokyo 100-8185 (JP)
(72) Inventor: MASUDA, Kimie, Tokyo 194-0032 (JP); NAKAMURA, Noriko, Tokyo 100-8185 (JP); MIZOGUCHI, Hiroshi, Tokyo 100-8185 (JP); MORI, Hideo, Tokyo 194-8533 (JP)
(74) Representative: O'Brien, Simon Warwick
(86) International application number: PCT/JP2009/055267
(87) International publication number: WO 2009/116566

(57) **Abstract**

An object of the present invention is to provide an *E*. *coli* mutant strain having a chromosomal DNA not less than 1040 kbp shorter than that of a wild-type *Escherichia coli,* and a production method of a useful substance using the variant.

## Description

### Technical Field

The present invention relates to an *E. coli* mutant strain having chromosomal DNA shorter than that of a wild-type *E. coli* strain and a process for producing a useful substance using such mutant strain.

### Background Art

All nucleotide sequences of the chromosomal DNAs of *E*. *coli* have been elucidated (Non-Patent Documents 1 and 2). Also, a method wherein a given gene or a given region on chromosomal DNA of *E*. *coli* is deleted by homologous recombination has been known (Non-Patent Document 3).

As reduced-genome strains defective in plural regions on the chromosome, MDS43 strain defective in 708 kbp (non-patent document 4), Δ16 strain defective in 1377 kbp (non-patent document 5) and Step28 strain defective in 1027 kbp (patent document 1) are already known.

MDS43 strains are known to grow equivalently to wild-type Escherichia coli strain MG1655, which is a parental strain, in a minimal medium or LB medium. In addition, Step28 strain shows an increased bacterial cell amount on termination of culture as compared to parental strain W3110red strain free of defective region on the chromosome, and is known to have a high ATP regeneration activity. Moreover, threonine production using Step28T strain, wherein threonine biosynthetic gene is reinforced, shows improved threonine productivity as compared to the use of W3110red strain.

However, Δ16 strain is known to show low growth rate as compared to the parental strain, MG1655 strain. Thus, the phenotype of a reduced-genome strain varies depending on the strain, and how the deletion of a chromosomal region exceeding 1000 kbp changes the properties of Escherichia coli is difficult to predict. Therefore, Escherichia coli defective in a region of not less than 1000 kbp in the chromosomal DNA does not necessarily show useful properties.

In addition, an *E. coli* mutant, which lacks regions of 1040kbp or more on chromosomal DNA and a process for producing a useful substance using such mutant have not yet been known.
non-patent document 1: Science, 277, 1453 (1997)
non-patent document 2: Nucleic Acids Research, 34, 1 (2006)
non-patent document 3: J. Bacteriol., 180, 2063 (1998)
non-patent document 4: Science, 312, 1044 (2006)
non-patent document 5: Mol. Microbiol., 55, 137 (2005)
patent document 1: WO2006/057341

### Disclosure of the Invention

### Problems to be Solved by the Invention

It is an object of the present invention to provide an industrially useful *E. coli* mutant strain that lacks regions of 1040kbp or more on chromosomal DNA and a process for producing a useful substance using such mutant.

### Means of Solving the Problems

The present invention relates to the following (1) - (28).
(1) An *E. coli* mutant strain comprising chromosomal DNA comprising the following genes [1] or homologous genes thereof, not comprising the genes [2] or homologous genes thereof, and being at least 1040 kbp shorter than that of a wild-type *E. coli* strain, provided that when the following genes [1] or homologous genes thereof comprise genes that wild-type *E. coli* strains do not originally contain (hereafter referred to as "NC genes"), the aforementioned *E. coli* mutant strain derived from the wild-type *E. coli* strain does not necessarily have the NC gene.
   [1] thrL gene, thrA gene, thrB gene, thrC gene, talB gene, mog gene, htgA gene, dnaK gene, dnaJ gene, mokC gene, hokC gene, sokC gene, nhaA gene, nhaR gene, rpsT gene, ribF gene, ileS gene, lspA gene, fkpB gene, ispH gene, rihC gene, dapB gene, carA gene, carB gene, caiF gene, caiE gene, caiD gene, caiC gene, caiB gene, caiA gene, caiT gene, fixA gene, fixB gene, fixC gene, fixX gene, kefF gene, kefC gene, folA gene, apaH gene, apaG gene, ksgA gene, pdxA gene, surA gene, imp gene, djlA gene, rluA gene, hepA gene, polB gene, leuD gene, leuC gene, leuB gene, leuA gene, leuL gene, leuO gene, ilvI gene, ilvH gene, fruR gene, mraZ gene, mraW gene, ftsL gene, ftsI gene, murE gene, murF gene, mraY gene, murD gene, ftsW gene, murG gene, murC gene, ddlB gene, ftsQ gene, ftsA gene, ftsZ gene, lpxC gene, secM gene, secA gene, mutT gene, coaE gene, guaC gene, hofC gene, hofB gene, ppdD gene, nadC gene, ampD gene, ampE gene, aroP gene, pdhR gene, aceE gene, aceF gene, lpd gene, acnB gene, speD gene, speE gene, cueO gene, gcd gene, hpt gene, can gene, panD gene, panC gene, panB gene, htrE gene, ecpD gene, folK gene, pcnB gene, dksA gene, sfsA gene, ligT gene, hrpB gene, mrcB gene, hemL gene, clcA gene, btuF gene, pfs gene, dgt gene, degP gene, cdaR gene, dapD gene, glnD gene, map gene, tff gene, rpsB gene, tsf gene, pyrH gene, frr gene, dxr gene, ispU gene, cdsA gene, hlpA gene, lpxD gene, fabZ gene, lpxA gene, lpxB gene, rnhB gene, dnaE gene, accA gene, ldcC gene, tilS gene, rof gene, nlpE gene, proS gene, rcsF gene, metQ gene, metI gene, metN gene, gmhB gene, rrsH gene, ileV gene, alaV gene, rrlH gene, rrfH gene, aspU gene, dkgB gene, mltD gene, gloB gene, rnhA gene, dnaQ gene, aspV gene, ivy gene, fadE gene, lpcA gene, pepD gene, gpt gene, frsA gene, crl gene, phoE gene, proB gene, proA gene, thrW gene, hemB gene, proC gene, aroL gene, aroM gene, rdgC gene, mak gene, araJ gene, sbcC gene, sbcD gene, phoB gene, phoR gene, brnQ gene, proY gene, malZ gene, queA gene, tgt gene, secD gene, secF gene, tsx gene, ribD gene, ribE gene, nusB gene, thiL gene, pgpA gene, dxs gene, ispA gene, xseB gene, thiI gene, panE gene, cyoE gene, cyoD gene, cyoC gene, cyoB gene, cyoA gene, ampG gene, bolA gene, tig gene, clpP gene, clpX gene, lon gene, hupB gene, ppiD gene, cof gene, mdlA gene, mdlB gene, glnK gene, amtB gene, tesB gene, ffs gene, maa gene, hha gene, acrB gene, acrA gene, acrR gene, kefA gene, priC gene, apt gene, dnaX gene, recR gene, htpG gene, adk gene, hemH gene, aes gene, gsk gene, fsr gene, ushA gene, copA gene, cueR gene, gcl gene, hyi gene, purK gene, purE gene, lpxH gene, ppiB gene, cysS gene, folD gene, sfmA gene, sfmC gene, sfmD gene, sfmH gene, sfmF gene, fimZ gene, argU gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, tatE gene, lipA gene, lipB gene, dacA gene, rlpA gene, mrdB gene, mrdA gene, cobC gene, nadD gene, holA gene, rlpB gene, leuS gene, lnt gene, miaB gene, ubiF gene, glnX gene, glnV gene, metU gene, glnW gene, glnU gene, leuW gene, metT gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, nei gene, abrB gene, gltA gene, sdhC gene, sdhD gene, sdhA gene, sdhB gene, sucA gene, sucB gene, sucC gene, sucD gene, mngR gene, mngA gene, mngB gene, cydA gene, cydB gene, tolQ gene, tolR gene, tolA gene, tolB gene, pal gene, lysT gene, valT gene, lysW gene, valZ gene, lysY gene, lysZ gene, lysQ gene, nadA gene, pnuC gene, zitB gene, aroG gene, gpmA gene, galM gene, galK gene, galT gene, galE gene, modF gene, modE gene, modA gene, modB gene, mode gene, bioA gene, bioB gene, bioF gene, bioC gene, bioD gene, uvrB gene, moaA gene, moaB gene, moaC gene, moaD gene, moaE gene, rhlE gene, dps gene, rhtA gene, ompX gene, rybA gene, mntR gene, fsaA gene, moeB gene, moeA gene, iaaA gene, cmr gene, rybB gene, grxA gene, poxB gene, hcr gene, hcp gene, aqpZ gene, macA gene, macB gene, cspD gene, clpS gene, clpA gene, serW gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, ihfB gene, msbA gene, lpxK gene, kdsB gene, smtA gene, mukF gene, mukE gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, serT gene, hyaA gene, hyaB gene, hyaC gene, hyaD gene, hyaE gene, hyaF gene, appC gene, appB gene, appA gene, etp gene, serX gene, csgG gene, csgF gene, csgE gene, csgD gene, csgB gene, csgA gene, csgC gene, mdoC gene, mdoG gene, mdoH gene, msyB gene, mdtG gene, IpxL gene, solA gene, dinI gene, pyrC gene, grxB gene, mdtH gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, sraB gene, rpmF gene, plsX gene, fabH gene, fabD gene, fabG gene, acpP gene, fabF gene, pabC gene, tmk gene, holB gene, ptsG gene, fhuE gene, hinT gene, nagZ gene, ndh gene, mfd gene, lolC gene, lolD gene, lolE gene, cobB gene, potD gene, potC gene, potB gene, potA gene, pepT gene, phoQ gene, phoP gene, purB gene, hflD gene, trmU gene, minE gene, minD gene, minC gene, hlyE gene, umuD gene, umuC gene, dsbB gene, nhaB gene, fadR gene, dadA gene, dadX gene, cvrA gene, ldcA gene, emtA gene, treA gene, dhaH gene, dhaL gene, dhaK gene, dhaR gene, pth gene, prsA gene, ispE gene, lolB gene, hemA gene, prfA gene, prmC gene, kdsA gene, ldrA gene, rdlA gene, ldrB gene, rdlB gene, ldrC gene, rdlC gene, chaA gene, chaB gene, chaC gene, narL gene, narX gene, narK gene, narG gene, narH gene, narJ gene, narI gene, rttR gene, tpr gene, tyrV gene, tyrT gene, purU gene, rssA gene, rssB gene, galU gene, hns gene, tdk gene, adhE gene, oppA gene, oppB gene, oppC gene, oppD gene, oppF gene, cls gene, kch gene, tonB gene, ompW gene, trpA gene, trpB gene, trpC gene, trpD gene, trpE gene, trpL gene, rluB gene, btuR gene, sohB gene, topA gene, cysB gene, acnA gene, ribA gene, pgpB gene, pyrF gene, osmB gene, gmr gene, rnb gene, fabI gene, sapF gene, sapD gene, sapC gene, sapB gene, sapA gene, puuP gene, puuA gene, puuD gene, puuR gene, puuC gene, puuB gene, puuE gene, pspF gene, pspA gene, pspB gene, pspC gene, pspD gene, pspE gene, ompG gene, tyrR gene, tpx gene, mpaA gene, mppA gene, uspE gene, fnr gene, ogt gene, abgT gene, abgB gene, abgA gene, abgR gene, isrA gene, dbpA gene, uspF gene, ompN gene, micC gene, hslJ gene, ldhA gene, feaR gene, feaB gene, tynA gene, maoC gene, paaA gene, paaB gene, paaC gene, paaD gene, paaE gene, paaF gene, paaG gene, paaH gene, paaI gene, paaJ gene, paaK gene, paaX gene, paaY gene, azoR gene, hrpA gene, aldA gene, gapC_2 gene, gapC_1 gene, cybB gene, hokB gene, mokB gene, sokB gene, trg gene, mdoD gene, rimL gene, tehA gene, tehB gene, ansP gene, nhoA gene, narV gene, narW gene, narY gene, narZ gene, narU gene, fdnG gene, fdnH gene, fdnI gene, adhP gene, sfcA gene, sra gene, bdm gene, osmC gene, ddpF gene, ddpD gene, ddpC gene, ddpB gene, ddpA gene, ddpX gene, dos gene, gadC gene, gadB gene, pqqL gene, hipA gene, hipB gene, ego gene, lsrC gene, lsrD gene, lsrB gene, lsrF gene, lsrG gene, tam gene, uxaB gene, marC gene, marR gene, marA gene, marB gene, eamA gene, dcp gene, rspB gene, rspA gene, speG gene, dmsD gene, clcB gene, dgsA gene, asr gene, mdtI gene, mdtJ gene, pntB gene, pntA gene, folM gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, rsxA gene, rsxB gene, rsxC gene, rsxD gene, rsxG gene, rsxE gene, nth gene, gst gene, pdxY gene, tyrS gene, pdxH gene, slyB gene, slyA gene, sodC gene, nemA gene, gloA gene, rnt gene, lhr gene, sodB gene, purR gene, cfa gene, ribC gene, mdtK gene, valV gene, valW gene, pykF gene, lpp gene, sufE gene, sufS gene, sufD gene, sufC gene, sufB gene, sufA gene, rydB gene, rprA gene, aroD gene, pps gene, aroH gene, nlpC gene, btuD gene, btuE gene, btuC gene, ihfA gene, pheT gene, pheS gene, pheM gene, rplT gene, rpmI gene, infC gene, thrS gene, arpB_1 gene, arpB_2 gene, pfkB gene, cedA gene, katE gene, chbG gene, chbF gene, chbR gene, chbA gene, chbC gene, chbB gene, osmE gene, nadE gene, spy gene, astE gene, astB gene, astD gene, astA gene, astC gene, xthA gene, nudG gene, gdhA gene, topB gene, selD gene, sppA gene, ansA gene, gapA gene, mipA gene, rnd gene, fadD gene, pabB gene, sdaA gene, manX gene, manY gene, manZ gene, rrmA gene, cspC gene, kdgR gene, htpX gene, prc gene, proQ gene, pphA gene, ryeA gene, ryeB gene, holE gene, exoX gene, ptrB gene, purT gene, eda gene, edd gene, zwf gene, pykA gene, lpxM gene, znuA gene, znuC gene, znuB gene, ruvB gene, ruvA gene, ruvC gene, nudB gene, aspS gene, torZ gene, torY gene, cutC gene, argS gene, leuZ gene, cysT gene, glyW gene, pgsA gene, dsrA gene, serU gene, asnT gene, shiA gene, amn gene, asnW gene, asnU gene, cbl gene, nac gene, asnV gene, erfK gene, cobT gene, cobs gene, cobU gene, sbmC gene, dacD gene, sbcB gene, hisL gene, hisG gene, hisD gene, hisC gene, hisB gene, hisH gene, hisA gene, hisF gene, hisI gene, cld gene, ugd gene, gnd gene, galF gene, wcaM gene, wcaL gene, wcaK gene, wzxC gene, wcaJ gene, cpsG gene, cpsB gene, wcaI gene, nudD gene, fcl gene, gmd gene, wcaF gene, wcaE gene, wcaD gene, wcaC gene, wcaB gene, wcaA gene, wzc gene, wzb gene, wza gene, asmA gene, dcd gene, udk gene, thiD gene, thiM gene, mrp gene, metG gene, dusC gene, cdd gene, sanA gene, mglC gene, mglA gene, mglB gene, galS gene, folE gene, cirA gene, lysP gene, nfo gene, fruA gene, fruK gene, fruB gene, setB gene, spr gene, rtn gene, bcr gene, rsuA gene, rplY gene, proL gene, narP gene, ccmH gene, ccmG gene, ccmF gene, ccmE gene, ccmD gene, ccmC gene, ccmB gene, ccmA gene, napC gene, napB gene, napH gene, napG gene, napA gene, napD gene, napF gene, eco gene, mqo gene, alkB gene, ada gene, ompC gene, micF gene, rcsD gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, ais gene, arnT gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoC gene, nuoB gene, nuoA gene, lrhA gene, ackA gene, pta gene, ubiX gene, purF gene, cvpA gene, dedD gene, folC gene, accD gene, dedA gene, truA gene, usg gene, pdxB gene, flk gene, fabB gene, trmC gene, mepA gene, aroC gene, prmB gene, sixA gene, fadL gene, vacJ gene, argW gene, dsdC gene, dsdX gene, dsdA gene, emrY gene, emrK gene, evgA gene, evgS gene, oxc gene, frc gene, ddg gene, glk gene, mntH gene, nupC gene, alaX gene, alaN gene, gltX gene, valU gene, valX gene, valY gene, lysV gene, xapR gene, xapB gene, xapA gene, ligA gene, zipA gene, cysZ gene, cysK gene, ptsH gene, ptsI gene, crr gene, pdxK gene, cysM gene, cysA gene, cysW gene, cysU gene, cysP gene, ucpA gene, murP gene, amiA gene, hemF gene, eutL gene, eutC gene, eutB gene, eutA gene, eutH gene, eutG gene, eutJ gene, eutE gene, cchB gene, cchA gene, eutI gene, eutT gene, eutQ gene, eutP gene, maeB gene, talA gene, tktB gene, aegA gene, narQ gene, acrD gene, dapE gene, purC gene, nlpB gene, dapA gene, gcvR gene, bcp gene, hda gene, uraA gene, upp gene, purM gene, purN gene, ppk gene, ppx gene, guaA gene, guaB gene, xseA gene, der gene, hisS gene, ispG gene, ndk gene, pbpC gene, sseA gene, ryfA gene, sseB gene, pepB gene, fdx gene, hscA gene, hscB gene, iscA gene, iscU gene, iscS gene, iscR gene, suhB gene, csiE gene, hcaT gene, hcaR gene, hcaE gene, hcaF gene, hcaC gene, hcaB gene, hcaD gene, glyA gene, hmp gene, glnB gene, sroF gene, purL gene, tadA gene, acpS gene, pdxJ gene, recO gene, era gene, rnc gene, lepB gene, lepA gene, rseC gene, rseB gene, rseA gene, rpoE gene, nadB gene, srmB gene, ung gene, trxC gene, pssA gene, kgtP gene, rrfG gene, rrlG gene, gltw gene, rrsG gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, ssrA gene, gabD gene, gabT gene, gabP gene, nrdF gene, mprA gene, emrA gene, emrB gene, luxS gene, sraD gene, gshA gene, argQ gene, argZ gene, argY gene, argV gene, serV gene, csrA gene, alaS gene, recX gene, recA gene, mltB gene, norR gene, norV gene, norW gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, pphB gene, rpoS gene, nlpD gene, pcm gene, surE gene, truD gene, ispF gene, ispD gene, ftsB gene, cysC gene, cysN gene, cysD gene, iap gene, cysH gene, cysI gene, cysJ gene, eno gene, pyrG gene, mazG gene, chpR gene, relA gene, rumA gene, barA gene, gudD gene, gudX gene, gudP gene, mltA gene, metZ gene, metW gene, metV gene, amiC gene, argA gene, recD gene, recB gene, ptr gene, recC gene, ppdC gene, ppdB gene, ppdA gene, thyA gene, lgt gene, ptsP gene, nudH gene, mutH gene, tas gene, aas gene, rygA gene, rygB gene, galR gene, lysA gene, lysR gene, araE gene, kduD gene, kduI gene, lysS gene, prfB gene, recJ gene, dsbC gene, xerD gene, fldB gene, bglA gene, gcvP gene, gcvH gene, gcvT gene, visC gene, ubiH gene, pepP gene, zapA gene, ssrS gene, rygC gene, serA gene, rpiA gene, argP gene, argK gene, argO gene, mscS gene, fbaA gene, pgk gene, epd gene, tktA gene, speB gene, speA gene, metK gene, galP gene, sprT gene, endA gene, gshB gene, ansB gene, mutY gene, mltC gene, nupG gene, speC gene, pheV gene, pitB gene, gss gene, hybG gene, hybF gene, hybE gene, hybD gene, hybC gene, hybB gene, hybA gene, hybO gene, exbD gene, exbB gene, metC gene, dkgA gene, sufI gene, plsC gene, parC gene, parE gene, cpdA gene, nudF gene, tolC gene, zupT gene, ribB gene, glgS gene, rygD gene, rfaE gene, glnE gene, htrG gene, cca gene, bacA gene, folB gene, ttdA gene, ttdB gene, rpsU gene, dnaG gene, rpoD gene, ileX gene, aer gene, ebgR gene, ebgA gene, ebgC gene, uxaA gene, uxaC gene, exuT gene, exuR gene, tdcG gene, tdcF gene, tdcE gene, tdcD gene, tdcC gene, tdcB gene, tdcA gene, tdcR gene, rnpB gene, garK gene, garR gene, garL gene, garP gene, garD gene, sohA gene, agaR gene, kbaZ gene, agaV gene, agaW gene, agaA gene, agaS gene, kbaY gene, agaB gene, agaC gene, agaD gene, agaI gene, mtr gene, deaD gene, nlpI gene, pnp gene, sraG gene, rpsO gene, truB gene, rbfA gene, infB gene, nusA gene, metY gene, argG gene, leuU gene, secG gene, glmM gene, folP gene, ftsH gene, rrmJ gene, greA gene, dacB gene, obgE gene, rpmA gene, rplU gene, ispB gene, sfsB gene, murA gene, kdsD gene, kdsC gene, rpoN gene, ptsN gene, npr gene, mtgA gene, elbB gene, ryhA gene, arcB gene, gltB gene, gltD gene, nanT gene, nanA gene, nanR gene, dcuD gene, sspB gene, sspA gene, rpsI gene, rplM gene, degQ gene, degS gene, mdh gene, argR gene, aaeB gene, aaeA gene, aaeX gene, aaeR gene, tldD gene, rng gene, mreD gene, mreC gene, mreB gene, accB gene, accC gene, panF gene, prmA gene, dusB gene, fis gene, rrfF gene, thrV gene, rrfD gene, rrlD gene, gltV gene, rrsD gene, purH gene, purD gene, zraR gene, zraS gene, zraP gene, hupA gene, nfi gene, hemE gene, nudC gene, rsd gene, thiC gene, thiE gene, thiF gene, this gene, thiG gene, thiH gene, htrC gene, rpoC gene, rpoB gene, rplL gene, rplJ gene, rplA gene, rplK gene, nusG gene, secE gene, tufB gene, thrT gene, glyT gene, tyrU gene, thrU gene, coaA gene, birA gene, murB gene, rrfB gene, rrlB gene, gltT gene, rrsB gene, murI gene, btuB gene, trmA gene, fabR gene, sthA gene, oxyR gene, oxyS gene, argH gene, argB gene, argC gene, argE gene, ppc gene, fsaB gene, gldA gene, katG gene, metF gene, metL gene, metB gene, metJ gene, rpmE gene, priA gene, cytR gene, ftsN gene, hslV gene, hslU gene, menA gene, rraA gene, glpf gene, glpK gene, glpX gene, fpr gene, tpiA gene, cdh gene, sbp gene, pfkA gene, fieF gene, cpxP gene, cpxR gene, cpxA gene, kdgT gene, sodA gene, fdhD gene, fdoG gene, fdoH gene, fdoI gene, fdhE gene, bipA gene, glnA gene, glnL gene, glnG gene, hemN gene, csrC gene, spf gene, polA gene, dsbA gene, mobA gene, mobB gene, rrfA gene, rrlA gene, alaT gene, ileT gene, rrsA gene, hemG gene, trkH gene, pepQ gene, fadB gene, fadA gene, fre gene, ubiD gene, rfaH gene, tatD gene, tatC gene, tatB gene, tatA gene, ubiB gene, ubiE gene, rmuC gene, udp gene, metE gene, metR gene, pldB gene, rhtB gene, rhtC gene, recQ gene, pldA gene, rarD gene, corA gene, uvrD gene, xerC gene, dapF gene, cyaY gene, cyaA gene, hemC gene, hemD gene, hemX gene, hemY gene, ryiA gene, aslA gene, aslB gene, proM gene, leuT gene, hisR gene, argX gene, rffM gene, wzyE gene, rffT gene, wzxE gene, rffA gene, rffC gene, rffH gene, rffG gene, rffD gene, rffE gene, wzzE gene, rfe gene, rho gene, rhoL gene, trxA gene, rhlB gene, gpp gene, rep gene, ppiC gene, ilvC gene, ilvY gene, ilvA gene, ilvD gene, ilvE gene, ilvM gene, ilvG_2 gene, ilvG_1 gene, ilvL gene, hdfR gene, trpT gene, aspT gene, rrfC gene, rrlC gene, gltU gene, rrsC gene, asnC gene, mioC gene, oriC gene, gidA gene, gidB gene, atpI gene, atpB gene, atpE gene, atpF gene, atpH gene, atpA gene, atpG gene, atpD gene, atpC gene, glmU gene, glmS gene, pstS gene, pstC gene, pstA gene, pstB gene, phoU gene, bglG gene, bglF gene, bglB gene, bglH gene, cbrC gene, mdtL gene, tnaB gene, tnaA gene, tnaC gene, trmE gene, rnpA gene, rpmH gene, dnaA gene, dnaN gene, recF gene, gyrB gene, ibpA gene, ibpB gene, emrD gene, ivbL gene, ilvB gene, ilvN gene, uhpA gene, uhpB gene, uhpC gene, uhpT gene, selC gene, glts gene, recG gene, trmH gene, spoT gene, rpoZ gene, gmk gene, ligB gene, dinD gene, rph gene, pyrE gene, ttk gene, dut gene, dfp gene, rpmB gene, rpmG gene, mutM gene, coaD gene, kdtA gene, rfaQ gene, rfaG gene, rfaP gene, rfaS gene, rfaB gene, rfaI gene, rfaJ gene, rfaY gene, rfaZ gene, rfaK gene, rfaL gene, rfaC gene, rfaF gene, rfaD gene, htrL gene, kbl gene, tdh gene, envC gene, gpmI gene, grxC gene, secB gene, gpsA gene, cysE gene, lldD gene, lldR gene, lldP gene, mtlR gene, mtlD gene, mtlA gene, selA gene, selB gene, aldB gene, sgbE gene, sgbU gene, sgbH gene, lyxK gene, avtA gene, malS gene, bax gene, xylR gene, xylH gene, xylG gene, xylF gene, xylA gene, xylB gene, glyQ gene, glyS gene, hokA gene, cspA gene, tiaE gene, bisC gene, tag gene, eptB gene, proK gene, dppA gene, dppB gene, dppC gene, dppD gene, dppF gene, rdlD gene, ldrD gene, bcsG gene, bcsF gene, bcsE gene, bcsA gene, bcsB gene, bcsZ gene, bcsC gene, dctA gene, kdgK gene, treF gene, gadA gene, gadX gene, gadY gene, gadW gene, mdtF gene, mdtB gene, gadE gene, hdeD gene, hdeA gene, hdeB gene, slp gene, arsC gene, arsB gene, arsR gene, gor gene, prlC gene, uspA gene, pitA gene, nikR gene, nikE gene, nikD gene, nikC gene, nikB gene, nikA gene, acpT gene, dcrB gene, zntA gene, ftsY gene, ftsE gene, ftsX gene, rpoH gene, livJ gene, livK gene, livH gene, livM gene, livG gene, livF gene, ugpB gene, ugpA gene, ugpE gene, ugpC gene, ugpQ gene, ggt gene, gntR gene, gntK gene, gntU gene, asd gene, glgB gene, glgX gene, glgC gene, glgA gene, glgP gene, glpD gene, glpE gene, glpG gene, glpR gene, rtcR gene, rtcB gene, rtcA gene, malT gene, malP gene, malQ gene, gntT gene, gntY gene, gntX gene, bioH gene, feoB gene, feoA gene, greB gene, ompR gene, envZ gene, pck gene, hslO gene, hslR gene, nudE gene, mrcA gene, hofQ gene, aroK gene, aroB gene, damX gene, dam gene, rpe gene, gph gene, trpS gene, cysG gene, nirC gene, nirD gene, nirB gene, tsgA gene, ppiA gene, fic gene, pabA gene, argD gene, crp gene, prkB gene, kefG gene, kefB gene, slyD gene, slyX gene, fkpA gene, rpsL gene, rpsG gene, fusA gene, tufA gene, rpsJ gene, rplC gene, rplD gene, rplW gene, rplB gene, rpsS gene, rplV gene, rpsC gene, rplP gene, rpmC gene, rpsQ gene, rplN gene, rplX gene, rplE gene, rpsN gene, rpsH gene, rplF gene, rplR gene, rpsE gene, rpmD gene, rplO gene, secY gene, rpmJ gene, rpsM gene, rpsK gene, rpsD gene, rpoA gene, rplQ gene, zntR gene, mscL gene, trkA gene, rsmB gene, fmt gene, def gene, smf gene, smg gene, aroE gene, rrsE gene, ileU gene, alaU gene, rrlE gene, rrfE gene, metA gene, aceB gene, aceA gene, aceK gene, arpA gene, iclR gene, metH gene, pepE gene, lysC gene, pgi gene, ubiC gene, ubiA gene, plsB gene, dgkA gene, lexA gene, dinF gene, zur gene, qor gene, dnaB gene, alr gene, tyrB gene, aphA gene, uvrA gene, ssb gene, soxS gene, soxR gene, ryjA gene, actP gene, acs gene, nrfA gene, nrfB gene, nrfC gene, nrfD gene, nrfE gene, nrfF gene, nrfG gene, gltP gene, fdhF gene, rpiR gene, rpiB gene, proP gene, basS gene, basR gene, eptA gene, adiC gene, adiY gene, adiA gene, melR gene, melA gene, melB gene, fumB gene, dcuB gene, dcuR gene, dcuS gene, lysU gene, cadA gene, cadB gene, cadC gene, pheU gene, dipZ gene, cutA gene, dcuA gene, aspA gene, fxsA gene, groS gene, groL gene, efp gene, ecnA gene, ecnB gene, sugE gene, blc gene, ampC gene, frdD gene, frdC gene, frdB gene, frdA gene, poxA gene, psd gene, rsgA gene, orn gene, glyV gene, glyX gene, glyY gene, amiB gene, mutL gene, miaA gene, hfq gene, hflX gene, hflK gene, hflC gene, purA gene, rnr gene, rlmB gene, ulaF gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrL gene, argI gene, valS gene, holC gene, pepA gene, idnR gene, idnT gene, idnO gene, idnD gene, idnK gene, leuX gene, dnaC gene, dnaT gene, bglJ gene, fhuF gene, leuV gene, leuP gene, leuQ gene, rsmC gene, holD gene, rimI gene, prfC gene, osmY gene, deoC gene, deoA gene, deoB gene, deoD gene, lplA gene, serB gene, radA gene, nadR gene, slt gene, trpR gene, rob gene, creA gene, creB gene, creC gene, creD gene, arcA gene
   [2]fhuA gene, fhuC gene, fhuD gene, fhuB gene, yafJ gene, yafK gene, yafQ gene, dinJ gene, yafL gene, yafM gene, fhiA gene, mbhA gene, dinB gene, yafN gene, yafO gene, yafP gene, ykfJ gene, prfH gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM(JW5034) gene, ykgM(JW5035) gene, eaeH gene, ykgA gene, ykgB gene, ykgI gene, ykgC gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, yahI gene, yahJ gene, yahK gene, yahL gene, yahM gene, yahN gene, yahO gene, prpR gene, prpB gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, uhpT gene, yaiL gene, frmB gene, frmA gene, frmR gene, yaiO gene, yaiX gene, yaiF gene, yaiP gene, yaiS gene, tauA gene, tauB gene, tauC gene, tauD gene, yaiT gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, ylbG gene, ybbB gene, ybbS gene, allA gene, allR gene, glxR gene, ybbV gene, ybbW gene, allB gene, ybbY gene, glxK gene, ylbA gene, allC gene, allD gene, fdrA gene, ylbE_1 gene, ylbE_2 gene, ylbF gene, ybcF gene, envY gene, ybcH gene, nfrA gene, nfrB gene, cusS gene, cusR gene, cusC gene, cusF gene, cusB gene, cusA gene, pheP gene, ybdG gene, nfnB gene, ybdF gene, ybdJ gene, ybdK gene, hokE gene, ybeL gene, ybeQ gene, ybeR gene, djlB gene, ybeT gene, ybeU gene, djlC gene, hscC gene, rihA gene, gltL gene, gltK gene, gltJ gene, gltI gene, kdpF gene, ybfA gene, ybgA gene, phr gene, ybiA gene, dinG gene, ybiB gene, ybiC gene, ybiJ gene, ybiI gene, ybiX gene, fiu gene, ybiM gene, ybiN gene, ybiO gene, glnQ gene, glnP gene, glnH gene, yliA gene, yliB gene, yliC gene, yliD gene, yliE gene, yliF gene, yliG gene, yliH gene, yliI gene, yliJ gene, dacC gene, deoR gene, ybjG gene, ybjC gene, nfsA gene, rimK gene, ybjN gene, potF gene, potG gene, potH gene, potI gene, ybjO gene, rumB gene, artJ gene, artM gene, artQ gene, artI gene, artP gene, ybjP gene, ybjQ gene, ybjR gene, ybjS gene, ybjT gene, ItaE gene, ymcC gene, ymcD gene, cspH gene, cspG gene, ymcE gene, gnsA gene, yccM gene, torS gene, torT gene, torR gene, torC gene, torA gene, torD gene, cbpM gene, cbpA gene, yccE gene, agp gene, yccJ gene, wrbA gene, ymdF gene, ycdG gene, ycdH gene, ycdI gene, rarA gene, ycdK gene, ycdL gene, ycdM gene, ycdC gene, putA gene, putP gene, ycdN_1 gene, ycdN_2 gene, ycdO gene, ycdB gene, phoH gene, ycdP gene, ycdQ gene, ycdR gene, ycdS gene, ycdT gene, ymdE gene, ycdU gene, elbA gene, ycgX gene, ycgE gene, ycgF gene, ycgZ gene, ymgA gene, ymgB gene, ymgC gene, ycgG gene, ymgF gene, ycgH_1 gene, ycgH_2 gene, ymgD gene, ymgG gene, ymgH gene, ycgI gene, ydfH gene, ydfZ gene, ydfI gene, ydfJ gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, yecG gene, otsA gene, otsB gene, araH gene, araG gene, araF gene, yecI gene, yecJ gene, isrB gene, yecR gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN_2 gene, yedN_1 gene, yedM gene, intG gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, yodD gene, yedA gene, vsr gene, dcm gene, yedJ gene, yedR gene, yeds_1 gene, yedS_2 gene, yedS_3 gene, hchA gene, yedV gene, yedW gene, yedX gene, yedY gene, yedZ gene, yodA gene, wbbL_2 gene, wbbL_1 gene, wbbK gene, wbbJ gene, wbbI gene, wbbH gene, glf gene, rfbX gene, rfbC gene, rfbA gene, rfbD gene, rfbB gene, yegE gene, alkA gene, yegD gene, yegI gene, yegJ gene, yegK gene, yegL gene, ryeC gene, ryeD gene, mdtA gene, mdtB gene, mdtC gene, mdtD gene, baeS gene, baeR gene, yegP gene, yegQ gene, ryeE gene, ogrK gene, yegZ gene, yegR gene, yegS gene, gatR_1 gene, gatR_2 gene, gatD gene, gatC gene, gatB gene, gatA gene, gatZ gene, gatY gene, fbaB gene, yegT gene, yegU gene, yegV gene, yegW gene, yegX gene, molR_1 gene, molR_2 gene, molR 3 gene, yehI gene, yehK gene, yehL gene, yehM gene, yehP gene, yehQ gene, yehR gene, yehS gene, yehT gene, yehU gene, mlrA gene, yohO gene, yehW gene, yehX gene, yehY gene, yehZ gene, bglX gene, dld gene, pbpG gene, yohC gene, yell gene, yeiJ gene, rihB gene, yeiL gene, yeiM gene, yeiN gene, yeiC gene, elaB gene, elaA gene, elaC gene, elaD gene, yfbK gene, yfbL gene, yfbM gene, yfbN gene, yfbO gene, yfbP gene, yfcC gene, yfcD gene, yfcE gene, yfcF gene, yfcG gene, folX gene, yfcH gene, yfcI gene, hisP gene, hisM gene, hisQ gene, hisJ gene, argT gene, ypdA gene, ypdB gene, ypdC gene, ypdD gene, ypdE gene, ypdF gene, ypdG gene, ypdH gene, pinH gene, ypjB gene, ypjC gene, ileY gene, ygaQ gene, ygaR gene, yqaC gene, yqaD gene, ygaT gene, csiR gene, ygaU gene, yqaE gene, ygaV gene, ygaP gene, stpA gene, ygaW gene, ygaC gene, ygaM gene, nrdH gene, nrdI gene, nrdE gene, proV gene, proW gene, proX gene, ygaX gene, ygaY gene, ygaZ gene, ygaH gene, srlA gene, srlE gene, srlB gene, srlD gene, gutM gene, srlR gene, gutQ gene, yqeG gene, yqeH gene, yqeI gene, yqeJ gene, yqeK gene, ygeF gene, ygeG gene, ygeH gene, ygeI gene, pbl gene, ygeK gene, ygeL gene, ygeM gene, ygeN gene, ygeO gene, ygeP gene, ygeQ gene, glyU gene, ygeR gene, xdhA gene, xdhB gene, xdhC gene, ygeV gene, ygeW gene, ygeX gene, ygeY gene, hyuA gene, yqeA gene, yqeB gene, yqeC gene, ygfJ gene, ygfK gene, ssnA gene, ygfM gene, xdhD gene, ygfO gene, guaD gene, ygfQ gene, ygfS gene, ygfT gene, ygfU gene, idi gene, cmtA gene, cmtB gene, yghD gene, yghE gene, yghF gene, yghG gene, pppA gene, yghJ gene, yghK gene, glcB gene, glcG gene, glcF gene, glcE gene, glcD gene, glcC gene, yghO gene, yghQ gene, yghR gene, yghS gene, yghT gene, ygiS gene, ygiT gene, ygiU gene, ygiV gene, ygiW gene, qseB gene, qseC gene, ygiZ gene, mdaB gene, ygiN gene, gltF gene, yhcA gene, yhcD gene, yhcE gene, yhcF gene, yhcG gene, yhcH gene, nanK gene, nanE gene, yhdJ gene, yhdU gene, envR gene, acrE gene, acrF gene, yhdV gene, yhdW gene, yhdX gene, yhdY gene, yijP gene, yijO gene, frwD gene, pflc gene, pflD gene, frwB gene, frwC gene, ptsA gene, rhaT gene, rhaR gene, rhaS gene, rhaB gene, rhaA gene, rhaD gene, yiiL gene, frvA gene, frvB gene, frvX gene, frvR gene, yiiG gene, yiiF gene, yiiE gene, yiiD gene, dtd gene, rbn gene, yihX gene, yihW gene, yihV gene, yihU gene, yihT gene, yihS gene, yihR gene, yihQ gene, yihP gene, yihO gene, ompL gene, yihN gene, yihM gene, yihL gene, hsrA gene, rbsR gene, rbsK gene, rbsB gene, rbsC gene, rbsA gene, rbsD gene, trkD gene, yieN gene, yieM gene, asnA gene, yidB gene, yidA gene, yidX gene, dgoR gene, dgoK gene, dgoA gene, dgoD gene, dgoT gene, cbrA gene, yidR gene, yidQ gene, yidE gene, yidP gene, glvC gene, glvB gene, glvG gene, ysdC gene, yidL gene, yidK gene, yidJ gene, yidI gene, yidH gene, yidG gene, yidF gene, yhiN gene, yhiM gene, yhiL gene, yhiK gene, yhiJ gene, yhiI gene, rbbA gene, yhhJ gene, chiA gene, bfd gene, bfr gene, gspO gene, gspM gene, gspL gene, gspK gene, gspJ gene, gspI gene, gspH gene, gspG gene, gspF gene, gspE gene, gspD gene, gspC gene, gspA gene, pioO gene, yjbE gene, yjbF gene, yjbG gene, yjbH gene, yjbA gene, xylE gene, malG gene, malF gene, malE gene, malK gene, lamB gene, maIM gene, yjbI gene, yjcP gene, yjcQ gene, yjcR gene, yjcS gene, alsK gene, alsE gene, alsC gene, alsA gene, alsB gene, yjdP gene, phnP gene, phnO gene, phnN gene, phnM gene, phnL gene, phnK gene, phnJ gene, phnI gene, phnH gene, phnG gene, phnF gene, phnE(JW4064) gene, phnE(JW4065) gene, phnD gene, phnC gene, phnB gene, phnA gene, yjdA gene, yjcZ gene, yjfI gene, yjfJ gene, yjfK gene, yjfL gene, yjfM gene, yjfC gene, aidB gene, yjfN gene, yjfO gene, yjfP gene, ulaR gene, ulaG gene, ulaA gene, ulaB gene, ulaC gene, ulaD gene, ulaE gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, kptA gene, yjiJ gene, yjiK gene, yjiL gene, yjiM gene, yjiN gene, yjiO gene, yjiP gene, yjiQ gene, yjiR gene, yjiS gene, yjiT gene, yjiV gene, mcrC gene, mcrB gene, yjiW gene, hsdS gene, hsdM gene, hsdR gene, mrr gene, yjiA gene, yjiX gene, yjiY gene, tsr gene, yjiZ gene, yjjM gene, yjjN gene, mdoB gene, yjjA gene.
(2) The *E. coli* mutant strain according to above (1) comprising chromosomal DNA that comprises DNA having the nucleotide sequences as shown in [1] to [83] below of the nucleotide sequence as shown in SEQ ID NO: 1 or DNA equivalent thereto:
   [1] a nucleotide sequence of nucleotides 1 to 15298
   [2] a nucleotide sequence of nucleotides 16751 to 19620
   [3] a nucleotide sequence of nucleotides 20815 to 65857
   [4] a nucleotide sequence of nucleotides 78797 to 167400
   [5] a nucleotide sequence of nucleotides 173316 to 244300
   [6] a nucleotide sequence of nucleotides 253747 to 262299
   [7] a nucleotide sequence of nucleotides 387868 to 389474
   [8] a nucleotide sequence of nucleotides 404040 to 518365
   [9] a nucleotide sequence of nucleotides 533048 to 535840
   [10] a nucleotide sequence of nucleotides 550552 to 564276
   [11] a nucleotide sequence of nucleotides 608455 to 640668
   [12] a nucleotide sequence of nucleotides 659193 to 675439
   [13] a nucleotide sequence of nucleotides 689711 to 729156
   [14] a nucleotide sequence of nucleotides 739930 to 832888
   [15] a nucleotide sequence of nucleotides 848427 to 867974
   [16] a nucleotide sequence of nucleotides 883811 to 891334
   [17] a nucleotide sequence of nucleotides 909717 to 1049224
   [18] a nucleotide sequence of nucleotides 1097358 to 1105534
   [19] a nucleotide sequence of nucleotides 1106991 to 1196528
   [20] a nucleotide sequence of nucleotides 1225485 to 1295721
   [21] a nucleotide sequence of nucleotides 1297023 to 1300822
   [22] a nucleotide sequence of nucleotides 1302896 to 1397636
   [23] a nucleotide sequence of nucleotides 1399079 to 1413662
   [24] a nucleotide sequence of nucleotides 1436899 to 1469664
   [25] a nucleotide sequence of nucleotides 1472404 to 1504839
   [26] a nucleotide sequence of nucleotides 1506619 to 1528866
   [27] a nucleotide sequence of nucleotides 1534766 to 1630050
   [28] a nucleotide sequence of nucleotides 1654610 to 1853604
   [29] a nucleotide sequence of nucleotides 1864144 to 1875287
   [30] a nucleotide sequence of nucleotides 1888500 to 1963662
   [31] a nucleotide sequence of nucleotides 1992215 to 1995800
   [32] a nucleotide sequence of nucleotides 2027349 to 2032587
   [33] a nucleotide sequence of nucleotides 2043516 to 2068287
   [34] a nucleotide sequence of nucleotides 2081066 to 2103531
   [35] a nucleotide sequence of nucleotides 2115204 to 2145402
   [36] a nucleotide sequence of nucleotides 2186995 to 2199807
   [37] a nucleotide sequence of nucleotides 2228990 to 2255033
   [38] a nucleotide sequence of nucleotides 2262631 to 2292234
   [39] a nucleotide sequence of nucleotides 2293834 to 2324498
   [40] a nucleotide sequence of nucleotides 2322075 to 2411311
   [41] a nucleotide sequence of nucleotides 2413007 to 2422503
   [42] a nucleotide sequence of nucleotides 2433238 to 2471990
   [43] a nucleotide sequence of nucleotides 2482140 to 2504213
   [44] a nucleotide sequence of nucleotides 2513697 to 2519690
   [45] a nucleotide sequence of nucleotides 2521089 to 2599856
   [46] a nucleotide sequence of nucleotides 2613439 to 2754814
   [47] a nucleotide sequence of nucleotides 2788619 to 2794311
   [48] a nucleotide sequence of nucleotides 2800029 to 2803445
   [49] a nucleotide sequence of nucleotides 2809286 to 2824451
   [50] a nucleotide sequence of nucleotides 2829399 to 2909409
   [51] a nucleotide sequence of nucleotides 2909710 to 2921193
   [52] a nucleotide sequence of nucleotides 2943639 to 2984497
   [53] a nucleotide sequence of nucleotides 3032267 to 3076223
   [54] a nucleotide sequence of nucleotides 3078056 to 3109245
   [55] a nucleotide sequence of nucleotides 3133480 to 3164769
   [56] a nucleotide sequence of nucleotides 3172082 to 3184621
   [57] a nucleotide sequence of nucleotides 3186056 to 3224889
   [58] a nucleotide sequence of nucleotides 3240401 to 3261074
   [59] a nucleotide sequence of nucleotides 3262220 to 3361080
   [60] a nucleotide sequence of nucleotides 3370892 to 3411501
   [61] a nucleotide sequence of nucleotides 3421222 to 3486426
   [62] a nucleotide sequence of nucleotides 3496906 to 3536155
   [63] a nucleotide sequence of nucleotides 3549687 to 3556477
   [64] a nucleotide sequence of nucleotides 3576239 to 3697304
   [65] a nucleotide sequence of nucleotides 3709527 to 3742812
   [66] a nucleotide sequence of nucleotides 3744293 to 3748980
   [67] a nucleotide sequence of nucleotides 3747997 to 3750486
   [68] a nucleotide sequence of nucleotides 3751906 to 3762908
   [69] a nucleotide sequence of nucleotides 3772817 to 3774086
   [70] a nucleotide sequence of nucleotides 3785257 to 3794687
   [71] a nucleotide sequence of nucleotides 3803459 to 3868134
   [72] a nucleotide sequence of nucleotides 3878460 to 3918087
   [73] a nucleotide sequence of nucleotides 3919815 to 3986454
   [74] a nucleotide sequence of nucleotides 3988342 to 4003004
   [75] a nucleotide sequence of nucleotides 4021347 to 4055371
   [76] a nucleotide sequence of nucleotides 4061466 to 4128290
   [77] a nucleotide sequence of nucleotides 4141016 to 4170562
   [78] a nucleotide sequence of nucleotides 4186909 to 4239493
   [79] a nucleotide sequence of nucleotides 4255871 to 4303000
   [80] a nucleotide sequence of nucleotides 4316735 to 4318540
   [81] a nucleotide sequence of nucleotides 4334946 to 4414758
   [82] a nucleotide sequence of nucleotides 4428378 to 4501437
   [83] a nucleotide sequence of nucleotides 4604873 to 4646332.
(3) The *E. coli* mutant strain according to (1) or (2) comprising chromosomal DNA that does not comprise uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, and ybeM gene, or homologous gene thereof.

(4) The *E*. *coli* mutant strain according to any one of (1) to (3) comprising chromosomal DNA that does not comprise DNA having a nucleotide sequence of nucleotides 640669 to 659192 in the nucleotide sequence as shown in SEQ ID NO: 1 or DNA equivalent thereto.
(5) The *E. coli* mutant strain according to any one of (1) to (4) comprising chromosomal DNA that does not comprise yqcA gene, yqcB gene, yqcC gene, syd gene, yqcD gene, ygdH gene, sdaC gene, sdaB gene, exo gene, fucO gene, fucA gene, fucP gene, fuel gene, fucK gene, fucU gene, fucR gene, ygdE gene, ygdD gene, gcvA gene, ygdI gene, csdA gene, and ygdK gene, or homologous gene thereof and comprise chpA gene, or homologous gene thereof.

(6) The *E. coli* mutant strain according to any one of (1) to (5) comprising chromosomal DNA that does not comprise DNA having a nucleotide sequence of nucleotides 2921194 to 2943638 in the nucleotide sequence as shown in SEQ ID NO: 1 or DNA equivalent thereto.
(7) The *E. coli* mutant strain according to any one of (1) to (6) comprising chromosomal DNA that does not comprise araD gene, araA gene, araB gene, araC gene, yabI gene, thiQ gene, thiP gene, tbpA gene, sgrR gene, and setA gene, or homologous gene thereof.

(8) The *E. coli* mutant strain according to any one of (1) to (7) comprising chromosomal DNA that does not comprise DNA having a nucleotide sequences of nucleotides 65858 to 78796 in the nucleotide sequence as shown in SEQ ID NO: 1 or DNA equivalent thereto.
(9) The *E. coli* mutant strain according to any one of (1) to (8) comprising chromosomal DNA that does not comprise yhfZ gene, yhfY gene, yhfX gene, yhfW gene, php gene, yhfU gene, yhfT gene, yhfS gene, frlR gene, frlD gene, frlC gene, frlB gene, frlA gene, and yhfL gene, or homologous gene thereof.

(10) The *E. coli* mutant strain according to any one of (1) to (9) comprising chromosomal DNA that does not comprise DNA having a nucleotide sequence of nucleotides 4128291 to 4141015 in the nucleotide sequence as shown in SEQ ID NO: 1 or DNA equivalent thereto.
(11) The *E. coli* mutant strain according to any one of (1) to (10) comprising chromosomal DNA that does not comprise pncA gene, ydjE gene, ydjF gene, ydjG gene, ydjH gene, ydjI gene, ydjJ gene, ydjK gene, ydjL gene, yeaC gene, and yeaA gene, or homologous gene thereof.

(12) The *E. coli* mutant strain according to any one of (1) to (11) comprising chromosomal DNA that does not comprise DNA having a nucleotide sequence of nucleotides 1853605 to 1864143 in the nucleotide sequence as shown in SEQ ID NO: 1 or DNA equivalent thereto.
(13) The *E. coli* mutant strain according to any one of (1) to (12) comprising chromosomal DNA that does not comprise ade gene, yicO gene, yicN gene, yicM gene, yicS gene, nlpA gene, yicL gene, and setC gene, or homologous gene thereof.

(14) The *E. coli* mutant strain according to any one of (1) to (13) comprising chromosomal DNA that does not comprise DNA having a nucleotide sequence of nucleotides 3794688 to 3803458 in the nucleotide sequence as shown in SEQ ID NO: 1 or DNA equivalent thereto.
(15) The *E. coli* mutant strain according to any one of (1) to (14) comprising chromosomal DNA that does not comprise ygjI gene, ygjJ gene, ygjK gene, fadH gene, ygjM gene, ygjN gene, ygjO gene, ygjP gene, ygjQ gene, ygjR gene, alx gene, sstT gene, and ygjV gene, or homologous gene thereof.

(16) The *E. coli* mutant strain according to any one of (1) to (15) comprising chromosomal DNA that does not comprise DNA having a nucleotide sequence of nucleotides 3224890 to 3240400 in the nucleotide sequence as shown in SEQ ID NO: 1 or DNA equivalent thereto.
(17) The *E. coli* mutant strain according to any one of (1) to (16) comprising chromosomal DNA that does not comprise yrhB gene, yrhA gene, yhhZ gene, yhhY gene, yhhX gene, and yhhW gene, or homologous gene thereof.

(18) The *E. coli* mutant strain according to any one of (1) to (17) comprising chromosomal DNA that does not comprise DNA having a nucleotide sequence of nucleotides 4055372 to 4061465 in the nucleotide sequence as shown in SEQ ID NO: 1 or DNA equivalent thereto.
(19) The *E. coli* mutant strain according to any one of (1) to (18) comprising chromosomal DNA that does not comprise yeaK gene, yoaI gene, yeaL gene, yeaM gene, yeaN gene, yeaO gene, yoaF gene, yeaP gene, yeaQ gene, yoaG gene, yeaR gene, yeaS gene, yeaT gene, yeaU gene, yeaV gene, yeaW gene, and yeaX gene, or homologous gene thereof.

(20) The *E. coli* mutant strain according to any one of (1) to (19) comprising chromosomal DNA that does not comprise DNA having a nucleotide sequence of nucleotides 1875288 to 1888499 in the nucleotide sequence as shown in SEQ ID NO: 1 or DNA equivalent thereto.
(21) The *E. coli* mutant strain according to any one of (1) to (20) comprising chromosomal DNA that does not comprise hyfA gene, hyfB gene, hyfC gene, hyfD gene, hyfE gene, hyfF gene, hyfG gene, hyfH gene, hyfI gene, hyfJ gene, hyfR gene, and focB gene, or homologous gene thereof.

(22) The *E. coli* mutant strain according to any one of (1) to (21) comprising chromosomal DNA that does not comprise DNA having a nucleotide sequence of nucleotides 2599857 to 2613438 in the nucleotide sequence as shown in SEQ ID NO: 1 or DNA equivalent thereto.
(23) An *E. coli* mutant strain comprising chromosomal DNA comprising the following genes [1] or homologous genes thereof, comprising not more than 10% of the genes [2] and [3] or homologous genes thereof, and being at least 1040 kbp shorter than that of a wild-type *E. coli* strain, provided that when the following genes [1] or homologous genes thereof comprise NC genes, the aforementioned *E. coli* mutant strain derived from the wild-type *E. coli* strain does not necessarily have the NC gene.
   [1] thrL gene, thrA gene, thrB gene, thrC gene, talB gene, mog gene, htgA gene, dnaK gene, dnaJ gene, mokC gene, hokC gene, sokC gene, nhaA gene, nhaR gene, rpsT gene, ribF gene, ileS gene, IspA gene, fkpB gene, ispH gene, rihC gene, dapB gene, carA gene, carB gene, caiF gene, caiE gene, caiD gene, caiC gene, caiB gene, caiA gene, caiT gene, fixA gene, fixB gene, fixC gene, fixX gene, kefF gene, kefC gene, folA gene, apaH gene, apaG gene, ksgA gene, pdxA gene, surA gene, imp gene, djlA gene, rluA gene, hepA gene, polB gene, leuD gene, leuC gene, leuB gene, leuA gene, leuL gene, leuO gene, ilvI gene, ilvH gene, fruR gene, mraZ gene, mraW gene, ftsL gene, ftsI gene, murE gene, murF gene, mraY gene, murD gene, ftsW gene, murG gene, murC gene, ddlB gene, ftsQ gene, ftsA gene, ftsZ gene, lpxC gene, secM gene, secA gene, mutT gene, coaE gene, guaC gene, hofC gene, hofB gene, ppdD gene, nadC gene, ampD gene, ampE gene, aroP gene, pdhR gene, aceE gene, aceF gene, lpd gene, acnB gene, speD gene, speE gene, cueO gene, gcd gene, hpt gene, can gene, panD gene, panC gene, panB gene, htrE gene, ecpD gene, folK gene, pcnB gene, dksA gene, sfsA gene, ligT gene, hrpB gene, mrcB gene, hemL gene, clcA gene, btuF gene, pfs gene, dgt gene, degP gene, cdaR gene, dapD gene, glnD gene, map gene, tff gene, rpsB gene, tsf gene, pyrH gene, frr gene, dxr gene, ispU gene, cdsA gene, hlpA gene, lpxD gene, fabZ gene, lpxA gene, lpxB gene, rnhB gene, dnaE gene, accA gene, ldcC gene, tilS gene, rof gene, nlpE gene, proS gene, rcsF gene, metQ gene, metI gene, metN gene, gmhB gene, rrsH gene, ileV gene, alaV gene, rrlH gene, rrfH gene, aspU gene, dkgB gene, mltD gene, gloB gene, rnhA gene, dnaQ gene, aspV gene, ivy gene, fadE gene, lpcA gene, pepD gene, gpt gene, frsA gene, crl gene, phoE gene, proB gene, proA gene, thrW gene, hemB gene, proC gene, aroL gene, aroM gene, rdgC gene, mak gene, araJ gene, sbcC gene, sbcD gene, phoB gene, phoR gene, brnQ gene, proY gene, malZ gene, queA gene, tgt gene, secD gene, secF gene, tsx gene, ribD gene, ribE gene, nusB gene, thiL gene, pgpA gene, dxs gene, ispA gene, xseB gene, thiI gene, panE gene, cyoE gene, cyoD gene, cyoC gene, cyoB gene, cyoA gene, ampG gene, bolA gene, tig gene, clpP gene, clpX gene, lon gene, hupB gene, ppiD gene, cof gene, mdlA gene, mdlB gene, glnK gene, amtB gene, tesB gene, ffs gene, maa gene, hha gene, acrB gene, acrA gene, acrR gene, kefA gene, priC gene, apt gene, dnaX gene, recR gene, htpG gene, adk gene, hemH gene, aes gene, gsk gene, fsr gene, ushA gene, copA gene, cueR gene, gcl gene, hyi gene, purK gene, purE gene, lpxH gene, ppiB gene, cysS gene, folD gene, sfmA gene, sfmC gene, sfmD gene, sfmH gene, sfmF gene, fimZ gene, argU gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, tatE gene, lipA gene, lipB gene, dacA gene, rlpA gene, mrdB gene, mrdA gene, cobC gene, nadD gene, holA gene, rlpB gene, leuS gene, lnt gene, miaB gene, ubiF gene, glnX gene, glnV gene, metU gene, glnW gene, glnU gene, leuW gene, metT gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, nei gene, abrB gene, gltA gene, sdhC gene, sdhD gene, sdhA gene, sdhB gene, sucA gene, sucB gene, sucC gene, sucD gene, mngR gene, mngA gene, mngB gene, cydA gene, cydB gene, tolQ gene, tolR gene, tolA gene, tolB gene, pal gene, lysT gene, valT gene, lysW gene, valZ gene, lysY gene, lysZ gene, lysQ gene, nadA gene, pnuC gene, zitB gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, rhtA gene, ompX gene, rybA gene, mntR gene, fsaA gene, moeB gene, moeA gene, iaaA gene, cmr gene, rybB gene, grxA gene, poxB gene, hcr gene, hcp gene, aqpZ gene, macA gene, macB gene, cspD gene, clpS gene, clpA gene, serW gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, ihfB gene, msbA gene, IpxK gene, kdsB gene, smtA gene, mukF gene, mukE gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, serT gene, hyaA gene, hyaB gene, hyaC gene, hyaD gene, hyaE gene, hyaF gene, appC gene, appB gene, appA gene, etp gene, serX gene, csgG gene, csgF gene, csgE gene, csgD gene, csgB gene, csgA gene, csgC gene, mdoC gene, mdoG gene, mdoH gene, msyB gene, mdtG gene, lpxL gene, solA gene, dinI gene, pyrC gene, grxB gene, mdtH gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, sraB gene, rpmF gene, plsX gene, fabH gene, fabD gene, fabG gene, acpP gene, fabF gene, pabC gene, tmk gene, holB gene, ptsG gene, fhuE gene, hinT gene, nagZ gene, ndh gene, mfd gene, lolC gene, lolD gene, lolE gene, cobB gene, potD gene, potC gene, potB gene, potA gene, pepT gene, phoQ gene, phoP gene, purB gene, hflD gene, trmU gene, minE gene, minD gene, minC gene, hlyE gene, umuD gene, umuC gene, dsbB gene, nhaB gene, fadR gene, dadA gene, dadX gene, cvrA gene, ldcA gene, emtA gene, treA gene, dhaH gene, dhaL gene, dhaK gene, dhaR gene, pth gene, prsA gene, ispE gene, lolB gene, hemA gene, prfA gene, prmC gene, kdsA gene, IdrA gene, rdlA gene, IdrB gene, rdlB gene, IdrC gene, rdlC gene, chaA gene, chaB gene, chaC gene, narL gene, narX gene, narK gene, narG gene, narH gene, narJ gene, narI gene, rttR gene, tpr gene, tyrV gene, tyrT gene, purU gene, rssA gene, rssB gene, galU gene, hns gene, tdk gene, adhE gene, oppA gene, oppB gene, oppC gene, oppD gene, oppF gene, cls gene, kch gene, tonB gene, ompW gene, trpA gene, trpB gene, trpC gene, trpD gene, trpE gene, trpL gene, rluB gene, btuR gene, sohB gene, topA gene, cysB gene, acnA gene, ribA gene, pgpB gene, pyrF gene, osmB gene, gmr gene, rnb gene, fabI gene, sapF gene, sapD gene, sapC gene, sapB gene, sapA gene, puuP gene, puuA gene, puuD gene, puuR gene, puuC gene, puuB gene, puuE gene, pspF gene, pspA gene, pspB gene, pspC gene, pspD gene, pspE gene, ompG gene, tyrR gene, tpx gene, mpaA gene, mppA gene, uspE gene, fnr gene, ogt gene, abgT gene, abgB gene, abgA gene, abgR gene, isrA gene, dbpA gene, uspF gene, ompN gene, micC gene, hslJ gene, ldhA gene, feaR gene, feaB gene, tynA gene, maoC gene, paaA gene, paaB gene, paaC gene, paaD gene, paaE gene, paaF gene, paaG gene, paaH gene, paaI gene, paaJ gene, paaK gene, paaX gene, paaY gene, azoR gene, hrpA gene, aldA gene, gapC_2 gene, gapC_1 gene, cybB gene, hokB gene, mokB gene, sokB gene, trg gene, mdoD gene, rimL gene, tehA gene, tehB gene, ansP gene, nhoA gene, narV gene, narW gene, narY gene, narZ gene, narU gene, fdnG gene, fdnH gene, fdnI gene, adhP gene, sfcA gene, sra gene, bdm gene, osmC gene, ddpF gene, ddpD gene, ddpC gene, ddpB gene, ddpA gene, ddpX gene, dos gene, gadC gene, gadB gene, pqqL gene, hipA gene, hipB gene, ego gene, IsrC gene, IsrD gene, lsrB gene, lsrF gene, lsrG gene, tam gene, uxaB gene, marC gene, marR gene, marA gene, marB gene, eamA gene, dcp gene, rspB gene, rspA gene, speG gene, dmsD gene, clcB gene, dgsA gene, asr gene, mdtl gene, mdtJ gene, pntB gene, pntA gene, folM gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, rsxA gene, rsxB gene, rsxC gene, rsxD gene, rsxG gene, rsxE gene, nth gene, gst gene, pdxY gene, tyrS gene, pdxH gene, slyB gene, slyA gene, sodC gene, nemA gene, gloA gene, rnt gene, lhr gene, sodB gene, purR gene, cfa gene, ribC gene, mdtK gene, valV gene, valW gene, pykF gene, lpp gene, sufE gene, sufS gene, sufD gene, sufC gene, sufB gene, sufA gene, rydB gene, rprA gene, aroD gene, pps gene, aroH gene, nlpC gene, btuD gene, btuE gene, btuC gene, ihfA gene, pheT gene, pheS gene, pheM gene, rplT gene, rpmI gene, infC gene, thrS gene, araB_1 gene, arpB_2 gene, pfkB gene, cedA gene, katE gene, chbG gene, chbF gene, chbR gene, chbA gene, chbC gene, chbB gene, osmE gene, nadE gene, spy gene, astE gene, astB gene, astD gene, astA gene, astC gene, xthA gene, nudG gene, gdhA gene, topB gene, selD gene, sppA gene, ansA gene, gapA gene, mipA gene, rnd gene, fadD gene, pabB gene, sdaA gene, manX gene, manY gene, manZ gene, rrmA gene, cspC gene, kdgR gene, htpX gene, prc gene, proQ gene, pphA gene, ryeA gene, ryeB gene, holE gene, exoX gene, ptrB gene, purT gene, eda gene, edd gene, zwf gene, pykA gene, lpxM gene, znuA gene, znuC gene, znuB gene, ruvB gene, ruvA gene, ruvC gene, nudB gene, aspS gene, torZ gene, torY gene, cutC gene, argS gene, leuZ gene, cysT gene, glyW gene, pgsA gene, dsrA gene, serU gene, asnT gene, shiA gene, amn gene, asnW gene, asnU gene, cbl gene, nac gene, asnV gene, erfK gene, cobT gene, cobS gene, cobU gene, sbmC gene, dacD gene, sbcB gene, hisL gene, hisG gene, hisD gene, hisC gene, hisB gene, hisH gene, hisA gene, hisF gene, hisI gene, cld gene, ugd gene, gnd gene, galF gene, wcaM gene, wcaL gene, wcaK gene, wzxC gene, wcaJ gene, cpsG gene, cpsB gene, wcaI gene, nudD gene, fcl gene, gmd gene, wcaF gene, wcaE gene, wcaD gene, wcaC gene, wcaB gene, wcaA gene, wzc gene, wzb gene, wza gene, asmA gene, dcd gene, udk gene, thiD gene, thiM gene, mrp gene, metG gene, dusC gene, cdd gene, sanA gene, mglC gene, mglA gene, mglB gene, galS gene, folE gene, cirA gene, lysP gene, nfo gene, fruA gene, fruK gene, fruB gene, setB gene, spr gene, rtn gene, bcr gene, rsuA gene, rplY gene, proL gene, narP gene, ccmH gene, ccmG gene, ccmF gene, ccmE gene, ccmD gene, ccmC gene, ccmB gene, ccmA gene, napC gene, napB gene, napH gene, napG gene, napA gene, napD gene, napF gene, eco gene, mqo gene, alkB gene, ada gene, ompC gene, micF gene, rcsD gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, ais gene, arnT gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoC gene, nuoB gene, nuoA gene, lrhA gene, ackA gene, pta gene, ubiX gene, purF gene, cvpA gene, dedD gene, folC gene, accD gene, dedA gene, truA gene, usg gene, pdxB gene, flk gene, fabB gene, trmC gene, mepA gene, aroC gene, prmB gene, sixA gene, fadL gene, vacJ gene, argW gene, dsdC gene, dsdX gene, dsdA gene, emrY gene, emrK gene, evgA gene, evgS gene, oxc gene, frc gene, ddg gene, glk gene, mntH gene, nupC gene, alaX gene, alaW gene, gltX gene, valU gene, valX gene, valY gene, lysV gene, xapR gene, xapB gene, xapA gene, ligA gene, zipA gene, cysZ gene, cysK gene, ptsH gene, ptsI gene, crr gene, pdxK gene, cysM gene, cysA gene, cysW gene, cysU gene, cysP gene, ucpA gene, murP gene, amiA gene, hemF gene, eutL gene, eutC gene, eutB gene, eutA gene, eutH gene, eutG gene, eutJ gene, eutE gene, cchB gene, cchA gene, eutI gene, eutT gene, eutQ gene, eutP gene, maeB gene, talA gene, tktB gene, aegA gene, narQ gene, acrD gene, dapE gene, purC gene, nlpB gene, dapA gene, gcvR gene, bcp gene, hda gene, uraA gene, upp gene, purM gene, purN gene, ppk gene, ppx gene, guaA gene, guaB gene, xseA gene, der gene, hisS gene, ispG gene, ndk gene, pbpC gene, sseA gene, ryfA gene, sseB gene, pepB gene, fdx gene, hscA gene, hscB gene, iscA gene, iscU gene, iscS gene, iscR gene, suhB gene, csiE gene, hcaT gene, hcaR gene, hcaE gene, hcaF gene, hcaC gene, hcaB gene, hcaD gene, glyA gene, hmp gene, glnB gene, sroF gene, purL gene, tadA gene, acpS gene, pdxJ gene, recO gene, era gene, rnc gene, lepB gene, lepA gene, rseC gene, rseB gene, rseA gene, rpoE gene, nadB gene, srmB gene, ung gene, trxC gene, pssA gene, kgtP gene, rrfG gene, rrlG gene, gltw gene, rrsG gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, ssrA gene, gabD gene, gabT gene, gabP gene, nrdF gene, mprA gene, emrA gene, emrB gene, luxS gene, sraD gene, gshA gene, argQ gene, argZ gene, argY gene, argV gene, serV gene, csrA gene, alas gene, recX gene, recA gene, mltB gene, norR gene, norV gene, norW gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, pphB gene, rpoS gene, nlpD gene, pcm gene, surE gene, truD gene, ispF gene, ispD gene, ftsB gene, cysC gene, cysN gene, cysD gene, iap gene, cysH gene, cysI gene, cysJ gene, eno gene, pyrG gene, mazG gene, chpR gene, relA gene, rumA gene, barA gene, gudD gene, gudX gene, gudP gene, mltA gene, metZ gene, metW gene, metV gene, amiC gene, argA gene, recD gene, recB gene, ptr gene, recC gene, ppdC gene, ppdB gene, ppdA gene, thyA gene, lgt gene, ptsP gene, nudH gene, mutH gene, tas gene, aas gene, rygA gene, rygB gene, galR gene, lysA gene, lysR gene, araE gene, kduD gene, kduI gene, lysS gene, prfB gene, recJ gene, dsbC gene, xerD gene, fldB gene, bglA gene, gcvP gene, gcvH gene, gcvT gene, visC gene, ubiH gene, pepP gene, zapA gene, ssrS gene, rygC gene, serA gene, rpiA gene, argP gene, argK gene, argO gene, mscS gene, fbaA gene, pgk gene, epd gene, tktA gene, speB gene, speA gene, metK gene, galP gene, sprT gene, endA gene, gshB gene, ansB gene, mutY gene, mltC gene, nupG gene, speC gene, pheV gene, pitB gene, gss gene, hybG gene, hybF gene, hybE gene, hybD gene, hybC gene, hybB gene, hybA gene, hybO gene, exbD gene, exbB gene, metC gene, dkgA gene, sufI gene, plsC gene, parC gene, parE gene, cpdA gene, nudF gene, tolC gene, zupT gene, ribB gene, glgS gene, rygD gene, rfaE gene, glnE gene, htrG gene, cca gene, bacA gene, folB gene, ttdA gene, ttdB gene, rpsU gene, dnaG gene, rpoD gene, ileX gene, aer gene, ebgR gene, ebgA gene, ebgC gene, uxaA gene, uxaC gene, exuT gene, exuR gene, tdcG gene, tdcF gene, tdcE gene, tdcD gene, tdcC gene, tdcB gene, tdcA gene, tdcR gene, rnpB gene, garK gene, garR gene, garL gene, garP gene, garD gene, sohA gene, agaR gene, kbaZ gene, agaV gene, agaW gene, agaA gene, agaS gene, kbaY gene, agaB gene, agaC gene, agaD gene, agaI gene, mtr gene, deaD gene, nlpI gene, pnp gene, sraG gene, rpsO gene, truB gene, rbfA gene, infB gene, nusA gene, metY gene, argG gene, leuU gene, secG gene, glmM gene, folP gene, ftsH gene, rrmJ gene, greA gene, dacB gene, obgE gene, rpmA gene, rplU gene, ispB gene, sfsB gene, murA gene, kdsD gene, kdsC gene, rpoN gene, ptsN gene, npr gene, mtgA gene, elbB gene, ryhA gene, arcB gene, gltB gene, gltD gene, nanT gene, nanA gene, nanR gene, dcuD gene, sspB gene, sspA gene, rpsI gene, rplM gene, degQ gene, degS gene, mdh gene, argR gene, aaeB gene, aaeA gene, aaeX gene, aaeR gene, tldD gene, rng gene, mreD gene, mreC gene, mreB gene, accB gene, accC gene, panF gene, prmA gene, dusB gene, fis gene, rrfF gene, thrV gene, rrfD gene, rrlD gene, gltv gene, rrsD gene, purH gene, purD gene, zraR gene, zraS gene, zraP gene, hupA gene, nfi gene, hemE gene, nudC gene, rsd gene, thiC gene, thiE gene, thiF gene, this gene, thiG gene, thiH gene, htrC gene, rpoC gene, rpoB gene, rplL gene, rplJ gene, rplA gene, rplK gene, nusG gene, secE gene, tufB gene, thrT gene, glyT gene, tyrU gene, thrU gene, coaA gene, birA gene, murB gene, rrfB gene, rrlB gene, gltT gene, rrsB gene, murI gene, btuB gene, trmA gene, fabR gene, sthA gene, oxyR gene, oxyS gene, argH gene, argB gene, argC gene, argE gene, ppc gene, fsaB gene, gldA gene, katG gene, metF gene, metL gene, metB gene, metJ gene, rpmE gene, priA gene, cytR gene, ftsN gene, hslV gene, hslU gene, menA gene, rraA gene, glpf gene, glpK gene, glpX gene, fpr gene, tpiA gene, cdh gene, sbp gene, pfkA gene, fieF gene, cpxP gene, cpxR gene, cpxA gene, kdgT gene, sodA gene, fdhD gene, fdoG gene, fdoH gene, fdoI gene, fdhE gene, bipA gene, glnA gene, glnL gene, glnG gene, hemN gene, csrC gene, spf gene, polA gene, dsbA gene, mobA gene, mobB gene, rrfA gene, rrlA gene, alaT gene, ileT gene, rrsA gene, hemG gene, trkH gene, pepQ gene, fadB gene, fadA gene, fre gene, ubiD gene, rfaH gene, tatD gene, tatC gene, tatB gene, tatA gene, ubiB gene, ubiE gene, rmuC gene, udp gene, metE gene, metR gene, pldB gene, rhtB gene, rhtC gene, recQ gene, pldA gene, rarD gene, corA gene, uvrD gene, xerC gene, dapF gene, cyaY gene, cyaA gene, hemC gene, hemD gene, hemX gene, hemY gene, ryiA gene, aslA gene, aslB gene, proM gene, leuT gene, hisR gene, argX gene, rffM gene, wzyE gene, rffT gene, wzxE gene, rffA gene, rffC gene, rffH gene, rffG gene, rffD gene, rffE gene, wzzE gene, rfe gene, rho gene, rhoL gene, trxA gene, rhlB gene, gpp gene, rep gene, ppiC gene, ilvC gene, ilvY gene, ilvA gene, ilvD gene, ilvE gene, ilvM gene, ilvG_2 gene, ilvG_1 gene, ilvL gene, hdfR gene, trpT gene, aspT gene, rrfC gene, rrlC gene, gltU gene, rrsC gene, asnC gene, mioC gene, oriC gene, gidA gene, gidB gene, atpI gene, atpB gene, atpE gene, atpF gene, atpH gene, atpA gene, atpG gene, atpD gene, atpC gene, glmU gene, glmS gene, pstS gene, pstC gene, pstA gene, pstB gene, phoU gene, bglG gene, bglF gene, bglB gene, bglH gene, cbrC gene, mdtL gene, tnaB gene, tnaA gene, tnaC gene, trmE gene, rnpA gene, rpmH gene, dnaA gene, dnaN gene, recF gene, gyrB gene, ibpA gene, ibpB gene, emrD gene, ivbL gene, ilvB gene, ilvN gene, uhpA gene, uhpB gene, uhpC gene, uhpT gene, selC gene, gltS gene, recG gene, trmH gene, spoT gene, rpoZ gene, gmk gene, ligB gene, dinD gene, rph gene, pyrE gene, ttk gene, dut gene, dfp gene, rpmB gene, rpmG gene, mutM gene, coaD gene, kdtA gene, rfaQ gene, rfaG gene, rfaP gene, rfaS gene, rfaB gene, rfaI gene, rfaJ gene, rfaY gene, rfaZ gene, rfaK gene, rfaL gene, rfaC gene, rfaF gene, rfaD gene, htrL gene, kbl gene, tdh gene, envC gene, gpmI gene, grxC gene, secB gene, gpsA gene, cysE gene, lldD gene, lldR gene, lldP gene, mtlR gene, mtlD gene, mtlA gene, selA gene, selB gene, aldB gene, sgbE gene, sgbU gene, sgbH gene, lyxK gene, avtA gene, malS gene, bax gene, xylR gene, xylH gene, xylG gene, xylF gene, xylA gene, xylB gene, glyQ gene, glyS gene, hokA gene, cspA gene, tiaE gene, bisC gene, tag gene, eptB gene, proK gene, dppA gene, dppB gene, dppC gene, dppD gene, dppF gene, rdlD gene, ldrD gene, bcsG gene, bcsF gene, bcsE gene, bcsA gene, bcsB gene, bcsZ gene, bcsC gene, dctA gene, kdgK gene, treF gene, gadA gene, gadX gene, gadY gene, gadW gene, mdtF gene, mdtE gene, gadE gene, hdeD gene, hdeA gene, hdeB gene, slp gene, arsC gene, arsB gene, arsR gene, gor gene, prlC gene, uspA gene, pitA gene, nikR gene, nikE gene, nikD gene, nikC gene, nikB gene, nikA gene, acpT gene, dcrB gene, zntA gene, ftsY gene, ftsE gene, ftsX gene, rpoH gene, livJ gene, livK gene, livH gene, livM gene, livG gene, livF gene, ugpB gene, ugpA gene, ugpE gene, ugpC gene, ugpQ gene, ggt gene, gntR gene, gntK gene, gntU gene, asd gene, glgB gene, glgX gene, glgC gene, glgA gene, glgP gene, glpD gene, glpE gene, glpG gene, glpR gene, rtcR gene, rtcB gene, rtcA gene, malT gene, malP gene, malQ gene, gntT gene, gntY gene, gntX gene, bioH gene, feoB gene, feoA gene, greB gene, ompR gene, envZ gene, pck gene, hslO gene, hslR gene, nudE gene, mrcA gene, hofQ gene, aroK gene, aroB gene, damX gene, dam gene, rpe gene, gph gene, trpS gene, cysG gene, nirC gene, nirD gene, nirB gene, tsgA gene, ppiA gene, fic gene, pabA gene, argD gene, crp gene, prkB gene, kefG gene, kefB gene, slyD gene, slyX gene, fkpA gene, rpsL gene, rpsG gene, fusA gene, tufA gene, rpsJ gene, rplC gene, rplD gene, rplW gene, rplB gene, rpsS gene, rplV gene, rpsC gene, rplP gene, rpmC gene, rpsQ gene, rplN gene, rplX gene, rplE gene, rpsN gene, rpsH gene, rplF gene, rplR gene, rpsE gene, rpmD gene, rplo gene, secY gene, rpmJ gene, rpsM gene, rpsK gene, rpsD gene, rpoA gene, rplQ gene, zntR gene, mscL gene, trkA gene, rsmB gene, fmt gene, def gene, smf gene, smg gene, aroE gene, rrsE gene, ileU gene, alaU gene, rrlE gene, rrfE gene, metA gene, aceB gene, aceA gene, aceK gene, arpA gene, iclR gene, metH gene, pepE gene, lysC gene, pgi gene, ubiC gene, ubiA gene, plsB gene, dgkA gene, lexA gene, dinF gene, zur gene, qor gene, dnaB gene, alr gene, tyrB gene, aphA gene, uvrA gene, ssb gene, soxS gene, soxR gene, ryjA gene, actP gene, acs gene, nrfA gene, nrfB gene, nrfC gene, nrfD gene, nrfE gene, nrfF gene, nrfG gene, gltP gene, fdhF gene, rpiR gene, rpiB gene, proP gene, basS gene, basR gene, eptA gene, adiC gene, adiY gene, adiA gene, melR gene, melA gene, melB gene, fumB gene, dcuB gene, dcuR gene, dcuS gene, lysU gene, cadA gene, cadB gene, cadC gene, pheU gene, dipZ gene, cutA gene, dcuA gene, aspA gene, fxsA gene, groS gene, groL gene, efp gene, ecnA gene, ecnB gene, sugE gene, blc gene, ampC gene, frdD gene, frdC gene, frdB gene, frdA gene, poxA gene, psd gene, rsgA gene, orn gene, glyV gene, glyX gene, glyY gene, amiB gene, mutL gene, miaA gene, hfq gene, hflX gene, hflK gene, hflC gene, purA gene, rnr gene, rlmB gene, ulaF gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrL gene, argI gene, valS gene, holC gene, pepA gene, idnR gene, idnT gene, idnO gene, idnD gene, idnK gene, leuX gene, dnaC gene, dnaT gene, bglJ gene, fhuF gene, leuV gene, leuP gene, leuQ gene, rsmC gene, holD gene, rimI gene, prfC gene, osmY gene, deoC gene, deoA gene, deoB gene, deoD gene, lplA gene, serB gene, radA gene, nadR gene, slt gene, trpR gene, rob gene, creA gene, creB gene, creC gene, creD gene, arcA gene
   [2] fhuA gene, fhuC gene, fhuD gene, fhuB gene, yafJ gene, yafK gene, yafQ gene, dinJ gene, yafL gene, yafM gene, fhiA gene, mbhA gene, dinB gene, yafN gene, yafO gene, yafP gene, ykfJ gene, prfH gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM(JW5034) gene, ykgM(JW5035) gene, eaeH gene, ykgA gene, ykgB gene, ykgI gene, ykgC gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, yahI gene, yahJ gene, yahK gene, yahL gene, yahM gene, yahN gene, yahO gene, prpR gene, prpB gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, frmB gene, frmA gene, frmR gene, yaiO gene, yaiX gene, yaiF gene, yaiP gene, yaiS gene, tauA gene, tauB gene, tauC gene, tauD gene, yaiT gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, ylbG gene, ybbB gene, ybbS gene, allA gene, allR gene, glxR gene, ybbV gene, ybbW gene, allB gene, ybbY gene, glxK gene, ylbA gene, allC gene, allD gene, fdrA gene, ylbE_1 gene, ylbE_2 gene, ylbF gene, ybcF gene, envY gene, ybcH gene, nfrA gene, nfrB gene, cusS gene, cusR gene, cusC gene, cusF gene, cusB gene, cusA gene, pheP gene, ybdG gene, nfnB gene, ybdF gene, ybdJ gene, ybdK gene, hokE gene, ybeL gene, ybeQ gene, ybeR gene, djlB gene, ybeT gene, ybeU gene, djlc gene, hscC gene, rihA gene, gltL gene, gltK gene, gltJ gene, gltI gene, kdpF gene, ybfA gene, ybgA gene, phr gene, ybiA gene, dinG gene, ybiB gene, ybiC gene, ybiJ gene, ybiI gene, ybiX gene, fiu gene, ybiM gene, ybiN gene, ybiO gene, glnQ gene, glnP gene, glnH gene, yliA gene, yliB gene, yliC gene, yliD gene, yliE gene, yliF gene, yliG gene, yliH gene, yliI gene, yliJ gene, dacC gene, deoR gene, ybjG gene, ybjC gene, nfsA gene, rimK gene, ybjN gene, potF gene, potG gene, potH gene, potI gene, ybjO gene, rumB gene, artJ gene, artM gene, artQ gene, artI gene, artP gene, ybjP gene, ybjQ gene, ybjR gene, ybjS gene, ybjT gene, ltaE gene, ymcC gene, ymcD gene, cspH gene, cspG gene, ymcE gene, gnsA gene, yccM gene, torS gene, torT gene, torR gene, torC gene, torA gene, torD gene, cbpM gene, cbpA gene, yccE gene, agp gene, yccJ gene, wrbA gene, ymdF gene, ycdG gene, ycdH gene, ycdI gene, rarA gene, ycdK gene, ycdL gene, ycdM gene, ycdC gene, putA gene, putP gene, ycdN_1 gene, ycdN_2 gene, ycdO gene, ycdB gene, phoH gene, ycdP gene, ycdQ gene, ycdR gene, ycdS gene, ycdT gene, ymdE gene, ycdU gene, elbA gene, ycgX gene, ycgE gene, ycgF gene, ycgZ gene, ymgA gene, ymgB gene, ymgC gene, ycgG gene, ymgF gene, ycgH_1 gene, ycgH_2 gene, ymgD gene, ymgG gene, ymgH gene, ycgI gene, ydfH gene, ydfZ gene, ydfI gene, ydfJ gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, yecG gene, otsA gene, otsB gene, araH gene, araG gene, araF gene, yecI gene, yecJ gene, isrB gene, yecR gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN 2 gene, yedN_1 gene, yedM gene, intG gene, fliE gene, fliF gene, fliG gene, fliH gene, fill gene, fliJ gene, fliK gene, fliL gene, fliM gene, fliN gene, fliO gene, fliP gene, fliQ gene, fliR gene, rcsA gene, dsrB gene, yodD gene, yedA gene, vsr gene, dcm gene, yedJ gene, yedR gene, yedS_1 gene, yedS_2 gene, yedS_3 gene, hchA gene, yedV gene, yedw gene, yedX gene, yedY gene, yedZ gene, yodA gene, wbbL_2 gene, wbbL_1 gene, wbbK gene, wbbJ gene, wbbI gene, wbbH gene, glf gene, rfbX gene, rfbC gene, rfbA gene, rfbD gene, rfbB gene, yegE gene, alkA gene, yegD gene, yegI gene, yegJ gene, yegK gene, yegL gene, ryeC gene, ryeD gene, mdtA gene, mdtB gene, mdtC gene, mdtD gene, baeS gene, baeR gene, yegP gene, yegQ gene, ryeE gene, ogrK gene, yegZ gene, yegR gene, yegS gene, gatR_1 gene, gatR_2 gene, gatD gene, gatC gene, gatB gene, gatA gene, gatZ gene, gatY gene, fbaB gene, yegT gene, yegU gene, yegV gene, yegW gene, yegX gene, molR_1 gene, molR_2 gene, molR_3 gene, yehI gene, yehK gene, yehL gene, yehM gene, yehP gene, yehQ gene, yehR gene, yehs gene, yehT gene, yehU gene, mlrA gene, yohO gene, yehW gene, yehX gene, yehY gene, yehZ gene, bglX gene, dld gene, pbpG gene, yohC gene, yeiI gene, yeiJ gene, rihB gene, yeiL gene, yeiM gene, yeiN gene, yeiC gene, elaB gene, elaA gene, elaC gene, elaD gene, yfbK gene, yfbL gene, yfbM gene, yfbN gene, yfbO gene, yfbP gene, yfcC gene, yfcD gene, yfcE gene, yfcF gene, yfcG gene, folX gene, yfcH gene, yfcI gene, hisP gene, hisM gene, hisQ gene, hisJ gene, argT gene, ypdA gene, ypdB gene, ypdC gene, ypdD gene, ypdE gene, ypdF gene, ypdG gene, ypdH gene, pinH gene, ypjB gene, ypjC gene, ileY gene, ygaQ gene, ygaR gene, yqaC gene, yqaD gene, ygaT gene, csiR gene, ygaU gene, yqaE gene, ygaV gene, ygaP gene, stpA gene, ygaW gene, ygaC gene, ygaM gene, nrdH gene, nrdI gene, nrdE gene, proV gene, proW gene, proX gene, ygaX gene, ygaY gene, ygaZ gene, ygaH gene, srlA gene, srlE gene, srlB gene, srlD gene, gutM gene, srlR gene, gutQ gene, yqeG gene, yqeH gene, yqeI gene, yqeJ gene, yqeK gene, ygeF gene, ygeG gene, ygeH gene, ygeI gene, pbl gene, ygeK gene, ygeL gene, ygeM gene, ygeN gene, ygeO gene, ygeP gene, ygeQ gene, glyU gene, ygeR gene, xdhA gene, xdhB gene, xdhC gene, ygeV gene, ygeW gene, ygeX gene, ygeY gene, hyuA gene, yqeA gene, yqeB gene, yqeC gene, ygfJ gene, ygfK gene, ssnA gene, ygfM gene, xdhD gene, ygfO gene, guaD gene, ygfQ gene, ygfS gene, ygfT gene, ygfU gene, idi gene, cmtA gene, cmtB gene, yghD gene, yghE gene, yghF gene, yghG gene, pppA gene, yghJ gene, yghK gene, glcB gene, glcG gene, glcF gene, glcE gene, glcD gene, glcC gene, yghO gene, yghQ gene, yghR gene, yghS gene, yghT gene, ygiS gene, ygiT gene, ygiU gene, ygiV gene, ygiW gene, qseB gene, qseC gene, ygiZ gene, mdaB gene, ygiN gene, gltF gene, yhcA gene, yhcD gene, yhcE gene, yhcF gene, yhcG gene, yhcH gene, nanK gene, nanE gene, yhdJ gene, yhdU gene, envR gene, acrE gene, acrF gene, yhdV gene, yhdW gene, yhdX gene, yhdY gene, yijP gene, yijO gene, frwD gene, pflC gene, pflD gene, frwB gene, frwC gene, ptsA gene, rhaT gene, rhaR gene, rhaS gene, rhaB gene, rhaA gene, rhaD gene, yiiL gene, frvA gene, frvB gene, frvX gene, frvR gene, yiiG gene, yiiF gene, yiiE gene, yiiD gene, dtd gene, rbn gene, yihX gene, yihW gene, yihV gene, yihU gene, yihT gene, yihS gene, yihR gene, yihQ gene, yihP gene, yihO gene, ompL gene, yihN gene, yihM gene, yihL gene, hsrA gene, rbsR gene, rbsK gene, rbsB gene, rbsC gene, rbsA gene, rbsD gene, trkD gene, yieN gene, yieM gene, asnA gene, yidB gene, yidA gene, yidX gene, dgoR gene, dgoK gene, dgoA gene, dgoD gene, dgoT gene, cbrA gene, yidR gene, yidQ gene, yidE gene, yidP gene, glvC gene, glvB gene, glvG gene, ysdC gene, yidL gene, yidK gene, yidJ gene, yidI gene, yidH gene, yidG gene, yidF gene, yhiN gene, yhiM gene, yhiL gene, yhiK gene, yhiJ gene, yhiI gene, rbbA gene, yhhJ gene, chiA gene, bfd gene, bfr gene, gspO gene, gspM gene, gspL gene, gspK gene, gspJ gene, gspI gene, gspH gene, gspG gene, gspF gene, gspE gene, gspD gene, gspC gene, gspA gene, pioO gene, yjbE gene, yjbF gene, yjbG gene, yjbH gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, yjdP gene, phnP gene, phnO gene, phnN gene, phnM gene, phnL gene, phnK gene, phnJ gene, phnI gene, phnH gene, phnG gene, phnF gene, phnE(JW4064) gene, phnE(JW4065) gene, phnD gene, phnC gene, phnB gene, phnA gene, yjdA gene, yjcZ gene, yjfI gene, yjfJ gene, yjfK gene, yjfL gene, yjfM gene, yjfC gene, aidB gene, yjfN gene, yjfO gene, yjfP gene, ulaR gene, ulaG gene, ulaA gene, ulaB gene, ulaC gene, ulaD gene, ulaE gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, kptA gene, yjiJ gene, yjiK gene, yjiL gene, yjiM gene, yjiN gene, yjiO gene, yjiP gene, yjiQ gene, yjiR gene, yjiS gene, yjiT gene, yjiV gene, mcrC gene, mcrB gene, yjiW gene, hsdS gene, hsdM gene, hsdR gene, mrr gene, yjiA gene, yjiX gene, yjiY gene, tsr gene, yjiZ gene, yjjM gene, yjjN gene, mdoB gene, yjjA gene
   [3] chpA gene, uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, ybeM gene, yqcA gene, yqcB gene, yqcC gene, syd gene, yqcD gene, ygdH gene, sdaC gene, sdaB gene, exo gene, fucO gene, fucA gene, fucP gene, fuel gene, fucK gene, fucU gene, fucR gene, ygdE gene, ygdD gene, gcvA gene, ygdI gene, csdA gene, ygdK gene, araD gene, araA gene, araB gene, araC gene, yabI gene, thiQ gene, thiP gene, tbpA gene, sgrR gene, setA gene, yhfZ gene, yhfY gene, yhfX gene, yhfW gene, php gene, yhfU gene, yhfT gene, yhfS gene, frlR gene, frlD gene, frlc gene, frlB gene, frlA gene, yhfL gene, pncA gene, ydjE gene, ydjF gene, ydjG gene, ydjH gene, ydjI gene, ydjJ gene, ydjK gene, ydjL gene, yeaC gene, yeaA gene, ade gene, yicO gene, yicN gene, yicM gene, yicS gene, nlpA gene, yicL gene, setC gene, ygjI gene, ygjJ gene, ygjK gene, fadH gene, ygjM gene, ygjN gene, ygjO gene, ygjP gene, ygjQ gene, ygjR gene, alx gene, sstT gene, ygjV gene, yrhB gene, yrhA gene, yhhZ gene, yhhY gene, yhhX gene, yhhW gene, yeaK gene, yoaI gene, yeaL gene, yeaM gene, yeaN gene, yeaO gene, yoaF gene, yeaP gene, yeaQ gene, yoaG gene, yeaR gene, yeaS gene, yeaT gene, yeaU gene, yeaV gene, yeaW gene, yeaX gene, hyfA gene, hyfB gene, hyfC gene, hyfD gene, hyfE gene, hyfF gene, hyfG gene, hyfH gene, hyfI gene, hyfJ gene, hyfR gene, and focB gene.
(24) The *E*. *coli* mutant strain obtained via modification of the *E*. *coli* mutant strain according to any one of (1) to (23) by a method selected from among the following [1] to [5]:
   [1] a method for reducing or removing at least one mechanism for regulating biosynthesis of the useful substance;
   [2] a method for increasing the expression of at least one enzyme associated with biosynthesis of the useful substance;
   [3] a method for increasing the copy number of at least one enzyme gene associated with biosynthesis of the useful substance;
   [4] a method for attenuating or blocking at least one metabolic pathway which branches from the biosynthetic pathway of the useful substance into metabolites other than the useful substance; or
   [5] a method for selecting a strain that exhibits higher resistance to an analogue of a useful substance than a wild-type *E. coli* strain.
(25) The *E. coli* mutant strain according to any one of (1) to (24), wherein the *E. coli* strain is the *E. coli* K-12, B, or W strain.
(26) The *E. coli* mutant strain according to (25), wherein the *E. coli* K-12 strain is the *E. coli* W3110 strain (ATCC27325) or the *E. coli* MG1655 strain (ATCC47076).
(27) A method for producing a useful substance comprising culturing the *E. coli* mutant strain according to any one of (1) to (26) in a medium so as to generate and accumulate the useful substance in the culture and recovering the useful substance from the culture.
(28) The method according to any one of (24) to (27), wherein the useful substance is selected from the group consisting of proteins, amino acids, nucleic acids, vitamins, saccharides, organic acids, and lipids.

### Effect of the Invention

The present invention can provide an *E. coli* mutant, the amount of *E. coli* cells of which, after a given period of culture, is larger than that of a wild-type *E. coli* strain, and a process for producing a useful substance using such mutant.

### Best Modes for Carrying out the Invention

In the present description, the wild-type *E. coli* strains may be any *E. coli* strains that are found in natural populations at the highest frequency. Examples thereof include the *E. coli* W3110 strain (ATCC27325) and the *E. coli* MG1655 strain (ATCC47076) that belong to the *E. coli* K-12 strain, the ATCC11303 strain that belongs to the *E. coli* B strain, and the ATCC9637 strain that belongs to the *E. coli* W strain.
The nucleotide sequence as shown in SEQ ID NO: 1 in the present specification is the full-length nucleotide sequence of chromosomal DNA of the *E. coli* W3110 strain registered as of August 16, 2007 on the internet site URL::http://www.ncbi.nlm.nih.gov/entrez/viewer.fcgi?db=nuccore &id=89106884. The above-mentioned nucleotide sequence shown in the internet site is a nucleotide sequence of Accession No. AC_000091 in NCBI (National Center for Biothechnology Information) GenBank.

In addition, the name of each gene on the Escherichia coli chromosome, which is used in the above-mentioned internet site, is used in Table 1 and Table 2 of the present description.
In Table 1, the gene shown by the gene name ykgM(JW5034) refers to nucleotides 311598 to 311738 of the nucleotide sequence as shown in SEQ ID NO: 1, the gene shown by the gene name ykgM(JW5035) refers to nucleotides 311738 to 312001 of the nucleotide sequence as shown in SEQ ID NO: 1, the gene shown by the gene name phnE(JW4064) refers to nucleotides 4327121 to 4327489 of the nucleotide sequence, and the gene shown by the gene name phnE(JW4065) refers to nucleotides 4327339 to 4327959 of the nucleotide sequence as shown in SEQ ID NO: 1.

The term chromosomal DNA that contains genes refers to chromosomal DNA that contains a specific structural gene and a region containing a specific regulatory function such as a promoter, operator or the like.
In the present specification, the term chromosomal DNA that does not contain gene refers to chromosomal DNA that does not contain a part of or an entire structural gene of a given gene or chromosomal DNA that does not contain a region having a specific regulatory function, such as a promoter and/or operator of such gene.

The chromosomal DNA that does not contain part of a structural gene is DNA that does not contain part of a structural gene, and a functional protein is not transcribed and expressed from the structural gene. Examples thereof include DNA that has no initiation codon due to a lack of the 5' end region of the structural gene and DNA that lacks most of the 3' end region, such as 10% or more, preferably 20% or more, more preferably 30% or more, and further preferably 40% or more of the entire 3' end region. Chromosomal DNA that does not contain two adjacent genes may be chromosomal DNA that does not contain DNA consisting of the nucleotide sequence located between such genes.

The homologous gene refers to a gene that shows sequence homology of the nucleotide sequence of the gene in the Escherichia coli species, or a gene encoding a protein having an amino acid sequence showing sequence homology. Showing sequence homology means that a nucleotide sequence shows sequence similarity of not less than 85%, preferably not less than 90%, more preferably not less than 95%, still more preferably not less than 98%, particularly preferably not less than 99%, or an amino acid sequence shows sequence similarity of not less than 80%, preferably not less than 90%, more preferably not less than 95%, still more preferably not less than 98%, particularly preferably not less than 99%.

The *E. coli* mutant strain having a chromosomal DNA containing not more than 10% of a particular set of genes or homologous gene thereof means an *E. coli* mutant strain having a chromosomal DNA lacking not less than 90%, preferably not less than 95%, more preferably not less than 98%, still more preferably not less than 99%, of a gene selected from a set of genes or homologous gene thereof.

The DNA equivalent to DNA comprising a nucleotide sequence represented by given nucleotide numbers in the nucleotide sequence as shown in SEQ ID NO: 1 may be any DNA, as far as it is substantially the same as DNA having a nucleotide sequence represented by such given nucleotide numbers of an *E*. *coli* species in terms of the composition of the gene contained and the position on chromosomal DNA. When compositions of genes contained in DNA are substantially the same as each other, genes contained in such DNA are composed of genes identical or homologous to each other, and arranged in the same order. In that case, nucleotide sequences may differ among genes. When the DNA having a nucleotide sequence represented by given nucleotide numbers contains an insertion sequence, however, such insertion sequence may be absent in DNA equivalent to the aforementioned DNA.

Substantially the same positions on chromosomal DNA refers to the following conditions. When adenine of the initiation codon of the thrL gene on *E. coli* chromosomal DNA is designated as No. 190 and the nucleotide sequence of chromosomal DNA is numbered along the coding chain of the thrL gene, differences between such nucleotide numbers and the given nucleotide numbers are 50 or smaller, preferably 30 or smaller, more preferably 10 or smaller, and further preferably 5 or smaller. When the DNA having a nucleotide sequence represented by given nucleotide numbers contains an insertion sequence, however, DNA equivalent to the aforementioned DNA does not necessarily contain such insertion sequence. Thus, such insertion sequence and nucleotide numbers may be different. An example of DNA equivalent to DNA having a nucleotide sequence represented by given nucleotide numbers of the nucleotide sequence as shown in SEQ ID NO: 1 is DNA having sequence similarity of 80% or more, preferably 90% or more, more preferably 95% or more, further preferably 98% or more, and particularly preferably 99% or more with the nucleotide sequence represented by given nucleotide numbers.

Sequence similarity of amino acid sequence or nucleotide sequence can be determined with the utilization of the BLAST algorithm of Karlin and Altschul (Pro. Natl. Acad. Sci., U.S.A., 90, 5873, 1993) or the FASTA algorithm (Methods Enzymol., 183, 63, 1990). Based on the BLAST algorithm, programs referred to as BLASTN or BLASTX have been developed (J. Mol. Biol., 215, 403, 1990). When analyzing nucleotide sequences by BLASTN based on BLAST, the score parameter is set to 100 and the word length parameter is set to 12, for example. When analyzing amino acid sequences via BLASTX based on BLAST, the score parameter is set to 50 and the word length parameter is set to 3, for example. When using BLAST and Gapped BLAST programs, the default parameters thereof are used. Specific procedures of such analytical methods are known (http://www.ncbi.nlm.nih.gov.).

### 1. E. coli mutant strain of the present invention

The *E*. *coli* mutant strain of the present invention may be
(1) any *E*. *coli* mutant strain comprising chromosomal DNA that comprise any gene excluding the genes [2] that are present on chromosomal DNA of a wild-type *E. coli* strain or any *E. coli* mutant strain comprising chromosomal DNA that does not comprise any gene excluding the genes [1], provided that it comprises the genes [1], and does not comprise the genes [2] and having chromosomal DNA that is at least 1040 kbp shorter than that of a wild-type *E*. *coli* strain,
   [1] thrL gene, thrA gene, thrB gene, thrC gene, talB gene, mog gene, htgA gene, dnaK gene, dnaJ gene, hokC gene, mokC gene, sokC gene, nhaA gene, nhaR gene, rpsT gene, ribF gene, ileS gene, lspA gene, fkpB gene, ispH gene, rihC gene, dapB gene, carA gene, carB gene, caiF gene, caiE gene, caiD gene, caiC gene, caiB gene, caiA gene, caiT gene, fixA gene, fixB gene, fixC gene, fixX gene, kefF gene, kefC gene, folA gene, apaH gene, apaG gene, ksgA gene, pdxA gene, surA gene, imp gene, djlA gene, rluA gene, hepA gene, polB gene, leuD gene, leuC gene, leuB gene, leuA gene, leuL gene, leuO gene, ilvI gene, ilvH gene, fruR gene, mraZ gene, mraW gene, ftsL gene, ftsI gene, murE gene, murF gene, mraY gene, murD gene, ftsW gene, murG gene, murC gene, ddlB gene, ftsQ gene, ftsA gene, ftsZ gene, IpxC gene, secM gene, secA gene, mutT gene, coaE gene, guaC gene, hofC gene, hofB gene, ppdD gene, nadC gene, ampD gene, ampE gene, aroP gene, pdhR gene, aceE gene, aceF gene, lpd gene, acnB gene, speD gene, speE gene, cueO gene, gcd gene, hpt gene, can gene, panD gene, panC gene, panB gene, htrE gene, ecpD gene, folK gene, pcnB gene, dksA gene, sfsA gene, ligT gene, hrpB gene, mrcB gene, hemL gene, clcA gene, btuF gene, pfs gene, dgt gene, degP gene, cdaR gene, dapD gene, glnD gene, map gene, tff gene, rpsB gene, tsf gene, pyrH gene, frr gene, dxr gene, ispU gene, cdsA gene, hlpA gene, IpxD gene, fabZ gene, IpxA gene, IpxB gene, rnhB gene, dnaE gene, accA gene, IdcC gene, tilS gene, rof gene, nlpE gene, proS gene, rcsF gene, metQ gene, metI gene, metN gene, gmhB gene, rrsH gene, ileV gene, alaV gene, rrlH gene, rrfH gene, aspU gene, dkgB gene, mltD gene, gloB gene, rnhA gene, dnaQ gene, aspV gene, ivy gene, fadE gene, lpcA gene, pepD gene, gpt gene, frsA gene, crl gene, phoE gene, proB gene, proA gene, thrW gene, hemB gene, proC gene, aroL gene, aroM gene, rdgC gene, mak gene, araJ gene, sbcC gene, sbcD gene, phoB gene, phoR gene, brnQ gene, proY gene, malZ gene, queA gene, tgt gene, secD gene, secF gene, tsx gene, ribD gene, ribE gene, nusB gene, thiL gene, pgpA gene, dxs gene, ispA gene, xseB gene, thiI gene, panE gene, cyoE gene, cyoD gene, cyoC gene, cyoB gene, cyoA gene, ampG gene, bolA gene, tig gene, clpP gene, clpX gene, lon gene, hupB gene, ppiD gene, cof gene, mdlA gene, mdlB gene, glnK gene, amtB gene, tesB gene, ffs gene, maa gene, hha gene, acrB gene, acrA gene, acrR gene, kefA gene, priC gene, apt gene, dnaX gene, recR gene, htpG gene, adk gene, hemH gene, aes gene, gsk gene, fsr gene, ushA gene, copA gene, cueR gene, gcl gene, hyi gene, purK gene, purE gene, lpxH gene, ppiB gene, cysS gene, folD gene, sfmA gene, sfmC gene, sfmD gene, sfmH gene, sfmF gene, fimZ gene, argU gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, tatE gene, lipA gene, lipB gene, dacA gene, rlpA gene, mrdB gene, mrdA gene, cobC gene, nadD gene, holA gene, rlpB gene, leuS gene, lnt gene, miaB gene, ubiF gene, glnX gene, glnV gene, metU gene, glnW gene, glnU gene, leuW gene, metT gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, nei gene, abrB gene, gltA gene, sdhC gene, sdhD gene, sdhA gene, sdhB gene, sucA gene, sucB gene, sucC gene, sucD gene, mngR gene, mngA gene, mngB gene, cydA gene, cydB gene, tolQ gene, tolR gene, tolA gene, tolB gene, pal gene, lysT gene, valT gene, lysW gene, valZ gene, lysY gene, lysZ gene, lysQ gene, nadA gene, pnuC gene, zitB gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, rhtA gene, ompX gene, rybA gene, mntR gene, fsaA gene, moeB gene, moeA gene, iaaA gene, cmr gene, rybB gene, grxA gene, poxB gene, hcr gene, hcp gene, aqpZ gene, macA gene, macB gene, cspD gene, clpS gene, clpA gene, serW gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, ihfB gene, msbA gene, lpxK gene, kdsB gene, smtA gene, mukF gene, mukE gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, serT gene, hyaA gene, hyaB gene, hyaC gene, hyaD gene, hyaE gene, hyaF gene, appC gene, appB gene, appA gene, etp gene, serX gene, csgG gene, csgF gene, csgE gene, csgD gene, csgB gene, csgA gene, csgC gene, mdoC gene, mdoG gene, mdoH gene, msyB gene, mdtG gene, lpxL gene, solA gene, dinI gene, pyrC gene, grxB gene, mdtH gene, rimJ gene, mviM gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, sraB gene, rpmF gene, plsX gene, fabH gene, fabD gene, fabG gene, acpP gene, fabF gene, pabC gene, tmk gene, holB gene, ptsG gene, fhuE gene, hinT gene, nagZ gene, ndh gene, mfd gene, lolC gene, lolD gene, lolE gene, cobB gene, potD gene, potC gene, potB gene, potA gene, pepT gene, phoQ gene, phoP gene, purB gene, hflD gene, trmU gene, minE gene, minD gene, minC gene, hlyE gene, umuD gene, umuC gene, dsbB gene, nhaB gene, fadR gene, dadA gene, dadX gene, cvrA gene, ldcA gene, emtA gene, treA gene, dhaH gene, dhaL gene, dhaK gene, dhaR gene, pth gene, prsA gene, ispE gene, lolB gene, hemA gene, prfA gene, prmC gene, kdsA gene, ldrA gene, rdlA gene, ldrB gene, rdlB gene, ldrC gene, rdlC gene, chaA gene, chaB gene, chaC gene, narL gene, narX gene, narK gene, narG gene, narH gene, narJ gene, narI gene, rttR gene, tpr gene, tyrV gene, tyrT gene, purU gene, rssA gene, rssB gene, galU gene, hns gene, tdk gene, adhE gene, oppA gene, oppB gene, oppC gene, oppD gene, oppF gene, cls gene, kch gene, tonB gene, ompW gene, trpA gene, trpB gene, trpC gene, trpD gene, trpE gene, trpL gene, rluB gene, btuR gene, sohB gene, topA gene, cysB gene, acnA gene, ribA gene, pgpB gene, pyrF gene, osmB gene, gmr gene, rnb gene, fabI gene, sapF gene, sapD gene, sapC gene, sapB gene, sapA gene, puuP gene, puuA gene, puuD gene, puuR gene, puuC gene, puuB gene, puuE gene, pspF gene, pspA gene, pspB gene, pspC gene, pspD gene, pspE gene, ompG gene, tyrR gene, tpx gene, mpaA gene, mppA gene, uspE gene, fnr gene, ogt gene, abgT gene, abgB gene, abgA gene, abgR gene, isrA gene, dbpA gene, uspF gene, ompN gene, micC gene, hslJ gene, ldhA gene, feaR gene, feaB gene, tynA gene, maoC gene, paaA gene, paaB gene, paaC gene, paaD gene, paaE gene, paaF gene, paaG gene, paaH gene, paaI gene, paaJ gene, paaK gene, paaX gene, paaY gene, azoR gene, hrpA gene, aldA gene, gapC_2 gene, gapC_1 gene, cybB gene, hokB gene, mokB gene, sokB gene, trg gene, mdoD gene, rimL gene, tehA gene, tehB gene, ansP gene, nhoA gene, narV gene, narW gene, narY gene, narZ gene, narU gene, fdnG gene, fdnH gene, fdnI gene, adhP gene, sfcA gene, sra gene, bdm gene, osmC gene, ddpF gene, ddpD gene, ddpC gene, ddpB gene, ddpA gene, ddpX gene, dos gene, gadC gene, gadB gene, pqqL gene, hipA gene, hipB gene, ego gene, lsrC gene, lsrD gene, lsrB gene, lsrF gene, lsrG gene, tam gene, uxaB gene, marC gene, marR gene, marA gene, marB gene, eamA gene, dcp gene, rspB gene, rspA gene, speG gene, dmsD gene, clcB gene, dgsA gene, asr gene, mdtI gene, mdtJ gene, pntB gene, pntA gene, folM gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, rsxA gene, rsxB gene, rsxC gene, rsxD gene, rsxG gene, rsxE gene, nth gene, gst gene, pdxY gene, tyrS gene, pdxH gene, slyB gene, slyA gene, sodC gene, nemA gene, gloA gene, rnt gene, lhr gene, sodB gene, purR gene, cfa gene, ribC gene, mdtK gene, valV gene, valW gene, pykF gene, lpp gene, sufE gene, sufS gene, sufD gene, sufC gene, sufB gene, sufA gene, rydB gene, rprA gene, aroD gene, pps gene, aroH gene, nlpC gene, btuD gene, btuE gene, btuC gene, ihfA gene, pheT gene, pheS gene, pheM gene, rplT gene, rpmI gene, infC gene, thrS gene, arpB_1 gene, arpB_2 gene, pfkB gene, cedA gene, katE gene, chbG gene, chbF gene, chbR gene, chbA gene, chbC gene, chbB gene, osmE gene, nadE gene, spy gene, astE gene, astB gene, astD gene, astA gene, astC gene, xthA gene, nudG gene, gdhA gene, topB gene, selD gene, sppA gene, ansA gene, gapA gene, mipA gene, rnd gene, fadD gene, pabB gene, sdaA gene, manX gene, manY gene, manZ gene, rrmA gene, cspC gene, kdgR gene, htpX gene, prc gene, proQ gene, pphA gene, ryeA gene, ryeB gene, holE gene, exoX gene, ptrB gene, purT gene, eda gene, edd gene, zwf gene, pykA gene, lpxM gene, znuA gene, znuC gene, znuB gene, ruvB gene, ruvA gene, ruvC gene, nudB gene, aspS gene, torZ gene, torY gene, cutC gene, argS gene, leuZ gene, cysT gene, glyW gene, pgsA gene, dsrA gene, serU gene, asnT gene, shiA gene, amn gene, asnW gene, asnU gene, cbl gene, nac gene, asnV gene, erfK gene, cobT gene, cobS gene, cobU gene, sbmC gene, dacD gene, sbcB gene, hisL gene, hisG gene, hisD gene, hisC gene, hisB gene, hisH gene, hisA gene, hisF gene, hisI gene, cld gene, ugd gene, gnd gene, galF gene, wcaM gene, wcaL gene, wcaK gene, wzxC gene, wcaJ gene, cpsG gene, cpsB gene, wcaI gene, nudD gene, fcl gene, gmd gene, wcaF gene, wcaE gene, wcaD gene, wcaC gene, wcaB gene, wcaA gene, wzc gene, wzb gene, wza gene, asmA gene, dcd gene, udk gene, thiD gene, thiM gene, mrp gene, metG gene, dusC gene, cdd gene, sanA gene, mglC gene, mglA gene, mglB gene, galS gene, folE gene, cirA gene, lysP gene, nfo gene, fruA gene, fruK gene, fruB gene, setB gene, spr gene, rtn gene, bcr gene, rsuA gene, rplY gene, proL gene, narP gene, ccmH gene, ccmG gene, ccmF gene, ccmE gene, ccmD gene, ccmC gene, ccmB gene, ccmA gene, napC gene, napB gene, napH gene, napG gene, napA gene, napD gene, napF gene, eco gene, mqo gene, alkB gene, ada gene, ompC gene, micF gene, rcsD gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, ais gene, arnT gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoC gene, nuoB gene, nuoA gene, lrhA gene, ackA gene, pta gene, ubiX gene, purF gene, cvpA gene, dedD gene, folC gene, accD gene, dedA gene, truA gene, usg gene, pdxB gene, flk gene, fabB gene, trmC gene, mepA gene, aroC gene, prmB gene, sixA gene, fadL gene, vacJ gene, argW gene, dsdC gene, dsdX gene, dsdA gene, emrY gene, emrK gene, evgA gene, evgS gene, oxc gene, frc gene, ddg gene, glk gene, mntH gene, nupC gene, alaX gene, alaW gene, gltX gene, valU gene, valX gene, valY gene, lysV gene, xapR gene, xapB gene, xapA gene, ligA gene, zipA gene, cysZ gene, cysK gene, ptsH gene, ptsI gene, crr gene, pdxK gene, cysM gene, cysA gene, cysW gene, cysU gene, cysP gene, ucpA gene, murP gene, amiA gene, hemF gene, eutL gene, eutC gene, eutB gene, eutA gene, eutH gene, eutG gene, eutJ gene, eutE gene, cchB gene, cchA gene, eutI gene, eutT gene, eutQ gene, eutP gene, maeB gene, talA gene, tktB gene, aegA gene, narQ gene, acrD gene, dapE gene, purC gene, nlpB gene, dapA gene, gcvR gene, bcp gene, hda gene, uraA gene, upp gene, purM gene, purN gene, ppk gene, ppx gene, guaA gene, guaB gene, xseA gene, der gene, hisS gene, ispG gene, ndk gene, pbpC gene, sseA gene, ryfA gene, sseB gene, pepB gene, fdx gene, hscA gene, hscB gene, iscA gene, iscU gene, iscS gene, iscR gene, suhB gene, csiE gene, hcaT gene, hcaR gene, hcaE gene, hcaF gene, hcaC gene, hcaB gene, hcaD gene, glyA gene, hmp gene, glnB gene, sroF gene, purL gene, tadA gene, acpS gene, pdxJ gene, recO gene, era gene, rnc gene, lepB gene, lepA gene, rseC gene, rseB gene, rseA gene, rpoE gene, nadB gene, srmB gene, ung gene, trxC gene, pssA gene, kgtP gene, rrfG gene, rrlG gene, gltW gene, rrsG gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, ssrA gene, gabD gene, gabT gene, gabP gene, nrdF gene, mprA gene, emrA gene, emrB gene, luxS gene, sraD gene, gshA gene, argQ gene, argZ gene, argY gene, argV gene, serV gene, csrA gene, alaS gene, recX gene, recA gene, mltB gene, norR gene, norV gene, norW gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, pphB gene, rpoS gene, nlpD gene, pcm gene, surE gene, truD gene, ispF gene, ispD gene, ftsB gene, cysC gene, cysN gene, cysD gene, iap gene, cysH gene, cysI gene, cysJ gene, eno gene, pyrG gene, mazG gene, chpR gene, relA gene, rumA gene, barA gene, gudD gene, gudX gene, gudP gene, mltA gene, metZ gene, metW gene, metV gene, amiC gene, argA gene, recD gene, recB gene, ptr gene, recC gene, ppdC gene, ppdB gene, ppdA gene, thyA gene, lgt gene, ptsP gene, nudH gene, mutH gene, tas gene, aas gene, rygA gene, rygB gene, galR gene, lysA gene, lysR gene, araE gene, kduD gene, kduI gene, lysS gene, prfB gene, recJ gene, dsbC gene, xerD gene, fldB gene, bglA gene, gcvP gene, gcvH gene, gcvT gene, visC gene, ubiH gene, pepP gene, zapA gene, ssrS gene, rygC gene, serA gene, rpiA gene, argP gene, argK gene, argO gene, mscS gene, fbaA gene, pgk gene, epd gene, tktA gene, speB gene, speA gene, metK gene, galP gene, sprT gene, endA gene, gshB gene, ansB gene, mutY gene, mltC gene, nupG gene, speC gene, pheV gene, pitB gene, gss gene, hybG gene, hybF gene, hybE gene, hybD gene, hybC gene, hybB gene, hybA gene, hybO gene, exbD gene, exbB gene, metC gene, dkgA gene, sufI gene, plsC gene, parC gene, parE gene, cpdA gene, nudF gene, tolC gene, zupT gene, ribB gene, glgS gene, rygD gene, rfaE gene, glnE gene, htrG gene, cca gene, bacA gene, folB gene, ttdA gene, ttdB gene, rpsU gene, dnaG gene, rpoD gene, ileX gene, aer gene, ebgR gene, ebgA gene, ebgC gene, uxaA gene, uxaC gene, exuT gene, exuR gene, tdcG gene, tdcF gene, tdcE gene, tdcD gene, tdcC gene, tdcB gene, tdcA gene, tdcR gene, rnpB gene, garK gene, garR gene, garL gene, garP gene, garD gene, sohA gene, agaR gene, kbaZ gene, agaV gene, agaW gene, agaA gene, agaS gene, kbaY gene, agaB gene, agaC gene, agaD gene, agaI gene, mtr gene, deaD gene, nlpI gene, pnp gene, sraG gene, rpsO gene, truB gene, rbfA gene, infB gene, nusA gene, metY gene, argG gene, leuU gene, secG gene, glmM gene, folP gene, ftsH gene, rrmJ gene, greA gene, dacB gene, obgE gene, rpmA gene, rplU gene, ispB gene, sfsB gene, murA gene, kdsD gene, kdsC gene, rpoN gene, ptsN gene, npr gene, mtgA gene, elbB gene, ryhA gene, arcB gene, gltB gene, gltD gene, nanT gene, nanA gene, nanR gene, dcuD gene, sspB gene, sspA gene, rpsI gene, rplM gene, degQ gene, degS gene, mdh gene, argR gene, aaeB gene, aaeA gene, aaeX gene, aaeR gene, tldD gene, rng gene, mreD gene, mreC gene, mreB gene, accB gene, accC gene, panF gene, prmA gene, dusB gene, fis gene, rrfF gene, thrV gene, rrfD gene, rrlD gene, gltv gene, rrsD gene, purH gene, purD gene, zraR gene, zras gene, zraP gene, hupA gene, nfi gene, hemE gene, nudC gene, rsd gene, thiC gene, thiE gene, thiF gene, thiS gene, thiG gene, thiH gene, htrC gene, rpoC gene, rpoB gene, rplL gene, rplJ gene, rplA gene, rplK gene, nusG gene, secE gene, tufB gene, thrT gene, glyT gene, tyrU gene, thrU gene, coaA gene, birA gene, murB gene, rrfB gene, rrlB gene, gltT gene, rrsB gene, murI gene, btuB gene, trmA gene, fabR gene, sthA gene, oxyR gene, oxyS gene, argH gene, argB gene, argC gene, argE gene, ppc gene, fsaB gene, gldA gene, katG gene, metF gene, metL gene, metB gene, metJ gene, rpmE gene, priA gene, cytR gene, ftsN gene, hslV gene, hslU gene, menA gene, rraA gene, glpF gene, glpK gene, glpX gene, fpr gene, tpiA gene, cdh gene, sbp gene, pfkA gene, fieF gene, cpxP gene, cpxR gene, cpxA gene, kdgT gene, sodA gene, fdhD gene, fdoG gene, fdoH gene, fdoI gene, fdhE gene, bipA gene, glnA gene, glnL gene, glnG gene, hemN gene, csrC gene, spf gene, polA gene, dsbA gene, mobA gene, mobB gene, rrfA gene, rrlA gene, alaT gene, ileT gene, rrsA gene, hemG gene, trkH gene, pepQ gene, fadB gene, fadA gene, fre gene, ubiD gene, rfaH gene, tatD gene, tatC gene, tatB gene, tatA gene, ubiB gene, ubiE gene, rmuC gene, udp gene, metE gene, metR gene, pldB gene, rhtB gene, rhtC gene, recQ gene, pldA gene, rarD gene, corA gene, uvrD gene, xerC gene, dapF gene, cyaY gene, cyaA gene, hemC gene, hemD gene, hemX gene, hemY gene, ryiA gene, aslA gene, aslB gene, proM gene, leuT gene, hisR gene, argX gene, rffM gene, wzyE gene, rffT gene, wzxE gene, rffA gene, rffC gene, rffH gene, rffG gene, rffD gene, rffE gene, wzzE gene, rfe gene, rho gene, rhoL gene, trxA gene, rhlB gene, gpp gene, rep gene, ppiC gene, ilvC gene, ilvY gene, ilvA gene, ilvD gene, ilvE gene, ilvM gene, ilvG_2 gene, ilvG_1 gene, ilvL gene, hdfR gene, trpT gene, aspT gene, rrfC gene, rrlC gene, gltU gene, rrsC gene, asnC gene, mioC gene, oriC gene, gidA gene, gidB gene, atpI gene, atpB gene, atpE gene, atpF gene, atpH gene, atpA gene, atpG gene, atpD gene, atpC gene, glmU gene, glms gene, pstS gene, pstC gene, pstA gene, pstB gene, phoU gene, bglG gene, bglF gene, bglB gene, bglH gene, cbrC gene, mdtL gene, tnaB gene, tnaA gene, tnaC gene, trmE gene, rnpA gene, rpmH gene, dnaA gene, dnaN gene, recF gene, gyrB gene, ibpA gene, ibpB gene, emrD gene, ivbL gene, ilvB gene, ilvN gene, uhpA gene, uhpB gene, uhpC gene, uhpT gene, selC gene, gltS gene, recG gene, trmH gene, spoT gene, rpoZ gene, gmk gene, ligB gene, dinD gene, rph gene, pyrE gene, ttk gene, dut gene, dfp gene, rpmB gene, rpmG gene, mutM gene, coaD gene, kdtA gene, rfaQ gene, rfaG gene, rfaP gene, rfaS gene, rfaB gene, rfaI gene, rfaJ gene, rfaY gene, rfaZ gene, rfaK gene, rfaL gene, rfaC gene, rfaF gene, rfaD gene, htrL gene, kbl gene, tdh gene, envC gene, gpmI gene, grxC gene, secB gene, gpsA gene, cysE gene, lldD gene, lldR gene, lldP gene, mtlR gene, mtlD gene, mtlA gene, selA gene, selB gene, aldB gene, sgbE gene, sgbU gene, sgbH gene, lyxK gene, avtA gene, malS gene, bax gene, xylR gene, xylH gene, xylG gene, xylF gene, xylA gene, xylB gene, glyQ gene, glyS gene, hokA gene, cspA gene, tiaE gene, bisC gene, tag gene, eptB gene, proK gene, dppA gene, dppB gene, dppC gene, dppD gene, dppF gene, rdlD gene, ldrD gene, bcsG gene, bcsF gene, bcsE gene, bcsA gene, bcsB gene, bcsZ gene, bcsC gene, dctA gene, kdgK gene, treF gene, gadA gene, gadX gene, gadY gene, gadW gene, mdtF gene, mdtE gene, gadE gene, hdeD gene, hdeA gene, hdeB gene, slp gene, arsC gene, arsB gene, arsR gene, gor gene, prlC gene, uspA gene, pitA gene, nikR gene, nikE gene, nikD gene, nikC gene, nikB gene, nikA gene, acpT gene, dcrB gene, zntA gene, ftsY gene, ftsE gene, ftsX gene, rpoH gene, livJ gene, livK gene, livH gene, livM gene, livG gene, livF gene, ugpB gene, ugpA gene, ugpE gene, ugpC gene, ugpQ gene, ggt gene, gntR gene, gntK gene, gntU gene, asd gene, glgB gene, glgX gene, glgC gene, glgA gene, glgP gene, glpD gene, glpE gene, glpG gene, glpR gene, rtcR gene, rtcB gene, rtcA gene, malT gene, malP gene, malQ gene, gntT gene, gntY gene, gntX gene, bioH gene, feoB gene, feoA gene, greB gene, ompR gene, envZ gene, pck gene, hslO gene, hslR gene, nudE gene, mrcA gene, hofQ gene, aroK gene, aroB gene, damX gene, dam gene, rpe gene, gph gene, trpS gene, cysG gene, nirC gene, nirD gene, nirB gene, tsgA gene, ppiA gene, fic gene, pabA gene, argD gene, crp gene, prkB gene, kefG gene, kefB gene, slyD gene, slyX gene, fkpA gene, rpsL gene, rpsG gene, fusA gene, tufA gene, rpsJ gene, rplC gene, rplD gene, rplW gene, rplB gene, rpsS gene, rplV gene, rpsC gene, rplP gene, rpmC gene, rpsQ gene, rplN gene, rplX gene, rplE gene, rpsN gene, rpsH gene, rplF gene, rplR gene, rpsE gene, rpmD gene, rplO gene, secY gene, rpmJ gene, rpsM gene, rpsK gene, rpsD gene, rpoA gene, rplQ gene, zntR gene, mscL gene, trkA gene, rsmB gene, fmt gene, def gene, smf gene, smg gene, aroE gene, rrsE gene, ileU gene, alaU gene, rrlE gene, rrfE gene, metA gene, aceB gene, aceA gene, aceK gene, arpA gene, iclR gene, metH gene, pepE gene, lysC gene, pgi gene, ubiC gene, ubiA gene, plsB gene, dgkA gene, lexA gene, dinF gene, zur gene, qor gene, dnaB gene, alr gene, tyrB gene, aphA gene, uvrA gene, ssb gene, soxS gene, soxR gene, ryjA gene, actP gene, acs gene, nrfA gene, nrfB gene, nrfC gene, nrfD gene, nrfE gene, nrfF gene, nrfG gene, gltP gene, fdhF gene, rpiR gene, rpiB gene, proP gene, basS gene, basR gene, eptA gene, adiC gene, adiY gene, adiA gene, melR gene, melA gene, melB gene, fumB gene, dcuB gene, dcuR gene, dcuS gene, lysU gene, cadA gene, cadB gene, cadC gene, pheU gene, dipZ gene, cutA gene, dcuA gene, aspA gene, fxsA gene, groS gene, groL gene, efp gene, ecnA gene, ecnB gene, sugE gene, blc gene, ampC gene, frdD gene, frdC gene, frdB gene, frdA gene, poxA gene, psd gene, rsgA gene, orn gene, glyV gene, glyX gene, glyY gene, amiB gene, mutL gene, miaA gene, hfq gene, hflX gene, hflK gene, hflC gene, purA gene, rnr gene, rlmB gene, ulaF gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrL gene, argI gene, valS gene, holC gene, pepA gene, idnR gene, idnT gene, idnO gene, idnD gene, idnK gene, leuX gene, dnaC gene, dnaT gene, bglJ gene, fhuF gene, leuV gene, leuP gene, leuQ gene, rsmC gene, holD gene, rimI gene, prfC gene, osmY gene, deoC gene, deoA gene, deoB gene, deoD gene, IplA gene, serB gene, radA gene, nadR gene, slt gene, trpR gene, rob gene, creA gene, creB gene, creC gene, creD gene, arcA gene or homologous gene thereof
   [2] fhuA gene, fhuC gene, fhuD gene, fhuB gene, yafJ gene, yafK gene, yafQ gene, dinJ gene, yafL gene, yafM gene, fhiA gene, mbhA gene, dinB gene, yafN gene, yafO gene, yafP gene, ykfJ gene, prfH gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM(JW5034) gene, ykgM(JW5035) gene, eaeH gene, ykgA gene, ykgB gene, ykgI gene, ykgC gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, yahI gene, yahJ gene, yahK gene, yahL gene, yahM gene, yahN gene, yahO gene, prpR gene, prpB gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, frmB gene, frmA gene, frmR gene, yaiO gene, yaiX gene, yaiF gene, yaiP gene, yaiS gene, tauA gene, tauB gene, tauC gene, tauD gene, yaiT gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, ylbG gene, ybbB gene, ybbS gene, allA gene, allR gene, glxR gene, ybbV gene, ybbW gene, allB gene, ybbY gene, glxK gene, ylbA gene, allC gene, allD gene, fdrA gene, ylbE_1 gene, ylbE_2 gene, ylbF gene, ybcF gene, envY gene, ybcH gene, nfrA gene, nfrB gene, cusS gene, cusR gene, cusC gene, cusF gene, cusB gene, cusA gene, pheP gene, ybdG gene, nfnB gene, ybdF gene, ybdJ gene, ybdK gene, hokE gene, ybeL gene, ybeQ gene, ybeR gene, djlB gene, ybeT gene, ybeU gene, djlC gene, hscC gene, rihA gene, gltL gene, gltK gene, gltJ gene, gltI gene, kdpF gene, ybfA gene, ybgA gene, phr gene, ybiA gene, dinG gene, ybiB gene, ybiC gene, ybiJ gene, ybiI gene, ybiX gene, fiu gene, ybiM gene, ybiN gene, ybiO gene, glnQ gene, glnP gene, glnH gene, yliA gene, yliB gene, yliC gene, yliD gene, yliE gene, yliF gene, yliG gene, yliH gene, yliI gene, yliJ gene, dacC gene, deoR gene, ybjG gene, ybjC gene, nfsA gene, rimK gene, ybjN gene, potF gene, potG gene, potH gene, potI gene, ybjO gene, rumB gene, artJ gene, artM gene, artQ gene, artI gene, artP gene, ybjP gene, ybjQ gene, ybjR gene, ybjS gene, ybjT gene, ItaE gene, ymcC gene, ymcD gene, cspH gene, cspG gene, ymcE gene, gnsA gene, yccM gene, torS gene, torT gene, torR gene, torC gene, torA gene, torD gene, cbpM gene, cbpA gene, yccE gene, agp gene, yccJ gene, wrbA gene, ymdF gene, ycdG gene, ycdH gene, ycdI gene, rarA gene, ycdK gene, ycdL gene, ycdM gene, ycdC gene, putA gene, putP gene, ycdN_1 gene, ycdN_2 gene, ycdO gene, ycdB gene, phoH gene, ycdP gene, ycdQ gene, ycdR gene, ycdS gene, ycdT gene, ymdE gene, ycdU gene, elbA gene, ycgX gene, ycgE gene, ycgF gene, ycgZ gene, ymgA gene, ymgB gene, ymgC gene, ycgG gene, ymgF gene, ycgH_1 gene, ycgH_2 gene, ymgD gene, ymgG gene, ymgH gene, ycgI gene, ydfH gene, ydfZ gene, ydfI gene, ydfJ gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, yecG gene, otsA gene, otsB gene, araH gene, araG gene, araF gene, yecI gene, yecJ gene, isrB gene, yecR gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN_2 gene, yedN_1 gene, yedM gene, intG gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, yodD gene, yedA gene, vsr gene, dcm gene, yedJ gene, yedR gene, yedS_1 gene, yedS_2 gene, yedS_3 gene, hchA gene, yedV gene, yedW gene, yedX gene, yedY gene, yedZ gene, yodA gene, wbbL_2 gene, wbbL_1 gene, wbbK gene, wbbJ gene, wbbI gene, wbbH gene, glf gene, rfbX gene, rfbC gene, rfbA gene, rfbD gene, rfbB gene, yegE gene, alkA gene, yegD gene, yegI gene, yegJ gene, yegK gene, yegL gene, ryeC gene, ryeD gene, mdtA gene, mdtB gene, mdtC gene, mdtD gene, baeS gene, baeR gene, yegP gene, yegQ gene, ryeE gene, ogrK gene, yegZ gene, yegR gene, yegS gene, gatR_1 gene, gatR_2 gene, gatD gene, gatC gene, gatB gene, gatA gene, gatZ gene, gatY gene, fbaB gene, yegT gene, yegU gene, yegV gene, yegW gene, yegX gene, molR_1 gene, molR_2 gene, molR_3 gene, yehI gene, yehK gene, yehL gene, yehM gene, yehP gene, yehQ gene, yehR gene, yehS gene, yehT gene, yehU gene, mlrA gene, yohO gene, yehW gene, yehX gene, yehY gene, yehZ gene, bglX,gene, dld gene, pbpG gene, yohC gene, yeiI gene, yeiJ gene, rihB gene, yeiL gene, yeiM gene, yeiN gene, yeiC gene, elaB gene, elaA gene, elaC gene, elaD gene, yfbK gene, yfbL gene, yfbM gene, yfbN gene, yfbO gene, yfbP gene, yfcC gene, yfcD gene, yfcE gene, yfcF gene, yfcG gene, folX gene, yfcH gene, yfcI gene, hisP gene, hisM gene, hisQ gene, hisJ gene, argT gene, ypdA gene, ypdB gene, ypdC gene, ypdD gene, ypdE gene, ypdF gene, ypdG gene, ypdH gene, pinH gene, ypjB gene, ypjC gene, ileY gene, ygaQ gene, ygaR gene, yqaC gene, yqaD gene, ygaT gene, csiR gene, ygaU gene, yqaE gene, ygaV gene, ygaP gene, stpA gene, ygaW gene, ygaC gene, ygaM gene, nrdH gene, nrdI gene, nrdE gene, proV gene, proW gene, proX gene, ygaX gene, ygaY gene, ygaZ gene, ygaH gene, srlA gene, srlE gene, srlB gene, srlD gene, gutM gene, srlR gene, gutQ gene, yqeG gene, yqeH gene, yqeI gene, yqeJ gene, yqeK gene, ygeF gene, ygeG gene, ygeH gene, ygeI gene, pbl gene, ygeK gene, ygeL gene, ygeM gene, ygeN gene, ygeO gene, ygeP gene, ygeQ gene, glyU gene, ygeR gene, xdhA gene, xdhB gene, xdhC gene, ygeV gene, ygeW gene, ygeX gene, ygeY gene, hyuA gene, yqeA gene, yqeB gene, yqeC gene, ygfJ gene, ygfK gene, ssnA gene, ygfM gene, xdhD gene, ygfO gene, guaD gene, ygfQ gene, ygfS gene, ygfT gene, ygfU gene, idi gene, cmtA gene, cmtB gene, yghD gene, yghE gene, yghF gene, yghG gene, pppA gene, yghJ gene, yghK gene, glcB gene, glcG gene, glcF gene, glcE gene, glcD gene, glcC gene, yghO gene, yghQ gene, yghR gene, yghS gene, yghT gene, ygiS gene, ygiT gene, ygiU gene, ygiV gene, ygiW gene, qseB gene, qseC gene, ygiZ gene, mdaB gene, ygiN gene, gltF gene, yhcA gene, yhcD gene, yhcE gene, yhcF gene, yhcG gene, yhcH gene, nanK gene, nanE gene, yhdJ gene, yhdU gene, envR gene, acrE gene, acrF gene, yhdV gene, yhdW gene, yhdX gene, yhdY gene, yijP gene, yijO gene, frwD gene, pflC gene, pflD gene, frwB gene, frwC gene, ptsA gene, rhaT gene, rhaR gene, rhaS gene, rhaB gene, rhaA gene, rhaD gene, yiiL gene, frvA gene, frvB gene, frvX gene, frvR gene, yiiG gene, yiiF gene, yiiE gene, yiiD gene, dtd gene, rbn gene, yihX gene, yihW gene, yihV gene, yihU gene, yihT gene, yihS gene, yihR gene, yihQ gene, yihP gene, yihO gene, ompL gene, yihN gene, yihM gene, yihL gene, hsrA gene, rbsR gene, rbsK gene, rbsB gene, rbsC gene, rbsA gene, rbsD gene, trkD gene, yieN gene, yieM gene, asnA gene, yidB gene, yidA gene, yidX gene, dgoR gene, dgoK gene, dgoA gene, dgoD gene, dgoT gene, cbrA gene, yidR gene, yidQ gene, yidE gene, yidP gene, glvC gene, glvB gene, glvG gene, ysdC gene, yidL gene, yidK gene, yidJ gene, yidI gene, yidH gene, yidG gene, yidF gene, yhiN gene, yhiM gene, yhiL gene, yhiK gene, yhiJ gene, yhiI gene, rbbA gene, yhhJ gene, chiA gene, bfd gene, bfr gene, gspO gene, gspM gene, gspL gene, gspK gene, gspJ gene, gspI gene, gspH gene, gspG gene, gspF gene, gspE gene, gspD gene, gspC gene, gspA gene, pioO gene, yjbE gene, yjbF gene, yjbG gene, yjbH gene, yjbA gene, xylE gene, malG gene, malF gene, malE gene, malK gene, lamB gene, maIM gene, yjbI gene, yjcP gene, yjcQ gene, yjcR gene, yjcS gene, alsK gene, alsE gene, alsC gene, alsA gene, alsB gene, yjdP gene, phnP gene, phnO gene, phnN gene, phnM gene, phnL gene, phnK gene, phnJ gene, phnI gene, phnH gene, phnG gene, phnF gene, phnE(JW4064) gene, phnE(JW4065) gene, phnD gene, phnC gene, phnB gene, phnA gene, yjdA gene, yjcZ gene, yjfI gene, yjfJ gene, yjfK gene, yjfL gene, yjfM gene, yjfC gene, aidB gene, yjfN gene, yjfO gene, yjfP gene, ulaR gene, ulaG gene, ulaA gene, ulaB gene, ulaC gene, ulaD gene, ulaE gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, kptA gene, yjiJ gene, yjiK gene, yjiL gene, yjiM gene, yjiN gene, yjiO gene, yjiP gene, yjiQ gene, yjiR gene, yjiS gene, yjiT gene, yjiV gene, mcrC gene, mcrB gene, yjiw gene, hsdS gene, hsdM gene, hsdR gene, mrr gene, yjiA gene, yjiX gene, yjiY gene, tsr gene, yjiZ gene, yjjM gene, yjjN gene, mdoB gene, yjjA gene or homologous gene thereof. When the above gene [1] or homologous gene thereof comprises genes that wild-type E. *coli* strains do not originally contain (hereafter referred to as "NC genes"), however, chromosomal DNA of the aforementioned E. coli mutant strain derived from the wild-type E. *coli* strain does not necessarily have the NC gene.

A preferable example of the *E. coli* mutant strain of the present invention is an *E. coli* mutant strain comprising chromosomal DNA that comprises DNA consisting of the nucleotide sequence of the genes of Table 1 and the above-mentioned [1] in the nucleotide sequences shown in SEQ ID NO: 1 or a DNA equivalent thereto, does not contain DNA consisting of the nucleotide sequence of chpA gene or DNA equivalent thereto, and does not contain DNA consisting of the nucleotide sequences of [3] chpA gene, uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, ybeM gene, yqcA gene, yqcB gene, yqcC gene, syd gene, yqcD gene, ygdH gene, sdaC gene, sdaB gene, exo gene, fucO gene, fucA gene, fucP gene, fucI gene, fucK gene, fucU gene, fucR gene, ygdE gene, ygdD gene, gcvA gene, ygdI gene, csdA gene, ygdK gene, araD gene, araA gene, araB gene, araC gene, yabI gene, thiQ gene, thiP gene, tbpA gene, sgrR gene, setA gene, yhfZ gene, yhfY gene, yhfX gene, yhfW gene, php gene, yhfU gene, yhfT gene, yhfS gene, frlR gene, frlD gene, frlc gene, frlB gene, frlA gene, yhfL gene, pncA gene, ydjE gene, ydjF gene, ydjG gene, ydjH gene, ydjI gene, ydjJ gene, ydjK gene, ydjL gene, yeaC gene, yeaA gene, ade gene, yicO gene, yicN gene, yicM gene, yicS gene, nlpA gene, yicL gene, setC gene, ygjI gene, ygjJ gene, ygjK gene, fadH gene, ygjM gene, ygjN gene, ygjO gene, ygjP gene, ygjQ gene, ygjR gene, alx gene, sstT gene, ygjV gene, yrhB gene, yrhA gene, yhhZ gene, yhhY gene, yhhX gene, yhhW gene, yeaK gene, yoaI gene, yeaL gene, yeaM gene, yeaN gene, yeaO gene, yoaF gene, yeaP gene, yeaQ gene, yoaG gene, yeaR gene, yeaS gene, yeaT gene, yeaU gene, yeaV gene, yeaW gene, yeaX gene, hyfA gene, hyfB gene, hyfC gene, hyfD gene, hyfE gene, hyfF gene, hyfG gene, hyfH gene, hyfI gene, hyfJ gene, hyfR gene, focB gene or homologous gene thereof, or DNA equivalent thereto. More preferred is
(2) the E. coli mutant strain according to above (1) comprising chromosomal DNA that comprises DNA consisting of the nucleotide sequences as shown in [1] to [83] below of the nucleotide sequence as shown in SEQ ID NO: 1 or DNA equivalent thereto:
   [1] a nucleotide sequence of nucleotides 1 to 15298
   [2] a nucleotide sequence of nucleotides 16751 to 19620
   [3] a nucleotide sequence of nucleotides 20815 to 65857
   [4] a nucleotide sequence of nucleotides 78797 to 167400
   [5] a nucleotide sequence of nucleotides 173316 to 244300
   [6] a nucleotide sequence of nucleotides 253747 to 262299
   [7] a nucleotide sequence of nucleotides 387868 to 389474
   [8] a nucleotide sequence of nucleotides 404040 to 518365
   [9] a nucleotide sequence of nucleotides 533048 to 535840
   [10] a nucleotide sequence of nucleotides 550552 to 564276
   [11] a nucleotide sequence of nucleotides 608455 to 640668
   [12] a nucleotide sequence of nucleotides 659193 to 675439
   [13] a nucleotide sequence of nucleotides 689711 to 729156
   [14] a nucleotide sequence of nucleotides 739930 to 832888
   [15] a nucleotide sequence of nucleotides 848427 to 867974
   [16] a nucleotide sequence of nucleotides 883811 to 891334
   [17] a nucleotide sequence of nucleotides 909717 to 1049224
   [18] a nucleotide sequence of nucleotides 1097358 to 1105534
   [19] a nucleotide sequence of nucleotides 1106991 to 1196528
   [20] a nucleotide sequence of nucleotides 1225485 to 1295721
   [21] a nucleotide sequence of nucleotides 1297023 to 1300822
   [22] a nucleotide sequence of nucleotides 1302896 to 1397636
   [23] a nucleotide sequence of nucleotides 1399079 to 1413662
   [24] a nucleotide sequence of nucleotides 1436899 to 1469664
   [25] a nucleotide sequence of nucleotides 1472404 to 1504839
   [26] a nucleotide sequence of nucleotides 1506619 to 1528866
   [27] a nucleotide sequence of nucleotides 1534766 to 1630050
   [28] a nucleotide sequence of nucleotides 1654610 to 1853604
   [29] a nucleotide sequence of nucleotides 1864144 to 1875287
   [30] a nucleotide sequence of nucleotides 1888500 to 1963662
   [31] a nucleotide sequence of nucleotides 1992215 to 1995800
   [32] a nucleotide sequence of nucleotides 2027349 to 2032587
   [33] a nucleotide sequence of nucleotides 2043516 to 2068287
   [34] a nucleotide sequence of nucleotides 2081066 to 2103531
   [35] a nucleotide sequence of nucleotides 2115204 to 2145402
   [36] a nucleotide sequence of nucleotides 2186995 to 2199807
   [37] a nucleotide sequence of nucleotides 2228990 to 2255033
   [38] a nucleotide sequence of nucleotides 2262631 to 2292234
   [39] a nucleotide sequence of nucleotides 2293834 to 2324498
   [40] a nucleotide sequence of nucleotides 2322075 to 2411311
   [41] a nucleotide sequence of nucleotides 2413007 to 2422503
   [42] a nucleotide sequence of nucleotides 2433238 to 2471990
   [43] a nucleotide sequence of nucleotides 2482140 to 2504213
   [44] a nucleotide sequence of nucleotides 2513697 to 2519690
   [45] a nucleotide sequence of nucleotides 2521089 to 2599856
   [46] a nucleotide sequence of nucleotides 2613439 to 2754814
   [47] a nucleotide sequence of nucleotides 2788619 to 2794311
   [48] a nucleotide sequence of nucleotides 2800029 to 2803445
   [49] a nucleotide sequence of nucleotides 2809286 to 2824451
   [50] a nucleotide sequence of nucleotides 2829399 to 2909409
   [51] a nucleotide sequence of nucleotides 2909710 to 2921193
   [52] a nucleotide sequence of nucleotides 2943639 to 2984497
   [53] a nucleotide sequence of nucleotides 3032267 to 3076223
   [54] a nucleotide sequence of nucleotides 3078056 to 3109245
   [55] a nucleotide sequence of nucleotides 3133480 to 3164769
   [56] a nucleotide sequence of nucleotides 3172082 to 3184621
   [57] a nucleotide sequence of nucleotides 3186056 to 3224889
   [58] a nucleotide sequence of nucleotides 3240401 to 3261074
   [59] a nucleotide sequence of nucleotides 3262220 to 3361080
   [60] a nucleotide sequence of nucleotides 3370892 to 3411501
   [61] a nucleotide sequence of nucleotides 3421222 to 3486426
   [62] a nucleotide sequence of nucleotides 3496906 to 3536155
   [63] a nucleotide sequence of nucleotides 3549687 to 3556477
   [64] a nucleotide sequence of nucleotides 3576239 to 3697304
   [65] a nucleotide sequence of nucleotides 3709527 to 3742812
   [66] a nucleotide sequence of nucleotides 3744293 to 3748980
   [67] a nucleotide sequence of nucleotides 3747997 to 3750486
   [68] a nucleotide sequence of nucleotides 3751906 to 3762908
   [69] a nucleotide sequence of nucleotides 3772817 to 3774086
   [70] a nucleotide sequence of nucleotides 3785257 to 3794687
   [71] a nucleotide sequence of nucleotides 3803459 to 3868134
   [72] a nucleotide sequence of nucleotides 3878460 to 3918087
   [73] a nucleotide sequence of nucleotides 3919815 to 3986454
   [74] a nucleotide sequence of nucleotides 3988342 to 4003004.
   [75] a nucleotide sequence of nucleotides 4021347 to 4055371
   [76] a nucleotide sequence of nucleotides 4061466 to 4128290
   [77] a nucleotide sequence of nucleotides 4141016 to 4170562
   [78] a nucleotide sequence of nucleotides 4186909 to 4239493
   [79] a nucleotide sequence of nucleotides 4255871 to 4303000
   [80] a nucleotide sequence of nucleotides 4316735 to 4318540
   [81] a nucleotide sequence of nucleotides 4334946 to 4414758
   [82] a nucleotide sequence of nucleotides 4428378 to 4501437
   [83] a nucleotide sequence of nucleotides 4604873 to 4646332.
(3) The E. *coli* mutant strain according to (1) or (2) above comprising chromosomal DNA that does not comprise uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, and ybeM gene, or homologous gene thereof and comprises chpA gene or homologous gene thereof.
(4) The E. *coli* mutant strain according to any one of (1) to (3) above comprising chromosomal DNA that does not comprise DNA consisting of a nucleotide sequence of nucleotides 640669 to 659192 in the nucleotide sequence as shown in SEQ ID NO: 1 or DNA equivalent thereto.
(5) The E. *coli* mutant strain according to any one of (1) to (4) above comprising chromosomal DNA that does not comprise yqcA gene, yqcB gene, yqcC gene, syd gene, yqcD gene, ygdH gene, sdaC gene, sdaB gene, exo gene, fucO gene, fucA gene, fucP gene, fucI gene, fucK gene, fucU gene, fucR gene, ygdE gene, ygdD gene, gcvA gene, ygdI gene, csdA gene, and ygdK gene, or homologous gene thereof.
(6) The E. *coli* mutant strain according to any one of (1) to (5) above comprising chromosomal DNA that does not comprise DNA consisting of a nucleotide sequence of nucleotides 2921194 to 2943638 in the nucleotide sequence as shown in SEQ ID NO: 1 or DNA equivalent thereto.
(7) The E. *coli* mutant strain according to any one of (1) to (6) above comprising chromosomal DNA that does not comprise araD gene, araA gene, araB gene, araC gene, yabI gene, thiQ gene, thiP gene, tbpA gene, sgrR gene, and setA gene, or homologous gene thereof.
(8) The E. coli mutant strain according to any one of (1) to (7) above comprising chromosomal DNA that does not comprise DNA consisting of a nucleotide sequences of nucleotides 65858 to 78796 in the nucleotide sequence as shown in SEQ ID NO: 1 or DNA equivalent thereto.
(9) The E. *coli* mutant strain according to any one of (1) to (8) above comprising chromosomal DNA that does not comprise yhfZ gene, yhfY gene, yhfX gene, yhfW gene, php gene, yhfU gene, yhfT gene, yhfS gene, frlR gene, frlD gene, frlc gene, frlB gene, frlA gene, and yhfL gene or homologous gene thereof.
(10) The E. *coli* mutant strain according to any one of (1) to (9) above comprising chromosomal DNA that does not comprise DNA consisting of a nucleotide sequence of nucleotides 4128291 to 4141015 in the nucleotide sequence as shown in SEQ ID NO: 1 or DNA equivalent thereto.
(11) The E. *coli* mutant strain according to any one of (1) to (10) above comprising chromosomal DNA that does not comprise pncA gene, ydjE gene, ydjF gene, ydjG gene, ydjH gene, ydjI gene, ydjJ gene, ydjK gene, ydjL gene, yeaC gene, and yeaA gene, or homologous gene thereof.
(12) The E. *coli* mutant strain according to any one of (1) to (11) above comprising chromosomal DNA that does not comprise DNA consisting of a nucleotide sequence of nucleotides 1853605 to 1864143 in the nucleotide sequence as shown in SEQ ID NO: 1 or DNA equivalent thereto.
(13) The E. *coli* mutant strain according to any one of (1) to (12) above comprising chromosomal DNA that does not comprise ade gene, yicO gene, yicN gene, yicM gene, yicS gene, nlpA gene, yicL gene, and setC gene, or homologous gene thereof.
(14) The E. *coli* mutant strain according to any one of (1) to (13) above comprising chromosomal DNA that does not comprise DNA consisting of a nucleotide sequence of nucleotides 3794688 to 3803458 in the nucleotide sequence as shown in SEQ ID NO: 1 or DNA equivalent thereto.
(15) The E. *coli* mutant strain according to any one of (1) to (14) above comprising chromosomal DNA that does not comprise ygjI gene, ygjJ gene, ygjK gene, fadH gene, ygjM gene, ygjN gene, ygjO gene, ygjP gene, ygjQ gene, ygjR gene, alx gene, sstT gene, and ygjV gene, or homologous gene thereof.
(16) The E. *coli* mutant strain according to any one of (1) to (15) above comprising chromosomal DNA that does not comprise DNA consisting of a nucleotide sequence of nucleotides 3224890 to 3240400 in the nucleotide sequence as shown in SEQ ID NO: 1 or DNA equivalent thereto.
(17) The *E. coli* mutant strain according to any one of (1) to (16) above comprising chromosomal DNA that does not comprise yrhB gene, yrhA gene, yhhZ gene, yhhY gene, yhhX gene, and yhhW gene, or homologous gene thereof.
(18) The E. *coli* mutant strain according to any one of (1) to (17) above comprising chromosomal DNA that does not comprise DNA consisting of a nucleotide sequence of nucleotides 4055372 to 4061465 in the nucleotide sequence as shown in SEQ ID NO: 1 or DNA equivalent thereto.
(19) The E. *coli* mutant strain according to any one of (1) to (18) above comprising chromosomal DNA that does not comprise yeaK gene, yoaI gene, yeaL gene, yeaM gene, yeaN gene, yeaO gene, yoaF gene, yeaP gene, yeaQ gene, yoaG gene, yeaR gene, yeaS gene, yeaT gene, yeaU gene, yeaV gene, yeaW gene, and yeaX gene, or homologous gene thereof.
(20) The *E. coli* mutant strain according to any one of (1) to (19) above comprising chromosomal DNA that does not comprise DNA consisting of a nucleotide sequence of nucleotides 1875288 to 1888499 in the nucleotide sequence as shown in SEQ ID NO: 1 or DNA equivalent thereto.
(21) The E. coli mutant strain according to any one of (1) to (20) above comprising chromosomal DNA that does not comprise hyfA gene, hyfB gene, hyfC gene, hyfD gene, hyfE gene, hyfF gene, hyfG gene, hyfH gene, hyfI gene, hyfJ gene, hyfR gene, and focB gene, or homologous gene thereof.
(22) The E. *coli* mutant strain according to any one of (1) to (21) above comprising chromosomal DNA that does not comprise DNA consisting of a nucleotide sequence of nucleotides 2599857 to 2613438 in the nucleotide sequence as shown in SEQ ID NO: 1 or DNA equivalent thereto.

**[Table 1-1]**

| Gene name | Coding strand | Start number in SEQ ID NO:1 (stop number Coding when encoded by complementary strand) | Stop number in SEQ ID NO:1 (start number when encoded by complementary strand) |
|---|---|---|---|
| thrL | | 190 | 255 |
| thrA | | 337 | 2799 |
| thrB | | 2801 | 3733 |
| thrC | | 3734 | 5020 |
| yaaX | | 5234 | 5530 |
| yaaA | Complementary strand | 5683 | 6459 |
| yaaJ | Complementary strand | 6529 | 7959 |
| talB | | 8238 | 9191 |
| mog | | 9306 | 9893 |
| yaaH | Complementary strand | 9928 | 10494 |
| yaaW | Complementary strand | 10643 | 11356 |
| htgA | | 10830 | 11315 |
| yaaI | Complementary strand | 11382 | 11786 |
| dnaK | | 12163 | 14079 |
| dnaJ | | 14168 | 15298 |
| insL-1 | | 15445 | 16557 |
| moRC | Complementary strand | 16751 | 16960 |
| hokC | Complementary strand | 16751. | 16903 |
| sokC | | 16952 | 17006 |
| nhaA | | 17489 | 18665 |
| nhaR | | 18715 | 19620 |
| insB-1 | Complementary strand | 19811 | 20314 |
| insA-1 | Complementary strand | 20233 | 20508 |
| rpsT | Complementary strand | 20815 | 21078 |
| yaaY | | 21181 | 21399 |
| ribF | | 21407 | 22348 |
| ileS | | 22391 | 25207 |
| ispA | | 25207 | 25701 |
| fkpB | | 25826 | 26275 |
| ispH | | 26277 | 27227 |
| rihC | | 27293 | 28207 |
| dapB | | 28374 | 29195 |
| carA | | 29651 | 30799 |
| carB | | 30817 | 34038 |
| caiF | | 34300 | 34695 |
| caiE | Complementary strand | 34781 | 35371 |
| caiD | Complementary strand | 35377 | 36270 |
| caiC | Complementary strand | 36271 | 37839 |
| caiB | Complementary strand | 37898 | 39115 |

**[Table 1-2]**

| Gene name | Coding strand | Start number in SEQ ID NO:1 (stop number when encoded by complementary strand) | Stop number in SEQ ID NO:1 (start number when encoded by complementary strand |
|---|---|---|---|
| caiA | Complementary strand | 39244 | 40386 |
| caiT | Complementary strand | 40417 | 41931 |
| fixA | | 42403 | 43173 |
| fixB | | 43188 | 44129 |
| fixC | | 44180 | 45466 |
| fixX | | 45463 | 45750 |
| yaaU | | 45807 | 47138 |
| kefF | | 47246 | 47776 |
| kefC | | 47769 | 49631 |
| folA | | 49823 | 50302 |
| apaH | Complementary strand | 50380 | 51222 |
| apaG | Complementary strand | 51229 | 51606 |
| ksgA | Complementary strand | 51609 | 52430 |
| pdxA | Complementary strand | 52427 | 53416 |
| surA | Complementary strand | 53416 | 54702 |
| imp | Complementary strand | 54755 | 57109 |
| djlA | | 57364 | 58179 |
| yabP | | 58474 | 59124 |
| yabQ | | 59121 | 59279 |
| rluA | Complementary strand | 59687 | 60346 |
| hepA | Complementary strand | 60358 | 63264 |
| polB | Complementary strand | 63429 | 65780 |
| araD | Complementary strand | 65855 | 66550 |
| araA | Complementary strand | 66835 | 68337 |
| araB | Complementary strand | 68348 | 70048 |
| araC | | 70387 | 71265 |
| yabl | | 71351 | 72115 |
| thiQ | Complementary strand | 72229 | 72927 |
| thiP | Complementary strand | 72911 | 74521 |
| tbpA | Complementary strand | 74497 | 75480 |
| sgrR | Complementary strand | 75644 | 77299 |
| sgrS | | 77367 | 77593 |
| setA | | 77621 | 78799 |
| leuD | Complementary strand | 78848 | 79453 |
| leuC | Complementary strand | 79464 | 80864 |
| leuB | Complementary strand | 80887 | 81958 |
| leuA | Complementary strand | 81958 | 83529 |
| leuL | Complementary strand | | 83622 83708 |
| leuO | | 84368 | 85312 |
| ilvl | | 85630 | 87354 |

**[Table 1-3]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ilvH | | 87357 | 87848 |
| fruR | | 88028 | 89032 |
| mraZ | | 89634 | 90092 |
| mraW | | 90094 | 91035 |
| ftsL | | 91032 | 91397 |
| ftsl | | 91413 | 93179 |
| murE | | 93166 | 94653 |
| murF | | 94650 | 96008 |
| mraY | | 96002 | 97084 |
| murD | | 97087 | 98403 |
| ftsW | | 98403 | 99647 |
| murG | | 99644 | 100711 |
| murC | | 100765 | 102240 |
| ddlB | | 102233 | 103153 |
| ftsQ | | 103155 | 103985 |
| ftsA | | 103982 | 105244 |
| ftsZ | | 105305 | 106456 |
| lpxC | | 106557 | 107474 |
| secM | | 107705 | |
| secA | | 108279 | 110984 |
| mulT | | 111044 | 111433 |
| yacG | Complementary strand | 111649 | 111646 |
| yacF | Complementary strand | 111856 | 112599 |
| coaE | Complementary strand | 112599 | 113219 |
| guaC | | 113444 | 114487 |
| hofC | Complementary strand | 114522 | 115724 |
| hofB | Complementary strand | 115714 | 117099 |
| ppdD | Complementary strand | 117109 | 117549 |
| nadC | Complementary strand | 117752 | 118645 |
| ampD | | 118733 | 119284 |
| ampE | | 119281 | 120135 |
| aroP | Complementary strand | 120178 | 121551 |
| pdhR | | 122092 | 122856 |
| aceE | | 123017 | 125680 |
| aceF | | 125695 | 127587 |
| lpd | | 127912 | 129336 |
| yacH | Complementary strand | 129407 | 131260 |
| acnB | | 131615 | 134212 |
| yacL | | 134388 | 134750 |
| speD | Complementary strand | 134788 | 135582 |

**[Table 1-4]**

| Gene name | Coding strand | Start number in SEQ ID NO:1 (stop number when encoded by complementary strand) | Stop number in SEQ ID NO:1 (start number when encoded by complementary strand) |
|---|---|---|---|
| speE | Complementary strand | 135598 | 136464 |
| yacC | Complementary strand | 136570 | 136917 |
| cueO | | 137083 | 138633 |
| gcd | Complementary strand | 138835 | 141225 |
| hpt | | 141431 | 141967 |
| can | Complementary strand | 142008 | 142670 |
| yadG | | 142779 | 143705 |
| yadH | | 143702 | 144472 |
| yadl | | 144577 | 145017 |
| yadE | | 145081 | 146310 |
| panD | Complementary strand | 146314 | 146694 |
| yadD | | 146968 | 147870 |
| panC | Complementary strand | 147944 | 148796 |
| panB | Complementary strand | 148807 | 149601 |
| yadC | Complementary strand | 149715 | 150953 |
| yadK | Complementary strand | 151003 | 151599 |
| yadL | Complementary strand | 151626 | 152231 |
| yadM | Complementary strand | 152243 | 152812 |
| htrE | Complementary strand | 152829 | 155426 |
| ecpD | Complementary strand | 155461 | 156201 |
| yadN | Complementary strand | 156299 | 156883 |
| folK | Complementary strand | 157253 | 157732 |
| pcnB | Complementary strand | 157729 | 159126 |
| yadB | Complementary strand | 159186 | 160112 |
| dksA | Complementary strand | 160149 | 160604 |
| sfsA | Complementary strand | 160782 | 161486 |
| lgT | Complementary strand | 161501 | 162031 |
| hrpB | | 162105 | 164534 |
| mrcB | | 164730 | 167264 |
| fhuA | | 167484 | 169727 |
| fhuC | | 169778 | 170575 |
| fhuD | | 170575 | 171465 |
| fhuB | | 171462 | 173444 |
| hemL | Complementary strand | 173602 | 174882 |
| clcA | | 175107 | 176528 |
| yadR | | 176610 | 176954 |
| yadS | Complementary strand | 177001 | 177624 |
| btuF | Complementary strand | 177662 | 178462 |
| pfs | Complementary strand | 178455 | 179153 |
| dgt | | 179237 | 180754 |

**[Table 1-5]**

| Gene name | Coding strand | Start number in SEQ ID NO:1 (stop number when encoded by complementary strand) | Stop number in SEQ ID NO:1 (start number when encoded by complementary strand) |
|---|---|---|---|
| degP | | 180884 | 182308 |
| cdaR | | 182463 | 183620 |
| yaeH | Complementary strand | 183709 | 184095 |
| yael | Complementary strand | 184257 | 185069 |
| dapD | Complementary strand | 185123 | 189947 |
| glnD | Complementary strand | 185978 | 188650 |
| map | | 188712 | 189506 |
| tff | | 189712 | 189847 |
| rpsB | | 189874 | 190599 |
| tsf | | 190857 | 191708 |
| pyrH | | 191855 | 192580 |
| frr | | 192872 | 193429 |
| dxr | | 193521 | 194717 |
| ispU | | 194903 | 195664 |
| cdsA | | 195677 | 196534 |
| yaeL | | 196546 | 187898 |
| yaeT | | 197928 | 200360 |
| hlpA | | 200482 | 200967 |
| lpxD | | 200971 | 201996 |
| fabZ | | 202101 | 202556 |
| lpxA | | 202560 | 203348 |
| lpxB | | 203348 | 204496 |
| rnhB | | 204493 | 205089 |
| dnaE | | 205126 | 208608 |
| accA | | 208621 | 209580 |
| ldcC | | 209679 | 211820 |
| yaeR | | 211877 | 212266 |
| tilS | | 212331 | 213629 |
| rcf | Complementary strand | 213678 | 213932 |
| yaeP | Complementary strand | 213925 | 214125 |
| yaeQ | | 214291 | 214836 |
| yaeJ | | 214833 | 215255 |
| nlpE | | 215269 | 215979 |
| yaeF | Complementary strand | 216179 | 217003 |
| proS | Complementary strand | 217057 | 218775 |
| yaeB | Complementary strand | 218887 | 219594 |
| rcsF | Complementary strand | 219591 | 219995 |
| metQ | Complementary strand | 220113 | 220928 |
| metI | Complementary strand | 220968 | 221621 |
| metN | Complementary strand | 221614 | 222645 |

**[Table 1-6]**

| Gene name | Coding strand | Start number in SEQ ID NO:1 (stop number when encoded by complementary strand) | Stop number in SEQ ID NO:1 (start number when encoded by complementary strand) |
|---|---|---|---|
| gmhB | | 222833 | 223408 |
| rrsH | | 223771 | 225312 |
| lleV | | 225381 | 225457 |
| alaV | | 225500 | 225575 |
| rrlH | | 225759 | 228662 |
| rrfH | | 228756 | 228875 |
| aspU | | 228928 | 229004 |
| dkgB | | 229167 | 229970 |
| yafC | Complementary strand | 229967 | 230881 |
| yafD | | 231122 | 231922 |
| yafE | | 231926 | 232549 |
| mltD | Complementary strand | 232597 | 233955 |
| gloB | Complementary strand | 234027 | 234782 |
| yafS | | 234816 | 235538 |
| rnhA | Complementary strand | 235535 | 236002 |
| dnaQ | | 236067 | 236798 |
| aspV | | 236931 | 237007 |
| yafT | | 237335 | 238120 |
| yafU | Complementary strand | 238746 | 239084 |
| yafF | | 239190 | 239378 |
| yafV | Complementary strand | 239419 | 240189 |
| ivy | | 240343 | 240816 |
| fadE | Complementary strand | 240859 | 243303 |
| lpcA | | 243543 | 244121 |
| yafJ | | 244327 | 245094 |
| yafK | Complementary strand | 245065 | 245805 |
| yafQ | Complementary strand | 245961 | 246239 |
| dinJ | Complementary strand | 246242 | 246502 |
| yafL | | 246712 | 247461 |
| yafM | | 247637 | 248134 |
| fhlA | Complementary strand | 248358 | 250070 |
| mbhA | | 250072 | 250827 |
| dlnB | | 250898 | 251953 |
| yafN | | 252005 | 252298 |
| yafO | | 252301 | 252699 |
| yafP | | 252709 | 253161 |
| ykfJ | | 253467 | 253733 |
| prfH | | 253702 | 254202 |
| pepD | Complementary strand | 254259 | 255716 |
| gpt | | 255977 | 256435 |

**[Table 1-7]**

| Gene name | Coding strand | Start number in SEQ ID NO:1 (stop number when encoded by complementary strand) | Stop number in SEQ ID NO:1 (start number when encoded by complementary strand) |
|---|---|---|---|
| frsA | | 256527 | 257771 |
| crl | | 257829 | 258230 |
| phoE | Complementary strand | 258269 | 259324 |
| proB | | 258612 | 260715 |
| proA | | 260727 | 261980 |
| thrW | | 262095 | 262170 |
| ykfl | Complementary strand | 262552 | 262893 |
| yafW | Complementary strand | 262914 | 263231 |
| ykfH | Complementary strand | 263250 | 263471 |
| ykfG | Complementary strand | 263480 | 263956 |
| yafX | Complementary strand | 253972 | 264430 |
| ykfF | Complementary strand | 264528 | 264737 |
| ykfB | Complementary strand | 264844 | 265311 |
| yafY | Complementary strand | 265334 | 266191 |
| yafZ | Complementary strand | 266408 | 287229 |
| ykfA | Complementary strand | 267321 | 268184 |
| perR | Complementary strand | 268513 | 269406 |
| insN-1 | | 269466 | 269870 |
| insl-1 | | 269827 | 270978 |
| insO-1 | | 271054 | 271479 |
| ykfC | | 272071 | 273216 |
| insH-1 | Complementary strand | 273325 | 274341, |
| mmuP | | 274549 | 276952 |
| mmuM | | 275939 | 276871 |
| afuC | Complementary strand | 276980 | 278026 |
| afuB | Complementary strand | 278038 | 278400 |
| insB-2 | Complementary strand | 278402 | 278905 |
| insA-2 | Complementary strand | 278824 | 279099 |
| ykgN | Complementary strand | 279248 | 279586 |
| yagB | Complementary strand | 279609 | 279959 |
| yagA | Complementary strand | 280053 | 281207 |
| yagE | | 281481 | 282410 |
| yagF | | 282425 | 284392 |
| yagG | | 284619 | 286001 |
| yagH | | 286013 | 287623 |
| yagl | Complementary strand | 287628 | 288386 |
| argF | Complementary strand | 268525 | 289529 |
| insB-3 | Complementary strand | 289873 | 290376 |
| insA-3 | Complementary strand | 290295 | 290570 |
| yagJ | | 290724 | 291455 |

**[Table 1-8]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yagK | Complementary strand | 291546 | 292172 |
| yagL | Complementary strand | 292444 | 293142 |
| yagM | Complementary strand | 293169 | 294023 |
| yagN | Complementary strand | 294363 | 294803 |
| intF | Complementary strand | 294920 | 296320 |
| yagP | Complementary strand | 296605 | 297015 |
| yagQ | Complementary strand | 296994 | 297950 |
| yagR | Complementary strand | 297960 | 300158 |
| yagS | Complementary strand | 300155 | 301111 |
| yagT | Complementary strand | 301108 | 301797 |
| yagU | | 302215 | 302829 |
| ykgJ | Complementary strand | 303077 | 303406 |
| yagV | Complementary strand | 303719 | 304429 |
| yagW | Complementary strand | 304398 | 306041 |
| yagX | Complementary strand | 306031 | 308556 |
| yagY | Complementary strand | 308582 | 309250 |
| yagZ | Complementary strand | 309308 | 309895 |
| ykgK | Complementary strand | 309970 | 310560 |
| ykgL | | 311336 | 311563 |
| ykgM(JW5034) | Complementary strand | 311598 | 311738 |
| ykgM(JW5035) | Complementary strand | 311738 | 312001 |
| eaeH | | 313581 | 314468 |
| lnsE-1 | | 314506 | 314814 |
| insF-1 | | 314811 | 315977 |
| yhgA | Complementary strand | 315674 | 316360 |
| ykgB | Complementary strand | 316950 | 317543 |
| ykgl | Complementary strand | 317565 | 317791 |
| ykgC | Complementary strand | 317900 | 319225 |
| ykgD | | 319451 | 320305 |
| ykgE | | 320832 | 321551 |
| ykgF | | 321562 | 322989 |
| ykgG | | 322982 | 323677 |
| ykgH | Complementary strand | 323920 | 324588 |
| betA | Complementary strand | 324801 | 326471 |
| betB | Complementary strand | 326485 | 327957 |
| betl | Complementary strand | 327971 | 328558 |
| betT | | 328687 | 330720 |
| yahA | | 331595 | 332683 |
| yahB | Complementary strand | 332725 | 333657 |
| yahC | Complementary strand | 333749 | 334246 |

**[Table 1-9]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yahD | | 334504 | 335109 |
| yahE | | 335149 | 336012 |
| yahF | | 336002 | 337549 |
| yahG | | 337549 | 338967 |
| yahH | | 339017 | 339313 |
| yahl | | 339389 | 340339 |
| yahJ | | 340349 | 341731 |
| yahK | | 342108 | 343157 |
| yahL | | 343400 | 344215 |
| yahM | | 344628 | 344873 |
| yahN | Complementary strand | 344890 | 345561 |
| yahO | | 345708 | 345983 |
| prpR | Complementary strand | 346081 | 347667 |
| prpB | | 347906 | 348796 |
| prpC | | 349236 | 350405 |
| prpD | | 350439 | 351890 |
| prpE | | 351930 | 353816 |
| codB | | 354146 | 355405 |
| codA | | 355395 | 356678 |
| cynR | Complementary strand | 357015 | 357914 |
| cynT | | 358023 | 358682 |
| cynS | | 358713 | 359183 |
| cynX | | 359216 | 360370 |
| lacA | Complementary strand | 360473 | 361084 |
| lacY | Complementary strand | 361150 | 362403 |
| lacZ | Complementary strand | 362455 | 365529 |
| lacl | Complementary strand | 365652 | 366734 |
| mhpR | Complementary strand | 366811 | 367758 |
| mhpA | | 367835 | 369499 |
| mhpB | | 369501 | 370445 |
| mhpC | | 370448 | 371329 |
| mhpD | | 371339 | 372148 |
| mhpF | | 372145 | 373095 |
| mhpE | | 373092 | 374105 |
| mhpT | | 374683 | 375894 |
| yalL | | 375996 | 376535 |
| frmB | Complementary strand | 376759 | 377592 |
| frmA | Complementary strand | 377686 | 378795 |
| frmR | Complementary strand | 378830 | 379105 |
| yalO | Complementary strand | 379293 | 380066 |

**[Table 1-10]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yaiX | Complementary strand | 380068 | 380283 |
| InsC-1 | | 380530 | 380940 |
| insD-1 | | 380898 | 381803 |
| yaiF | Complementary strand | 381728 | 382114 |
| yaiP | Complementary strand | 381963 | 383159 |
| yaiS | complementary Strand | 383283 | 383840 |
| tauA | | 384456 | 385418 |
| tauB | | 385431 | 386198 |
| tauC | | 386195 | 387022 |
| tauD | | 387019 | 387870 |
| hemB | Complementary strand | 387977 | 388951 |
| ykiB | Complementary strand | 389121 | 389339 |
| yaiT | | 389475 | 390935 |
| insF-2 | Complementary strand | 390963 | 391829 |
| InsE-2 | complementary strand | 391826 | 392134 |
| yaiU | | 392239 | 393642 |
| yaiV | | 393685 | 394353 |
| ampH | Complementary strand | 394354 | 395511 |
| sbmA | | 395863 | 397083 |
| yaiW | | 397096 | 338190 |
| yaiY | complementary strand | 398249 | 398557 |
| yaiZ | | 398817 | 399029 |
| ddlA | complementary strand | 399053 | 400147 |
| yalB | | 400610 | 400870 |
| phoA | | 400971 | 402386 |
| psiF | | 402505 | 402825 |
| yaiC | | 402927 | 404042 |
| proC | complementary strand | 404059 | 404868 |
| yail | | 404988 | 405446 |
| aroL | | 405629 | 406153 |
| yaiA | | 406203 | 406394 |
| aroM | | 406652 | 407329 |
| yalE | | 407401 | 407685 |
| ykiA | | 407893 | 408174 |
| rdgC | complementary strand | 408332 | 409243 |
| mak | | 409368 | 410276 |
| araJ | complementary strand | 410521 | 411705 |
| sbcC | complementary strand | 411831 | 414977 |
| sbcD | complementary strand | 414974 | 416176 |
| phoB | | 416366 | 417055 |

**[Table 1-11]**

| Gene name | Coding strand | Start number in SEQ ID NO:1 (stop number when encoded by complementary strand) | Stop number in SEQ ID NO:1 (start number when encoded by complementary strand) |
|---|---|---|---|
| phoR | | 417113 | 418408 |
| bmQ | | 418815 | 420134 |
| proY | | 420210 | 421583 |
| malZ | | 421739 | 423556 |
| yajB | Complementary strand | 423561 | 424142 |
| queA | | 424235 | 425305 |
| tgt | | 425361 | 426488 |
| yajC | | 426511 | 426843 |
| secD | | 426671 | 428718 |
| secF | | 428729 | 429700 |
| yajD | | 429829 | 430176 |
| tsx | Complementary strand | 430353 | 431237 |
| yajl | Complementary strand | 431536 | 432075 |
| ybaD | | 432226 | 432675 |
| ribD | | 432679 | 433782 |
| ribE | | 4333871 | 434341 |
| nusB | | 434361 | 434780 |
| thiL | | 434858 | 435835 |
| pgpA | | 435813 | 436331 |
| yajO | Complementary strand | 436385 | 437359 |
| dxs | Complementary strand | 437539 | 439401 |
| ispA | Complementary strand | 439426 | 440325 |
| xseB | Complementary strand | 440325 | 440567 |
| thiI | | 440773 | 442221 |
| yajL | Complementary strand | 442275 | 442865 |
| panE | Complementary strand | 442828 | 443739 |
| yajQ | | 443907 | 444398 |
| yajR | Complementary strand | 444526 | 445890 |
| cyoE | Complementary strand | 446039 | 446929 |
| cyoD | Complementary strand | 446941 | 447270 |
| cyoC | Complementary strand | 447270 | 447884 |
| cyoB | Complementary strand | 447874 | 449865 |
| cyoA | Complementary strand | 449887 | 450834 |
| ampG | Complementary strand | 451294 | 452769 |
| yajG | Complementary strand | 452813 | 453391 |
| bolA | | 453696 | 454013 |
| tig | | 454357 | 455655 |
| clpP | | 455901 | 456524 |
| clpX | | 456650 | 457924 |
| lon | | 458112 | 460466 |

**[Table 1-12]**

| Gene name | Coding strand | Start number in SEQ ID NO:1 (stop number when encoded by complementary strand) | Stop number in SEQ ID NO:1 (start number when encoded by complementary strand) |
|---|---|---|---|
| hupB | | 460675 | 460947 |
| ppiD | | 461139 | 463010 |
| ybaV | | 463161 | 463532 |
| ybaW | | 463626 | 464024 |
| ybaX | Complementary strand | 464076 | 464771 |
| ybaE | Complementary strand | 464836 | 466536 |
| cof | | 466636 | 467454 |
| ybaO | | 467607 | 468065 |
| mdlA | | 468095 | 469867 |
| mdlB | | 469860 | 471641 |
| glnK | | 471822 | 472160 |
| amtB | | 472190 | 473476 |
| tesB | Complementary strand | 473525 | 474385 |
| ybaY | | 474603 | 475175 |
| ybaZ | Complementary strand | 475206 | 475595 |
| ffs | | 475672 | 475785 |
| ybaA | | 475896 | 476249 |
| ylaB | Complementary strand | 476291 | 477841 |
| ylaC | Complementary strand | 478005 | 478475 |
| maa | Complementary strand | 478591 | 479142 |
| hha | Complementary strand | 479314 | 479532 |
| ybaJ | Complementary strand | 479558 | 479932 |
| acrB | Complementary strand | 480478 | 483627 |
| acrA | Complementary strand | 483650 | 484843 |
| acrR | | 484985 | 485632 |
| kefA | | 485760 | 489122 |
| ybaM | Complementary strand | 489334 | 489495 |
| priC | Complementary strand | 489509 | 490036 |
| ybaN | | 490106 | 490483 |
| apt | | 490636 | 491187 |
| dnaX | | 491316 | 493247 |
| ybaB | | 493300 | 493629 |
| recR | | 493629 | 494234 |
| htpG | | 494344 | 496218 |
| adk | | 496399 | 497043 |
| hemH | | 497279 | 498241 |
| aes | Complementary strand | 498238 | 499197 |
| gsk | | 499349 | 500653 |
| ybaL | Complementary strand | 500786 | 502462 |
| fsr | Complementary strand | 502700 | 503920 |

**[Table 1-13]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ushA | | 504138 | 505790 |
| ybaK | Complementary strand | 505827 | 506306 |
| ybaP | Complementary strand | 506510 | 507304 |
| ybaQ | | 507442 | 507783 |
| copA | Complementary strand | 508099 | 510603 |
| ybaS | | 510865 | 511797 |
| ybaT | | 511800 | 513092 |
| cueR | | 513217 | 513624 |
| ybbJ | Complementary strand | 513625 | 514080 |
| ybbK | Complementary strand | 514080 | 514997 |
| ybbL | | 515143 | 515820 |
| ybbM | | 515807 | 516586 |
| ybbN | Complementary strand | 516649 | 517503 |
| ybbO | Complementary strand | 517564 | 518373 |
| tesA | Complementary strand | 518363 | 518989 |
| ybbA | | 518957 | 519643 |
| ybbP | | 519640 | 622054 |
| rhsD | | 522485 | 526765 |
| ybbC | | 526805 | 527173 |
| ylbH | | 527173 | 527883 |
| ybbD | | 527864 | 528124 |
| ylbG | Complementary strand | 528869 | 529240 |
| ybbB | Complementary strand | 529356 | 530450 |
| ybbS | Complementary strand | 530519 | 531445 |
| allA | | 531675 | 532157 |
| allR | | 532235 | 533050 |
| gcl | | 533140 | 534921 |
| hyl | | 534934 | 535710 |
| glxR | | 535810 | 536688 |
| ybbV | | 536720 | 536998 |
| ybbW | | 536857 | 538311 |
| allB | | 538371 | 539732 |
| ybbY | | 539789 | 541090 |
| glxK | | 541112 | 542257 |
| ylbA | Complementary strand | 542485 | 543270 |
| allC | Complementary strand | 543281 | 544516 |
| allD | Complementary strand | 544538 | 545587 |
| fdrA | | 545904 | 547571 |
| ylbE_1 | | 547581 | 547841 |
| ylbE_2 | | 547838 | 548839 |

**[Table 1-14]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ylbF | | 548850 | 549665 |
| ybcF | | 549662 | 550555 |
| purK | Complementary strand | 550750 | 551817 |
| purE | Complementary strand | 551814 | 552323 |
| IpxH | Complementary strand | 552441 | 553163 |
| ppiB | Complementary strand | 553166 | 553660 |
| cysS | | 553834 | 555219 |
| ybcl | Complementary strand | 555255 | 555776 |
| ybcJ | Complementary strand | 555884 | 556096 |
| folD | Complementary strand | 556098 | 556964 |
| sfmA | | 557435 | 557977 |
| sfmC | | 558197 | 558889 |
| sfmD | | 558920 | 561523 |
| sfmH | | 561565 | 562542 |
| sfmF | | 562553 | 563068 |
| fimZ | Complementary strand | 563071 | 563703 |
| argU | | 563946 | 564022 |
| intD | Complementary strand | 564038 | 565201 |
| ybcC | Complementary strand | 565321 | 565584 |
| ybcD | | 565674 | 565910 |
| insE-3 | | 566056 | 566364 |
| InsF-3 | | 556361 | 567227 |
| renD | | 567285 | 567470 |
| emrE | | 567538 | 567870 |
| ybcK | | 568125 | 569651 |
| ybcL | | 570116 | 570667 |
| ybcM | | 570677 | 571474 |
| ybcN | | 571689 | 572144 |
| ninE | | 572144 | 572314 |
| ybcO | | 572307 | 572597 |
| rusA | | 572594 | 572956 |
| ylcG | | 572953 | 573093 |
| ybcQ | | 573179 | 573562 |
| insH-2 | Complementary strand | 573960 | 574976 |
| nmpC | Complementary strand | 574981 | 576108 |
| essD | | 576621 | 576836 |
| ybcS | | 576836 | 577333 |
| rzpD | | 577330 | 577791 |
| rzoD | | 577550 | 577732 |
| borD | Complementary strand | 577823 | 578116 |

**[Table 1-15]**

| Gene name | Coding strand | start number in SEQ ID NO:1 (stop number when encoded by complementary strand | Stop number in SEQ ID NO:1 (start number when encoded by complementary strand) |
|---|---|---|---|
| ybcV | Complementary strand | 578407 | 578817 |
| ybcW | | 579103 | 579309 |
| hchB | | 580057 | 580602 |
| tfaD | | 580757 | 581320 |
| ybcY | Complementary strand | 581375 | 581959 |
| ylcE | | 582098 | 582283 |
| appY | | 582904 | 583653 |
| ompT | Complementary strand | 583903 | 584856 |
| envY | Complementary strand | 585370 | 586131 |
| ybcH | Complementary strand | 586314 | 587204 |
| nfrA | Complementary strand | 587205 | 590177 |
| nfrB | Complementary strand | 590164 | 592401 |
| cusS | Complementary strand | 592551 | 593993 |
| cusR | Complementary strand | 593983 | 594666 |
| cusC | | 594823 | 596196 |
| cusF | | 596354 | 596686 |
| cusB | | 596702 | 597925 |
| cusA | | 597937 | 601080 |
| pheP | | 601182 | 602558 |
| ybdG | Complementary strand | 602639 | 603886 |
| nfnB | Complementary strand | 603994 | 604647 |
| ybdF | Complementary strand | 604741 | 605109 |
| ybdJ | Complementary strand | 605174 | 605422 |
| ybdK | Complementary strand | 605488 | 606606 |
| hokE | | 607059 | 607211 |
| insL-2 | | 607288 | 608400 |
| entD | Complementary strand | 608682 | 609311 |
| fepA | Complementary strand | 609477 | 611717 |
| fes | | 612038 | 613162 |
| ybdZ | | 613165 | 613383 |
| entF | | 613380 | 617261 |
| fepE | | 617477 | 618610 |
| fepC | Complementary strand | 618607 | 619422 |
| fepG | Complementary strand | 619419 | 620411 |
| fepD | Complementary strand | 620408 | 621412 |
| ybdA | | 621523 | 622773 |
| fepB | Complementary strand | 622777 | 623733 |
| entC | | 624108 | 625283 |
| entE | | 625293 | 626903 |
| entB | | 626917 | 627774 |

**[Table 1-16]**

| Gene name | Coding strand | Start number in SEQ ID NO:1 (stop number when encoded by complementary strand | Stop number in SEQ ID NO:1 (start number when encoded by complementary strand) |
|---|---|---|---|
| entA | | 627774 | 628520 |
| ybdB | | 628523 | 628936 |
| cstA | | 629117 | 631222 |
| ybdD | | 631405 | 631602 |
| ybdH | Complementary strand | 631612 | 632700 |
| ybdL | | 632809 | 633969 |
| ybdM | Complementary strand | 633970 | 634599 |
| ybdN | Complementary strand | 634572 | 635792 |
| ybdO | Complementary strand | 635939 | 636841 |
| dsbG | Complementary strand | 637050 | 637796 |
| ahpC | | 638168 | 638731 |
| ahpF | | 638976 | 640541 |
| uspG | Complementary strand | 640662 | 641090 |
| ybdR | | 641311 | 642549 |
| mk | Complementary strand | 642780 | 643190 |
| rna | Complementary strand | 643420 | 644226 |
| citT | Complementary strand | 644340 | 645803 |
| citG | Complementary strand | 645854 | 646732 |
| citX | Complementary strand | 646707 | 647258 |
| citF | Complementary strand | 647262 | 648794 |
| citE | Complementary strand | 648805 | 649713 |
| citD | Complementary strand | 649710 | 660006 |
| citC | Complementary strand | 650021 | 651079 |
| citA | | 651458 | 653116 |
| citB | | 653085 | 653765 |
| dcuC | Complementary strand | 653806 | 654282 |
| dcuC | Complementary strand | 653806 | 656390 |
| insH | | 654281 | 655261 |
| dcuC | Complementary strand | 655299 | 656390 |
| crcA | | 658979 | 657539 |
| cspE | | 657714 | 657923 |
| crcB | Complementary strand | 657977 | 658360 |
| ybeH | | 658453 | 658680 |
| ybeM | | 658677 | 659240 |
| tatE | | 659369 | 659572 |
| lipA | Complementary strand | 659673 | 660638 |
| ybeF | Complementary strand | 660847 | 661800 |
| lipB | Complementary strand | 662059 | 662700 |
| ybeD | Complementary strand | 662801 | 663064 |
| dacA | Complementary strand | 663174 | 664385 |

**[Table 1-17]**

| Gene name | Coding strand | Start number in SEQ ID NO:1 (stop number when encoded by complementary strand | Stop number in SEQ ID NO:1 (start number when encoded by complementary strand) |
|---|---|---|---|
| rlpA | Complementary strand | 664524 | 665612 |
| mrdB | Complementary strand | 665623 | 666735 |
| mrdA | Complementary strand | 666738 | 668639 |
| ybeA | Complementary strand | 668670 | 689137 |
| ybeB | Complementary strand | 669141 | 669458 |
| cobC | Complementary strand | 669718 | 670329 |
| nadD | Complementary strand | 670353 | 670994 |
| holA | Complementary strand | 670996 | 672027 |
| rlpB | Complementary strand | 672027 | 672608 |
| leuS | Complementary strand | 672623 | 675205 |
| ybeL | | 675440 | 675922 |
| ybeQ | Complementary strand | 675992 | 676969 |
| ybeR | | 677133 | 677840 |
| djlB | | 677837 | 679264 |
| ybeT | Complementary strand | 679274 | 679828 |
| ybeU | | 679930 | 680637 |
| djlC | | 680634 | 682085 |
| hscC | Complementary strand | 682145 | 683815 |
| rihA | Complementary strand | 683899 | 684834 |
| gltL | Complementary strand | 684952 | 685677 |
| gltK | Complementary strand | 685677 | 686351 |
| gltJ | Complementary strand | 686351 | 687091 |
| gltl | Complementary strand | 687261 | 688169 |
| insH-3 | Complementary strand | 688419 | 689435 |
| lnt | Complementary strand | 689765 | 691303 |
| ybeX | Complementary strand | 691328 | 692206 |
| ybeY | Complementary strand | 692296 | 692763 |
| ybeZ | Complementary strand | 692760 | 693839 |
| miaB | Complementary strand | 693953 | 695377 |
| ubiF | | 695523 | 696698 |
| glnX | Complementary strand | 696852 | 696926 |
| glnV | Complementary strand | 696964 | 697038 |
| metU | Complementary strand | 697086 | 697162 |
| glnW | Complementary strand | 697178 | 697252 |
| glnU | Complementary strand | 697287 | 697361 |
| leuW | Complementary strand | 697385 | 697469 |
| metT | Complementary strand | 697479 | 697555 |
| asnB | Complementary strand | 697935 | 699599 |
| nagD | Complementary strand | 699996 | 700748 |
| nagC | Complementary strand | 700796 | 702016 |

**[Table 1-18]**

| Gene name | Coding strand | Start number in SEQ ID NO: 1 (stop number when encoded by complementary strand) | Stop number in SEQ ID NO: 1 (start number when encoded by complementary strand) |
|---|---|---|---|
| nagA | Complementary strand | 702025 | 703173 |
| nagB | Complementary strand | 703233 | 704033 |
| nagE | | 704366 | 706312 |
| glnS | | 706515 | 708179 |
| ybfM | | 708756 | 710162 |
| ybFN | | 710212 | 710538 |
| fur | Complementary strand | 710622 | 711068 |
| fldA | Complementary strand | 711357 | 711887 |
| ybfE | Complementary strand | 712027 | 712320 |
| ybfF | Complementary strand | 712460 | 713224 |
| seqA | | 713409 | 713954 |
| pgm | | 713980 | 715820 |
| ybfP | | 715834 | 716328 |
| ybfG | Complementary strand | 716369 | 716779 |
| ybfH | Complementary strand | 716810 | 717019 |
| potE | Complementary strand | 717368 | 718687 |
| speF | Complementary strand | 718684 | 720882 |
| kdpE | Complementary strand | 721478 | 722155 |
| kdpD | Complementary strang | 722152 | 724836 |
| kdpC | Complementary strand | 724829 | 725401 |
| kdpB | Complementary strand | 725410 | 727458 |
| kdpA | Complementary strand | 727481 | 729154 |
| kdpF | Complementary strand | 729154 | 729243 |
| ybfA | | 729556 | 729762 |
| rhsC | | 730005 | 734198 |
| ybfB | | 734198 | 734524 |
| ybfO | | 734642 | 736075 |
| ybfC | | 736072 | 736641 |
| ybfQ | | 736867 | 737121 |
| ybfL | | 737322 | 738383 |
| ybfD | | 738514 | 739275 |
| ybgA | | 739423 | 739932 |
| phr | | 739929 | 741347 |
| ybgH | Complementary stand | 741497 | 742978 |
| ybgl | | 743249 | 743992 |
| ybgJ | | 744015 | 744671 |
| ybgK | | 744665 | 745597 |
| ybgL | | 745587 | 746321 |
| nei | | 746357 | 747148 |
| abrB | Complementary strand | 747145 | 748191 |

**[Table 1-19]**

| Gene name | Coding strand | Start number in SEQ ID NO:1 (stop number when encoded by complementary strand) | Stop number in SEQ ID NO:1 (start number ID when encoded by complemantary strand) |
|---|---|---|---|
| ybgO | Complementary strand | 748343 | 749404 |
| ybgP | Complementary strand | 749401 | 750129 |
| ybgQ | Complementary strand | 750144 | 752591 |
| ybgD | Complementary strand | 752651 | 753217 |
| gltA | Complementary strand | 753607 | 754890 |
| SdhC | | 755599 | 755988 |
| sdhD | | 755982 | 756329 |
| sdhA | | 756329 | 758095 |
| sdhB | | 758111 | 758827 |
| sucA | | 759128 | 761929 |
| sucB | | 761944 | 763161 |
| sucC | | 763436 | 764602 |
| sucD | | 764602 | 765471 |
| mngR | Complementary stand | 765575 | 766297 |
| mngA | | 766406 | 768382 |
| mngB | | 768400 | 771033 |
| cydA | | 771880 | 773448 |
| cydB | | 773464 | 774603 |
| ybgT | | 774618 | 774731 |
| ybgE | | 774731 | 775024 |
| ybgC | | 775174 | 775578 |
| tolQ | | 775575 | 776267 |
| tolR | | 776271 | 776699 |
| tolA | | 776764 | 778029 |
| tolB | | 778162 | 779454 |
| pal | | 779489 | 780010 |
| ybgF | | 780020 | 780811 |
| lysT | | 780976 | 781051 |
| valT | | 781167 | 781262 |
| lysW | | 781265 | 781340 |
| valZ | | 781490 | 781565 |
| lysY | | 781569 | 781644 |
| lysZ | | 781791 | 761866 |
| lysQ | | 781999 | 782074 |
| nadA | | 782507 | 783550 |
| pnuC | | 783588 | 784307 |
| zitB | Complementary strand | 784304 | 785245 |
| ybgS | | 785359 | 785739 |
| aroG | | 786055 | 787107 |
| gpmA | Complementary stand | 787265 | 788017 |

**[Table 1-20]**

| Gene name | Coding strand | Start number in SEQ ID NO:1 (stop number when encoded by complementary strand) | Stop number in SEQ ID NO:1 (start number when encoded by complementary strand) |
|---|---|---|---|
| galM | Complementary strand | 788219 | 789259 |
| galK | Complementary strand | 789253 | 790401 |
| galT | Complementary strand | 790405 | 791451 |
| galE | Complementary strand | 791461 | 792477 |
| modF | Complementary strand | 792738 | 794210 |
| modE | Complementary strand | 794278 | 795066 |
| ybhT | | 795195 | 795344 |
| modA | | 795511 | 796284 |
| modB | | 796284 | 796973 |
| modC | | 796976 | 798034 |
| ybhA | Complementary strand | 798035 | 798853 |
| ybhE | | 799008 | 800003 |
| ybhD | Complementary strand | 800044 | 801060 |
| ybhH | | 801181 | 802233 |
| ybhl | | 802309 | 803742 |
| ybhJ | | 803925 | 806186 |
| ybhC | Complementary strand | 808420 | 807703 |
| ybhB | Complementary strand | 807855 | 808331 |
| bioA | Complementary strand | 808390 | 809679 |
| bioB | | 809766 | 810806 |
| bioF | | 810803 | 811957 |
| bioC | | 811944 | 812699 |
| bioD | | 812692 | 813369 |
| uvrB | | 813948 | 815969 |
| ybhK | Complementary strand | 816161 | 817069 |
| moaA | | 817466 | 818455 |
| moaB | | 818477 | 818989 |
| moaC | | 818992 | 819477 |
| moaD | | 819470 | 819715 |
| moaE | | 819717 | 820169 |
| ybhL | | 820306 | 821010 |
| ybhM | | 821215 | 821928 |
| ybhN | Complementary strand | 821964 | 822920 |
| ybhO | Complementary strand | 822920 | 824161 |
| ybhP | Complementary strand | 824158 | 824919 |
| ybhQ | | 825052 | 825462 |
| ybdR | Complementary strand | 825424 | 826530 |
| ybhS | Complementary strand | 826541 | 827674 |
| ybhF | Complementary strand | 827667 | 629403 |
| ybhG | Complementary strand | 829396 | 830394 |

**[Table 1-21]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yblH | Complementary strand | 830394 | 831065 |
| rhlE | | 831294 | 832658 |
| yblA | Complementary strand | 632890 | 833372 |
| dinG | | 833492 | 835642 |
| yblB | | 835670 | 836632 |
| ybiC | | 836773 | 837858 |
| ybiJ | Complementary strand | 838087 | 838347 |
| ybil | Complementary strand | 838612 | 838878 |
| ybiX | Complementary strand | 838952 | 839629 |
| flu | Complementary strand | 839671 | 841953 |
| ybiM | Complementary strand | 842218 | 842478 |
| ybiN | | 842754 | 843880 |
| ybiO | Complementary strand | 843677 | 845902 |
| glnQ | Complementary strand | 846163 | 846885 |
| glnP | Complementary strand | 846882 | 847541 |
| glnH | Complementary strand | 847680 | 848426 |
| dps | Complementary strand | 848830 | 849333 |
| rhtA | Complementary strand | 849632 | 850519 |
| ompX | . | 850872 | 851387 |
| ybiP | Complementary strand | 851436 | 853019 |
| yliL | | 853093 | 853362 |
| rybA | Complementary strand | 853374 | 853462 |
| mntR | | 853605 | 854072 |
| ybiR | | 854069 | 855187 |
| ybiS | Complementary strand | 855246 | 856166 |
| ybiT | | 856385 | 857977 |
| ybiU | Complementary strand | 858218 | 859483 |
| ybiV | | 869635 | 860450 |
| ybiW | Complementary strand | 860596 | 863028 |
| ybiY | Complementary strand | 863034 | 863960 |
| fsaA | | 864064 | 864726 |
| moeB | Complementary strand | 864802 | 865551 |
| moeA | Complementary strand | 865551 | 866786 |
| iaaA | | 866990 | 867955 |
| yliA | | 867975 | 869813 |
| yliB | | 869833 | 871371 |
| yliC | | 871389 | 872309 |
| yliD | | 872312 | 873223 |
| yliE | | 873401 | 875749 |
| yliF | | 875757 | 877085 |

**[Table 1-22]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yliG | Complementary strand | 877132 | B78457 |
| yliH | | 878670 | 879053 |
| ylil | | 879164 | 880279 |
| yliJ | Complementary strand | 860276 | 800902 |
| dacC | | 881149 | 882351 |
| deoR | Complementary strand | 882398 | 883156 |
| ybjG | Complementary strand | 863214 | 883810 |
| cmr | | 884095 | 885327 |
| ybjH | Complementary strand | 885368 | 885652 |
| ybjl | Complementary strand | 885738 | 886553 |
| ybjJ | Complementary strand | 886553 | 887761 |
| ybjK | | 887845 | 888381 |
| rybB | Complementary strand | 888398 | 888476 |
| ybjL | Complementary strand | 888556 | 890241 |
| ybjM | | 890511 | 890888 |
| grxA | Complementary strand | 890918 | 891175 |
| ybjC | | 891335 | 891622 |
| nfsA | | 891606 | 892328 |
| rlmK | | 892389 | 893291 |
| ybjN | | 893379 | 893855 |
| potF | | 894206 | 895318 |
| potG | | 895413 | 896546 |
| potH | | 896556 | 897509 |
| potl | | 897506 | 898351 |
| ybjO | | 898411 | 898899 |
| rumB | | 898940 | 900067 |
| artJ | Complementary strand | 900266 | 900997 |
| artM | Complementary strand | 901288 | 901956 |
| artQ | Complementary strand | 901956 | 902672 |
| artl | Complementary strand | 902679 | 903410 |
| artP | Complementary strand | 903428 | 904156 |
| ybjP | Complementary strand | 904374 | 904889 |
| ybjQ | | 905015 | 905338 |
| ybjR | | 905335 | 906165 |
| ybjS | Complementary strand | 906162 | 907175 |
| ybJT | Complementary strand | 907274 | 908704 |
| ltaE | Complementary strand | 908715 | 909716 |
| poxB | Complementary strand | 909753 | 911471 |
| hcr | Complementary strand | 911604 | 912572 |
| hcp | Complementary strand | 912584 | 914236 |

**[Table 1-23]**

| Gene name | Coding when strand | Start number in SEQ ID NO:1 (stop number encoded by Complementary strand) | Stop number in SEQ ID NO:1 (start number when encoded by complementary strand) |
|---|---|---|---|
| ybjE | Complementary strand | 914380 | 915279 |
| aqpZ | Complementary strand | 915774 | 916469 |
| ybjD | | 916895 | 918553 |
| ybjX | Complementary strand | 918550 | 919542 |
| macA | | 919657 | 920772 |
| macB | | 920769 | 922715 |
| cspD | Complementary strand | 922788 | 923012 |
| clpS | | 923335 | 923655 |
| clpA | | 923686 | 925962 |
| serW | Complementary strand | 926306 | 926393 |
| infA | Complementary strand | 926647 | 926865 |
| aat | Complementary strand | 927150 | 927854 |
| cydC | Complementary strand | 927896 | 929617 |
| cydD | Complementary strand | 929618 | 931384 |
| trxB | Complementary strand | 931507 | 932472 |
| lrp | | 933017 | 933511 |
| ftsK | | 933646 | 937635 |
| lolA | | 937791 | 938405 |
| ycaJ | | 938416 | 939759 |
| serS | | 939850 | 941142 |
| dmsA | | 941381 | 943825 |
| dmsB | | 943836 | 944453 |
| dmsC | | 944455 | 945318 |
| ycaC | Complementary strand | 945353 | 945979 |
| ycaD | | 946293 | 947441 |
| ycaM | | 947651 | 949081 |
| ycaN | Complementary strand | 949082 | 949990 |
| ycaK | | 950090 | 950680 |
| pflA | Complementary strand | 950762 | 951502 |
| pflB | Complementary strand | 951694 | 953976 |
| focA | Complementary strand | 954031 | 954888 |
| ycaO | Complementary strand | 955294 | 957054 |
| ycaP | | 957184 | 957876 |
| serC | | 958075 | 959163 |
| aroA | | 959234 | 960517 |
| ycaL | | 960686 | 901450 |
| cmk | | 961623 | 962306 |
| rpsA | | 962417 | 964090 |
| ihfB | | 964250 | 964534 |
| ycal | | 964742 | 967006 |

**[Table 1-24]**

| Gene name | Coding strand | Start number in SEQ ID NO:1 (stop number when encoded by Complementary strand) | Stop number in SEQ ID NO:1 (start number ID when encoded by Complementary strand) |
|---|---|---|---|
| msbA | | 867043 | 968791 |
| lpxK | | 968788 | 969774 |
| ycaQ | | 969811 | 971043 |
| ycaR | | 971095 | 971277 |
| kdsB | | 971274 | 972020 |
| ycbJ | | 972174 | 973067 |
| ycbC | Complementary strand | 973044 | 973823 |
| smtA | | 973959 | 974744 |
| mukF | | 974741 | 976063 |
| mukE | | 976071 | 976748 |
| mukB | | 976748 | 981208 |
| ycbB | | 981469 | 983316 |
| ycbK | | 983497 | 984045 |
| ycbL | | 984072 | 984719 |
| aspC | Complementary strand | 984941 | 986131 |
| ompF | Complementary strand | 986316 | 987404 |
| asnS | Complementary strand | 988007 | 959407 |
| pncB | Complementary strand | 989576 | 990778 |
| pepN | | 991044 | 923656 |
| ssuB | Complementary strand | 993699 | 994466 |
| ssuC | Complementary strand | 994483 | 995254 |
| ssuD | Complementary strand | 995265 | 996410 |
| ssUA | Complementary strand | 996407 | 997366 |
| ssuE | Complementary strand | 997359 | 997934 |
| ycbQ | | 998290 | 998829 |
| ycbR | | 998912 | 999613 |
| ycbS | | 999638 | 1002238 |
| ycbT | | 1002229 | 1003299 |
| ycbU | | 1003311 | 1003853 |
| ycbV | | 1003861 | 1004376 |
| ycbF | | 1004369 | 1005079 |
| pyrD | | 1005190 | 1006200 |
| ycbW | | 1006374 | 1006916 |
| ycbX | Complementary strand | 1006913 | 1008022 |
| ycbY | | 1008266 | 1010374 |
| uup | | 1010386 | 1012293 |
| pqiA | | 1012423 | 1013676 |
| ppiB | | 1013681 | 1015321 |
| ymbA | | 1015333 | 1015881 |
| rmf | | 1016137 | 1016304 |

**[Table 1-25]**

| Gene name | Coding strand | Start number in SEQ ID NO:1 (stop number when encoded by complementary strand) | Stop number in SEQ ID NO:1 (start number when encoded by complementary strand) |
|---|---|---|---|
| fabA | Complementary strand | 1016374 | 1016892 |
| ycbZ | Complementary strand | 1016961 | 1018721 |
| ycbG | | 1018907 | 1019359 |
| ompA | Complementary strand | 1019435 | 1020475 |
| sulA | Complementary strand | 1020832 | 1021341 |
| yccR | | 1021660 | 1022189 |
| yccS | Complementary strand | 1022152 | 1024305 |
| yccF | Complementary strand | 1024324 | 1024770 |
| helD | | 1024893 | 1026947 |
| mgsA | Complementary strand | 1026979 | 1027437 |
| yccT | Complementary strand | 1027533 | 1028195 |
| yccU | | 1028368 | 1028781 |
| yccV | Complementary strand | 1028826 | 1029143 |
| yccW | Complementary strand | 1029201 | 1030304 |
| yccX | | 1030486 | 1030764 |
| yccK | Complementary strand | 1030761 | 1031090 |
| yccA | Complementary strand | 1031181 | 1031840 |
| serT | Complementary strand | 1032047 | 1032134 |
| hyaA | | 1032561 | 1033679 |
| hyaB | | 1033676 | 1035469 |
| hyaC | | 1035488 | 1033195 |
| hyaD | | 1036192 | 1036779 |
| hyaE | | 1036776 | 1037174 |
| hyaF | | 1037171 | 1038028 |
| appC | | 1038162 | 1039706 |
| appB | | 1039718 | 1040854 |
| appA | | 1041039 | 1042337 |
| yccC | Complementary strand | 1042452 | 1044632 |
| etp | Complementary strand | 1044652 | 1045098 |
| yccZ | Complementary strand | 1045086 | 1046225 |
| ymcA | Complementary strand | 1046271 | 1048367 |
| ymcB | Complementary strand | 1048367 | 1049113 |
| ymcC | Complementary strand | 1049110 | 1049754 |
| ymcD | Complementary strand | 1049861 | 1050166 |
| insA-4 | | 1050255 | 1050530 |
| insB-4 | | 1050449 | 1050952 |
| cspH | Complementary strand | 1051385 | 1051597 |
| cspG | | 1051883 | 1052095 |
| ymcE | | 1052269 | 1052499 |
| gnsA | | 1052489 | 1052662 |

**[Table 1-26]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yccM | Complementary strand | 1052711 | 1053784 |
| torS | Complementary strand | 1053866 | 1056600 |
| torT | | 1056683 | 1057711 |
| torR | Complementary strand | 1057684 | 1058376 |
| torC | | 1058506 | 1059678 |
| torA | | 1059678 | 1062224 |
| torD | | 1062221 | 1062820 |
| cbpM | Complementary strand | 1062972 | 1063277 |
| cbpA | Complementary strand | 1063277 | 1064197 |
| yccE | | 1064458 | 1065714 |
| agp | | 1066007 | 1067248 |
| yccJ | Complementary strand | 1067286 | 1067513 |
| wrbA | Complementary strand | 1067534 | 1068130 |
| ymdF | | 1068503 | 1068676 |
| ycdG | Complementary strand | 1068933 | 1070261 |
| ycdH | Complementary strand | 1070282 | 1070776 |
| ycdl | Complementary strand | 1070787 | 1071377 |
| rarA | Complementary strand | 1071387 | 1072181 |
| ycdK | Complementary strand | 1072195 | 1072581 |
| ycdL | Complementary strand | 1072593 | 1073285 |
| ycdM | Complementary strand | 1073285 | 1074433 |
| ycdC | | 1074664 | 1075302 |
| putA | Complementary strand | 1076342 | 1079304 |
| putP | | 1079727 | 1081235 |
| ycdN_1 | | 1081778 | 1081888 |
| ycdN_2 | | 1081876 | 1082607 |
| ycdO | | 1082665 | 1083792 |
| ycdB | | 1083798 | 1085069 |
| phoH | | 1085414 | 1086478 |
| ycdP | Complementary strand | 1086528 | 1086941 |
| ycdQ | Complementary strand | 1086943 | 1088268 |
| ycdR | Complementary strand | 1088261 | 1090279 |
| ycdS | Complementary strand | 1090288 | 1092711 |
| ycdT | | 1093298 | 1094656 |
| insF-4 | Complementary strand | 1094697 | 1095563 |
| insE-4 | Complementary strand | 1095560 | 1095868 |
| ymdE | | 1095966 | 1096268 |
| ycdU | | 1096265 | 1097251 |
| serX | Complementary strand | 1097806 | 1097893 |
| ycdW | | 1098127 | 1099065 |

**[Table 1-27]**

| Gene name | Coding strand | Start number in SEQ ID NO:1 (stop number when encoded by complementary strand) | stop number in SEQ ID NO:1 (start number when encoded by complementary strand) |
|---|---|---|---|
| ycdX | | 1099120 | 1099857 |
| ycdY | | 1099881 | 1100435 |
| ycdZ | | 1100537 | 1101028 |
| csgG | Complementary strand | 1101092 | 1101925 |
| csgF | Complementary strand | 1101952 | 1102368 |
| csgE | Complementary strand | 1102393 | 1102782 |
| csgD | Complementary strand | 1102787 | 1103437 |
| csgB | | 1104192 | 1104647 |
| csgA | | 1104688 | 1105143 |
| csgC | | 1105202 | 1105534 |
| insD | Complementary strand | 1105633 | 1106556 |
| insC | Complementary strand | 1106496 | 1106861 |
| ymdA | | 1106991 | 1107302 |
| ymdB | | 1107397 | 1107930 |
| ymdC | | 1107932 | 1109353 |
| mdoC | Complementary strand | 1109361 | 1110518 |
| mdoG | | 1110912 | 1112447 |
| mdoH | | 1112440 | 1114983 |
| yceK | | 1115156 | 1115383 |
| msyB | Complementary strand | 1115384 | 1115758 |
| mdtG | Complementary strand | 1115841 | 1117067 |
| lpxL | Complementary strand | 1117239 | 1118159 |
| yceA | | 1118384 | 1119436 |
| ycel | Complementary strand | 1119478 | 1120053 |
| yceJ | Complementary strand | 1120057 | 1120623 |
| yceO | Complementary strand | 1120884 | 1121024 |
| solA | Complementary strand | 1121045 | 1122163 |
| yceP | Complementary strand | 1122278 | 1122532 |
| dinl | Complementary strand | 1122819 | 1123064 |
| pyrC | Complementary strand | 1123138 | 1124184 |
| yceB | Complementary strand | 1124290 | 1124850 |
| grxB | Complementary strand | 1124984 | 1125631 |
| mdtH | Complementary strand | 1125695 | 1126903 |
| rimJ | | 1127139 | 1127723 |
| yceH | | 1127734 | 1128381 |
| mviM | | 1128383 | 1129306 |
| mviN | | 1129416 | 1130951 |
| flgN | Complementary strand | 1130991 | 1131407 |
| flgm | Complementary strand | 1131412 | 1131705 |
| flgA | Complementary strand | 1131781 | 1132440 |

**[Table 1-28]**

| Gene name | Coding strand | Start number in SEQ ID NO:1 (stop number when encoded by complementary strand) | Stop number in SEQ ID NO:1 (start number when encoded by complementary strand) |
|---|---|---|---|
| flgB | | 1132595 | 1133011 |
| flgC | | 1133015 | 1133419 |
| flgD | | 1133431 | 1134126 |
| flgE | | 1134151 | 1135359 |
| flgF | | 1135379 | 1136134 |
| flgG | | 1136306 | 1137088 |
| flgH | | 1137141 | 1137839 |
| flgI | | 1137851 | 1138948 |
| flgJ | | 1138948 | 1139889 |
| flgK | | 1139955 | 1141598 |
| ffgL | | 1141610 | 1142563 |
| me | Complementary strand | 1142759 | 1145944 |
| yceQ | | 1146079 | 1146399 |
| rluC | | 1146517 | 1147476 |
| yceF | Complementary strand | 1147588 | 1148172 |
| sraB | | 1148166 | 1148334 |
| yceD | | 1148371 | 1148892 |
| rpmF | | 1148944 | 1149117 |
| plsX | | 1149198 | 1150268 |
| fabH | | 1150336 | 1151289 |
| fabD | | 1151305 | 1152234 |
| fabG | | 1152247 | 1152981 |
| acpP | | 1153192 | 1153428 |
| fabF | | 1153516 | 1154757 |
| pabC | | 1154877 | 1155686 |
| yceG | | 1155689 | 1156711 |
| tmk | | 1156701 | 1157342 |
| holB | | 1157339 | 1158343 |
| ycfH | | 1158354 | 1159151 |
| ptsG | | 1159446 | 1160879 |
| fhuE | Complementary strand | 1160939 | 1163128 |
| hinT | | 1163462 | 1163821 |
| ycfL | | 1163824 | 1164201 |
| ycfM | | 1164215 | 1164856 |
| ycfN | | 1164837 | 1165661 |
| nagZ | | 1165672 | 1166697 |
| ycfP | | 1166720 | 1167262 |
| ndh | | 1167662 | 1168966 |
| ycfJ | | 1169176 | 1169715 |
| ycfQ | Complementary strand | 1169777 | 1170409 |

**[[Table 1-29]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ycfR | | 1170650 | 1170907 |
| ycfS | Complementary strand | 1170989 | 1171951 |
| mfd | Complementary strand | 1172095 | 1175541 |
| ycfT | Complementary strand | 1175669 | 1176742 |
| lolC | | 1177004 | 1178203 |
| lolD | | 1178196 | 1178897 |
| IolE | | 1178897 | 1180141 |
| ycfX | | 1180170 | 1181081 |
| cobB | | 1181097 | 1181936 |
| ycfZ | Complementary strand | 1182056 | 1182844 |
| ymfA | Complementary strand | 1182841 | 1183302 |
| potD | Complementary strand | 1183360 | 1184406 |
| potC | Complementary strand | 1184403 | 1185197 |
| potB | Complementary strand | 1185194 | 1186021 |
| potA | Complementary strand | 1186035 | 1187171 |
| pepT | | 1187421 | 1188647 |
| ycfD | Complementary strand | 1188696 | 1189817 |
| phoQ | Complementary strand | 1189893 | 1191353 |
| phoP | Complementary strand | 1191353 | 1192024 |
| purB | Complementary strand | 1192193 | 1193563 |
| hflD | Complementary strand | 1193567 | 1194208 |
| trmU | Complementary strand | 1194244 | 1195350 |
| ymfB | Complementary strand | 1195404 | 1195335 |
| ymfC | Complementary strand | 1195875 | 1196528 |
| icd | | 1196700 | 1197950 |
| ymfD | Complementary strand | 1198444 | 1199109 |
| ymfE | Complementary strand | 1199110 | 1199814 |
| lit | | 1200272 | 1201165 |
| lntE | Complementary strand | 1201256 | 1202383 |
| ymfG | Complementary strand | 1202364 | 1202609 |
| ymfH | Complementary strand | 1202646 | 1202957 |
| ymfl | | 1203074 | 1203415 |
| ymfJ | Complementary strand | 1203353 | 1203661 |
| ymfK | Complementary strand | 1203836 | 1204510 |
| ymfT | | 1204601 | 1204801 |
| ymfL | | 1204833 | 1205402 |
| ymfM | | 1205399 | 1205737 |
| ymfN | | 1205747 | 1207114 |
| ymfR | | 1207126 | 1207308 |
| ymfO | | 1207308 | 1207781 |

**[Table 1-30]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ymfP | | 1207837 | 1208499 |
| ymfQ | | 1208490 | 1209074 |
| ycfK | | 1209078 | 1209707 |
| ymfS | | 1209709 | 1210122 |
| tfaE | Complementary strand | 1210094 | 1210696 |
| stfE | Complementary strand | 1210696 | 1211232 |
| pln | | 1211262 | 1211816 |
| mcrA | | 1211923 | 1212756 |
| icdC | | 1213002 | 1213154 |
| elbA | Complementary strand | 1213257 | 1213580 |
| ycgX | Complementary strand | 1214280 | 1214684 |
| ycgE | Complementary strand | 1214905 | 1215636 |
| ycgF | Complementary strand | 1215841 | 1217052 |
| ycgZ | | 1217366 | 1217602 |
| ymgA | | 1217645 | 1217917 |
| ymgB | | 1217946 | 1218212 |
| ymgC | | 1218325 | 1218573 |
| ycgG | | 1218905 | 1220428 |
| ymgF | | 1220560 | 1220778 |
| ycgH_1 | | 1222378 | 1222698 |
| ycgH_2 | | 1222783 | 1223799 |
| ymgD | Complementary strand | 1223882 | 1224211 |
| ymgG | Complementary strand | 1224221 | 1224496 |
| ymgH | | 1225042 | 1225263 |
| ycgl | | 1225272 | 1225484 |
| minE | Complementary strand | 1225856 | 1226122 |
| minD | Complementary strand | 1226126 | 1226938 |
| minC | Complementary strand | 1226962 | 1227657 |
| ycgJ | | 1228177 | 1228545 |
| ycgK | Complementary strand | 1228648 | 1229049 |
| ycgL | | 1229258 | 1229584 |
| ycgM | | 1229656 | 1230315 |
| ycgN | | 1230407 | 1230853 |
| hlyE | Complementary strand | 1231060 | 1231971 |
| umuD | | 1232344 | 1232763 |
| umuC | | 1232763 | 1234031 |
| dsbB | Complementary strand | 1234077 | 1234607 |
| nhaB | Complementary strand | 1234763 | 1236294 |
| fadR | | 1236515 | 1237234 |
| ycgB | Complementary strand | 1237286 | 1238818 |

**[Table 1-31]**

| Gene name | Coding strand | Start number in SEQ ID NO:1 (stop number when encoded by complementary strand | Stop number in SEQ ID NO:1 (start number when encoded by complementary strand) |
|---|---|---|---|
| dadA | | 1239148 | 1240446 |
| dadX | | 1240456 | 1241526 |
| cvrA | Complementary strand | 1241912 | 1243648 |
| ldcA | Complementary strand | 1243743 | 1244657 |
| emtA | | 1244757 | 1245368 |
| ycgR | Complementary strand | 1245370 | 1246104 |
| ymgE | | 1246305 | 1246559 |
| ycgY | | 1246737 | 1247177 |
| treA | Complementary strand | 1247256 | 1248953 |
| dhaH | Complementary strand | 1249273 | 1250691 |
| dhaL | Complementary strand | 1250702 | 1251334 |
| dhaK | Complementary strand | 1251345 | 1252415 |
| dhaR | | 1252643 | 1254562 |
| ycgV | Complementary strand | 1254662 | 1257529 |
| ychF | Complementary strand | 1258298 | 1259389 |
| pth | Complementary strand | 1259506 | 1260090 |
| ychH | | 1260368 | 1260646 |
| ychM | Complementary strand | 1260701 | 1262380 |
| prsA | Complementary strand | 1262505 | 1263452 |
| ispE | Complementary strand | 1263603 | 1264454 |
| lolB | Complementary strand | 1264454 | 1265077 |
| hemA | | 1265291 | 1266547 |
| prfA | | 1266589 | 1267671 |
| prmC | | 1267671 | 1268504 |
| ychQ | | 1268501 | 1268893 |
| ychA | | 1268887 | 1269706 |
| kdsA | | 1269742 | 1270596 |
| IdrA | Complementary strand | 1270745 | 1270852 |
| rdlA | | 1270900 | 1270966 |
| ldrB | Complementary strand | 1271280 | 1271387 |
| rdlB | | 1271435 | 1271500 |
| ldrC | Complementary strand | 1271815 | 1271922 |
| rdlC | | 1271970 | 1272037 |
| chaA | Complementary strand | 1272326 | 1273426 |
| chaB | | 1273696 | 1273926 |
| chaC | | 1274063 | 1274779 |
| ychN | Complementary strand | 1274823 | 1275176 |
| ychP | | 1275502 | 1276755 |
| narL | Complementary strand | 1276756 | 1277406 |
| narX | Complementary strand | 1277399 | 1279195 |

**[Table 1-32]**

| Gene name | Coding strand | Start number in SEQ ID NO:1 (stop number when encoded by when complementary | Stop number in SEQ ID NO:1 [start number encoded by strand) complementary strand) |
|---|---|---|---|
| narK | | 1279534 | 1280925 |
| narG | | 1281441 | 1285184 |
| narH | | 1285181 | 1286719 |
| narJ | | 1286716 | 1287426 |
| narl | | 1287426 | 1288103 |
| ychS | | 1288286 | 1288561 |
| rttR | Complementary strand | 1288643 | 1288813 |
| tpr | Complementary strand | 1288664 | 1288765 |
| tyrV | Complementary strand | 1288821 | 1288905 |
| tyrT | Complementary strand | 1289115 | 1289199 |
| purU | Complementary strand | 1289359 | 1290201 |
| ychJ | Complementary strand | 1290251 | 1290709 |
| rssA | | 1290783 | 1291727 |
| rssB | | 1291819 | 1292832 |
| galU | | 1293034 | 1293942 |
| hns | Complementary strand | 1294086 | 1294499 |
| tdk | | 1295104 | 1295721 |
| ychG_2 | Complementary strand | 1296003 | 1296593 |
| ychG_1 | Complementary strand | 1296545 | 1296775 |
| adhE | Complementary strand | 1297023 | 1299698 |
| ychE | | 1300175 | 1300822 |
| insC | | 1301130 | 1301495 |
| insD | | 1301435 | 1302358 |
| oppA | | 1302896 | 1304527 |
| oppB | | 1304613 | 1305533 |
| oppC | | 1305548 | 1306456 |
| oppD | | 1306468 | 1307481 |
| oppF | | 1307478 | 1308482 |
| yciU | Complementary strand | 1308535 | 1308864 |
| cls | Complementary strand | 1308899 | 1310359 |
| kch | Complementary strand | 1310730 | 1311983 |
| ycil | Complementary strand | 1312283 | 1312579 |
| tcnB | | 1312803 | 1313522 |
| yciA | Complementary strand | 1313662 | 1313960 |
| yciB | Complementary strand | 1314065 | 1314604 |
| yciC | Complementary strand | 1314634 | 1315377 |
| ompW | | 1315734 | 1316372 |
| yciE | Complementary strand | 1316432 | 1316938 |
| yciF | Complementary strand | 1316984 | 1317484 |
| yciG | Complementary strand | 1317570 | 1317749 |

**[Table 1-33]**

| Gene name | Coding strand | Start number in SEQ ID NO:1 (stop number when encoded by complementary strand) | Stop number in SEQ ID NO:1(start number when encoded by complementary strand) |
|---|---|---|---|
| trpA | Complementary strand | 1318130 | 1318936 |
| trpB | Complementary strand | 1318936 | 1320129 |
| trpC | Complementary strand | 1320141 | 1321499 |
| trpD | Complementary strand | 1321503 | 1323098 |
| trpE | Complementary strand | 1323098 | 1324680 |
| trpL | Complementary strand | 1324752 | 1324796 |
| yciV | | 1324934 | 1325815 |
| yciO | | 1325812 | 1326432 |
| yciQ | | 1326460 | 1328355 |
| rluB | | 1328566 | 1329441 |
| btuR | Complementary strand | 1329481 | 1330071 |
| yciK | Complementary strand | 1330068 | 1330626 |
| sohB | | 1331046 | 1332095 |
| yciN | Complementary strand | 1332131 | 1332382 |
| topA | | 1332762 | 1335359 |
| cysB | | 1335569 | 1336543 |
| yciX_1 | | 1336874 | 1337002 |
| ycDC2 | | 1336984 | 1337172 |
| acnA | | 1337545 | 1340220 |
| ribA | Complementary strand | 1340284 | 1340874 |
| pgpB | | 1341044 | 1341808 |
| yciS | | 1341957 | 1342265 |
| yciM | | 1342272 | 1343441 |
| pyrF | | 1343635 | 1344372 |
| yciH | | 1344372 | 1344698 |
| osmB | Complementary strand | 1344824 | 1345042 |
| yciT | Complementary strand | 1345311 | 1346060 |
| gmr | Complementary strand | 1346471 | 1348456 |
| rnb | Complementary strand | 1348692 | 1350626 |
| yciW | Complementary strand | 1350694 | 1351821 |
| fabl | Complementary strand | 1351965 | 1352753 |
| yojD | Complementary strand | 1353121 | 1353474 |
| sapF | Complementary strand | 1353542 | 1354348 |
| sapD | Complementary strand | 1354350 | 1355342 |
| sapC | Complementary strand | 1355342 | 1356232 |
| sapB | Complementary strand | 1356219 | 1357184 |
| sapA | Complementary strand | 1357181 | 1358824 |
| ymjA | Complementary strand | 1359137 | 1359382 |
| puuP | Complementary strand | 1369516 | 1360901 |
| puuA | Complementary strand | 1361204 | 1362622 |

**[Table 1-34]**

| Gene name | Coding strand | Start number in SEQ ID NO:1 (stop number when encoded by complementary strand) | Stop number in SEQ ID NO:1 (start number when encoded by complementary strand) |
|---|---|---|---|
| puuD | | 1362834 | 1363598 |
| puuR | | 1363625 | 1364182 |
| puuC | | 1364457 | 1365944 |
| puuB | | 1365946 | 1367226 |
| puuE | | 1367264 | 1368529 |
| pspF | Complementary strand | 1368649 | 1369626 |
| pspA | | 1369793 | 1370461 |
| pspB | | 1370515 | 1370739 |
| pspC | | 1370739 | 1371098 |
| pspD | | 1371107 | 1371328 |
| pspE | | 1371403 | 1371717 |
| ycjM | | 1371930 | 1373609 |
| ycjN | | 1373623 | 1374915 |
| ycjO | | 1374936 | 1375817 |
| ycjP | | 1375804 | 1376646 |
| ycjQ | | 1376677 | 1377729 |
| ycjR | | 1377748 | 1378536 |
| ycjS | | 1378546 | 1379601 |
| ycjT | | 1379598 | 1381865 |
| ycjU | | 1381862 | 1382521 |
| ycjV | | 1382535 | 1383503 |
| ymjB | | 1383503 | 1383616 |
| ompG | | 1383661 | 1384566 |
| ycjW | Complementary strand | 1384677 | 1385675 |
| ycjX | | 1385831 | 1387228 |
| ycjF | | 1387225 | 1388286 |
| tyrR | | 1388434 | 1389975 |
| tpx | Complementary strand | 1390019 | 1390525 |
| ycjG | | 1390644 | 1391609 |
| mpaA | Complementary strand | 1391584 | 1392372 |
| ymjC | Complementary strand | 1392394 | 1392576 |
| ycjY | Complementary strand | 1392647 | 1393579 |
| yojZ | | 1393705 | 1394604 |
| mppA | | 1394941 | 1396554 |
| ynal | Complementary strand | 1396605 | 1397636 |
| lnsH-4 | | 1397790 | 1398806 |
| ynaJ | | 1399079 | 1399336 |
| uspE | Complementary strand | 1399386 | 1400336 |
| fnr | Complementary strand | 1400488 | 1401240 |
| ogt | Complementary strand | 1401435 | 1401950 |

**[Table 1-35]**

| Gene name | Coding strand | Start number in SEQ ID NO:1 (stop number when encoded by complementary strand) | Stop number in SEQ ID NO:1 (start number when encoded by complementary strand) |
|---|---|---|---|
| abgT | Complementary strand | 1401961 | 1403487 |
| abgB | Complementary strand | 1403524 | 1404969 |
| abgA | Complementary strand | 1404969 | 1406279 |
| abgR | | 1406455 | 1407363 |
| isrA | Complementary strand | 1407366 | 1407523 |
| ydaL | | 1407693 | 1408256 |
| ydaM | Complementary strand | 1408277 | 1409509 |
| ydaN | | 1409764 | 1410747 |
| dbpA | | 1411225 | 1412598 |
| ydaO | Complementary strand | 1412727 | 1413662 |
| intR | Complementary strand | 1413714 | 1414949 |
| ydaQ | Complementary strand | 1414951 | 1415166 |
| ydaC | Complementary strand | 1415245 | 1415454 |
| lar | Complementary strand | 1415447 | 1415641 |
| recT | Complementary strand | 1415698 | 1416507 |
| recE | Complementary strand | 1416500 | 1419100 |
| racC | Complementary strand | 1419202 | 1419477 |
| ydaE | Complementary strand | 1419552 | 1419722 |
| kll | Complementary strand | 1419722 | 1419943 |
| sleB | | 1420385 | 1420873 |
| ydaF | Complementary strand | 1420870 | 1421025 |
| ydaG | Complementary strand | 1421036 | 1421170 |
| racR | Complementary strand | 1421479 | 1421955 |
| ydaS | | 1422079 | 1422375 |
| ydaT | | 1422398 | 1422820 |
| ydaU | | 1422833 | 1423690 |
| ydaV | | 1423697 | 1424443 |
| ydaW | | 1424466 | 1425026 |
| rzpR | | 1425059 | 1425358 |
| rzcR | | 1425114 | 1425299 |
| trkG | | 1425496 | 1426953 |
| ynaK | | 1427091 | 1427354 |
| ydaY | | 1427335 | 1427694 |
| ynaA | | 1428168 | 1429196 |
| lomR_1 | | 1429172 | 1429327 |
| insH-5 | Complementary strand | 1429460 | 1430440 |
| lomR_2 | | 1430528 | 1430698 |
| stfR | | 1430763 | 1434125 |
| tfaR | | 1434125 | 1434700 |
| plnR | Complementary strand | 1434798 | 1435388 |

**[Table 1-36]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ynaE | Complementary strand | 1435705 | 1435971 |
| uspF | Complementary strand | 1436899 | 1437333 |
| ompN | Complementary strand | 1437414 | 1438607 |
| micC | | 1438835 | 1438942 |
| ydbK | Complementary strand | 1438974 | 1442498 |
| ydbJ | | 1442772 | 1443038 |
| hslJ | Complementary strand | 1443035 | 1443457 |
| ldhA | Complementary strand | 1443568 | 1444557 |
| ydbH | | 1444765 | 1447404 |
| ynbE | | 1441401 | 1447586 |
| ydbL | | 1447594 | 1447920 |
| feaR | Complementary strand | 1448092 | 1448997 |
| feaB | | 1449233 | 1450732 |
| tynA | Complementary strand | 1450790 | 1453063 |
| macC | Complementary strand | 1463311 | 1455356 |
| paaA | | 1455641 | 1456570 |
| paaB | | 1456582 | 1456869 |
| paaC | | 1456878 | 1457624 |
| paaD | | 1457639 | 1458136 |
| paaE | | . 1458144 | 1459214 |
| paaF | | 1459211 | 1459978 |
| paaG | | 1459978 | 1460766 |
| paaH | | 1460768 | 1462195 |
| paaI | | 1462185 | 1462607 |
| paaJ | | 1462607 | 1463812 |
| paaK | | 1463839 | 1465152 |
| paaX | | 1465253 | 1466203 |
| paaY | | 1466185 | 1466775 |
| ydbA_1 | | 1467106 | 1469664 |
| insD-2 | Complementary strand | 1469635 | 1470540 |
| insC-2 | Complementary strand | 1470498 | 1470908 |
| insI-2 | | 1471072 | 1472223 |
| ydbA_2 | | 1472404 | 1475727 |
| ydbC | | 1475935 | 1476795 |
| ydbD | | 1476858 | 1479164 |
| ynbA | | 1479335 | 1479940 |
| ybbB | | 1479940 | 1480836 |
| ynbC | | 1480852 | 1482609 |
| ynbD | | 1482623 | 1483915 |
| azoR | Complementary strand | 1483969 | 1484574 |

**[Table 1-37]**

| Gene name | Coding strand | Start number in SEQ ID NO:1 (stop number when encoded by complementary strand) | Stop number in SEQ ID NO:1 (start number when encoded by complementary strand) |
|---|---|---|---|
| hrpA | | 1484832 | 1488677 |
| ydcF | | 1488949 | 1489749 |
| aldA | | 1489946 | 1491385 |
| gapC_2 | Complementary strand | 1491427 | 1491678 |
| gapC_1 | Complementary strand | 1491675 | 1492427 |
| cybB | | 1492616 | 1493146 |
| ydcA | | 1493391 | 1493564 |
| hokB | Complementary strand | 1493636 | 1493785 |
| mokB | Complementary strand | 1493676 | 1493843 |
| sokB | | 1493833 | 1493885 |
| trg | | 1494184 | 1495824 |
| ydcl | Complementary strand | 1495862 | 1496785 |
| ydcJ | | 1497002 | 1498345 |
| mdoD | | 1498600 | 1500225 |
| ydcH | | 1500365 | 1500589 |
| rimL | | 1500652 | 1501191 |
| ydcK | Complementary strand | 1501183 | 1502163 |
| tehA | | 1502287 | 1503279 |
| tehB | | 1503276 | 1503869 |
| ydcL | | 1504171 | 1504839 |
| yncK_1 | Complementary strand | 1504857 | 1505024 |
| yncK_2 | Complementary strand | 1505076 | 1505363 |
| ydcM | | 1505371 | 1506579 |
| ydcO | Complementary strand | 1506619 | 1507794 |
| ydcN | | 1507886 | 1508422 |
| ydcP | | 1508495 | 1510456 |
| yncJ | Complementary strand | 1510648 | 1510778 |
| yncN | | 1511000 | 1511176 |
| ydcQ | | 1511201 | 1511638 |
| ydcR | | 1511717 | 1513123 |
| ydcS | | 1513368 | 1514513 |
| ydcT | | 1514531 | 1515544 |
| ydcU | | 1515545 | 1516486 |
| ydcV | | 1516476 | 1517270 |
| ydcW | | 1511292 | 1518716 |
| ydcX | | 1519103 | 1519276 |
| ydcY | | 1519362 | 1519595 |
| ydcZ | Complementary strand | 1519596 | 1520045 |
| yncA | Complementary strand | 1520042 | 1520560 |
| yncB | | 1520741 | 1521778 |

**[Table 1-38]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yncC | | 1521976 | 1522641 |
| yncD | Complementary strand | 1522677 | 1524779 |
| yncE | | 1525021 | 1526082 |
| ansP | Complementary strand | 1526195 | 1527694 |
| yncG | | 1527961 | 1528578 |
| yncH | | 1528654 | 1528866 |
| rhsE | | 1529604 | 1531652 |
| ydcD | | 1531636 | 1532118 |
| yncl | | 1532300 | 1533046 |
| yncM | | 1533090 | 1533290 |
| ydcC | | 1533530 | 1534666 |
| ydcE | | 1534766 | 1534999 |
| yddH | Complementary strand | 1534996 | 1535565 |
| nhoA | | 1535738 | 1536583 |
| yddE | Complementary strand | 1536679 | 1537572 |
| narV | Complementary strand | 1537651 | 1538331 |
| narW | Complementary strand | 1538328 | 1539023 |
| narY | Complementary strand | 1539023 | 1540567 |
| narZ | Complementary strand | 1540564 | 1544304 |
| narU | Complementary strand | 1544386 | 1545774 |
| yddJ | Complementary strand | 1546098 | 1546433 |
| yddK | Complementary strand | 1546472 | 1547428 |
| yddL | Complementary strand | 1547452 | 1547742 |
| yddG | Complementary strand | 1548002 | 1548826 |
| fdnG | | 1549115 | 1552162 |
| fdnH | | 1552175 | 1553059 |
| fdnl | | 1553052 | 1553705 |
| yddM | Complementary strand | 1554112 | 1554474 |
| adhP | Complementary strand | 1554542 | 1555552 |
| sfcA | Complementary strand | 1555686 | 1557383 |
| sra | Complementary strand | 1557540 | 1557677 |
| bdm | Complementary strand | 1557779 | 1557994 |
| osmC | | 1558339 | 1558770 |
| ddpF | Complementary strand | 1558826 | 1559752 |
| ddpD | Complementary strand | 1559745 | 1560731 |
| ddpC | Complementary strand | 1560728 | 1561624 |
| ddpB | Complementary strand | 1561621 | 1562643 |
| ddpA | Complementary strand | 1562645 | 1564195 |
| ddpX | Complementary strand | 1564209 | 1564790 |
| dos | Complementary strand | 1565048 | 1567447 |

**[Table 1-39]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yddV | Complementary strand | 1567472 | 1568515 |
| yddW | Complementary strand | 1569218 | 1570537 |
| gadC | Complementary strand | 1570668 | 1572203 |
| gadB | | 1572359 | 1573759 |
| pqqL | Complementary strand | 1574121 | 1576916 |
| yddB | Complementary strand | 1576961 | 1579333 |
| yddA | Complementary strand | 1579371 | 1581056 |
| ydeM | Complementary strand | 1581347 | 1582504 |
| ydeN | Complementary strand | 1582556 | 1584238 |
| ydeO | Complementary strand | 1584640 | 1585401 |
| ydeP | Complementary strand | 1585921 | 1588200 |
| ydeQ | Complementary strand | 1588534 | 1589448 |
| ydeR | Complementary strand | 1589507 | 1590010 |
| ydeS | Complementary strand | 1590023 | 1590553 |
| ydeT | Complementary strand | 1590567 | 1591115 |
| yneL | Complementary strand | 1592071 | 1592250 |
| hipA | Complementary strand | 1592568 | 1593890 |
| hipB | Complementary strand | 1593890 | 1594156 |
| ydeU | Complementary strand | 1594379 | 1595779 |
| ydeK | Complementary strand | 1595823 | 1599800 |
| ydeV | Complementary strand | 1600331 | 1601923 |
| ydeW | Complementary strand | 1602002 | 1602955 |
| ego | | 1603204 | 1604739 |
| lsrC | | 1604733 | 1605761 |
| lsrD | | 1605761 | 1606753 |
| lsrB | | 1606765 | 1607787 |
| lsrF | | 1601814 | 1608689 |
| lsrG | | 1608713 | 1609003 |
| tam | | 1609060 | 1609818 |
| yneE | Complementary strand | 1609822 | 1610736 |
| uxaB | Complementary strand | 1610943 | 1612394 |
| yneF | Complementary strand | 1612621 | 1613568 |
| yneG | Complementary strand | 1613680 | 1614039 |
| yneH | Complementary strand | 1614039 | 1614965 |
| yneI | Complementary strand | 1615029 | 1616417 |
| yneJ | | 1616518 | 1617399 |
| yneK | | 1617477 | 1618592 |
| ydeA | | 1618742 | 1619932 |
| marC | Complementary strand | 1619957 | 1620622 |
| marR | | 1620834 | 1621268 |

**[Table 1-40]**

| Gene name | Coding strand | Start number in SEQ ID NO:1 (stop number when encoded by complementary strand) | Stop number in SEQ ID NO:1 (start number when encoded by complementary strand) |
|---|---|---|---|
| marA | | 1621288 | 1621671 |
| marB | | 1621703 | 1621921 |
| eamA | Complementary strand | 1621952 | 1622851 |
| ydeE | | 1623046 | 1624233 |
| ydeH | Complementary strand | 1624674 | 1625564 |
| ydeI | Complementary strand | 1625819 | 1626211 |
| ydeJ | | 1626487 | 1627005 |
| dcp | Complementary strand | 1627049 | 1629094 |
| ydfG | | 1629231 | 1629977 |
| ydfH | | 1630066 | 1630752 |
| ydfz | | 1630929 | 1631132 |
| ydfl | Complementary strand | 1631167 | 1632627 |
| ydfJ | Complementary strand | 1632716 | 1633999 |
| ydfK | | 1634753 | 1635019 |
| pinQ | | 1635336 | 1635926 |
| tfaQ | Complementary strand | 1636024 | 1636539 |
| stfQ | Complementary strand | 1636599 | 1637561 |
| nohA | Complementary strand | 1637512 | 1638081 |
| ynfO | | 1638470 | 1638703 |
| ydfO | | 1638761 | 1639171 |
| gnsB | Complementary strand | 1639323 | 1639496 |
| ynfN | Complementary strand | 1639668 | 1639823 |
| cspl | Complementary strand | 1640169 | 1640381 |
| ydfP | Complementary strand | 1640744 | 1641241 |
| ydfQ | Complementary strand | 1641238 | 1641771 |
| ydfR | Complementary strand | 1641768 | 1642079 |
| essQ | Complementary strand | 1642084 | 1642299 |
| cspB | Complementary strand | 1643053 | 1643268 |
| cspF | | 1643569 | 1643781 |
| ydfT | Complementary strand | 1644203 | 1644965 |
| ydfU | Complementary strand | 1644969 | 1646018 |
| rem | Complementary strand | 1646365 | 1646616 |
| hokD | Complementary strand | 1646833 | 1646988 |
| relE | Complementary strand | 1647060 | 1647347 |
| relB | Complementary strand | 1647347 | 1647586 |
| ydfV | | 1647611 | 1647916 |
| fixA | | 1648119 | 1648451 |
| ydfW | Complementary strand | 1648688 | 1649037 |
| ydfX | Complementary strand | 1649060 | 1649350 |
| dicC | Complementary strand | 1649334 | 1649564 |

**[Table 1-41]**

| Gene name | Coding strand | Start number in SEQ ID NO: 1 (stop number when encoded by complementary strand) | Stop number in SEQ ID NO: 1 (start number ID when encoded by strand) complementary strand) |
|---|---|---|---|
| dicA | | 1649648 | 1650055 |
| ydfA | | 1650222 | 1650377 |
| ydfB | | 1650379 | 1650807 |
| ydfC | | 1650537 | 1650755 |
| dicF | | 1651096 | 1651148 |
| dicB | | 1651323 | 1651511 |
| ydfD | | 1651508 | 1651699 |
| ydfE | | 1651792 | 1662712 |
| insD | | 1652595 | 1653251 |
| intQ | | 1653226 | 1654422 |
| ynfP | Complementary strand | 1654442 | 1654552 |
| rspB | Complementary strand | 1654610 | 1655629 |
| rspA | Complementary strand | 1655641 | 1656855 |
| ynfA | Complementary strand | 1657061 | 1657387 |
| ynfB | | 1657522 | 1657863 |
| speG | | 1657898 | 1658458 |
| ynfC | Complementary strand | 1658461 | 1659171 |
| ynfD | | 1659279 | 1659584 |
| ynfE | | 1669783 | 1662209 |
| ynfF | | 1662270 | 1664693 |
| ynfG | | 1664704 | 1665321 |
| ynfH | | 1665323 | 1666177 |
| dmsD | | 1666220 | 1666834 |
| clcB | | 1667029 | 1668285 |
| ynfK | Complementary strand | 1668238 | 1668933 |
| dgsA | Complementary strand | 1669058 | 1670278 |
| ynfL | Complementary strand | 1670413 | 1671306 |
| ynfM | | 1671413 | 1672666 |
| asr | | 1673090 | 1673398 |
| ydgD | | 1673674 | 1674495 |
| mdtl | Complementary strand | 1674534 | 1674863 |
| mdtJ | Complementary strand | 1674850 | 1675215 |
| ydgG | | 1675627 | 1676681 |
| pntB | Complementary strand | 1676686 | 1678074 |
| pntA | Complementary strand | 1678085 | 1679617 |
| ydgH | | 1680141 | 1681085 |
| ydgl | | 681271 | 1582653 |
| folM | | 1682690 | 1683412 |
| ydgC | Complementary strand | 1683409 | 1683744 |
| rstA | | 1683864 | 1684892 |

**[Table 1-42]**

| Gene name | Coding strand | Start number in SEQ ID NO:1 (stop mumber when encoded by complementary strand) | Stop number in SEQ ID NO:1 (start number when encoded by complementary strand) |
|---|---|---|---|
| rstB | | 1684696 | 1688897 |
| tus | | 1685973 | 1686902 |
| fumC | Complementary strand | 1686699 | 1688302 |
| fumA | Complementary strand | 1688445 | 1690091 |
| manA | | 1690290 | 1691465 |
| ydgA | | 1691566 | 1693074 |
| uidC | Complementary strand | 1693300 | 1694565 |
| uidB | Complementary strand | 1694604 | 1695977 |
| uidA | Complementary strand | 1695974 | 1697785 |
| uidR | Complementary strand | 1698176 | 1698766 |
| hdhA | Complementary strand | 1698987 | 1699754 |
| mall | Complementary strand | 1699866 | 1700894 |
| malX | | 1701069 | 1702661 |
| malY | | 1702671 | 1703843 |
| add | | 1703947 | 1704948 |
| ydgJ | Complementary strand | 1704982 | 1706022 |
| blr | | 1706265 | 1706390 |
| ydgT | | 1706663 | 1706878 |
| ydgK | | 1706964 | 1707404 |
| rsxB | | 1707481 | 1708062 |
| rsxB | | 1708062 | 1708640 |
| rsxC | | 1708633 | 1710855 |
| rsxD | | 1710856 | 1711914 |
| rsxG | | 1711918 | 1712538 |
| rsxE | | 1712542 | 1713237 |
| nth | | 1713237 | 1713872 |
| ydgR | | 1714483 | 1716985 |
| gst | | 1716091 | 1716696 |
| pdxY | Complementary strand | 1716740 | 1717603 |
| tyrs | Complementary strand | 1717662 | 1718936 |
| pdxH | Complementary strand | 1719065 | 1719721 |
| ydhA | Complementary strand | 1719780 | 1720109 |
| ydhH | Complementary strand | 1720207 | 1721316 |
| slyB | | 1721590 | 1722057 |
| slyA | Complementary strand | 1722104 | 1722638 |
| ydhl | | 1722739 | 1722975 |
| ydhJ | | 1722978 | 1723835 |
| ydhK | | 1723835 | 1725847 |
| sodC | Complementary strand | 1725848 | 1726369 |
| ydhF | Complementary strand | 1726450 | 1727346 |

**[Table 1-43]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ydhL | Complementary strand | 1727395 | 1727634 |
| ydhM | | 1727821 | 1728336 |
| nemA | | 1728373 | 1729470 |
| gloA | | 1729551 | 1729958 |
| mt | | 1730061 | 1730708 |
| lhr | | 1730801 | 1735417 |
| ydhD | Complementary strand | 1735466 | 1735815 |
| ydhO | | 1736149 | 1736964 |
| sodB | | 1737092 | 1737673 |
| ydhP | Complementary strand | 1737835 | 1739004 |
| purR | | 1739558 | 1740583 |
| ydhB | Complementary strand | 1.740580 | 1741512 |
| ydhC | | 1741625 | 1742836 |
| cfa | | 1743127 | 1744275 |
| rlbc | Complementary strand | 1744315 | 1744956 |
| mdtK | | 1745171 | 1746544 |
| ydhQ | Complementary strand | 1746585 | 1747841 |
| valV | | 1748149 | 1748225 |
| valW | | 1748230 | 1748306 |
| ydhR | | 1748414 | 1748719 |
| ydhS | | 1748845 | 1750449 |
| ydhT | Complementary strand | 1750461 | 1751273 |
| ydhU | Complementary strand | 1751277 | 1752062 |
| ydhX | Complementary strand | 1752059 | 1752727 |
| ydhW | Complementary strand | 1752791 | 1753438 |
| ydhV | Complementary strand | 1753442 | 1755544 |
| ydhY | Complementary strand | 1765565 | 1756191 |
| ydhZ | Complementary strand | 1756646 | 1756855 |
| pykF | | 1757412 | 1758824 |
| lpp | | 1759135 | 1769371 |
| ynhG | Complementary strand | 1759435 | 1760439 |
| sufE | Complementary strand | 1760588 | 1761004 |
| sufS | Complementary strand | 1761017 | 1762237 |
| sufD | Complementary strand | 1762234 | 1763505 |
| sufC | Complementary strand | 1763480 | 1764226 |
| sufB | Complementary strand | 1764236 | 1765723 |
| sufA | Complementary strand | 1765732 | 1766100 |
| rydB | Complementary strand | 1766427 | 1766494 |
| ydiH | Complementary strand | 1766648 | 1766917 |
| ydil | Complementary strand | 1766936 | 1767346 |

**[Table 1-44]**

| Gene name | Coding strand | Start number in SEQ ID NO:1 (stop number when encoded by complementary strand) | Stop number in SEQ ID NO:1 start number when encoded by complementary strand) |
|---|---|---|---|
| ydiJ | Complementary strand | 1767343 | 1770399 |
| ydiK | | 1770783 | 1771900 |
| rprA | | 1772080 | 1772190 |
| ydiL | | 1772329 | 1772685 |
| ydJM | | 1772785 | 1773999 |
| ydiN | | 1774226 | 1775491 |
| ydiB | | 1775503 | 1776369 |
| aroD | | 1776400 | 1777158 |
| ydiF | | 1777301 | 1778896 |
| ydiO | | 1778910 | 1780061 |
| ydiP | Complementary strand | 1780104 | 1781015 |
| ydiQ | | 1781331 | 1782095 |
| ydiR | | 1782115 | 1783053 |
| ydiS | | 1783109 | 1784398 |
| ydiT | | 1784395 | 1784688 |
| ydiD | | 1784691 | 1786391 |
| pps | Complementary strand | 1786448 | 1788826 |
| ydiA | | 1739159 | 1789992 |
| aroH | | 1790149 | 1791195 |
| ydiE | | 1791327 | 1791518 |
| ydiU | Complementary strand | 1791522 | 1792958 |
| ydiV | Complementary strand | 1793021 | 1793734 |
| nipC | Complementary strand | 1793981 | 1794445 |
| btuD | Complementary strand | 1794523 | 1795272 |
| btuE | Complementary strand | 1795272 | 1795823 |
| btuC | Complementary strand | 1795886 | 1796866 |
| ihfA | Complementary strand | 1796367 | 1797266 |
| pheT | Complementary strand | 1797271 | 1799658 |
| pheS | Complementary strand | 1799673 | 1800656 |
| pheM | Complementary strand | 1800940 | 1800984 |
| rpiT | Complementary strand | 1801107 | 1801463 |
| rpml | Complementary strand | 1801516 | 1801713 |
| infC | Complementary strand | 1801810 | 1802352 |
| thrS | Complementary strand | 1802356 | 1804284 |
| araB_1 | | 1804808 | 1805281 |
| arpB_2 | | 1805292 | 1806707 |
| yniD | | 1806864 | 1806986 |
| ydiY | Complementary strand | 1807039 | 1807757 |
| pfkB | | 1808084 | 1809013 |
| ydiZ | | 1809114 | 1809404 |

**[Table 1-45]**

| Gene name | Coding strand | Start number in SEQ ID NO: 1 (stop number when encoded by complementary strand) | Stop number in SEQ ID NO:1 (start number when encoded by complementary strand) |
|---|---|---|---|
| yniA | | 1809510 | 1810370 |
| yniB | Complementary strand | 1810411 | 1810947 |
| yniC | | 1811034 | 1811762 |
| ydjM | | 1811925 | 1812515 |
| ydjN | | 1812648 | 1814039 |
| ydjO | Complementary strand | 1814043 | 1814858 |
| cedA | Complementary strand | 1815135 | 1815398 |
| katE | | 1815581 | 1817842 |
| chbG | Complementary strand | 1818100 | 1818849 |
| chbF | Complementary strand | 1818862 | 1820214 |
| chbR | Complementary strand | 1820319 | 1821161 |
| chbA | Complementary strand | 1821169 | 1821519 |
| chbC | Complementary strand | 1821570 | 1822928 |
| chbB | Complementary strand | 1823013 | 1823333 |
| osmE | Complementary strand | 1823632 | 1823970 |
| nadE | | 1824112 | 1624999 |
| ydjQ | | 1825229 | 1826116 |
| ydjR | Complementary strand | 1826076 | 1826651 |
| spy | Complementary strand | 1826854 | 1827339 |
| astE | Complementary strand | 1827669 | 1828637 |
| astB | Complementary strand | 1828630 | 1829973 |
| astD | Complementary strand | 1829970 | 1831448 |
| astA | Complementary strand | 1831445 | 1832479 |
| astC | Complementary strand | 1832476 | 1833896 |
| xthA | | 1834142 | 1834948 |
| ydjX | | 1835115 | 1835825 |
| ydjY | | 1835830 | 1836507 |
| ydjZ | | 1836522 | 1837229 |
| ynjA | | 1837229 | 1837777 |
| ynjB | | 1837787 | 1838953 |
| ynjC | | 1838971 | 1840461 |
| ynjD | | 1840461 | 1841114 |
| ynjE | | 1841181 | 1842488 |
| ynjF | Complementary strand | 1842497 | 1843117 |
| nudG | | 1843204 | 1843611 |
| ynjH | Complementary strand | 1843577 | 1843849 |
| gdhA | | 1844085 | 1845428 |
| ynjl | Complementary strand | 1845545 | 1846585 |
| topB | Complementary strand | 1846713 | 1848674 |
| selD | Complementary strand | 1848679 | 1849722 |

**[Table 1-46]**

| Gene name | Coding strand | Start number in SEQ ID NO: 1 (stop number when encoded by complementary | Stop number in SEQ ID NO:1 (start number when encoded by strand) complementary strand) |
|---|---|---|---|
| ydJA | Complementary strand | 1849839 | 1860390 |
| sppA | | 1850551 | 1852401 |
| ansA | | 1852574 | 1653590 |
| pncA | | 1853601 | 1854242 |
| ydjE | Complementary strand | 1854335 | 1855693 |
| ydjF | Complementary strand | 1855810 | 1856568 |
| ydjG | Complementary strand | 1856705 | 1857685 |
| ydjH | Complementary strand | 1857695 | 1858642 |
| ydjl | Complementary strand | 1858647 | 1859483 |
| ydjJ | Complementary strand | 1859504 | 1860547 |
| ydjK | Complementary strand | 1660564 | 1861943 |
| ydjL | Complementary strand | 1861970 | 1863046 |
| yeaC | Complementary strand | 1863416 | 1863688 |
| yeaA | Complementary strand | 1863730 | 1864143 |
| gapA | | 1864485, | 1865480 |
| yeaD | | 1865564 | 1866448 |
| yeaE | Complementary strand | 1866496 | 1867350 |
| mipA | Complementary strand | 1867440 | 1868186 |
| yeaG | | 1868622 | 1870556 |
| yeaH | | 1870669 | 1871952 |
| yeal | | 1872099 | 1873574 |
| yeaJ | | 1873755 | 1378245 |
| yeaK | | 1875288 | 1875791 |
| yeal | Complementary strand | 1875792 | 1875896 |
| yeaL | | 1876066 | 1876512 |
| yeaM | Complementary strand | 1876469 | 1877290 |
| yeaN | | 1877387 | 1878568 |
| yeaO | | 1878623 | 1878970 |
| yeaF | Complementary strand | 1878992 | 1879246 |
| yeaP | | 1879429 | 1860454 |
| yeaQ | Complementary strand | 1880721 | 1880969 |
| yeaG | Complementary strand | 1881117 | 1881299 |
| yeaR | Complementary strand | 1881303 | 1881662 |
| yeaS | Complementary strand | 1881835 | 1882473 |
| yeaT | Complementary strand | 1882600 | 1883523 |
| yeaU | | 1883626 | 1884711 |
| yeaV | | 1884902 | 1886347 |
| yeaW | | 1886379 | 1887503 |
| yeaX | | 1887559 | 1888524 |
| rnd | Complementary strand | 1888578 | 1889705 |

**[Table 1-47]**

| Gene name | Coding strand | Start number in SEQ ID NO:1 (stop number when encoded by complementary strand) | Stop number in SEQ ID NO:1 (start number when encoded by complementary strand) |
|---|---|---|---|
| fadD | Complementary strand | 1889775 | 1891460 |
| yeaY | Complementary strand | 1891665 | 1892246 |
| yeaZ | Complementary strand | 1892286 | 1892981 |
| yoaA | Complementary strand | 1893039 | 1894949 |
| yoaB | | 1895081 | 1895425 |
| yoaC | | 1895787 | 1896146 |
| yoaH | Complementary strand | 1896266 | 1896445 |
| pabB | | 1896519 | 1897880 |
| yeaB | | 1897884 | 1898462 |
| sdaA | | 1898646 | 1900010 |
| yoaD | | 1900141 | 1901739 |
| yoaE | Complementary strand | 1901743 | 1903289 |
| manX | | 1903762 | 1904733 |
| manY | | 1904796 | 1905596 |
| manZ | | 1905600 | 1906460 |
| yobD | | 1906515 | 1906973 |
| yebN | | 1907402 | 1907968 |
| rrmA | Complementary strand | 1907965 | 1906774 |
| cspC | Complementary strand | 1908940 | 1909149 |
| yobF | Complementary strand | 1909162 | 1909305 |
| yobO | Complementary strand | 1909975 | 1910262 |
| yobG | Complementary strand | 1910337 | 1910480 |
| yobH | | 1910639 | 1910876 |
| kdgR | Complementary strand | 1911022 | 1911813 |
| yebQ | | 1911990 | 1913363 |
| htpX | Complementary strand | 1913409 | 1914290 |
| prc | Complementary strand | 1914482 | 1916530 |
| proQ | Complementary strand | 1916550 | 1917248 |
| yebR | Complementary strand | 1917345 | 1917896 |
| yebS | | 1917972 | 1919255 |
| yebT | | 1919224 | 1921857 |
| yebU | | 1921937 | 1923376 |
| yebV | | 1923494 | 1923730 |
| yebW | | 1923835 | 1924026 |
| pphA | Complementary strand | 1924027 | 1924683 |
| ryeA | | 1924780 | 1925028 |
| ryeB | Complementary strand | 1924878 | 1924998 |
| yebY | Complementary strand | 1925079 | 1925420 |
| yebZ | Complementary strand | 1925433 | 1926305 |
| yobA | Complementary strand | 1926309 | 1926683 |

**[Table 1-48]**

| Gene name | Coding strand | Start number in SEQ ID NO:1 (stop number when encoded by complementary strand) | Stop number in SEQ ID NO:1 (start number when encoded by complementary strand) |
|---|---|---|---|
| holE | | 1926822 | 1927052 |
| yobB | | 1927154 | 1927810 |
| exoX | | 1927834 | 1928496 |
| ptrB | Complementary strand | 1928493 | 1930553 |
| yebE | Complementary strand | 1939762 | 1931421 |
| yebF | Complementary strand | 1931748 | 1932116 |
| YebG | Complementary strand | 1932171 | 1932461 |
| purT | | 1932595 | 1933773 |
| eda | Complementary strand | 1933829 | 1934470 |
| edd | | 1934507 | 1936318 |
| zwf | Complementary strand | 1936553 | 1938028 |
| yebK | | 1938366 | 1939235 |
| pykA | | 1939363 | 1940805 |
| lpxM | Complementary strand | 1940936 | 1941907 |
| yebA | Complementary strand | 1942027 | 1943349 |
| znuA | Complementary strand | 1943365 | 1944297 |
| znuC | | 1944376 | 1945131 |
| znuB | | 1945128 | 1945913 |
| ruvB | Complementary strand | 1946060 | 1947070 |
| ruvA | Complementary strand | 1947079 | 1947690 |
| yebB | | 1947965 | 1948567 |
| ruvB | Complementary strand | 1948569 | 1949090 |
| yabC | Complementary strand | 1949125 | 1949865 |
| nudB | Complementary strand | 1949894 | 1950346 |
| aspS | Complementary strand | 1950464 | 1952236 |
| yecD | | 1952546 | 1953112 |
| yecE | | 1953109 | 1993927 |
| yecN | | 1953980 | 1954375 |
| yecO | | 1954416 | 1955159 |
| yecP | | 1955156 | 1950127 |
| torZ | Complementary strand | 1956292 | 1958721 |
| torY | Complementary strand | 1958746 | 1959846 |
| cutC | Complementary strand | 1960234 | 1960980 |
| yecM | Complementary strand | 1960994 | 1961560 |
| argS | | 1961776 | 1963509 |
| yecT | | 1963686 | 1964174 |
| flhE | Complementary strand | 1964294 | 1964686 |
| flhA | Complementary strand | 1964686 | 1966764 |
| flhB | Complementary strand | 1966757 | 1967905 |
| cheZ | Complementary strand | 1968107 | 1968751 |

**[Table 1-49]**

| Gene name | Coding strand | Start number in SEQ ID NO:1 (stop number when encoded by complementary strand) | Stop number in SEQ ID NO:1 (start number when encoded by complementary strand) |
|---|---|---|---|
| cheY | Complementary strand | 1968762 | 1959151 |
| cheB | Complementary strand | 1963160 | 1970215 |
| cheR | Complementary strand | 1970218 | 1971078 |
| tap | Complementary strand | 1971091 | 1972698 |
| tar | Complementary strand | 1972744 | 1974405 |
| cheW | Complementary strand | 1974550 | 1975063 |
| cheA | Complementary strand | 1975074 | 1977038 |
| motB | Complementary strand | 1977043 | 1977969 |
| motA | Complementary strand | 1977966 | 1978853 |
| flhC | Complementary strand | 1978980 | 1979558 |
| flhD | Complementary strand | 1979561 | 1979920 |
| insH | Complementary strand | 1980355 | 1981371 |
| yecG | | 1981890 | 1982318 |
| otsA | Complementary strand | 1982325 | 19883749 |
| otsB | Complementary strand | 1983724 | 1984524 |
| araH | Complementary strand | 1984691 | 1985680 |
| araG | Complementary strand | 1985692 | 1987206 |
| araF | Complementary strand | 1987276 | 1988265 |
| yecl | | 1989062 | 1989565 |
| yecJ | Complementary strand | 1989644 | 1989895 |
| isrB | Complementary strand | 1989976 | 1990135 |
| yecR | | 1990359 | 1990682 |
| ftn | | 1990853 | 1991350 |
| yecH | Complementary strand | 1991388 | 1991627 |
| tyrP | | 1991818 | 1993029 |
| yecA | Complementary strand | 1993091 | 1993756 |
| leuZ | Complementary strand | 1993952 | 1994038 |
| cysT | Complementary strand | 1994051 | 1994124 |
| glyW | Complementary strand | 1994179 | 1994254 |
| pgsA | Complementary strand | 1994406 | 1994954 |
| uviC | Complementary strand | 1995011 | 1996843 |
| uvrY | Complementary strand | 1996840 | 1997496 |
| yecF | | 1997955 | 1998179 |
| sdiA | Complementary strand | 7998247 | 1998969 |
| yecC | Complementary strand | 1999199 | 1999951 |
| yecS | Complementary strand | 1999948 | 2000616 |
| yedO | complementary strand | | 2001617 |
| fliY | Complementary strand | 2001722 | 2002522 |
| fliZ | Complementary strand | 2002610 | 2003161 |
| fliA | Complementary strand | 2003207 | 2003936 |

**[Table 1-50]**

| Gene name | Coding strand | Start number in SEQ ID NO:1 (stop number when encoded by complementary strand) | Stop number in SEQ ID NO:1 (start number when encoded by complementary strand) |
|---|---|---|---|
| fliC | Complementary strand | 2004247 | 2005743 |
| fliD | | 2006006 | 2007415 |
| fliS | | 2007440 | 2007850 |
| fliT | | 2007850 | 2008215 |
| amyA | | 2008293 | 2009780 |
| yedD | Complementary strand | 2009814 | 2010227 |
| yedE | | 2010414 | 2011619 |
| yedF | | 2011616 | 2011649 |
| yedK | | 2011958 | 2012626 |
| yedL | | 2012737 | 2013216 |
| yedN_2 | Complementary strand | 2013485 | 2013676 |
| yedN_1 | | 20131566 | 2014006 |
| yedM | Complementary strand | 2014138 | 2014188 |
| intG | | 2014639 | 2014917 |
| fliE | Complementary strand | 2014837 | 2015151 |
| fliF | | 2015366 | 2017024 |
| fliG | | 2017017 | 2018012 |
| fliH | | 2018005 | 2016691 |
| fliI | | 2018691 | 2020064 |
| fliJ | | 2020083 | 2020526 |
| fliK | | 2020523 | 2021650 |
| fliL | | 2021755 | 2022219 |
| filM | | 2022224 | 2023228 |
| fliN | | 2023225 | 2023638 |
| fliO | | 2023641 | 2024006 |
| fliP | | 2024006 | 2024743 |
| fliQ | | 2024753 | 2025022 |
| fliR | | 2025030 | 2025815 |
| rcsA | | 2026105 | 2020728 |
| dsrB | Complementary strand | 2026772 | 2026960 |
| yodD | | 2027123 | 2027350 |
| dsrA | Complementary strand | 2027364 | 2027450 |
| yedP | | 2027648 | 2028463 |
| yedQ | Complementary strand | 2028460 | 2030154 |
| yodC | Complementary strand | 2030325 | 2030507 |
| yedl | Complementary strand | 2030586 | 2031503 |
| yedA | | 2031676 | 2032596 |
| vsr | Complementary strand | 2032585 | 2033055 |
| dern | Complementary strand | 2033036 | 2034454 |
| yedJ | Complementary strand | 2034521 | 2035216 |

**[Table 1-51]**

| Gene nane | Coding strand | Start number in SEQ ID NO:1 (stop number when encoded by complementary strand) | Stop number in SEQ ID NO:1 (start number when encoded by complementary strand) |
|---|---|---|---|
| yedR | Complementary strand | 2035256 | 2035621 |
| yedS_1 | | 2036188 | 2036673 |
| yedS_2 | | 2036683 | 2036392 |
| yedS_3 | | 2036976 | 2037380 |
| hchA | | 2037972 | 2038823 |
| yedV | Complementary strand | 2038931 | 2040289 |
| yedW | Complementary strand | 2040289 | 2040960 |
| yedX | | 2041093 | 2041506 |
| yedY | | 2041615 | 2042619 |
| yedZ | | 2042620 | 2043255 |
| yodA | | 2o43512 | 2644162 |
| yodB | | 2044505 | 2045035 |
| serU | Complementary strand | 2045605 | 2045694 |
| yeel | | 2045788 | 2046585 |
| asnT | | 2046686 | 2046761 |
| yeeJ | | 2047048 | 2054151 |
| yelL_2 | Complementary strand | 2064413 | 2054739 |
| yeeL_1 | Complementary strand | 2054761 | 2055465 |
| shiA | | 2055780 | 2057096 |
| amn | | 2057198 | 2058652 |
| yeeN | | 2058995 | 2059711 |
| asnW | Complementary strand | 3060164 | |
| yeeO | Complementary strand | 2060340 | 2061983 |
| asnU | | 2061988 | 2062063 |
| cbl | Complementary strand | 2062101 | 2083051 |
| nac | Complementary strand | 2063153 | 2064070 |
| asnV | | 2064397 | 2064472 |
| erfK | Complementary strand | 2064528 | 2065450 |
| cobT | Complementary strand | 2065525 | 2068604 |
| cobS | Complementary strand | 2066616 | 2067359 |
| cobU | Complementary strand | 2067356 | 2067901 |
| insH-6 | Complementary strand | 2068442 | 2069458 |
| yoeA | | 2070772 | 2071164 |
| insD-3 | Complementary strand | 2071089 | 2071994 |
| insC-3 | Complementary strand | 2071952 | 2072362 |
| yoeE | | 2072381 | 2072641 |
| yeeP | | 2072923 | 2073348 |
| isrC | | 2073452 | 2073655 |
| flu | | 2073676 | 2076795 |
| yeeR | | 2076916 | 2078448 |

**[Table 1-52]**

| Gene name | Coding strand | Start number in SEQ ID NO:1 (stop number when encoded by complementary strand) | Stop number in SEQ ID NO:1 (start number when encoded by strand) complementary strand) |
|---|---|---|---|
| yeeS | | 2078445 | 2070891 |
| yeeT | | 2078954 | 2079176 |
| yeeU | | 2079249 | 2079617 |
| yeeV | | 2079706 | 2080080 |
| yeeW | | 2080077 | 2080271 |
| yoeF | | 2080712 | 2081068 |
| yeeX | Complementary strand | 2081169 | 2081564 |
| yeeA | Complementary strand | 2081670 | 2082728 |
| sbmC | Complementary strand | 2082926 | 2083399 |
| dacD | Complementary strand | 2083518 | 2084684 |
| sbcB | | 2084893 | 2086320 |
| yeeD | Complementary strand | 2086363 | 206S590 |
| yeeE | Complementary strand | 2086604 | 2087662 |
| yeeF | Complementary strand | 2087841 | 2083199 |
| yeeY | Complementary strand | 2039466 | 2090395 |
| yeeZ | Complementary strand | 2090441 | 2091265 |
| yoeB | Complementary strand | 2091348 | 2091802 |
| yefM | Complementary strand | 2091599 | 2091850 |
| hisL | | 2092133 | 2092183 |
| MsG | | 2092329 | 2093228 |
| hisD | | 2093234 | 2094538 |
| hisC | | 2094535 | 2095605 |
| hisB | | 2095605 | 2096672 |
| hisH | | 2096672 | 2097262 |
| hisA | | 2097262 | 2097999 |
| hisF | | 2097981 | 2098757 |
| hisl | | 2098751 | 2099362 |
| cld | Complementary strand | 2099458 | 2100438 |
| ugd | Complementary strand | 2100584 | 2101750 |
| gnd | Complementary strand | 2101999 | 2103405 |
| wbb_2 | Complementary strand | 2103533 | 2103814 |
| insH-7 | Complementary strand | 2104032 | 2105048 |
| wbbL_1 | Complementary strand | 2105053 | 2105526 |
| WbbK | Complementary strand | 2105528 | 2106646 |
| wbbJ | Complementary strand | 2106631 | 2107221 |
| wbbl | Complementary strand | 2107202 | 2100194 |
| wbbH | Complementary strand | 2108197 | 2109363 |
| glf | Complementary strand | 2109363 | 2110466 |
| rfbX | Complementary strand | 2110474 | 2111721 |
| rfbC | Complementary strand | 2111718 | 2112275 |

**[Table 1-53]**

| Gene name | Coding strand | Start number in SEQ ID NO:1 (stop number when encoded by complementary strand) | Stop number in SEQ ID NO:1 (start number when encoded by complementary strand) |
|---|---|---|---|
| rfbA | Complementary strand | 2112275 | 2113156 |
| rfbD | Complementary strand | 2113214 | 2114113 |
| rfbB | Complementary strand | 2114113 | 2115198 |
| galF | Complementary strand | 2115571 | 2116464 |
| wcaM | Complementary strand | 2116639 | 2118033 |
| wcaL | Complementary strand | 2118044 | 2119264 |
| wcaK | Complementary strand | 2119261 | 2120541 |
| wzxC | Complementary strand | 2120817 | 2122295 |
| wcaJ | Complementary strand | 2122297 | 2123691 |
| cpsG | Complementary strand | 2123746 | 2125116 |
| cpsB | Complementary strand | 2125221 | 2126657 |
| wcal | Complementary strand | 2126660 | 2127883 |
| nudD | Complementary strand | 2127880 | 2128359 |
| fcl | Complementary strand | 2128362 | 2129327 |
| gmd | Complementary strand | 2129330 | 2130451 |
| wcaF | Complementary strand | 2130477 | 2131025 |
| wcaE | Complementary strand | 2131041 | 2131787 |
| wcaD | Complementary strand | 2131798 | 2133015 |
| wcaC | Complementary strand | 2132990 | 2134207 |
| wcaB | Complementary strand | 2134204 | 2134692 |
| wcaA | Complementary strand | 2134695 | 2135534 |
| wzc | Complementary strand | 2135627 | 2137789 |
| wzb | Complementary strand | 137792 | 2138235 |
| wza | Complementary strand | 2138241 | 2139380 |
| yegH | | 2140039 | 2141622 |
| asmA | Complementary strand | 2141896 | 2143749 |
| dcd | Complementary strand | 2143771 | 2144352 |
| udk | Complementary strand | 2444444 | 2145085 |
| yegE | | 2145403 | 2148720 |
| alkA | Complementary strand | 2146829 | 2149677 |
| yegD | | 2149811 | 2151163 |
| yegl | Complementary strand | 2151176 | 2153122 |
| yegJ | | 2153322 | 2153783 |
| yegK | Complementary strand | 2153848 | 2154609 |
| yegL | Complementary strand | 2154606 | 2155265 |
| ryeC | | 2155412 | 2155560 |
| ryeD | | 2155747 | 2155889 |
| mdtA | | 2156153 | 2157400 |
| mdtB | | 2157400 | 2160522 |
| mdtC | | 2100523 | 2163600 |

**[Table 1-54]**

| Gene name | Coding strand | Start number in SEQ ID NO:1 (stop Number when encoded by complementary strand) | Stop number in SEQ ID NO:1 (start number when encoded by complementary strand) |
|---|---|---|---|
| mdtD | | 2163601 | 2165016 |
| baes | | 2165013 | 2166416 |
| baeR | | 2166413 | 2167135 |
| yegP | | 2167326 | 2167658 |
| yegQ | | 2167805 | 2100166 |
| ryeE | | 2109249 | 2169334 |
| cgrK | Complementary strand | 2169439 | 2169657 |
| yegZ | Complementary strand | 2169739 | 2169957 |
| yegR | Complementary strand | 2170126 | 2170443 |
| yegS | | 2170849 | 2171748 |
| gatR_1 | Complementary strand | 2171830 | 2172276 |
| insE-5 | | 2172364 | 2172672 |
| insF-5 | | 2172669 | 2173535 |
| gatR_2 | Complementary strand | 2173532 | 2173870 |
| gatD | Complementary strand | 2173970 | 2176010 |
| gatC | Complementary strand | 2175058 | 2176413 |
| gatB | Complementary strand | 2176417 | 2176701 |
| gatA | Complementary strand | 2176732 | 2178383 |
| inSH | | 2177051 | 2178031 |
| gatZ | Complementary strand | 2178393 | 2179655 |
| gatY | Complementary strand | 2179684 | 2180538 |
| fbaB | Complementary strand | 2180846 | 2161898 |
| yegT | | 2182155 | 2183432 |
| yegU | | 2183429 | 2184433 |
| yegV | | 2184430 | 2185395 |
| yegW | Complementary strand | 2185369 | 2186115 |
| yegX | Complementary strand | 2186167 | 2166985 |
| thiD | Complementary strand | 2187050 | 2187850 |
| thiM | Complementary strand | 2187847 | 2168636 |
| yohL | Complementary strand | 2188858 | 2189130 |
| yohM | | 2189251 | 2190075 |
| yohN | | 2190294 | 2190632 |
| yehA | Complementary strand | 2190714 | 2191748 |
| yehB | Complementary strand | 2191764 | 2194244 |
| yehC | Complementary strand | 2194260 | 2194979 |
| yehD | Complementary strand | 2195014 | 2195556 |
| yehE | Complementary strand | 2195849 | 2196130 |
| mrp | Complementary strand | 2196393 | 2197502 |
| metG | | 2197634 | 2199667 |
| molR_1 | | 2199808 | 2200632 |

**[Table 1-55]**

| Gene name | Coding strand | Start number in SEQ ID NO:1 (stop number when encoded by complementary strand) | Stop number in SEQ ID NO:1 (start number when encoded by complementary strand) |
|---|---|---|---|
| molR_2 | | 2200744 | 2202681 |
| molR_3 | | 2202656 | 2203603 |
| yehl | | 2203613 | 2207245 |
| yehK | | 2207306 | 2207623 |
| yehL | | 2207930 | 2209018 |
| yehM | | 2209029 | 2211303 |
| yehK | | 2211301 | 2212437 |
| yehQ | | 2212434 | 2214278 |
| yehP | | 2214559 | 2215020 |
| yehS | Complementary strand | 2215060 | 2215530 |
| yehT | Complementary strand | 2215577 | 2216296 |
| yehK | Complementary strand | 2216293 | 2217978 |
| mtrA | | 2218200 | 2218931 |
| yohO | | 22189991 | 2219098 |
| yehW | Complementary strand | 2219079 | 2219810 |
| yehX | Complementary strand | 2219815 | 2220741 |
| yehY | Complementary strand | 2220734 | 2221891 |
| | | | |
| yehZ | Complementary strand | 2221898 | 2222815 |
| bglX | Complementary strand | 2223026 | 2225323 |
| dld | | 2225519 | 2227234 |
| pbpG | Complementary strand | 2227272 | 2228204 |
| yohC | Complementary strand | 2228376 | 2228965 |
| yohD | | 2229135 | 2229713 |
| yohF | Complementary strand | 2229843 | 2230604 |
| york | Complementary strand | 2230657 | 2231745 |
| yohH | Complementary strand | 2231883 | 2232020 |
| dusC | Complementary strand | 2232772 | 2233719 |
| yehJ | | 2233958 | 2234356 |
| yohK | | 2234353 | 2235048 |
| cdd | | 2235178 | 2236082 |
| sanA | | 2236212 | 2236931 |
| yeiS | | 2236934 | 2237173 |
| yeiT | | 2237367 | 2238605 |
| yeiA | | 2238599 | 2239834 |
| mglC | Complementary strand | 2240077 | 2241087 |
| mglA | Complementary strand | 2241103 | 2242623 |
| mglb | Complementary strand | 2242684 | 2243682 |
| gals | Complementary strand | 2243962 | 2245002 |
| yeiB | Complementary strand | 2245144 | 2246301 |
| folE | Complementary strand | 2246318 | 2246986 |

**[Table 1-56]**

| Gene name | Coding strand | Start number in SEQ ID NO:1 (stop number when encoded by complementary stand) | Stop number in SEQ ID NO:1 (start number when encoded by complementary strand) |
|---|---|---|---|
| yelG | | 2247244 | 2248580 |
| cirA | Complementary strand | 2248112 | 2250103 |
| lysP | Complementary strand | 2260397 | 2251866 |
| yeiE | Complementary strand | 2262071 | 2262952 |
| yelh | | 2253051 | 2254100 |
| nfo | | 2254174 | 2255031 |
| yeil | | 2255034 | 2256122 |
| yeiJ | Complementary strand | 2256229 | 2257479 |
| rihB | Complementary strand | 2267579 | 2258620 |
| yeiL | | 2258689 | 2259348 |
| yeiM | Complementary strand | 2259419 | 2260669 |
| yeiN | Complementary strand | 2260763 | 2261701 |
| yeiC | Complementary strand | 2261689 | 2262630 |
| fruA | Complementary strand | 2263053 | 2264744 |
| fruK | Complementary strand | 2264761 | 2265699 |
| fruB | Complementary strand | 2265699 | 2266829 |
| setB | | 2267137 | 2268378 |
| yeiW | Complementary strand | 2268375 | 2268629 |
| yeiP | | 2268784 | 2269356 |
| yeiQ | | 2269579 | 2271045 |
| yeiR | | 2271183 | 2272149 |
| yeiU | | 2272188 | 2272901 |
| spr | | 2273313 | 227S879 |
| rtn | | 2274060 | 2275616 |
| yejA | | 2275698 | 2277512 |
| yejB | | 2277513 | 2278607 |
| yejE | | 2278607 | 2279632 |
| yejF | | 2279634 | 2281223 |
| yejG | Complementary strand | 2281227 | 2281571 |
| bcr | Complementary strand | 2281904 | 2283094 |
| rsuA | Complementary strand | 2283122 | 2283817 |
| yejH | | 2283086 | 2285726 |
| rplY | | 2285851 | 2286135 |
| yejK | Complementary strand | 2286274 | 2287261 |
| yejL | | 2297463 | 2287690 |
| yejM | | 2287710 | 2289470 |
| proL | | 2289545 | 2289621 |
| yejO | Complementary strand | 2289724 | 2292234 |
| insH-8 | Complementary strand | 2292399 | 2293415 |
| narP | | 2293834 | 2294401 |

**[Table 1-57]**

| Gene name | Coding strand | Start number in SEQ ID. NO:1 (stop number when encoded by complementary strand) | Stop number in SEQ ID NO:1 (start number when encoded by complementary strand] |
|---|---|---|---|
| ccmH | Complementary strand | 2294692 | 2295744 |
| ccmG | Complementary strand | 2295741 | 2296298 |
| ccmF | Complementary strand | 2296295 | 2298238 |
| ccmE | Complementary strand | 2298235 | 2298714 |
| ccmD | Complementary strand | 229871 | 2298930 |
| ccmC | Complementary strand | 2298917 | 2299654 |
| ccmB | Complementary strand | 2299696 | 2300358 |
| ccmA | Complementary strand | 2300355 | 2300978 |
| napC | Complementary strand | 2300991 | 2301593 |
| napB | Complementary strand | 2301603 | 2302052 |
| napH | Complementary strand | 2302049 | 2302912 |
| napG | Complementary strand | 2302899 | 2303594 |
| napA | Complementary strand | 2303601 | 2306087 |
| napD | Complementary strand | 2306084 | 2306347 |
| napF | Complementary strand | 2306337 | 2306831 |
| eco | | 2307239 | 2307727 |
| mqo | Complementary strand | 2308442 | 2310088 |
| yojl | Complementary strand | 2310306 | 2311949 |
| alkB | Complementary strand | 2312025 | 2312675 |
| ada | Complementary strand | 2312675 | 2313739 |
| yojL | Complementary strand | 2313813 | 2314868 |
| ompC | Complementary strand | 2314980 | 2316083 |
| micF | | 2316418 | 2316510 |
| rcsD | | 2316822 | 2319494 |
| rcsB | | 2319511 | 2320161 |
| rcsC | Complementary strand | 2320361 | 2324498 |
| insD | Complementary strand | 2320805 | 2321728 |
| insC | Complementary strand | 2321668 | 2322033 |
| atoS | | 2324713 | 2326539 |
| atoC | | 2326536 | 2327921 |
| atoD | | 2328117 | 2328779 |
| atoA | | 2328779 | 2329429 |
| atoE | | 2329426 | 2330748 |
| atoB | | 2330779 | 2331963 |
| yfaP | Complementary strand | 2332037 | 2332813 |
| yfaQ | Complementary strand | 2332818 | 2334467 |
| yfaS_2 | Complementary strand | 2334468 | 2334953 |
| yfaS_1 | Complementary strand | 2334969 | 2339072 |
| yfaT | Complementary strand | 2339006 | 2339629 |
| yfaA | Complementary strand | 2339626 | 2341314 |

**[Table 1-58]**

| Gene name | Coding strand | Start number in SEQ ID NO.1 (stop number when encoded by complementary strand) | Stop number in SEQ ID NO:1 (start number when encoded by complementary strand) |
|---|---|---|---|
| gyrA | Complementary strand | 2341463 | 2344090 |
| ubiG | | 2344237 | 2344959 |
| yfaL | Complementary strand | 2345087 | 2348839 |
| nrdA | | 2349539 | 2351820 |
| nrdB | | 2352054 | 2353184 |
| yfaE | | 2353184 | 2353438 |
| inaA | Complementary strand | 2353492 | 2354142 |
| yfaH | | 2354357 | 2354563 |
| glpQ | Complementary strand | 2364605 | 2355681 |
| glpT | Complementary strand | 2356666 | 2357044 |
| glpA | | 2357317 | 2358945 |
| glpB | | 2358935 | 2360194 |
| glpC | | 2360191 | 2361381 |
| yfaD | | 2361574 | 2362473 |
| ypaA | | 2362496 | 2362671 |
| yfaU | Complementary strand | 2362712 | 2363515 |
| yfaV | Complementary strand | 2363533 | 2364822 |
| yfaW | Complementary strand | 2364879 | 2366096 |
| yfaX | Complementary strand | 2386099 | 2366881 |
| yfaY | Complementary strand | 2367101 | 2368303 |
| rfaZ | Complementary strand | 2368403 | 2368945 |
| yfaO | | 2369224 | 2369649 |
| ais | Complementary strand | 2369688 | 2370290 |
| yfbE | | 2370580 | 2371737 |
| yfbF | | 2371741 | 2372709 |
| yfbG | | 2372709 | 2374691 |
| yfbH | | 2374688 | 2375578 |
| amT | | 2375578 | 2377230 |
| yfbW | | 2377227 | 2377562 |
| yfbJ | | 2377562 | 2377948 |
| pmrD | Complementary strand | 2377942 | 2378208 |
| menE | Complementary strand | 2378318 | 2379673 |
| menC | Complementary strand | 2379670 | 2380632 |
| menB | Complementary strand | 2380632 | 2381489 |
| yfbB | Complementary strand | 2381504 | 2382262 |
| menD | Complementary strand | 2382259 | 2383929 |
| menF | Complementary strand | 2384018 | 2385313 |
| elaB | Complementary strand | 2385392 | 2385697 |
| elaA | Complementary strand | 2385752 | 2386213 |
| elaC | | 2386278 | 2387195 |

**[Table 1-59]**

| Gene name | Coding strand | Start number in SEQ ID NO:1 (stop number when encoded by complementary strand) | Stop number in SEQ In NO:1 (start number wheat encoded by complementary strand) |
|---|---|---|---|
| elaD | | 2387383 | 2388594 |
| yfbK | Complementary strand | 3368665 | 2390392 |
| yfbL | | 2390530 | 2391501 |
| yfbM | | 2391604 | 2392107 |
| yfbN | Complementary strand | 2392380 | 2393096 |
| yfbO | | 2393251 | 2393727 |
| yfbP | | 2393783 | 2394634 |
| nuoN | Complementary strand | 2394718 | 3395996 |
| nuoM | Complementary strand | 2396182 | 2397711 |
| nuoL | Complementary strand | 2397875 | 2399716 |
| nuoK | Complementary strand | 2399713 | 2400015 |
| nuoJ | Complementary strand | 2400012 | 2400566 |
| nuol | Complementary strand | 2400578 | 2401120 |
| nuoH | Complementary strand | 2401135 | 2402112 |
| nuoG | Complementary strand | 2402109 | 2404841 |
| nuoF | Complementary strand | 2404886 | 2406225 |
| nuoE | Complementary strand | 2406222 | 2406722 |
| nuoC | Complementary strand | 2406725 | 2408527 |
| nuoB | Complementary strand | 2408621 | 2409283 |
| nuoA | Complementary strand | 2409299 | 2409742 |
| lrhA | Complementary strand | 2410373 | 2411311 |
| insB | Complementary strand | 2411473 | 2411976 |
| insA | Complementary strand | 2411895 | 2412170 |
| yfbQ | | 2413007 | 2414224 |
| yfbR | | 2414308 | 2414907 |
| yfbS | Complementary strand | 2414966 | 2416798 |
| yfbT | Complementary strand | 2416865 | 2417535 |
| yfbU | Complementary strand | 2417546 | 2418040 |
| yfbV | Complementary strand | 2418123 | 2418578 |
| ackA | | 2418916 | 2420118 |
| pta | | 2420193 | 2422337 |
| yfcC | | 2422527 | 242400 |
| yfcD | Complementary strand | 2424080 | 2424622 |
| yfoE | Complementary strand | 2424680 | 2425234 |
| yfcF | Complementary strand | 2425287 | 2425931 |
| yfcG | | 2428067 | 2426714 |
| folX | | 2426771 | 2427133 |
| yfoH | | 2427154 | 2428047 |
| yfcl | Complementary strand | 2428095 | 2428985 |
| hisP | Complementary strand | 2429182 | 2429955 |

**[Table 1-60]**

| Gene name | Coding strand | Start number in SEQ ID No:1 (stop number when encoded by complementary strand) | Stop number in SEQ ID NO:1 (start number ID when encoded by complementary strand) |
|---|---|---|---|
| hisM | Complementary strand | 2429963 | 2430679 |
| hisQ | Complementary strand | 2430676 | 2431362 |
| hisJ | Complementary strand | 2431452 | 2432234 |
| argT | Complementary strand | 2432465 | 2433237 |
| ubiX | Complementary strand | 2433503 | 2434072 |
| purF | Complementary strand | 2434167 | 24-35684 |
| cvpA | Complementary strand | 2435721 | 2436209 |
| dedD | Complementary strand | 2436468 | 2437130 |
| folC | Complementary strand | 2437120 | 2438388 |
| accD | Complementary strand | 2438458 | 2439372 |
| dedA | Complementary strand | 2439528 | 2440187 |
| truA | Complementary strand | 2440370 | 2441082 |
| usg | Complementary strand | 2441082 | 2442095 |
| pdxB | Complementary strand | 2442161 | 2443297 |
| flk | | 2443396 | 2444391 |
| yfoJ | Complementary strand | 2444388 | 244 5566 |
| fabB | Complementary strand | 2445831 | 2447051 |
| trmC | | 2447210 | 2449216 |
| yfcL | Complementary strand | 2449337 | 2448615 |
| yfcM | Complementary strand | 2449649 | 2460197 |
| fcA | Complementary strand | 2450197 | 2451006 |
| mepA | Complementary strand | 2451006 | 2451830 |
| aroC | Complementary strand | 2451834 | 2452910 |
| prmB | Complementary strand | 2452954 | 2453886 |
| YfcN | | 2454052 | 2454603 |
| yfcO | Complementary strand | 2454674 | 2455495 |
| yfcP | Complementary strand | 2455497 | 2456036 |
| yfcQ | Complementary strand | 2456033 | 2456521 |
| yfcR | Complementary strand | 2456518 | 2457030 |
| yfcS | Complementary strand | 2457030 | 2457782 |
| yfcT | Complementary strand | 2457802 | 2458698 |
| yfcU | Complementary strand | 2458711 | 2460447 |
| yfcV | Complementary strand | 2460529 | 2461092 |
| siXA | Complementary strand | 2461773 | 2462258 |
| yfcX | Complementary strand | 2462461 | 2464605 |
| yfcY | Complementary strand | 2464605 | 2465915 |
| yfcZ | Complementary strand | 2466096 | 2466380 |
| fadL | | 2466746 | 2468092 |
| yfdF | | 2468458 | 2469516 |
| vacJ | Complementary strand | 2469698 | 2470453 |

**[Table 1-61]**

| Gene name | Coding strand | Start number in SEQ ID NO : 1 (stop number when encoded by complementary strand) | Stop number in SEQ ID NO :1 (start number when encoded by complementary strand) |
|---|---|---|---|
| yfdC | | 2470747 | 2471679 |
| argW | | 2471755 | 2471829 |
| intS | | 2471991 | 2473148 |
| yfdG | | 2473301 | 2473663 |
| yfdH | | 2473660 | 2474580 |
| yfdl | | 2474577 | 2475908 |
| tfaS | | 2476249 | 2476551 |
| yfdK | Complementary stand | 2476523 | 2476963 |
| yfdL | Complementary strand | 2476990 | 2477508 |
| yfdM | Complementary strand | 2477558 | 2477833 |
| yfdN | Complementary strand | 2477833 | 2478327 |
| yfdO | Complementary strand | 2478324 | 2478692 |
| yfdP | | 2478966 | 2479412 |
| yfdQ | | 2479478 | 2480302 |
| yfdR | | 2480430 | 2480966 |
| yfdS | | 2480957 | 2481319 |
| yfdT | | 2481319 | 2481624 |
| ypdJ | | 2481540 | 2481680 |
| torl | | 2481756 | 2481956 |
| sdC | Complementary strand | 2482140 | 2483075 |
| dsdX | | 2483293 | 2484630 |
| dsdA | | 2484648 | 2485976 |
| emrY | Complementary strand | 2486084 | 2487622 |
| emrK | Complementary strand | 2487622 | 2488785 |
| evgA | | 2489201 | 2489815 |
| evgS | | 2489820 | 2493413 |
| yfdE | Complementary strand | 2493469 | 2494614 |
| yfdV | Complementary strand | 2494688 | 2495632 |
| oxc | Complementary strand | 2495702 | 2497396 |
| frc | Complementary strand | 2497450 | 2498700 |
| yfdX | Complementary strand | 2499213 | 2499848 |
| ypdl | | 2500144 | 2500419 |
| yfdY | Complementary strand | 2500496 | 2500738 |
| ddg | | 2501091 | 2502011 |
| yfdZ | Complementary strand | 2502503 | 2503741 |
| ypdA | | 2504117 | 2505814 |
| ypdB | | 2505829 | 2506563 |
| ypdC | | 2506576 | 2507433 |
| ypdD | Complementary strand | 2507436 | 2509931 |
| ypdE | Complementary strand | 2509956 | 2510993 |

**[Table 1-62]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ypdF | Complementary strand | 2510993 | 2512078 |
| ypdG | Complementary strand | 2512093 | 2513340 |
| ypdH | Complementary strand | 2513362 | 2513688 |
| glk | Complementary strand | 2513907 | 2514872 |
| yfeO | | 2515076 | 2516332 |
| ypeC | | 2516447 | 2516773 |
| mntH | Complementary strand | 2516914 | 2518152 |
| nupC | | 2518488 | 2519690 |
| insL-3 | | 2519777 | 2520889 |
| yfeA | Complementary strand | 2521089 | 2623278 |
| alaX | Complementary strand | 2523487 | 2523562 |
| alaW | Complementary strand | 2523602 | 2523677 |
| yfeC | | 2523913 | 2624257 |
| yfeD | | 2524259 | 2524651 |
| gltX | Complementary strand | 2524703 | 2526118 |
| valU | | 2526377 | 2526452 |
| valX | | 2526497 | 2626572 |
| valY | | 2526819 | 2526694 |
| lysV | | 2526699 | 2526774 |
| xapR | Complementary strand | 2527039 | 2527923 |
| xapB | Complementary strand | 2528175 | 2529431 |
| xapA | Complementary strand | 2529491 | 2530324 |
| yfeN | | 2530573 | 2531337 |
| yfeR | Complementary strand | 2531376 | 2532302 |
| yfeH | | 2532392 | 2533390 |
| ypeB | Complementary strand | 2533387 | 2533605 |
| llgA | Complementary strand | 2533607 | 2535622 |
| zipA | Complementary strand | 2535693 | 2536679 |
| cysZ | | 2536909 | 2537670 |
| cysK | | 2537855 | 2S38826 |
| ptsH | | 2539210 | 2539467 |
| ptsl | | 2539512 | 2541239 |
| crr | | 2541280 | 2541789 |
| pdxK | Complementary strand | 2541832 | 2542683 |
| yfeK | | 2542788 | 2543162 |
| yfeS | | 2543195 | 2543929 |
| cysM | Complementary strand | 2544118 | 2545029 |
| cysA | Complementary strand | 2545163 | 2546260 |
| cysW | Complementary strand | 2546250 | 2647125 |
| cysU | Complementary strand | 2547125 | 2547958 |

**[Table 1-63]**

| Gene name | Coding strand | Start number in SEQ ID NO:1 (stop number when encoded by complementary strand) | Stop number in SEQ ID NO:1 (start number when encoded by complementary strand) |
|---|---|---|---|
| cysP | Complementary strand | 2547958 | 2548974 |
| ucpA | Complementary strand | 2549278 | 2550069 |
| yfeT | Complementary strand | 2550198 | 2551055 |
| yfeU | | 2551219 | 2552115 |
| murP | | 2552119 | 2553543 |
| yfeW | | 2553548 | 2554852 |
| yfeX | Complementary strand | 2555092 | 2555991 |
| yfeY | Complementary strand | 2556087 | 2556662 |
| yfeZ | Complementary strand | 2556723 | 2557172 |
| ypeA | Complementary strand | 2557159 | 2557584 |
| amiA | | 2557798 | 2558667 |
| hemF | | 2558671 | 2559570 |
| yfeG | Complementary strand | 2559576 | 2560628 |
| yffl | Complementary strand | 2560674 | 2561174 |
| eutL | Complementart strand | 2561187 | 2561846 |
| eutC | Complementary strand | 3561856 | 2562743 |
| eutB | Complementary strand | 2562764 | 2564125 |
| eutA | Complementary strand | 2564137 | 2565540 |
| eutH | Complementary strand | 2565537 | 2566763 |
| eutG | Complementary strand | 2566980 | 2568167 |
| eutJ | Complementary strand | 2568157 | 2568993 |
| eutE | Complementary strand | 2569004 | 2570407 |
| cchB | Complementary strand | 2570419 | 2570706 |
| cchA | Complementary strand | 2570813 | 2571106 |
| eutl | Complementary strand | 2571145 | 2572161 |
| eutT | Complementary strand | 2572158 | 2572961 |
| eutQ | Complementary strand | 2572958 | 2573659 |
| eutP | Complementary strand | 2573634 | 2574113 |
| ypfE | Complementary strand | 2574126 | 2574461 |
| maeB | Complementary strand | 2674754 | 2577033 |
| talA | | 2577322 | 2578272 |
| tktB | | 2578292 | 2580295 |
| ypfG | Complementary strand | 2580390 | 2581433 |
| yffH | Complementary strand | 2581559 | 2582134 |
| aegA | Complementary strand | 2582202 | 2584181 |
| narQ | | 2584387 | 2586087 |
| acrD | | 2586251 | 2589364 |
| yffB | | 2589903 | 2590259 |
| dapE | | 2590263 | 2591390 |
| ypfN | | 2591418 | 2591618 |

**[Table 1-64]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ypfH | Complementary strand | 2591728 | 2592426 |
| ypfl | Complementary strand | 2592500 | 2594515 |
| ypfJ | Complementary strand | 2594530 | 2595393 |
| purC | Complementary strand | 2595561 | 2596274 |
| nlpB | Complementary strand | 2596487 | 2597521 |
| dapA | Complementary strand | 2597538 | 2598416 |
| gcvR | | 2598562 | 2599134 |
| bcp | | 2599134 | 2599604 |
| hyfA | | 2599857 | 2600474 |
| hyfB | | 2600474 | 2602492 |
| hyfC | | 2602503 | 2603450 |
| hyfD | | 2603467 | 2604906 |
| hyfE | | 2604918 | 2605568 |
| hyfF | | 2605573 | 2607153 |
| hyfG | | 2607143 | 2608810 |
| hyfH | | 2608820 | 2609365 |
| hyfl | | 2609362 | 2610120 |
| hyfJ | | 2610113 | 2610526 |
| hyfR | | 2610556 | 2612568 |
| focB | | 2612590 | 2613438 |
| yfgO | Complementary strand | 2613476 | 2614537 |
| yfgC | | 2614750 | 2616213 |
| yfgD | | 2616234 | 2616593 |
| hda | Complementary strand | 2616731 | 3617477 |
| uraA | Complementary strand | 2617527 | 2618816 |
| upp | Complementary strand | 2018902 | 2619528 |
| purM | | 2619853 | 2620890 |
| purN | | 2620890 | 2621528 |
| ppk | | 2621700 | 2623766 |
| ppx | | 2623771 | 2625312 |
| yfgF | Complementary strand | 2625351 | 2627594 |
| yfgG | | 2627946 | 2628137 |
| yfgH | | 2628448 | 2628966 |
| yfgl | | 2628982 | 2629521 |
| guaA | Complementary strand | 2629614 | 2631191 |
| guaB | Complementary strand | 2631260 | 2632736 |
| xseA | | 2632888 | 2634258 |
| yfgJ | Complementary strand | 2634255 | 2634470 |
| der | Complementary strand | 2634540 | 2636012 |
| yfgL | Complementary strand | 2636130 | 2637308 |

**[Table 1-65]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yfgM | Complementary strand | 2637319 | 2637939 |
| hisS | Complementary strand | 2637957 | 2639231 |
| ispG | Complementary strand | 2639342 | 2640460 |
| yfgA | Complementary strand | 2640487 | 2041500 |
| yfgB | Complementary strand | 2641785 | 2642939 |
| ndk | Complementary strand | 2643089 | 2643520 |
| pbpC | Complementary strand | 2643669 | 2645981 |
| yfhM | Complementary strand | 2645982 | 2650943 |
| sseA | | 2651150 | 2651995 |
| ryfA | | 2652511 | 2652814 |
| sseB | Complementary strand | 2652813 | 2653589 |
| pepB | | 2653731 | 2655014 |
| yfhJ | Complementary strand | 2655192 | 2655392 |
| fdx | Complementary strand | 2055404 | 2655739 |
| hscA | Complementary strand | 2655741 | 2657591 |
| hscB | Complementary strand | 2657608 | 2658123 |
| iscA | Complementary strand | 2658219 | 2658542 |
| iscU | Complementary strand | 2658559 | 2658945 |
| iscS | Complementary strand | 2658973 | 2660187 |
| iscR | Complementary strand | 2660299 | 2660787 |
| yfhQ | Complementary strand | 2661239 | 2661979 |
| suhB | | 2662098 | 2662901 |
| yfhR | | 2663019 | 3663900 |
| csiE | | 2664091 | 3S66371 |
| hcaT | Complementary strand | 2665363 | 2666502 |
| hcaR | Complementary strand | 2666662 | 2667552 |
| hcaE | | 2667688 | 2669049 |
| hcaF | | 2669046 | 2669564 |
| hcaC | | 2669564 | 2669884 |
| hcaB | | 2669881 | 2670693 |
| hcaD | | 2670703 | 2671905 |
| yphA | | 2671930 | 2672424 |
| yphB | Complementary strand | 2672472 | 2673344 |
| yphC | Complementary strand | 2673356 | 2674417 |
| yphD | Complementary strand | 2674483 | 2615481 |
| yphE | Complementary strand | 2675506 | 2677017 |
| yphF | Complementary strand | 2677040 | 2678023 |
| yphG | Complementary strand | 2676120 | 2681401 |
| yphH | | 2681519 | 2682712 |
| glyA | Complementary strand | 2682910 | 2684163 |

**[Table 1-66]**

| Gene name | Coding strand | Start number in SEQ ID NO:1 (stop number when encoded by complementary | Stop number in SEQ ID NO:1 (start number when encoded by strand) complementary strand) |
|---|---|---|---|
| hmp | | 2684491 | 2685681 |
| glnB | Complementary strand | 3685726 | 2686064 |
| yfhA | Complementary strand | 2686125 | 2687459 |
| yfhG | Complementary strand | 2687449 | 2688162 |
| yfhk | Complementary strand | 2688327 | 2689754 |
| sroF | Complementary strand | 2689848 | 2689996 |
| purL | Complementary strand | 2690312 | 2694199 |
| yfhD | | 2694595 | 2696013 |
| tadA | Complementary strand | 2696010 | 2696546 |
| yfhB | Complementary strand | 2696571 | 2697206 |
| yfhH | | 2637415 | 2698263 |
| yfhL | | 2698319 | 2698579 |
| acpS | Complementary strand | 2699274 | 2699654 |
| pdxJ | Complementary strand | 2699654 | 2700385 |
| recO | Complementary strand | 2700397 | 2701125 |
| era | Complementary strand | 2701137 | 2702042 |
| rnc | Complementary strand | 2702039 | 2702719 |
| lepB | Complementary strand | 2702991 | 2703965 |
| lepA | Complementary strand | 2703981 | 2705780 |
| rseC | Complementary strand | 2705978 | 2706457 |
| rseB | Complementary strand | 2706454 | 2707410 |
| rseA | Complementary strand | 2707410 | 2708060 |
| rpoE | Complementary strand | 2708093 | 2708668 |
| nadB | | 2709076 | 2710698 |
| yfiC | Complementary strand | 2710683 | 3711420 |
| srmB | | 2711552 | 2712886 |
| yfiE | Complementary strand | 2713095 | 2713976 |
| yfiK | | 2714079 | 2714666 |
| yfiD | Complementary strand | 2714722 | 2715105 |
| ung | | 2715410 | 2716099 |
| yfiF | Complementary strand | 2716147 | 2717184 |
| trxC | | 2717391 | 2717810 |
| yfiP | | 2717879 | 2718577 |
| yfiQ | | 2718609 | 2721269 |
| pssA | | 2721383 | 2722738 |
| yfiM | | 2722835 | 2723107 |
| kgtP | Complementary strand | 2723104 | 2724402 |
| rrfG | Complementary strand | 2724725 | 2724844 |
| rriG | Complementary strand | 2724937 | 2727840 |
| gltW | Complementary strand | 2728025 | 2728100 |

**[Table 1-67]**

| Gene name | Coding strand | Start number in SEQ ID NO:1 (stop number when encoded by complementary strand) | Stop number in SEQ ID NO:1 (start number when encoded by complementary strand) |
|---|---|---|---|
| rrsG | Complementary strand | 2728272 | 2729813 |
| clpB | Complementary strand | 2730256 | 2732829 |
| yfiH | Complementary strand | 2732959 | 2733690 |
| rluD | Complementary strand | 2733687 | 2734667 |
| yfiO | | 2734802 | 2735539 |
| yfiA | | 2735810 | 2736151 |
| pheL | | 2736255 | 2736302 |
| pheA | | 2736401 | 2737561 |
| tyrA | Complementary strand | 2737604 | 2738725 |
| aroF | Complementary strand | 2738736 | 2739806 |
| yfiL | | 2740016 | 2740381 |
| yfiR | | 2740531 | 2741049 |
| yfiN | | 2741039 | 2742265 |
| yfiB | | 2742281 | 2742763 |
| rplS | Complementary strand | 2742839 | 2743186 |
| trmD | Complementary strand | 2743228 | 2743995 |
| rimM | Complementary strand | 2744026 | 2744574 |
| rpsP | Complementary strand | 2744593 | 2744841 |
| ffh | Complementary strand | 2745090 | 2746451 |
| ypjD | | 2746543 | 2747409 |
| yfjD | | 2747520 | 2748716 |
| grpE | Complementary strand | 2748771 | 2749364 |
| yfjB | | 2749487 | 2750365 |
| recN | | 2750451 | 2752112 |
| smpA | | 2752261 | 2752602 |
| yfjF | Complementary strand | 2752664 | 2752954 |
| yfjG | Complementary strand | 2752944 | 2753420 |
| smpB | | 2753552 | 2754034 |
| ssrA | | 2754249 | 2754611 |
| intA | | 2754815 | 2756056 |
| yfjH | Complementary strand | 2756300 | 2757256 |
| alpA | | 2757300 | 2757512 |
| yfjl | | 2757641 | 2759050 |
| yfjJ | | 2759203 | 2759829 |
| yrjK | Complementary strand | 2760007 | 2762196 |
| yfjL | Complementary strand | 2762193 | 2763809 |
| yfjM | Complementary strand | 2764169 | 2764432 |
| yfjN | | 2764574 | 2765647 |
| yfjO | | 2765640 | 2766011 |
| yfjP | | 2766366 | 2767229 |

**[Table 1-68]**

| Gene name | Coding strand | Start number in SEQ ID NO: (stop number when encoded by complementary strand) | Stop number in SEQ ID NO:1 (start number when snooded by complementary strand] |
|---|---|---|---|
| yfjQ | | 2757321 | 2768142 |
| yfjR | | 2766359 | 2769090 |
| ypjK | | 2769101 | 2769337 |
| yfjS | | 2769337 | 2769780 |
| yfjT | | 2769804 | 2770271 |
| yfjU | Complementary strand | 2770496 | 2770810 |
| ypjL | Complementary strand | 2770823 | 2771341 |
| yfjV | Complementary strand | 2771492 | 2771692 |
| YpjM | Complementary strand | 2771632 | 2771814 |
| yfjW | | 2771974 | 2773677 |
| yfjX | | 2774575 | 2775033 |
| yfjY | | 2775042 | 2775524 |
| ypjJ | | 2775533 | 2775733 |
| yfjZ | | 2775771 | 2776088 |
| ypjF | | 2776109 | 2776438 |
| ypjA | Complementary strand | 2776802 | 2781382 |
| pinH | Complementary strand | 2781721 | 2781864 |
| ypjB | Complementary strand | 2782294 | 2783085 |
| ypjC | Complementary strand | 2783185 | 2783667 |
| ileY | Complementary strand | 2784418 | 2784493 |
| ygaQ | | 2785053 | 2785385 |
| ygaR | | 2785404 | 2786090 |
| yqaC | | 2786298 | 2786894 |
| yqaD | | 2787033 | 2787305 |
| ygaT | | 2787641 | 2788618 |
| ygaF | | 2788638 | 2789906 |
| gabD | | 2789929 | 2791377 |
| gabT | | 2791391 | 2792671 |
| gabP | | 2792909 | 2794309 |
| csiR | | 2794330 | 2794992 |
| ygaU | Complementary strand | 2794993 | 2795442 |
| yqaE | Complementary strand | 2795526 | 2795684 |
| ygaV | | 2795867 | 2796166 |
| ygaP | | 2796176 | 2796700 |
| stpA | Complementary strand | 2796747 | 2797151 |
| ygaW | | 2797820 | 2798269 |
| ygaC | Complementary strand | 2798306 | 2798650 |
| ygaM | | 2796790 | 2799131 |
| nrdH | | 2799379 | 2799624 |
| nrdl | | 2199621 | 2800031 |

**[Table 1-69]**

| Gene name | Coding strand | Start number in SEQ No: 1 (stop number when encode complementary strand) | Stop number in SEQ ID NO:1 (start number when encoded by complementary strand) |
|---|---|---|---|
| nrdE | | 2800004 | 2802148 |
| nrdF | | 2802158 | 2803117 |
| proV | | 2803471 | 2804673 |
| proW | | 3804666 | 2805730 |
| proX | | 2805788 | 2806780 |
| ygaX | | 2806972 | 2807238 |
| ygaY | | 2807268 | 2808149 |
| ygaZ | | 2808273 | 2809010 |
| ygaH | | 2609000 | 2809335 |
| mprA | | 2809426 | 3809996 |
| emrA | | 2810083 | 2811255 |
| emrB | | 2811272 | 2812810 |
| luxS | Complementary strand | 2812874 | 2813389 |
| sraD | | 2813457 | 2813531 |
| gshA | Complementary strand | 2813539 | 2815095 |
| yqaA | Complementary strand | 2815168 | 2815596 |
| yqaB | Complementary strand | 2815593 | 2816159 |
| argQ | Complementary strand | 2816440 | 2816516 |
| argZ | Complementary strand | 2816715 | 2816791 |
| argY | Complementary strand | 2816854 | 2816930 |
| argV | Complementary strand | 2817129 | 2817205 |
| serV | Complementary strand | 2617209 | 2817301 |
| csrA | Complementary strand | 28176H7 | 2817802 |
| alaS | Complementary strand | 2818037 | 2820667 |
| recX | Complementary strand | 2820795 | 2821295 |
| recA | Complementary strand | 2821364 | 2622425 |
| ygaD | Complementary strand | 2822505 | 2823002 |
| mltB | Complementary strand | 2823147 | 2824232 |
| srlA | | 2824488 | 2825051 |
| srlE | | 2825048 | 2626007 |
| srlB | | 2826018 | 2826389 |
| srlD | | 2626393 | 2827172 |
| gutM | | 2827277 | 2827636 |
| srlR | | 2827703 | 2826476 |
| gutQ | | 2828469 | 2829434 |
| norR | Complementary strand | 2829431 | 2830945 |
| norV | | 2831132 | 2832571 |
| norW | | 2832568 | 2833701 |
| hypF | Complementary strand | 2833829 | 2836081 |
| hydN | Complementary strand | 2836234 | 2836761 |

**[Table 1-70]**

| Gene name | Coding strand | Start number in SEQ ID NO:1 (stop number when encoded by Complementary strand) | Stop number in SEQ ID NO:1 (start number when encoded by complementary strand) |
|---|---|---|---|
| ascG | Complementary strand | 2836910 | 2837923 |
| ascF | | 2838180 | 2839637 |
| ascB | | 2839646 | 2841070 |
| hycl | Complementary strand | 2841229 | 2841699 |
| hycH | Complementary strand | 2841682 | 2842102 |
| hycG | Complementary strand | 2842099 | 2842866 |
| hycB | Complementary strand | 2842866 | 2843408 |
| hycE | Complementary strand | 2643418 | 2845127 |
| hycD | Complementary strand | 2845145 | 2849068 |
| hycC | Complementary strand | 2846071 | 2847897 |
| hycB | Complementary strand | 2947834 | 2848005 |
| hycA | Complementary strand | 2848630 | 2849091 |
| hypA | | 2849303 | 2849653 |
| hypB | | 2849657 | 285D529 |
| hypC | | 2850520 | 2850792 |
| hypD | | 2850792 | 2851913 |
| hypE | | 2851952 | 2852920 |
| fhlA | | 2852994 | 2855072 |
| ygbA | Complementary strand | 2855109 | 2855462 |
| mutS | | 2855749 | 2858310 |
| pphB | | 2658416 | 2859072 |
| ygbl | Complementary strand | 2859123 | 2859920 |
| ygbJ | | 2860086 | 2860994 |
| ygbK | | 2860991 | 2862157 |
| ygbL | | 2662249 | 2862887 |
| ygbM | | 2862892 | 2863668 |
| ygbN | | 2863757 | 2865121 |
| rpoS | Complementary strand | 2865215 | 2866090 |
| nlpD | Complementary strand | 2866270 | 2867409 |
| pcm | Complementary strand | 2867549 | 2868175 |
| surE | Complementary strand | 2868169 | 2868930 |
| truD | Complementary strand | 2868911 | 2869960 |
| IspF | Complementary strand | 2669957 | 2870436 |
| ispD | Complementary strand | 2870436 | 2871146 |
| ftsB | Complementary strand | 2871165 | 2871476 |
| ygbE | Complementary strand | 3871670 | 2871993 |
| cysC | Complementary strand | 2872043 | 2872648 |
| cysN | Complementary strand | 2872648 | 2874075 |
| cysD | Complementary strand | 2674077 | 2874985 |
| lap | | 2875237 | 2876274 |

**[Table 1-71]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ygbF | Complementary strand | 2877225 | 2877509 |
| ygbT | Complementary strand | 2871511 | 2878428 |
| ygcH | Complementary strand | 2878444 | 2879043 |
| ygcl | Complementary strand | 2879030 | 2879704 |
| ygcJ | Complementary strand | 2879707 | 2880798 |
| ygcK | Complementary strand | 2880811 | 2881293 |
| ygcL | Complementary strand | 2881286 | 2882794 |
| ygcB | Complementary strand | 2883209 | 2885875 |
| cysH | Complementary strand | 2886234 | 2886968 |
| cysl | Complementary strand | 2887043 | 2888755 |
| cysJ | Complementary strand | 2885755 | 2890554 |
| ygcM | | 2890870 | 2891235 |
| ygcN | | 2891313 | 2892584 |
| ygcO | | 2892575 | 2892835 |
| ygcP | | 2692852 | 2893427 |
| ygcQ | Complementary strand | 2893675 | 2894435 |
| ygcR | Complementary strand | 2894432 | 2895211 |
| ygcS | Complementary strand | 2895189 | 2836526 |
| ygcU | Complementary strand | 2895620 | 2898074 |
| ygcW | Complementary strand | 2898144 | 2698929 |
| yqcE | | 2859248 | 2900525 |
| ygcE | | 2900652 | 2902030 |
| ygcF | Complementary strand | 2903403 | 2904074 |
| ygcG | | 2904367 | 2905239 |
| eno | Complementary strand | 2905299 | 2906597 |
| pyrG | Complementary strand | 2906685 | 2908322 |
| mazG | | 2908550 | 2909341 |
| chpA | Complementary strand | 2009412 | 2909747 |
| chpR | Complementary strand | 2909747 | 2909995 |
| relA | Complementary strand | 2910073 | 2912307 |
| rumA | Complementary strand | 2912355 | 2913656 |
| barA | | 2913713 | 2916469 |
| gudD | Complementary strand | 2916701 | 2918041 |
| gudX | Complementary strand | 2918062 | 2919402 |
| gudP | Complementary strand | 2919404 | 2920756 |
| yqcA | Complementary strand | 2921191 | 2921640 |
| yqcB | Complementary strand | 2921658 | 2922440 |
| yqcC | Complementary strand | 2922440 | 2922769 |
| csrB | Complementary strand | 2922812 | 2923171 |
| syd | Complementary strand | 2923391 | 2923936 |

**[Table 1-72]**

| Gene name | Coding strand | Start number in SEQ ID NO:1 (stop number when encoded by complementary strand) | Stop number in SEQ ID NO: 1 (start number when encoded by complementary strand) |
|---|---|---|---|
| yqcD | | 2924004 | 29248S2 |
| ygdH | | 2924964 | 2926328 |
| sdaC | | 2926885 | 2928174 |
| sdaB | | 2928232 | 2929599 |
| exo | | 2929621 | 2930466 |
| fucO | Complementary strand | 2930521 | 2931872 |
| fucA | Complementary strand | 2931697 | 2932344 |
| fucP | | 2932891 | 2934207 |
| fucl | | 2934240 | 2936015 |
| fucK | | 2936094 | 2937542 |
| fucU | | 2937544 | 2937966 |
| fucR | | 2936024 | 2938755 |
| ygdE | Complementary strand | 2938789 | 2939899 |
| ygdD | Complementary strand | 2939892 | 2940287 |
| gcvA | complementary strand | 2940306 | 2941223 |
| gcvB | | 2941352 | 2941556 |
| ygdl | Complementary strand | 2941574 | 2941801 |
| csdA | | 2941993 | 2943198 |
| ygdK | | 2943198 | 2943641 |
| ygdL | Complementary strand | 2943692 | 2944498 |
| mltA | Complementary strand | 2944737 | 2945834 |
| metZ | | 2946043 | 2946119 |
| metW | | 2946153 | 2946229 |
| metV | | 2946263 | 2946339 |
| amiC | Complementary strand | 2946413 | 2947666 |
| argA | | 2947698 | 2949229 |
| recD | Complementary strand | 2949391 | 2951117 |
| recB | Complementary strand | 2951117 | 2954659 |
| ptr | Complementary strand | 2954652 | 2957540 |
| recC | Complementary strand | 2957716 | 2961084 |
| ppdC | Complementary strand | 2961097 | 2961420 |
| ygdB | Complementary strand | 2961405 | 2961812 |
| ppdB | Complementary strand | 2961809 | 2962372 |
| ppdA | Complementary strand | 2962363 | 2962833 |
| thyA | Complementary strand | 2963017 | 2863811 |
| lgt | Complementary strand | 2963618 | 2964693 |
| ptsP | Complementary strand | 2964844 | 2967090 |
| nudH | Complementary strand | 2967103 | 2967633 |
| mutH | | 2968318 | 2969007 |
| ygdQ | | 2969076 | 2969789 |

**[Table 1-73]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ygdR | | 2969927 | 2970145 |
| tas | | 2970253 | 2971293 |
| ygeD | Complementary strand | 2971325 | 2972518 |
| aas | Complementary strand | 2972511 | 2974670 |
| rygA | Complementary strand | 2974758 | 2974845 |
| rygB | Complementary strand | 2974966 | 2975041 |
| galR | | 2975255 | 2976286 |
| lysA | Complementary strand | 2976293 | 2977555 |
| lysR | | 2977677 | 2973612 |
| ygeA | Complementary strand | 2978599 | 2979291 |
| araE | Complementary strand | 2979420 | 2980838 |
| kduD | Complementary strand | 3981153 | 2981914 |
| kdul | Complementary strand | 2981944 | 2982780 |
| yqeF | Complementary strand | 2983067 | 2964246 |
| yqeG | | 2984503 | 2985732 |
| yqeH | | 2986192 | 2986824 |
| yqel | | 2987158 | 2987967 |
| yqeJ | | 2987960 | 2988442 |
| yqeK | Complementary strand | 2988591 | 2989016 |
| ygeF | | 2989210 | 2989656 |
| ygeG | | 2989924 | 2990415 |
| ygeH | | 2990750 | 2992126 |
| ygel | | 2992294 | 2992512 |
| pbt | | 2992697 | 2993071 |
| ygeK | Complementary strand | 2993116 | 2993559 |
| ygeL | Complementary strand | 2983583 | 2993748 |
| ygeM | Complementary strand | 2993970 | 2994401 |
| ygeN | Complementary strand | 2994404 | 2994676 |
| ygeG | Complementary strand | 2994618 | 2995043 |
| insD-4 | Complementary strand | 2995028 | 2995933 |
| insC-4 | Complementary strand | 2995891 | 2996301 |
| ygeP | | 2996345 | 2996644 |
| ygeQ | Complementary strand | 2996690 | 2997484 |
| gtyU | Complementary strand | 2997640 | 2997713 |
| ygeR | Complementary strand | 2997792 | 2998547 |
| xdhA | | 2999001 | 3061259 |
| xdhB | | 3001270 | 3002148 |
| xdhC | | 3002145 | 3002624 |
| ygeV | Complementary strand | 3002664 | 3004442 |
| ygeW | | 3004918 | 3005108 |

**[Table 1-74]**

| Gene name | Coding strand | Start number in SEQ ID NO:1 (stop number when encoded by complementary strand) | Stop number in SEQ ID NO:1 (start number ID when encoded by complementary strand) |
|---|---|---|---|
| ygeX | | 3006166 | 3007362 |
| ygeY | | 3007420 | 3008631 |
| hyuA | | 3008684 | 3010069 |
| yqeA | | 3010117 | 3011049 |
| yqeB | Complementary strand | 3011270 | 3612895 |
| yqeC | Complementary strand | 3012943 | 3013713 |
| ygfJ | | 3013816 | 3044394 |
| ygfK | | 3014716 | 3017814 |
| ssnA | | 3017817 | 3019145 |
| ygfM | | 3019196 | 3019975 |
| xdhD | | 3019672 | 3022842 |
| ygfO | | 3023007 | 3024407 |
| guaD | | 3024422 | 3025741 |
| ygfQ | | 3025777 | 3027144 |
| ygfS | Complementary strand | 3027180 | 3027600 |
| ygfT | Complementary strand | 3027668 | 3029587 |
| ygfU | | 3030023 | 3031471 |
| idi | | 3031721 | 3032269 |
| lysS | Complementary strand | 3032313 | 3033830 |
| prfB | Complementary strand | 3033840 | 3034938 |
| recJ | Complementary strand | 3035029 | 3036762 |
| dsbC | Complementary strand | 3036768 | 3037478 |
| xerD | Complementary strand | 3037503 | 3038399 |
| fldB | | 3038611 | 3039032 |
| ygfX | Complementary strand | 3039072 | 3039479 |
| ygfY | Complementary strand | 3039460 | 3039726 |
| ygfZ | | 3039969 | 3040949 |
| yqfA | Complementary strand | 3041145 | 3041804 |
| yqfB | Complementary strand | 3041968 | 3042279 |
| bglA | | 3042318 | 3043757 |
| ygfF | Complementary strand | 3043814 | 3044557 |
| gcyP | Complementary strand | 3044834 | 3047697 |
| gcvH | Complementary strand | 3047816 | 3048205 |
| gcvT | Complementary strand | 3048229 | 3049323 |
| visC | Complementary strand | 3049771 | 3050973 |
| ubiH | Complementary strand | 3050996 | 3052174 |
| pepP | Complementary strand | 3052171 | 3053496 |
| VgfB | Complementary strand | 3053522 | 3054100 |
| zapA | | 3054268 | 3054597 |
| ssrS | | 3054639 | 3054821 |

**[Table 1-75]**

| Gene name | Coding strand | Start: number in SEQ NO:1 (stop number Coding when encoded by complementary strand) | Stop number in SEQ ID NO:1 (start number when encoded by complementary strand) |
|---|---|---|---|
| ygfA | | 3054897 | 3065445 |
| rygC | | 3055471 | 3055621 |
| serA | Complementary strand | 3055834 | 3057066 |
| rpiA | Complementary strand | 3057322 | 3057981 |
| yqfE | Complementary strand | 3058037 | 3058267 |
| argP | | 3058409 | 3059302 |
| yliK | | 3059506 | 3061650 |
| argK | | 3061643 | 3062638 |
| ygfG | | 3062649 | 3063434 |
| ygfH | | 3063458 | 3064936 |
| ygfl | Complementary strand | 306033 | 3065829 |
| yggE | Complementary strand | 3065996 | 3060736 |
| argO | | 3066829 | 3067464 |
| mscS | | 3067603 | 3068463 |
| fbaA | Complementary strand | 3068821 | 3069900 |
| pgk | Complementary strand | 3970115 | 3071278 |
| epd | Complementary strand | 3071328 | 3072347 |
| yggC | Complementary strand | 3072632 | 3073345 |
| yggD | Complementary strand | 3073342 | 3073851 |
| yggF | Complementary strand | 3073873 | 3074838 |
| yggP | Complementary strand | 3074835 | 3076112 |
| cmtA | Complementary strand | 3076127 | 3077515 |
| cmtB | Complementary strand | 3077843 | 3077986 |
| tktA | Complementary strand | 3078300 | 3080291 |
| yggG | | 3080569 | 3081327 |
| speB | Complementary strand | 3081533 | 3082453 |
| speA | Complementary strand | 3082591 | 3084567 |
| yqgB | Complementary strand | 3084576 | 3064722 |
| yqgC | | 3084843 | 3085058 |
| yqgD | Complementary strand | 3085055 | 3085306 |
| metK | | 3085362 | 3086516 |
| galP | | 3086940 | 3088334 |
| sprT | | 3088411 | 3088908 |
| endA | | 3069003 | 3089710 |
| yggJ | | 3089790 | 3090521 |
| gshB | | 3090534 | 3091484 |
| yqgE | | 3091593 | 3092156 |
| yqgF | | 3092156 | 3092572 |
| yggR | Complementary strand | 3092756 | 3093736 |
| yggS | | 3093754 | 3094458 |

**[Table 1-76]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yggT | | 3094476 | 3095042 |
| yggU | | 3095039 | 3095329 |
| yggV | | 3095337 | 3095930 |
| yggW | | 3095923 | 3097059 |
| yggM | Complementary strand | 3097214 | 3098221 |
| ansB | Complementary strand | 3098338 | 3099384 |
| yggN | Complementary strand | 3099560 | 3100279 |
| yggL | Complementary strand | 3100463 | 3100789 |
| yggH | Complementary strand | 3100789 | 3101508 |
| mutY | | 3101669 | 3102721 |
| yggX | | 3102749 | 3103024 |
| mltC | | 3103089 | 3104168 |
| nupG | | 3104370 | 3105626 |
| speC | Complementary strand | 3105676 | 3107811 |
| yqgA | | 3108209 | 3108916 |
| pheV | | 3109022 | 3109097 |
| yghD | | 3109246 | 3109782 |
| yghE | Complementary strand | 3109784 | 3110644 |
| yghF | Complementary strand | 3110710 | 3111576 |
| yghG | Complementary strand | 3111723 | 3112133 |
| pppA | Complementary strand | 3112199 | 3113008 |
| yghJ | Complementary strand | 3113206 | 3117768 |
| yghK | Complementary strand | 3118253 | 3119935 |
| glcB | Complementary strand | 3120290 | 3122461 |
| glcG | Complementary strand | 3122483 | 3122887 |
| glcF | | 3122892 | 3124115 |
| glcE | Complementary strand | 3124126 | 3125178 |
| glcD | Complementary strand | 3125178 | 3126677 |
| glcC | Complementary strand | 3126928 | 3127692 |
| yghO | Complementary strand | 3127699 | 3128871 |
| insH-9 | | 3128834 | 3129850 |
| yghQ | Complementary strand | 3129997 | 3131064 |
| YghR | Complementary strand | 3131110 | 3131868 |
| yghS | Complementary strand | 3131900 | 3132813 |
| yghT | | 3132787 | 3133479 |
| pltB | Complementary strand | 3133528 | 3135027 |
| gss | Complementary strand | 3135319 | 3137178 |
| yghU | | 3137383 | 3138249 |
| hybG | Complementary strand | 3138372 | 3138620 |
| hybF | Complementary strand | 3138633 | 3138974 |

**[Table 1-77]**

| Gene name | Coding strand | Start number in SEQ ID NO:1 (stop number when encoded by complementary strand) | Stop number in SEQ ID NO:1 (start number when encoded by complementary strand) |
|---|---|---|---|
| hybE | Complementary strand | 3138967 | 3139455 |
| hybD | Complementary strand | 3139448 | 3139942 |
| hybC | Complementary strand | 3139942 | 3141645 |
| hybB | Complementary strand | 3141642 | 3142820 |
| hybA | Complementary strand | 3142810 | 3143796 |
| hybO | Complementary strand | 3143799 | 3144917 |
| yghW | Complementary strand | 3145106 | 3145393 |
| yghX | Complementary strand | 3145512 | 3145922 |
| yghY | Complementary strand | 3145922 | 3146275 |
| yghZ | | 3146553 | 3147593 |
| yqhA | Complementary strand | 3147633 | 3148127 |
| yghA | | 3148318 | 3149202 |
| exbD | Complementary strand | 3149474 | 3149899 |
| exbB | Complementary strand | 3149906 | 3150640 |
| metC | | 3150892 | 3152079 |
| yghB | | 3152219 | 3152878 |
| yqhC | Complementary strand | 3152918 | 3153874 |
| yqhD | | 3154011 | 3155174 |
| dkgA | | 3155279 | 3156106 |
| yqhG | | 3156306 | 3157232 |
| yqhH | | 3157283 | 3157540 |
| ygiQ | Complementary strand | 3157583 | 3159802 |
| sufl | Complementary strand | 3159913 | 3161325 |
| plsC | Complementary strand | 3161400 | 3162137 |
| parC | Complementary strand | 3162371 | 3164629 |
| ygiS | Complementary strand | 3164767 | 3166374 |
| ygiT | Complementary strand | 3166507 | 3166902 |
| ygiU | Complementary strand | 3166904 | 3167200 |
| ygiV | Complementary strand | 3167405 | 3167887 |
| ygiW | Complementary strand | 3167940 | 3168332 |
| qseB | | 3168484 | 3169143 |
| qseC | | 3169140 | 3170489 |
| ygiZ | Complementary strand | 3170535 | 3170867 |
| mdaB | | 3171186 | 3171767 |
| ygiN | | 3171798 | 3172112 |
| parE | Complementary strand | 3172160 | 3174052 |
| yqiA | Complementary strand | 3174081 | 3174662 |
| cpdA | Complementary strand | 3174662 | 3175489 |
| yqiB | Complementary strand | 3175514 | 3175936 |
| nudF | Complementary strand | 3175937 | 3176566 |

**[Table 1-78]**

| Gene name | Coding strand | Start number in SEQ ID-NO:1 (stop number when encoded by complementary strand) | Stop number in SEQ ID NO:1 (Start number when encoded by complementary strand) |
|---|---|---|---|
| tolC | | 3176771 | 3178252 |
| yglA | | 3178252 | 3178512 |
| yglB | | 3178496 | 3179071 |
| ygiC | | 3179077 | 3180237 |
| zupT | Complementary strand | 3180275 | 3181090 |
| ygiE | | 3181206 | 3181979 |
| ribB | Complementary strand | 3182469 | 3183122 |
| yqiC | | 3183496 | 3183786 |
| ygiL | | 3184070 | 3184621 |
| insC-5 | | 3184798 | 3185208 |
| insD-5 | | 3185166 | 3186071 |
| yqiG | | 3186056 | 3188521 |
| yqiH | | 3188537 | 3189286 |
| yqil | | 3189288 | 3190352 |
| glgS | Complementary strand | 3190395 | 3190595 |
| yqiJ | | 3190864 | 3191493 |
| yqiK | | 3191520 | 3193181 |
| rygD | Complementary strand | 3193407 | 3193556 |
| rfaE | Complementary strand | 3193976 | 3195409 |
| glnE | Complementary strand | 3195457 | 3198297 |
| ygiF | Complementary strand | 3198320 | 3199621 |
| htrG | | 3199863 | 3200483 |
| cca | | 3200547 | 3201785 |
| bacA | Complementary strand | 3201966 | 3202787 |
| folB | Complementary strand | 3202877 | 3203245 |
| ygiH | | 3203350 | 3203967 |
| yglP | Complementary strand | 3203980 | 3204912 |
| ttdA | | 3205119 | 3206030 |
| ttdB | | 3206027 | 3206632 |
| ygjE | | 3206680 | 3208143 |
| ygjD | Complementary strand | 3208186 | 3209199 |
| rpsU | | 3209437 | 3209652 |
| dnaG | | 3209763 | 3211508 |
| rpoD | | 3211703 | 3213544 |
| ygjF | Complementary strand | 3213623 | 3214129 |
| ilex | | 3214254 | 3214329 |
| yqjH | Complementary strand | 3214383 | 3215147 |
| yqjl | | 3215435 | 3216058 |
| aer | Complementary strand | 3216212 | 3217732 |
| ygjG | | 3218123 | 3219529 |

**[Table 1-79]**

| Gene name | Coding strand | Start number in SEQ ID-NO:1 (stop number when encoded by complementary strand) | Stop number in SEQ ID NO:1 (Start number when encoded by complementary strand) |
|---|---|---|---|
| ygjH | Complementary strand | 3219571 | 3219903 |
| ebgR | | 3220122 | 3221105 |
| ebgA | | 3221289 | 3224381 |
| ebgC | | 3224378 | 3224827 |
| ygjl | | 3224890 | 3226323 |
| ygjJ | | 3226457 | 3227527 |
| ygjK | | 3227544 | 3229895 |
| fadH | | 3230321 | 3232339 |
| ygjM | Complementary strand | 3232384 | 3232800 |
| ygjN | Complementary strand | 3232797 | 3233111 |
| ygjO | Complementary strand | 3233395 | 3234531 |
| ygjP | | 3234616 | 3235119 |
| ygjQ | | 3235196 | 3235888 |
| ygjR | | 3235949 | 3236953 |
| sraF | | 3237030 | 3237217 |
| alx | | 3237236 | 3238201 |
| sstT | | 3238600 | 3239844 |
| ygjV | Complementary strand | 3239849 | 3240400 |
| uxaA | Complementary strand | 3240483 | 3241970 |
| uxaC | Complementary strand | 3241985 | 3243397 |
| exuT | | 324376D | 3245178 |
| exuR | | 3245308 | 3246084 |
| yqjA | | 3246429 | 3247091 |
| yqjB | | 3247095 | 3247478 |
| yqjC | | 3247625 | 3247993 |
| yqjD | | 3248031 | 3248336 |
| yqjE | | 3248339 | 3248743 |
| yqjK | | 3248733 | 3249032 |
| yqjF | | 3249218 | 3249610 |
| yqjG | | 3249680 | 3250666 |
| yhaH | | 3250960 | 3251325 |
| yhal | | 3251567 | 3251923 |
| yhaJ | Complementary strand | 3251974 | 3252870 |
| yhaK | | 3252975 | 3253676 |
| yhaL | | 3253699 | 3253863 |
| yhaM | Complementary strand | 3253997 | 3255307 |
| yhaO | Complementary strand | 3255335 | 3256603 |
| tdcG | Complementary strand | 3256941 | 3258305 |
| tdcF | Complementary strand | 3258377 | 3258766 |
| tdcE | Complementary strand | 3258780 | 3261074 |

**[Table 1-80]**

| Gene name | Coding strand | Start number in SEQ ID NO:1 (stop number when encoded by complementary strand) | Stop number in SEQ ID NO:1 (start number when encoded by complementary strand) |
|---|---|---|---|
| insH | | 3261202 | 3262182 |
| tdcD | Complementary strand | 3262220 | 3263515 |
| tdcC | Complementary strand | 3263541 | 3264872 |
| tdcB | Complementary strand | 3264894 | 3265833 |
| tdcA | Complementary strand | 3265982 | 3266920 |
| tdcR | | 3267235 | 3267453 |
| YhaB | | 3267709 | 3268248 |
| yhaC | | 3268270 | 3269457 |
| rnpB | Complementary strand | 3270071 | 3270447 |
| garK | Complementary strand | 3270480 | 3271706 |
| garR | Complementary strand | 3271722 | 3272612 |
| garL | Complementary strand | 3272642 | 3273412 |
| garP | Complementary strand | 3273428 | 3274762 |
| garD | | 3275137 | 3276708 |
| sohA | | 3276857 | 3277192 |
| yhaV | | 3277192 | 3277656 |
| agaR | Complementary strand | 3277711 | 3278520 |
| kbaZ | | 3278769 | 3200049 |
| agaV | | 3280072 | 3280545 |
| agaW | | 3280556 | 3280957 |
| agaA | | 3280977 | 3281480 |
| agaS | | 3281831 | 3282985 |
| kbaY | | 3262998 | 3283858 |
| agaB | | 3284025 | 3284501 |
| agaC | | 3284540 | 3285343 |
| agaD | | 3285333 | 3286124 |
| agal | | 3286125 | 3286880 |
| yraH | | 3287281 | 3287865 |
| yraI | | 3287945 | 3288640 |
| yraJ | | 3288669 | 3391185 |
| yraK | | 3291196 | 3292287 |
| yraL | Complementary strand | 3292330 | 3293190 |
| yraM | | 3293255 | 3296291 |
| yraN | | 3295249 | 3295644 |
| yraO | | 3295664 | 3296254 |
| yraP | | 3296264 | 3296839 |
| yraQ | Complementary strand | 3296953 | 3297993 |
| yraR | Complementary strand | 3298066 | 3298701 |
| yhbO | | 3298829 | 3299347 |
| yhbP | Complementary strand | 3299327 | 3299770 |

**[Table 1-81]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yhbQ | | 3299821 | 3300123 |
| yhbS | Complementary strand | 3300110 | 3300613 |
| yhbT | Complementary strand | 3300607 | 3301131 |
| yhbU | | 3301340 | 3302335 |
| yhbV | | 3302344 | 3303222 |
| yhbW | | 3303303 | 3304310 |
| mtr | Complementary strand | 3304428 | 3305672 |
| deaD | Complementary strand | 3305826 | 3307715 |
| nlpl | Complementary strand | 3307895 | 3308779 |
| pnp | Complementary strand | 3308888 | 3311092 |
| sraG | | 3311080 | 3311253 |
| rpsO | Complementary strand | 3311270 | 3311539 |
| truB | Complementary strand | 3311688 | 3312632 |
| rbfA | Complementary strand | 3312632 | 3313033 |
| infB | Complementary strand | 3313197 | 3315869 |
| nusA | Complementary strand | 3315894 | 3317331 |
| yhbC | Complementary strand | 3317409 | 3317861 |
| metY | Complementary strand | 3318068 | 3318144 |
| argG | | 3318492 | 3319635 |
| yhbX | Complementary strand | 3319843 | 3321468 |
| leuU | Complementary strand | 3321927 | 3322013 |
| secG | Complementary strand | 3322028 | 3322360 |
| glmM | Complementary strand | 3322588 | 3323925 |
| folP | Complementary strand | 3323918 | 3324766 |
| ftsH | Complementary strand | 3324856 | 3326790 |
| rrmJ | Complementary strand | 3326890 | 3327519 |
| yhbY | | 3327645 | 3327938 |
| greA | Complementary strand | 3328094 | 3328570 |
| dacB | | 3328818 | 3330251 |
| obgE | Complementary strand | 3330437 | 3331609 |
| yhbE | Complementary strand | 3331625 | 3332590 |
| rpmA | Complementary strand | 3332717 | 3332974 |
| rplU | Complementary strand | 3332995 | 3333306 |
| ispB | | 3333965 | 3334536 |
| sfsB | | 3334764 | 3335042 |
| murA | Complementary strand | 3335090 | 3336349 |
| yrbA | Complementary strand | 3336404 | 3336658 |
| yrhB | Complementary strand | 3336818 | 3337111 |
| yrbC | Complementary strand | 3337111 | 3337746 |
| yrbD | Complementary strand | 3337765 | 3338316 |

**[Table 1-82]**

| Gene name | Coding strand | Start number in SEQ ID NO:1 (stop number when encoded by complementary) | Stop number in SEQ ID NO:1 (start number when encoded by complementary strand) |
|---|---|---|---|
| yrbE | Complementary strand | 3338321 | 3339103 |
| yrbF | Complementary strand | 3339111 | 3339920 |
| yrbG | | 3340130 | 3341107 |
| kdsD | | 3341121 | 3342107 |
| kdsC | | 3342128 | 3342694 |
| yrbK | | 3342691 | 3343266 |
| yhbN | | 3343235 | 3343792 |
| yhbG | | 3343799 | 3344524 |
| rpoN | | 3344572 | 3346005 |
| yhbH | | 3346028 | 3346315 |
| ptsN | | 3345433 | 3346924 |
| yhbJ | | 0346970 | 3347824 |
| npr | | 3347821 | 3348093 |
| yrbL | | 3348307 | 3348939 |
| mtgA | Complementary strand | 3348936 | 3348664 |
| elbB | Complementary strand | 3350314 | 3349661 |
| ryhA | | 3350432 | 3350539 |
| arcB | Complementary strand | 3350544 | 3352880 |
| yhcC | Complementary strand | 3352976 | 33S3905 |
| gltB | | 3354487 | 3359040 |
| gltD | | 3359053 | 3360471 |
| gltF | | 3361031 | 3361795 |
| yhcA | | 3361967 | 3363641 |
| yhcD | | 3363662 | 3365043 |
| yhcE_1 | | 3365040 | 3365519 |
| insH-10 | Complementary strand | 3365557 | 3366573 |
| yhcE_2 | | 3366536 | 3366784 |
| yhcF | | 3366781 | 3367497 |
| yhcG | | 3367682 | 3368809 |
| yhcH | Complementary strand | 3368869 | 3369333 |
| nanK | Complementary strand | 3369330 | 3370205 |
| nanE | Complementary strand | 3370202 | 3370891 |
| nanT | Complementary strand | 3370935 | 3372429 |
| nanA | Complementary strand | 3372538 | 3373431 |
| nanR | Complementary strand | 3373553 | 3374344 |
| dcuD | | 3374724 | 3376091 |
| sspB | Complementary strand | 3376134 | 3376631 |
| sspA | Complementary strand | 3376637 | 3377275 |
| rpsl | Complementary strand | 3377670 | 3378062 |
| rplM | Complementary strand | 3378078 | 3378506 |

**[Table 1-83]**

| Gene name | Coding strand | Start number in SEQ ID NO:1 (stop number when encoded by Complementary strand) | Stop number in SEQ ID NO:1 (start number when encoded by Complementary strand) |
|---|---|---|---|
| yhcM | Complementary strand | 3378725 | 3379852 |
| yhcB | | 3380046 | 3380444 |
| degQ | | 3380598 | 3381965 |
| degS | | 3382055 | 3383122 |
| mdh | Complementary strand | 3383185 | 3384123 |
| argR | | 3384558 | 3385028 |
| yhcN | | 3385393 | 3385656 |
| yhcO | Complementary strand | 3385712 | 3385984 |
| aaeB | Complementary strand | 3386076 | 3388043 |
| aaeA | Complementary strand | 3388049 | 3388981 |
| aaeX | Complementary strand | 3388989 | 3388192 |
| aaeR | | 3389375 | 3390304 |
| tldD | Complementary strand | 3390438 | 3391883 |
| yhdP | Complementary strand | 3392313 | 3396113 |
| rng | Complementary strand | 3388181 | 3397650 |
| yhdE | | 3397640 | 3398233 |
| mreD | Complementary strand | 3398242 | 3398730 |
| mreC | Complementary strand | 3398730 | 3399833 |
| mreB | Complementary strand | 3399899 | 3400942 |
| yhdA | Complementary strand | 3401247 | 3403187 |
| yhdH | | 3403339 | 3404313 |
| accB | | 3405291 | 3405761 |
| accC | | 3405772 | 3407121 |
| yhdT | | 3407230 | 3407472 |
| panF | | 3407462 | 3408913 |
| prmA | | 3403925 | 3409806 |
| dusB | | 3410135 | 3411100 |
| fis | | 3411126 | 4411422 |
| yhdJ | | 3.411508 | 3412392 |
| yhdU | | 3412476 | 3412655 |
| envR | Complementary strand | 3412658 | 3413320 |
| acrE | | 3413719 | 3414876 |
| acrF | | 3414888 | 3417992 |
| yhdV | | 3418245 | 3418466 |
| yhdW | | 3419004 | 3419921 |
| yhdX | | 3419989 | 3421170 |
| yhdY | | 3421180 | 3422283 |
| yhdZ | | 3422291 | 3423049 |
| rrfF | Complementary strand | 3423278 | 3423397 |
| thrV | Complementary strand | 3423435 | 3423510 |

**[Table 1-84]**

| Gene name | Coding strand | Start number in SEQ ID NO:1 (stop number when encoded by Complementary strand) | Stop number in SEQ ID NO:1 (start number when encoded by Complementary strand) |
|---|---|---|---|
| rrfD | Complementary strand | 3423523 | 3423642 |
| rrlD | Complementary strand | 3423735 | 3426638 |
| gltV | Complementary strand | 3426832 | 3426907 |
| rrsD | Complementary strand | 3426993 | 3428534 |
| purH | | 3429149 | 3430738 |
| purD | | 3430750 | 3432039 |
| zraR | Complementary strand | 3432036 | 3433361 |
| zraS | Complementary strand | 3433358 | 3434755 |
| zraP | | 3434993 | 3435418 |
| yjaH | Complementary strand | 3435420 | 3436115 |
| hupA | Complementary strand | 3436128 | 3436400 |
| yjaG | Complementary strand | 3436587 | 3437177 |
| nfi | Complementary strand | 3437220 | 3437891 |
| hemE | Complementary strand | 3437901 | 3438965 |
| nudC | Complementary strand | 3439005 | 3439778 |
| rsd | | 3439873 | 3440349 |
| thiC | | 3440582 | 3342477 |
| thiE | | 3442477 | 3443112 |
| thiF | | 3443105 | 3443860 |
| thiS | | 3443844 | 3444044 |
| thiG | | 3444046 | 3444816 |
| thiH | | 3444813 | 3445946 |
| htrC | Complementary strand | 3446356 | 3446895 |
| rpoC | Complementary strand | 3447108 | 3451331 |
| rpoB | Complementary strand | 3451408 | 3455436 |
| rplL | Complementary strand | 3455756 | 3450121 |
| rplJ | Complementary strand | 3456188 | 3456685 |
| rplA | Complementary strand | 3457098 | 3457802 |
| rplK | Complementary strand | 3457806 | 3458234 |
| nusG | Complementary strand | 3456393 | 3458938 |
| secE | Complementary strand | 3458940 | 3459323 |
| tufB | Complementary strand | 3459553 | 3400737 |
| thrT | Complementary strand | 3460852 | 3460927 |
| glyT | Complementary strand | 3460934 | 3461008 |
| tyrU | Complementary strand | 3461125 | 3461209 |
| thrU | Complementary strand | 3461218 | 3461293 |
| coaA | | 3461655 | 3462605 |
| birA | Complementary strand | 3462634 | 3463599 |
| murB | Complementary strand | 3463596 | 3464624 |
| rrfb | Complementary strand | 3464925 | 3465044 |

**[Table 1-85]**

| Gene name | Coding strand | Start number in SEQ ID NO:1 (stop number when encoded by complementary strand) | Stop number in SEQ ID NO:1 (start number when encoded by complementary strand) |
|---|---|---|---|
| rrlB | Complementary strand | 3465137 | 3468040 |
| gltT | Complementary strand | 3468234 | 3468309 |
| rrsB | Complementary strand | 3468481 | 3470022 |
| murl | Complementary strand | 3470396 | 3471253 |
| btuB | Complementary strand | 3471198 | 3473042 |
| trmA | | 3473411 | 3474511 |
| yijD | Complementary strand | 3474551 | 3474910 |
| fabR | Complementary strand | 3474910 | 3475614 |
| sthA | | 3475891 | 3477291 |
| oxyR | Complementary strand | 3477274 | 3478191 |
| oxyS | | 3478287 | 3478396 |
| argH | Complementary strand | 3476458 | 3479831 |
| argB | Complementary strand | 3479892 | 3480665 |
| argC | Complementary strand | 3480676 | 3481680 |
| argE | | 3481834 | 3482985 |
| ppc | | 3433583 | 3486234 |
| yijP | | 3486416 | 3488149 |
| yijO | | 3488364 | 3489215 |
| frwD | Complementary strand | 3489202 | 3489543 |
| pflC | Complementary strand | 3489545 | 3490423 |
| pflD | Complementary strand | 3490389 | 3492686 |
| frwB | Complementary strand | 3492737 | 3493057 |
| frwC | Complementary strand | 3493072 | 3494151 |
| ptsA | | 3494460 | 3496961 |
| fsaB | | 3496973 | 3497635 |
| gldA | | 3497646 | 3498749 |
| yljF | | 3499024 | 3499641 |
| yijE | Complementary strand | 3499668 | 3500573 |
| katG | Complementary strand | 3500666 | 3502846 |
| metF | Complementary strand | 3503175 | 3504065 |
| metL | Complementary strand | 3504414 | 3506846 |
| metB | Complementary strand | 3508849 3 | 508009 |
| metJ | | 3508286 | 3508603 |
| yiiX | | 3508787 | 3509395 |
| rpmE | Complementary strand | 3509456 | 3509668 |
| priA | | 3509871 | 3512069 |
| cytR | | 3512225 | 3513250 |
| ftsN | | 3513342 | 3514301 |
| hslV | | 3514394 | 3514924 |
| hslU | | 3514934 | 3516265 |

**[Table 1-86]**

| Gene name | Coding strand | Start number in SEQ 10 NO:1 (stop number when encoded by complementary strand) | Stop number in SEQ ID NO:1 (start number when encoded by complementary strand |
|---|---|---|---|
| menA | | 3516332 | 3517258 |
| rraA | | 3517351 | 3617836 |
| yiiU | Complementary strand | 3517921 | 3518166 |
| glpF | | 3518591 | 3519436 |
| glpK | | 3519459 | 3520967 |
| glpX | | 3521102 | 3522112 |
| fpr | | 3522209 | 3522955 |
| yilT | Complementary strand | 3522960 | 3523388 |
| yiiS | Complementary strand | 3523415 | 3523714 |
| yiiR | Complementary strand | 3523926 | 3524366 |
| yilQ | | 3524467 | 3525066 |
| tpiA | | 3525174 | 3525941 |
| cdh | Complementary strand | 3525996 | 3526751 |
| sbp | Complementary strand | 3526858 | 3527847 |
| pfkA | Complementary strand | 3528167 | 3529129 |
| fieF | Complementary strand | 3529310 | 3530212 |
| cpxP | Complementary strand | 3530361 | 3530861 |
| cpxR | | 3531011 | 3531709 |
| cpxA | | 3531706 | 3533079 |
| yiiM | Complementary strand | 3633185 | 3533859 |
| kdgT | Complementary strand | 3S34008 | 3534991 |
| sodA | Complementary strand | 3535251 | 3535871 |
| rhaT | | 3536156 | 3637190 |
| rhaR | Complementary strand | 3537187 | 3538125 |
| rhaS | Complementary strand | 3538109 | 3538945 |
| rhaB | | 3539233 | 3540702 |
| rhaA | | 3540699 | 3541958 |
| rhaD | | 3542409 | 3543233 |
| yiiL | | 3643243 | 3643557 |
| frvA | | 3543858 | 3544304 |
| FrvB | | 3544315 | 3545766 |
| frvX | | 3545756 | 3546826 |
| frvR | | 3546826 | 3548574 |
| yiiG | Complementary strand | 3548624 | 3549679 |
| fdhD | Complementary strand | 3549832 | 3550665 |
| fdoG | | 3550859 | 3553909 |
| fdoH | | 3563922 | 3554824 |
| fdol | | 3554821 | 3555456 |
| fdhE | | 3555453 | 3556382 |
| yiiF | Complementary strand | 3556712 | 3556930 |

**[Table 1-87]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yiiE | Complementary strand | 3557172 | 3557390 |
| yiiD | Complementary strand | 3558243 | 3559232 |
| dtd | Complementary strand | 3559229 | 3559666 |
| rbn | Complementary strand | 3559663 | 3560535 |
| yihX | Complementary strand | 3560529 | 3561128 |
| yihW | Complementary strand | 3561227 | 3562012 |
| yihV | Complementary strand | 3562046 | 3562942 |
| yihU | | 3563110 | 3564006 |
| yihT | | 3564030 | 3564908 |
| yihS | | 3564925 | 3566166 |
| yihR | | 3566280 | 3567206 |
| yihQ | | 3567405 | 3569441 |
| yihP | | 3569466 | 3570872 |
| yihO | | 3570915 | 3572318 |
| ompL | | 3572386 | 3573078 |
| yihN | Complementary strand | 3573169 | 3574434 |
| yihM | Complementary strand | 3574536 | 3575516 |
| yihL | Complementary strand | 3575524 | 3576234 |
| bipA | Complementary strand | 3576451 | 3578274 |
| glnA | | 3578647 | 3580056 |
| glnL | | 3580342 | 3581391 |
| glnG | | 3581403 | 3582812 |
| hemN | Complementary strand | 3583263 | 3684636 |
| yihl | Complementary strand | 3584825 | 3585334 |
| csrC | Complementary strand | 3585401 | 3585645 |
| yihA | | 3585952 | 3586548 |
| spf | Complementary strand | 3586674 | 3586782 |
| polA | Complementary strand | 3586929 | 3589715 |
| yihG | | 3590079 | 3591011 |
| yihF | Complementary strand | 3591052 | 3592482 |
| dsbA | Complementary strand | 3592637 | 3593263 |
| yihE | Complementary strand | 3593280 | 3594266 |
| yihD | Complementary strand | 3594343 | 3594612 |
| mobA | | 3594682 | 3595266 |
| mobB | | 3595263 | 3595775 |
| rrfA | Complementary strand | 3596045 | 3696164 |
| rrlA | Complementary strand | 3596258 | 3599162 |
| alaT | Complementary strand | 3599346 | 3599421 |
| ileT | Complementary strand | 3S99464 | 3599540 |
| rrsA | Complementary strand | 3599609 | 3601150 |

**[Table 1-88]**

| Gene name | Coding strand | Start number in SEQ ID NO:1 (stop number when encoded by complementary) | Stop number in SEQ ID NO: 1 (start number when encoded by complementary strand) |
|---|---|---|---|
| hemG | Complementary strand | 3601528 | 3602073 |
| trkH | Complementary strand | 3602085 | 3600536 |
| yigZ | Complementary strand | 3603575 | 3604189 |
| pepQ | Complementary strand | 3604189 | 3605520 |
| fadB | | 3605710 | 3607899 |
| fadA | | 3607009 | 3609072 |
| fre | Complementary strand | 3609453 | 3610154 |
| ubiD | Complementary strand | 3610200 | 3611693 |
| rfaH | | 3611860 | 3612348 |
| tatD | Complementary strand | 3612345 | 3613127 |
| tatC | Complementary strand | 3613169 | 3613945 |
| tatB | Complementary strand | 3613848 | 3614463 |
| tatA | Complementary strand | 3614467 | 3614736 |
| ubiB | Complementary strand | 3614815 | 3616455 |
| yigP | Complementary strand | 3616452 | 3617057 |
| ubiE | Complementary strand | 1617071 | 3617326 |
| rmuC | Complementary strand | 3617921 | 3619348 |
| udp | Complementary strand | 3619489 | 3620250 |
| ysgA | | 3620512 | 3621327 |
| metE | Complementary strand | 3621367 | 3623628 |
| metR | | 3623865 | 3624818 |
| yigM | Complementary strand | 3624706 | 3625605 |
| yigL | Complementary strand | 3625681 | 3628481 |
| pidB | Complementary strand | 3626489 | 3627511 |
| rhtB | | 3627622 | 3626242 |
| rhtC | Complementary strand | 3628304 | 3628924 |
| recQ | Complementary strand | 3628988 | 3630817 |
| pldA | Complementary strand | 3630950 | 3631819 |
| yigl | | 3631984 | 3632451 |
| rarD | . | 3632503 | 3633393 |
| yigG | | 3633488 | 3633868 |
| yigF | | 3633882 | 3634262 |
| corA | Complementary strand | 3634305 | 3635255 |
| yigE | | 3535625 | 3636389 |
| uvrD | Complementary strand | 3636936 | 3638698 |
| yigB | Complementary strand | 3638782 | 3639498 |
| xerC | Complementary strand | 3639498 | 3640394 |
| yigA | Complementary strand | 3640391 | 3641098 |
| dapF | Complementary strand | 3641095 | 3641919 |
| yifL | Complementary strand | 3641956 | 3642159 |

**[Table 1-89]**

| Gene name | Coding strand | Start number: in SEQ NO: 1 (stop number when encoded by complementary strand) | Stop number in SEQ ID NO:1 (start number when encoded by complementary strand) |
|---|---|---|---|
| yzcX | Complementary strand | 3642346 | 3642831 |
| cyaY | | 36426224 | 3642942 |
| cyaA | Complementary strand | 3642982 | 3648528 |
| hemC | | 3645915 | 3646856 |
| hemD | | 364S853 | 3647593 |
| hemX | | 3647615 | 3648796 |
| hemY | | 3648799 | 3649995 |
| rylA | Complementary strand | 3650078 | 3650249 |
| aslA | | 3650674 | 3652329 |
| aslB | Complementary strand | 3652488 | 3653723 |
| proM | Complementary strand | 3653870 | 3853946 |
| leuT | Complementary strand | 3653989 | 3654075 |
| hisR | Complementary strand | 3654096 | 3654172 |
| argX | Complementary strand | 3654230 | 3654306 |
| yifK | Complementary strand | 3654409 | 3655794 |
| rffM | Complementary strand | 3655985 | 3658725 |
| wzyE | Complementary strand | 3656728 | 3658080 |
| rffT | Complementary strand | 3658077 | 3659156 |
| wzxE | Complementary strand | 3659153 | 3560409 |
| rffA | Complementary strand | 3660405 | 3861535 |
| rffC | Complementary strand | 3661540 | 3662085 |
| rffH | Complementary strand | 3662192 | 3663073 |
| rffG | Complementary strand | 3663092 | 3664159 |
| rffD | Complementary strand | 3664159 | 3665421 |
| rffE | Complementary strand | 3665418 | 3666548 |
| wzzE | Complementary strand | 3566604 | 3667650 |
| rfe | Complementary strand | 3667662 | 3668765 |
| rho | Complementary strand | 3669005 | 3670264 |
| rhoL | Complementary strand | 3670349 | 3670450 |
| trxA | Complementary strand | 3670591 | 3670920 |
| rhlB | | 3671051 | 3672316 |
| gpp | | 3672452 | 3673936 |
| rep | Complementary strand | 3673983 | 3676004 |
| yifN | | 3676221 | 3676439 |
| yifO | | 3676394 | 3876669 |
| ppiC | | 3676868 | 3677149 |
| ilvC | Complementary strand | 3677236 | 3678711 |
| ilvY | | 3678861 | 3679754 |
| ilvA | Complementary strand | 3679806 | 3681350 |
| ilvD | Complementary strand | 36B1353 | 3683203 |

**[Table 1-90]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ilvE | Complementary strand | 3683268 | 3684197 |
| ilvM | | 36M217 | 3684480 |
| ilVG_2 | Complementary strand | 3684477 | 3685058 |
| ilvG_1 | Complementary strand | 3885138 | 3686121 |
| ilvL | Complementary strand | 3686261 | 3686359 |
| yifB | | 3686682 | 3688232 |
| yifE | Complementary strand | 3688257 | 3680595 |
| hdfR | | 3686714 | 3689553 |
| trpT | Complementary strand | 3689649 | 3669724 |
| aspT | complementary strand | 3689733 | 3689809 |
| rrfC | Complementary strand | 3689862 | 3689981 |
| rrlC | Complementary strand | 3690074 | 3602077 |
| gltU | Complementary strand | 3693171 | 2693246 |
| rrsC | Complementary strand | 3693332 | 3694873 |
| yieP | | 3695354 | 3696046 |
| hsrA | | 3696069 | 3697496 |
| rbsR | Complementary strand | 3597462 | 3698454 |
| rbsK | Complementary strand | 3698458 | 3699387 |
| rbsB | Complementary strand | 3699513 | 3700403 |
| rbsC | complementary strand | 3700428 | 3701393 |
| rbsA | Complementary strand | 3701398 | 3702903 |
| rbsD | Complementary strand | 3102911 | 3703330 |
| trkD | Complementary strand | 3703497 | 3705365 |
| yieN | | 3705588 | 3707084 |
| yieM | | 3707078 | 3708529 |
| asnA | Complementary strand | 3708534 | 3709526 |
| asnC | | 3709678 | 3710136 |
| mioC | | 3710226 | 3710669 |
| oriC | Complementary strand | 3710706 | 3710937 |
| gidA | | 3711048 | 3712937 |
| gidB | | 3713001 | 3713624 |
| atpl | | 3714241 | 3714621 |
| atpB | | 3714630 | 3715445 |
| atpE | | 3715492 | 3715731 |
| atpF | | 3715793 | 3716263 |
| atpH | | 3716278 | 3716811 |
| atpA | | 3716824 | 3716365 |
| atpG | | 3718416 | 3719279 |
| atpD | | 3719306 | 3720688 |
| atpC | | 3720709 | 3721128 |

**[Table 1-91]**

| Gene name | Coding strand | Start number in SEQ ID NO:1 (stop number when encoded by complementary strand) | Stop number in SEQ ID NO:1 (start number when encoded by complementary strand) |
|---|---|---|---|
| glmU | | 3721481 | 3722851 |
| glmS | | 3723013 | 3724842 |
| pstS | | 3726156 | 3726196 |
| pstC | | 3726283 | 3727242 |
| pstA | | 3727242 | 3728132 |
| pstB | | 3728315 | 3729088 |
| phoU | | 3729103 | 3729628 |
| bglG | | 3730114 | 3730950 |
| bglF | | 3731084 | 3732961 |
| bglB | | 3732980 | 3734392 |
| bglH | | 3734461 | 3736077 |
| yieL | | 3736077 | 3737273 |
| yieK | | 3737288 | 3738010 |
| cbfC | Complementary strand | 3738072 | 3738659 |
| yiel | Complementary strand | 3738708 | 3739175 |
| yieH | Complementary strand | 3739242 | 3739907 |
| yieG | | 3740072 | 3741409 |
| yieF | Complementary strand | 3741463 | 3742029 |
| yieE | Complementary strand | 3742051 | 3742812 |
| insD | Complementary strand | 3742944 | 3743867 |
| InsC | Complementary strand | 3743807 | 3744172 |
| yidZ | Complementary strand | 3744293 | 3745252 |
| mdtL | Complementary strand | 3745227 | 3746402 |
| tnaB | Complementary strand | 3746534 | 3748980 |
| insH | | 3746979 | 3747959 |
| tnaA | Complementary strand | 3749071 | 3750486 |
| insH | | 3750659 | 3751639 |
| tnaC | Complementary strand | 3751906 | 3751980 |
| trmE | Complementary strand | 3752223 | 3753587 |
| yidC | Complementary strand | 3753693 | 3755339 |
| yidD | Complementary strand | 3755342 | 3755599 |
| rnpA | Complementary strand | 3755563 | 3755922 |
| rpmH | Complementary strand | 3755939 | 3756079 |
| dnaA | | 3756686 | 3758089 |
| dnaN | | 3758094 | 3759194 |
| recF | | 3759194 | 3760267 |
| gyrB | | 3760296 | 3762710 |
| yidB | | 3762950 | 3763348 |
| yidA | | 3763463 | 3764275 |
| yidX | Complementary strand | 3764321 | 3764977 |

**[Table 1-92]**

| Gene name | Coding strand | Start number in SEQ ID NO:1 (stop number when encoded by complementary strand) | Stop number in SEQ ID NO:1 (start number when encoded by complementary strand) |
|---|---|---|---|
| dgoR | | 3765255 | 3765944 |
| dgok | | 37659941 | 3796819 |
| dgoA | | 3766803 | 3767420 |
| dgoD | | 3767417 | 3768565 |
| dgoT | | 3768685 | 3769977 |
| cbrA | Complementary strand | 3769974 | 3711036 |
| yldR | | 3771139 | 3772353 |
| JidQ | Complementary strand | 3772355 | 3772687 |
| IbpA, | | 3772993 | 3773406 |
| ibpB | | 3773518 | 3773946 |
| yidE | | 3774142 | 3775803 |
| yidP | Complementary strand | 3775800 | 3776516 |
| glvC | | 3776812 | 3777918 |
| glvB | | 3777943 | 3778428 |
| glvG | | 3778428 | 3779066 |
| ysdC | | 3779066 | 3779242 |
| yidL | Complementary strand | 3779239 | 3780162 |
| yidK | | 3780299 | 3782014 |
| yidJ | | 3782011 | 5763504 |
| yidl | Complementary strand | 3783551 | 3784000 |
| yidH | | 3784108, | 3784456 |
| yidG | | 3784445 | 3784807 |
| yldF | | 3784804 | 3785301 |
| emrD | Complementary strand | 3785309 | 3786493 |
| ivbL | | 3787427 | 3787525 |
| ilvB | | 3757631 | 3789319 |
| ilvN | | 3789323 | 3789613 |
| uhpA | | 3789689 | 3790279 |
| uhpB | | 3790279 | 3791781 |
| uhpC | | 3791791 | 3793110 |
| uhpT | | 3793248 | 3794639 |
| ade | Complementary strand | 3794685 | 3796451 |
| yicO | | 3796626 | 3797960 |
| yicN | | 3798013 | 3798465 |
| yicM | | 3798511 | 3799866 |
| yicS | Complementary strand | 3799907 | 3800200 |
| nlpA | | 3800422 | 3801240 |
| yicL | Complementary strand | 3801244 | 3802167 |
| setC | Complementary strand | 3802278 | 3803462 |
| selC | Complementary strand | 3804099 | 3804193 |

**[Table 1-93]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yicJ | | 3804486 | 3805868 |
| yicl | | 3805878 | 3808196 |
| yicH | Complementary strand | 3808249 | 3809958 |
| YicE | Complementary strand | 3810079 | 3811470 |
| gltS | | 3811790 | 3812955 |
| recG | Complementary strand | 3813124 | 3815205 |
| trmH | Complementary strand | 3815211 | 3816900 |
| spoT | Complementary strand | 3815907 | 3818015 |
| rpoZ | Complementary strand | 3818034 | 3818309 |
| gmk | Complementary strand | 3818364 | 3818987 |
| llgB | | 3819245 | 3820927 |
| yicG | Complementary strand | 3820824 | 3821541 |
| dinD | Complementary strand | 3821831 | 3822655 |
| yicC | Complementary strand | 3822876 | 33823739 |
| rph | | 3823866 | 3824552, |
| pyrE | | 3824647 | 3825288 |
| ttk | Complementary strand | 3825325 | 3825921 |
| dut | Complementary strand | 3826028 | 3826483 |
| dfp | Complementary strand | 3826464 | 5827684 |
| yicR | | 3827856 | 3828524 |
| rpmB | | 3828741 | 3828977 |
| rpmG | | 3828998 | 3829165 |
| mutM | | 3829263 | 3830072 |
| coaD | Complementary strand | 3830111 | 3830590 |
| kdtA | Complementary strand | 3830698 | 3831675 |
| rfaQ | | 3832317 | 3833351 |
| rfaG | | 3833348 | 3834472 |
| rfaP | | 3834466 | 3835262 |
| rfaS | | 3835299 | 3836234 |
| rfaB | | 3836248 | 3837357 |
| rfal | | 3837357 | 3838376 |
| rfaJ | | 3838416 | 3839432 |
| rfaY | | 3839450 | 3640148 |
| rfaZ | | 3840219 | 3841070 |
| rfaK | | 3841103 | 3B42176 |
| rfaL | Complementary strand | 3842208 | 3643467 |
| rfaC | Complementary strand | 3643477 | 3844436 |
| rfaF | Complementary strand | 3844440 | 3845486 |
| rfaD | Complementary strand | 3845496 | 3846428 |
| htrL | | 3546732 | 3847589 |

**[Table 1-94]**

| Gene name | Coding strand | Start number in SEQ ID NO:1 (stop number when encoded by complementary strand) | Stop number in SEQ ID NO:1 (start number When encoded by complementary strand) |
|---|---|---|---|
| kbl | | 3847664 | 3649060 |
| tdh | | 3849070 | 3850095 |
| yibD | | 3850334 | 3851368 |
| ylbQ | Complementary strand | 3851355 | 3852314 |
| envC | Complementary strand | 3852318 | 3853577 |
| gpml | Complementary strand | 38S3611 | 3855155 |
| YibN | | 3863400 | 3855831 |
| grxC | | 3855973 | 3856224 |
| secB | | 3856287 | 3856754 |
| gpsA | | 3866754 | 3857773 |
| cysE | | 3857853 | 3858674 |
| yibk | Complementary strand | 3858737 | 3B59200 |
| lidD | Complementary strand | 3859398 | 3860688 |
| lldR | Complementary strand | 3860585 | 3861361 |
| lldP | Complementary strand | 3861361 | 3863016 |
| ylbL | Complementary strand | 3863388 | 3863750 |
| yibT | | 3864035 | 3864244 |
| mtlR | | 3864256 | 3864843 |
| mtlD | Complementary strand | 3864843 | 3865991 |
| mbA | Complementary strand | 3866221 | 3868134 |
| yibl | | 3868671 | 3869033 |
| yibH | | 3869036 | 3870172 |
| yibG | Complementary strand | 3871777 | 3872238 |
| yibJ | Complementary strand | 3872493 | 3873194 |
| yibA | Complementary strand | 3873236 | 3874078 |
| rhsA | Complementary strand | 3874099 | 3878232 |
| yibF | | 3878460 | 3879068 |
| selA | | 3879166 | 3880557 |
| selB | | 3B80554 | 3882398 |
| yiaY | | 3882588 | 3883739 |
| aldB | | 3883904 | 3885442 |
| yiaW | | 3885987 | 3886310 |
| yiaV | | 3886316 | 3887452 |
| yiaU | Complementary strand | 3887449 | 3888423 |
| yiaT | | 3888547 | 3889287 |
| sgbE | Complementary strand | 3889634 | 3890329 |
| sgbU | Complementary strand | 3890323 | 3891183 |
| sgbH | Complementary strand | 3891176 | 3891838 |
| lyxK | Complementary strand | 3891835 | 3893331 |
| yiaO | Complementary strand | 3893335 | 3894321 |

**[Table 1-95]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yiaN | complementary strand | 3894334 | 3895611 |
| yiaM | complementary strand | 3895614 | 3896087 |
| yiaL | complementary strand | 3896205 | 3856672 |
| yiaK | complementary strand | 3998964, | 3897682 |
| yiaJ | | 3897883 | 3898731 |
| yial | | 3898633 | 3899306 |
| avtA | Complementary strand | 3899457 | 3900710 |
| malS | Complementary strand | 3900888 | 3902918 |
| bax | | 3903238 | 3904062 |
| xylR | Complementary strand | 3904258 | 3905436 |
| xylH | Complementary strand | 3905514 | 3906695 |
| xylG | Complementary strand | 3906673 | 3908214 |
| xylF | Complementary strand | 3908292 | 3909284 |
| xylA | | 3909650 | 3910972 |
| xylB | | 3911044 | 3912498 |
| ylaB | | 3912667 | 3913008 |
| ylaA | | 3913054 | 3913491 |
| yiaH | Complementary strand | 3913533 | 3914528 |
| ysaB | | 3914703 | 3915002 |
| glyQ | | 3915097 | 3916008 |
| glyS | | 3916018 | 3918087 |
| insK | Complementary strand | 3918366 | 3919217 |
| insJ | Complementary strand | 3919214 | 3919735 |
| hokA | | 3919815 | 3919967 |
| cspA | Complementary strand | 3920154 | 3620366 |
| yiaG | Complementary strand | 3920647 | 3320937 |
| yiaF | | 3921371 | 3922081 |
| tiaE | Complementary strand | 3922131 | 3923105 |
| yiaD | Complementary strand | 3923209 | 3923868 |
| bisC | | 3924075 | 3926354 |
| ylaC | Complementary strand | 3926323 | 3926763 |
| tag | Complementary strand | 3026760 | 3927323 |
| yhjY | | 3927481 | 3928179 |
| yhjX | | 3928408 | 3929616 |
| eptB | | 3929940 | 3931631 |
| proK | | 3931723 | 3931799 |
| dppA | | 3932710 | 3934317 |
| dppB | | 3934625 | 3935644 |
| dppC | | 3935654 | 3936556 |
| dppD | | 3936567 | 3937550 |

**[Table 1-96]**

| Gene name | Coding strand | Start number in SEQ ID NO:1 (stop number when encoded by complementary strand) | Stop number in SEQ ID NO:1 (start number when encoded by complementary strand) |
|---|---|---|---|
| dppF | | 3937547 | 3938551 |
| yhjV | Complementary strand | 3938581 | 3938952 |
| rdID | Complementary strand | 3940216 | 3940279 |
| IdrD | | 3940328 | 3940435 |
| bcsG | Complementary strand | 3940522 | 3942201 |
| bcsF | Complementary strand | 3942198 | 3942389 |
| bcsE | Complementary strand | 3942336 | 3943957 |
| yhjR | | 3944230 | 3944418 |
| yhjQ | | 3944454 | 3045182 |
| bcsA | | 3945179 | 3947797 |
| bcsB | | 3947808 | 3950147 |
| bcsZ | | 3850154 | 3551260 |
| bcsC | | 3951242 | 3954715 |
| yhjK | | 3954836 | 3956785 |
| dcTA | | 3956968 | 3958254 |
| yhjJ | | 3958475 | 3959971 |
| kdgK | Complementary strand | 3960067 | 3960996 |
| yhjH | | 3361228 | 3981995 |
| yhjG | | 3962050 | 3964125 |
| yhjE | Complementary strand | 3964307 | 3965629 |
| yhjD | Complementary strand | 3966040 | 3967053 |
| yhjC | Complementary strand | 3967102 | 3968073 |
| yhjB | | 3968521 | 3969123 |
| treF | Complementary strand | 3969174 | 3970823 |
| yhjA | | 3971227 | 3972624 |
| gadA | | 3972835 | 3974235 |
| gadX | | 3974605 | 3975429 |
| gadY | Complementary strand | 3975447 | 3975551 |
| gadW | | 3975787 | 3976525 |
| mdtF | Complementary strand | 3976888 | 3980001 |
| mdtE | Complementary strand | 3980026 | 3981183 |
| gadE | Complementary strand | 3981822 | 3982049 |
| hdeD | Complementary strand | 3982848 | 3983420 |
| hdeA | | 3983675 | 3984007 |
| hdeB | | 3984123 | 3984449 |
| yhiD | | 3984513 | 3985180 |
| yhiF | Complementary strand | 3985202 | 3985732 |
| slp | Complementary strand | 3985888 | 3986454 |
| insH-11 | | 3987217 | 3988233 |
| yhlS | Complementary strand | 3988342 | 3989124 |

**[Table 1-97]**

| Gene name | Coding strand | Start number is SEQ ID NO:1 (stop number when encoded by complementary strand) | Stop number in SEQ ID NO:1 (start number when encoded by complementary strand) |
|---|---|---|---|
| arsC | Complementary strand | 3989753 | 3990178 |
| arsB | Complementary strand | 3990191 | 3991480 |
| arsR | Complementary strand | 3991534 | 3991887 |
| gor | Complementary strand | 3992764 | 3994116 |
| yhlR | Complementary strand | 3994188, | 3995030 |
| prlC | | 3995233 | 3997275 |
| yhiQ | | 3997283 | 3998035 |
| yhiP | Complementary strand | 3998084 | 3999553 |
| uspA | Complementary strand | 3999870 | 4000304 |
| yhiO | | 4000695 | 4001030 |
| pitA | Complementary strand | 4001274 | 4002773 |
| yhiN | | 4003005 | 4004207 |
| yhiM | | 4004522 | 4005574 |
| yhiL | | 4005957 | 4007195 |
| yhiK | | 4007171 | 4007563 |
| yhiJ | | 4007825 | 4009447 |
| yhil | | 4009813 | 4010880 |
| rbbA | | 4010877 | 4013612 |
| yhhJ | | 4013612 | 4014736 |
| yhhl | Complementary strand | 4014901 | 4016037 |
| yrhC | Complementary strand | 4016283 | 4016528 |
| yhhH | Complementary strand | 4016633 | 4017016 |
| rhsB | Complementary strand | 4016988 | 4021223 |
| nikR | Complementary strand | 4021426 | 4021827 |
| nikE | Complementary strand | 4021833 | 4022639 |
| nikD | Complementary strand | 4022636 | 4023400 |
| nikC | Complementary strand | 4023400 | 4024233 |
| nikB | Complementary strand | 4024230 | 4025174 |
| nikA | Complementary strand | 4025174 | 4026748 |
| acpT | Complementary strand | 4026859 | 4027446 |
| yhhT | Complementary strand | 4027501 | 4028550 |
| yhhS | | 4028682 | 4029899 |
| dcrB | Complementary strand | 4029903 | 4030460 |
| yhhQ | Complementary strand | 4030533 | 4031198 |
| yhhP | | 4031419 | 4031664 |
| zntA | Complementary strand | 4031766 | 4033964 |
| yhhN | Complementary strand | 4034038 | 4034664 |
| yhhM | | 4034805 | 4035164 |
| yhhL | | 4035167 | 4035436 |
| yhhF | | 4035426 | 4036022 |

**[Table 1-98]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ftsY | | 4036172 | 4037665 |
| ftsE | | 4037668 | 4038336 |
| ftsX | | 4038329 | 4039387 |
| rpoH | | 4039632 | 4040486 |
| livJ | | 4040757 | 4041860 |
| yhhK | Complementary strand | 4042048 | 4042431 |
| livK | | 4042855 | 4043964 |
| livH | | 4044012 | 4044938 |
| livM | | 4044935 | 4046212 |
| ljvG | | 4046209 | 4046976 |
| livF | | 4046978 | 4047691 |
| ugpB | | 4048090 | 4049406 |
| ugpA | | 4049504 | 4050391 |
| ugpE | | 4050388 | 4051233 |
| ugpC | | 4051235 | 4052305 |
| ugpQ | | 4052302 | 4053045 |
| yhhA | Complementary strand | 4053032 | 4053472 |
| ggt | | 4053592 | 4055334 |
| yrhB | Complementary strand | 4055372 | 4055656 |
| insB-6 | Complementary strand | 4056235 | 4056738 |
| insA-6 | Complementary strand | 4056657 | 4056932 |
| yrhA | Complementary strand | 4056961 | 4057374 |
| yhhZ | Complementary strand | 4057374 | 4058552 |
| yhhY | Complementary strand | 4058789 | 4059277 |
| ryhB | | 4059399 | 4059492 |
| yhhX | | 4059610 | 4060647 |
| yhhW | | 4060770 | 4061465 |
| gntR | | 4061689 | 4062684 |
| gntK | | 4062823 | 4063350 |
| gntU | | 4063354 | 4064694 |
| yhgN | Complementary strand | 4064751 | 4065344 |
| asd | | 4065537 | 4066640 |
| glgB | | 4066913 | 4069099 |
| glgX | | 4069096 | 4071069 |
| glgC | | 4071087 | 4072382 |
| glgA | | 4072382 | 4073815 |
| glgP | | 4073834 | 4076281 |
| yzgL | | 4076410 | 4076691 |
| glpD | Complementary strand | 4076897 | 4078402 |
| glpE | | 4078592 | 4078918 |

**[Table 1-99]**

| Gene name | Coding strand | Start number in SEQ ID NO:1 (stop number when encoded by complementary strand) | Stop number in SEQ ID NO:1 (start number when encoded by complementary strand) |
|---|---|---|---|
| glpG | | 4076963 | 4079793 |
| glpR | | 4079810 | 4080568 |
| rtcR | Complementary strand | 4080550 | 4082148 |
| rtcB | | 4082337 | 4083563 |
| rtcA | | 4083567 | 4084583 |
| malT | Complementary strand | 4084626 | 4087331 |
| malP | | 4087943 | 4090336 |
| malQ | | 4090346 | 4092430 |
| gntT | Complementary strand | 4092541 | 4093867 |
| gntY | Complementary strand | 4094217 | 4094792 |
| gntX | Complementary strand | 4094851 | 4095534 |
| bioH | | 4095572 | 4096342 |
| yhgA | Complementary strand | 4096371 | 4097249 |
| yhgG | Complementary strand | 4097452 | 4097688 |
| feoB | Complementary strand | 4097688 | 4100009 |
| feoA | Complementary strand | 4100026 | 4100253 |
| yhgF | Complementary strand | 4100710 | 4103031 |
| greB | Complementary strand | 4103128 | 4103604 |
| ompR | | 4103832 | 4104551 |
| envZ | | 4104548 | 4105900 |
| pck | Complementary strand | 4105976 | 4107598 |
| yhgE | | 4107977 | 4109701 |
| hslO | Complementary strand | 4109764 | 4110642 |
| hslR | Complementary strand | 4110667 | 4111068 |
| yrfG | Complementary strand | 4111079 | 4111747 |
| yrfF | Complementary strand | 4111812 | 4113947 |
| nudE | | 4114267 | 4114827 |
| mrcA | Complementary strand | 4114993 | 4117545 |
| yrtD | | 4117665 | 4118444 |
| yrfC | | 4118444 | 4118983 |
| yrfB | | 4118967 | 4119407 |
| yrtA | | 4119397 | 4119801 |
| hofQ | | 4119713 | 4120951 |
| aroK | | 4121352 | 4121873 |
| aroB | | 4121930 | 4123018 |
| damX | | 4123110 | 4124396 |
| dam | | 4124503 | 4125339 |
| rpe | | 4125357 | 4126034 |
| gph | | 4126027 | 4126785 |
| trpS | | 4126778 | 4127782 |

**[Table 1-100]**

| Gene name | Coding strand | Staff number in SEQ ID NO:1 (stop number when encoded by complementary strand) | stop number in SEQ ID NO:1 (start number when encoded by complementary strand) |
|---|---|---|---|
| yhfZ | | 4128072 | 4128977 |
| yhfY | | 4128994 | 4129356 |
| yhfX | | 4129440 | 4130603 |
| yhfW | | 4130603 | 4131829 |
| php | | 4131826 | 4132704 |
| yhfU | | 4132715 | 4133068 |
| yhfT | | 4133080 | 4134384 |
| yhfS | | 4134396 | 4135481 |
| frlR | Complementary strand | 4135633 | 4136364 |
| frlD | Complementary strand | 4136464 | 4137249 |
| friC | Complementary strand | 4137246 | 4138076 |
| frlB | Complementary strand | 4138126 | 4139148 |
| frlA | Complementary strand | 4130169 | 4140506 |
| yhfL | Complementary strand | 4140801 | 4140968 |
| cysG | Complementary strand | 4141215 | 4142588 |
| nirC | Complementary strand | 4142607 | 4143413 |
| nirD | Complementary strand | 4143539 | 4143865 |
| nirB | Complementary strand | 4143862 | 4146405 |
| tsgA | Complementary strand | 4146667 | 4147848 |
| pplA | | 4148119 | 4148691 |
| yhfG | | 4148796 | 4148963 |
| fic | | 4148953 | 4149555 |
| pabA | | 4149587 | 4150150 |
| argD | | 4150236 | 4151456 |
| yhlK | Complementary strand | 4151523 | 4153625 |
| crp | Complementary strand | 4153664 | 4154296 |
| yhfA | | 4154598 | 4155002 |
| prkB | Complementary strand | 4155057 | 4155926 |
| yheU | Complementary strand | 4155980 | 4156198 |
| yheT | Complementary strand | 4156192 | 4157214 |
| yheS | Complementary strand | 4157214 | 4159127 |
| kefG | | 4159255 | 4159809 |
| kefB | | 4159809 | 4161614 |
| yheV | | 4161624 | 4161824 |
| slyD | | 4161919 | 4162509 |
| slyX | Complementary strand | 4162558 | 4162776 |
| fkpA | | 4162997 | 4163809 |
| yheO | | 4163976 | 4164698 |
| yheN | | 4164698 | 4165084 |
| yheM | | 4165084 | 4165443 |

**[Table 1-101]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yheL | | 4165451 | 4165738 |
| rpsL | | 4165864 | 4166238 |
| rpsG | | 4166335 | 4166874, |
| fusA | | 4166902 | 4169016 |
| tufA | | 4169087 | 4170271 |
| chlA | | 4170563 | 4173256 |
| bfd | | 4173425 | 4173619 |
| bfr | | 4173691 | 4174167 |
| gspO | Complementary strand | 4174196 | 4174873 |
| gspM | Complementary strand | 4174873 | 4175334 |
| gspL | Complementary strand | 4175331 | 4176494 |
| gspK | Complementary strand | 4176509 | 4177492 |
| gspJ | Complementary strand | 4177485 | 4178072 |
| gspl | Complementary strand | 4178065 | 4178442 |
| gspH | Complementary strand | 4178439 | 4178948 |
| gapG | Complementary strand | 4178956 | 4119393 |
| gspF | Complementary strand | 4179403 | 4180599 |
| gspE | Complementary strand | 4180596 | 4182077 |
| gspD | Complementary strand | 4102087 | 4184039 |
| gspC | Complementary strand | 4184023 | 4184838 |
| gspA | | 4185018 | 4186487 |
| pioO | | 4186489 | 4186908 |
| rpsJ | | 4187146 | 4187457 |
| rplC | | 4187490 | 4188119 |
| rplD | | 4188130 | 4188735 |
| rplW | | 4188732 | 4189034 |
| rplB | | 4189052 | 4189873 |
| ripsS | | 4188890 | 4190168 |
| rplV | | 4190183 | 4190515 |
| rpsC | | 4190533 | 4191234 |
| rplP | | 4191247 | 4191657 |
| rpmC | | 4191657 | 4191848 |
| rpsQ | | 4191848 | 4192102 |
| rpIN | | 4192267 | 4192638 |
| rplX | | 4192649 | 4192963 |
| rplE | | 4192978 | 4193517 |
| rpsN | | 4193532 | 4193837 |
| rpsH | | 4193871 | 4194283 |
| rpIF | | 4194276 | 4194809 |
| rpIR | | 4194819 | 4195172 |

**[Table 1-102]**

| Gene name | Coding strand | Start number in SEQ ID NO:1 (stop number when encoded by complementary strand) | Stop number in SEQ ID NO.1 (start number when encoded by complementary strand) |
|---|---|---|---|
| rpsE | | 4195187 | 4195690 |
| rpmD | | 4195694 | 4195873 |
| rplO | | 4195877 | 4196311 |
| secY | | 4196319 | 4197650 |
| rpmJ | | 4197682 | 4197798 |
| rpsM | | 4197945 | 4198301 |
| rpsK | | 4198318 | 4198707 |
| rpsD | | 4198741 | 4199361 |
| rpoA | | 4199387 | 4200376 |
| rplQ | | 4200417 | 4200800 |
| yhdN | | 4200907 | 4201275 |
| zntR | | 4201286 | 4201711 |
| yhdL | | 4201767 | 4201985 |
| mscL | Complementary strand | 4201982 | 4202392 |
| trkA | Complementary strand | 4202522 | 4203898 |
| rsmB | Complementary strand | 4203920 | 4205209 |
| fmt | Complementary strand | 4205255 | 4206202 |
| def | Complementary strand | 4206217 | 4206726 |
| smf | | 4206856 | 4207980 |
| smg | | 4207952 | 4208425 |
| yrdD | | 4208454 | 4208996 |
| yrdC | | 4209001 | 4209573 |
| aroE | | 4209578 | 4210396 |
| yrdB | | 4210393 | 4210650 |
| yrdA | Complementary strand | 4210626 | 4211180 |
| rrsE | | 4211654 | 4213195 |
| ileU | | 4213264 | 4213340 |
| alaU | | 4213383 | 4213458 |
| rrlE | | 4213633 | 4216536 |
| rrfE | | 4216630 | 4216749 |
| yjaA | | 4216824 | 4217207 |
| yjaB | Complementary strand | 4217270 | 4217713 |
| metA | | 4217870 | 4218799 |
| aceB | | 4219068 | 4220669 |
| aceA | | 4220699 | 4222003 |
| aceK | | 4222186 | 4223922 |
| arpA | Complementary strand | 4223891 | 4226077 |
| idR | | 4226394 | 4227218 |
| metH | | 4227418 | 4231101 |
| yjbB | | 4231321 | 4232952 |

**[Table 1-103]**

| Gene name | Coding strand | Start number in SEQ ID NO:1 (stop number when encoded by complementary strand) | Stop number in SEQ ID NO.1 (start number when encoded by complementary strand) |
|---|---|---|---|
| pepE | Complementary strand | 4233043 | 4233732 |
| yjbC | | 4233944 | 4234816 |
| yjbD | Complementary strand | 4234949 | 4235221 |
| lysC | Complementary strand | 4235474 | 4236823 |
| pgl | | 4237348 | 4238997 |
| yjbE | | 4239496 | 4239738 |
| yjbF | | 4239852 | 4240490 |
| yjbG | | 4240487 | 4241224 |
| yjbH | | 4241224 | 4243320 |
| yjbA | | 4243915 | 4244325 |
| xylE | Complementary strand | 4244369 | 4245844 |
| malG | Complementary strand | 4246216 | 4247106 |
| malF | Complementary strand | 4247121 | 4248665 |
| malE | Complementary strand | 4248819 | 4250009 |
| malK | | 4250374 | 4251489 |
| lamB | | 4251561 | 4252901 |
| malM | | 4253144 | 4254064 |
| yjbl | | 4254545 | 4255873 |
| ubiC | | 4256096 | 4256593 |
| ubiA | | 4256806 | 4257478 |
| plsB | Complementary strand | 4257633 | 4260056 |
| dgkA | | 4260227 | 4260595 |
| lexA | | 4260705 | 4261313 |
| dinF | | 4261332 | 4262711 |
| yjbJ | | 4262827 | 4263036 |
| zur | Complementary strand | 4263078 | 4263593 |
| yjbL | | 4263911 | 4264165 |
| yjbM | | 4264189 | 4284896 |
| yjbN | | 4265289 | 4266296 |
| yjbO | | 4266430 | 4266672 |
| qor | Complementary strand | 4266838 | 4267821 |
| dnaB | | 4267904 | 4269319 |
| alr | | 4269372 | 4270451 |
| tyrB | | 4270704 | 4271897 |
| aphA | | 4273004 | 4273717 |
| yjbQ | | 4273828 | 4274244 |
| yjbR | | 4274248 | 4274604 |
| uvrA | Complementary strand | 4274639 | 4277461 |
| ssb | | 4277715 | 4279251 |
| yjcB | Complementary strand | 4278350 | 4278631 |

**[Table 1-104]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yjcC | | 4279061 | 4280647 |
| soxS | Complementary strand | 4280650 | 4280973 |
| soxR | | 4281059 | 4281523 |
| ryjA | Complementary strand | 4281517 | 4281656 |
| yjcD | | 4282069 | 4283418 |
| yjcE | | 4283570 | 4285219 |
| yjcF | Complementary strand | 4285373 | 4286865 |
| actP | Complementary strand | 4286843 | 4288492 |
| yjcH | Complementary strand | 4288489 | 4288803 |
| acs | Complementary strand | 4269003 | 4290961 |
| nrfA | | 4291354 | 4292790 |
| nrfB | | 4292835 | 4293401 |
| nrfC | | 4293398 | 4294069 |
| nrfD | | 4294066 | 4295022 |
| nrfE | | 4295102 | 4296760 |
| nrfF | | 4296753 | 4297136 |
| nrfG | | 4297133 | 4297729 |
| gltP | | 4298071 | 4299384 |
| yjcO | Complementary strand | 4299915 | 4300604 |
| fdhF | Complementary strand | 4300698 | 4302845 |
| yjcP | Complementary strand | 4303043 | 4304509 |
| yjcQ | Complementary strand | 4304506 | 4306557 |
| yjcR | Complementary strand | 4306557 | 4307588 |
| yjcS | Complementary strand | 4308091 | 4310076 |
| insH | | 4310431 | 4311411 |
| alsK | Complementary strand | 4311449 | 4312477 |
| alsE | Complementary strand | 4312461 | 4313156 |
| alsC | Complementary strand | 4313167 | 4314147 |
| alsA | Complementary strand | 4314126 | 4315658 |
| alsB | Complementary strand | 4315785 | 4316720 |
| rpiR | Complementary strand | 4316779 | 4317669 |
| rpiB | | 4318028 | 4318477 |
| yjdP | | 4318546 | 4318875 |
| phnP | Complementary strand | 4319022 | 4319780 |
| phnO | Complementary strand | 4319782 | 4320216 |
| phnN | Complementary strand | 4320203 | 4320760 |
| phnM | Complementary strand | 4320760 | 4321896 |
| phnL | Complementary strand | 4321893 | 4322573 |
| phnK | Complementary strand | 4322684 | 4323442 |
| phnJ | Complementary strand | 4323439 | 4324284 |

**[Table 1-105]**

| Gene name | Coding strand | Start number in SEQ ID NO:1 (stop number when encoded by complementary strand) | Stop number in SEQ ID NO:1 (start number when encoded by complementary strand) |
|---|---|---|---|
| phnl | Complementary strand | 4324277 | 4325341 |
| phnH | Complementary strand | 4325341 | 4325925 |
| phnG | Complementary strand | 4325922 | 4326374 |
| phnF | Complementary strand | 4326375 | 4327100 |
| phnE(JW4064) | Complementary strand | 4327121 | 4327489 |
| phnE(JW4065) | Complementary strand | 4327339 | 4327959 |
| phnD | Complementary strand | 4328014 | 4329030 |
| phnC | Complementary strand | 4329055 | 4329843 |
| phnB | Complementary strand | 4329976 | 4330419 |
| phnA | Complementary strand | 4331077 | 4331412 |
| yjdA | | 4331813 | 4334041 |
| yjcZ | | 4334038 | 4334916 |
| proP | | 4335180 | 4336682 |
| basS | Complementary strand | 4336859 | 4337950 |
| bssR | Complementary strand | 4337960 | 4338628 |
| eptA | Complementary strand | 4338625 | 4340268 |
| adiC | Complementary strand | 4340372 | 4341709 |
| adiY | Complementary strand | 4341846 | 4342607 |
| adiA | Complementary strand | 4342932 | 4345202 |
| melR | Complementary strand | 4345398 | 4346306 |
| melA | | 4346589 | 4347944 |
| melB | | 4348059 | 4349468 |
| yjdF | Complementary strand | 4349607 | 4350236 |
| fumB | Complementary strand | 4350358 | 4352004 |
| dcuB | Complementary strand | 4352082 | 4353422 |
| dcuR | Complementary strand | 4353993 | 4354712 |
| dcuS | Complementary strand | 4354709 | 4355340 |
| yjdl | | 4356521 | 4356751 |
| yjdJ | | 4356763 | 4357035 |
| yjdK | | 4357262 | 4357558 |
| yjdO | | 4357586 | 4357759 |
| lysU | Complementary strand | 4357878 | 4359395 |
| yjdL | Complementary strand | 4359632 | 4361089 |
| cadA | Complementary strand | 4361148 | 4363295 |
| cadB | Complementary strand | 4363375 | 4364709 |
| cadC | Complementary strand | 4365074 | 4366612 |
| pheU | Complementary strand | 4367229 | 4367304 |
| yjdC | Complementary strand | 4367411 | 4367986 |
| dipZ | Complementary strand | 4368023 | 4369720 |
| cutA | Complementary strand | 4369696 | 4370034 |

**[Table 1-106]**

| Gene name | Coding strand | Start number in SEQ ID NO:1 (stop number when encoded by Complementary strand) | Stop number in SEQ ID NO:1 (start number when encoded by complementary strand) |
|---|---|---|---|
| dcuA | Complementary strand | 4370150 | 4371265 |
| aspA | Complementary strand | 4371571 | 4373007 |
| fxsA | | 4373344 | 4373820 |
| yjeH | Complementary strand | 4373836 | 4375092 |
| groS | | 4375368 | 4375661 |
| groL | | 4375705 | 4377351 |
| yjel | | 4377489 | 4377842 |
| yjeJ | Complementary strand | 4378045 | 4378914 |
| yjeK | Complementary strand | 4379309 | 4380337 |
| efp | | 4380379 | 4380945 |
| ecnA | | 4380997 | 4381122 |
| ecnB | | 4381233 | 4381379 |
| sugE | | 4381555 | 4381872 |
| blc | Complementary strand | 4381869 | 4382402 |
| ampC | Complementary strand | 4382491 | 4383624 |
| frdD | Complementary strand | 4383687 | 4384046 |
| frdC | Complementary strand | 4364057 | 4384452 |
| frdB | Complementary strand | 4384463 | 4385197 |
| frdA | Complementary strand | 4385190 | 4386998 |
| poxA | | 4387323 | 4388300 |
| yjeM | | 4388519 | 4390021 |
| yjeN | | 4390073 | 4390387 |
| yjeO | | 4390384 | 4390698 |
| yjeP | Complementary strand | 4390727 | 4394050 |
| psd | Complementary strand | 4394072 | 4395040 |
| rsgA | Complementary strand | 4395137 | 4396189 |
| orn | | 4396284 | 4396829 |
| glyV | | 4397040 | 4397115 |
| glyX | | 4397152 | 4397227 |
| glyY | | 4397263 | 4397338 |
| yjeS | Complementary strand | 4397608 | 4398747 |
| yjeF | | 4398746 | 4400293 |
| yjeE | | 4400265 | 4400726 |
| amiB | | 4400745 | 4402082 |
| mutL | | 4402092 | 4403939 |
| miaA | | 4403932 | 4404882 |
| hfq | | 4404968 | 4405276 |
| hflX | | 4405352 | 4406632 |
| hflK | | 4406718 | 4407977 |
| hflC | | 4407980 | 4408984 |

**[Table 1-107]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yjeT | | 4409066 | 4409263 |
| purA | | 4409367 | 4410665 |
| yjeB | | 4410870 | 4411295 |
| mr | | 4411334 | 4413775 |
| rlmB | | 4413955 | 4414686 |
| yjfl | | 4414813 | 4415214 |
| yjfJ | | 4415233 | 4415931 |
| yjfK | | 4415982 | 4416641 |
| yjfL | | 4416659 | 4417057 |
| yjfM | | 4417067 | 4417705 |
| yjfC | | 4417708 | 4418871 |
| aidB | | 4418955 | 4420580 |
| yjfN | Complementary strand | 4420697 | 4420972 |
| yjfO | Complementary strand | 4421121 | 4421450 |
| yjfP | | 4421632 | 4422381 |
| ulaR | Complementary strand | 4422378 | 4423133 |
| ulaG | Complementary strand | 4423241 | 4424305 |
| ulaA | | 4424660 | 4426057 |
| ulaB | | 4426073 | 4426378 |
| ulaC | | 4426388 | 4426852 |
| ulaD | | 4426866 | 4427516 |
| ulaE | | 4427526 | 4428380 |
| ulaF | | 4428380 | 4429066 |
| yjfY | Complementary strand | 4429196 | 4429471 |
| rpsF | | 4429798 | 4430193 |
| priB | | 4430200 | 4430514 |
| rpsR | | 4430519 | 4430746 |
| rpll | | 4430788 | 4431237 |
| yjfZ | Complementary strand | 4431308 | 4432102 |
| ytfA | | 4432449 | 4432775 |
| ytfB | Complementary strand | 4432759 | 4433397 |
| fklB | | 4433615 | 4434235 |
| cycA | | 4434544 | 4435956 |
| ytfE | Complementary strand | 4436001 | 4436663 |
| ytfF | Complementary strand | 4436771 | 4437745 |
| ytfG | Complementary strand | 4437844 | 4438704 |
| ytfH | | 4438793 | 4439173 |
| cpdB | Complementary strand | 4439302 | 4441245 |
| cysQ | | 4441435 | 4442175 |
| ytfl | | 4442387 | 4443325 |

**[Table 1-108]**

| Gene name | Coding strand | Start number in SEQ ID NO:1 (stop number when encoded by complementary strand) | Stop number in SEQ ID NO:1 (start number when encoded by complementary strand) |
|---|---|---|---|
| ytfJ | Complementary strand | 4443388 | 4443942 |
| ytfK | | 4444267 | 4444473 |
| ytfL | Complementary strand | 4444552 | 4445895 |
| msrA | Complementary strand | 4446218 | 4446856 |
| ytfM | | 4447062 | 4448795 |
| ytfN | | 4448792 | 4452571 |
| ytfP | | 4452574 | 4462915 |
| yzfA | Complementary strand | 4452656 | 4452925 |
| chpS | | 4453127 | 4453378 |
| chpB | | 4453372 | 4453722 |
| ppa | Complementary strand | 4453802 | 4454332 |
| ytfQ | | 4454642 | 4455598 |
| ytfR | | 4455798 | 4457240 |
| ytfT | | 4457254 | 4458276 |
| yjfF | | 4458263 | 4459258 |
| fbp | Complementary strand | 4459291 | 4460289 |
| mpl | | 4460465 | 4461838 |
| yjgA | Complementary strand | 4461994 | 4462545 |
| pmbA | | 4462639 | 4463991 |
| cybC | | 4464233 | 4464535 |
| nrdG | Complementary strand | 4464580 | 4465044 |
| nrdD | Complementary strand | 4465202 | 4467340 |
| treC | Complementary strand | 4467734 | 4469389 |
| treB | Complementary strand | 4469439 | 4470860 |
| treR | Complementary strand | 4470979 | 4471926 |
| mgtA | | 4472305 | 4475001 |
| yjgF | Complementary strand | 4475207 | 4475593 |
| pyrl | Complementary strand | 4475666 | 4476127 |
| pyrB | Complementary strand | 4476140 | 4477075 |
| pyrL | Complementary strand | 4477079 | 4477213 |
| yjgH | Complementary strand | 4477494 | 4477889 |
| yjgI | Complementary strand | 4478020 | 4478733 |
| yjgJ | | 4478804 | 4479397 |
| yjgK | | 4479542 | 4479994 |
| yjgL | | 4480117 | 4481931 |
| argl | Complementary strand | 4481987 | 4482991 |
| yjgD | | 4483153 | 4483569 |
| yjgM | Complementary strand | 4483714 | 4484217 |
| yjgN | | 4484410 | 4485606 |
| valS | Complementary strand | 4485662 | 4488517 |

**[Table 1-109]**

| Genre name | Coding strand | Start number in SEQ ID NO:1 (stop number when encoded by complementary strand) | Stop number in SEQ ID NO:1 (start number when encoded by complementary strand) |
|---|---|---|---|
| holC | Complementary strand | 4488517 | 4488960 |
| pepA | Complementary strand | 4489120 | 4490631 |
| yjgP | | 4490898 | 4491998 |
| yjgQ | | 4491998 | 4493080 |
| yjgR | Complementary strand | 4493241 | 4494743 |
| idnR | Complementary strand | 4494821 | 4495819 |
| idnT | Complementary strand | 4495886 | 4497205 |
| idnO | Complementary strand | 4497267 | 4498031 |
| idnD | Complementary strand | 4498055 | 4499086 |
| idnK | | 4499303 | 4499866 |
| yjgB | Complementary strand | 4499870 | 4500889 |
| leuX | | 4501085 | 4501169 |
| intB | | 4501430 | 4502620 |
| insC-6 | | 4502907 | 4503317 |
| insD-6 | | 4603275 | 4504180 |
| yjgW | | 4504279 | 4504614 |
| yjgX_3 | Complementary strand | 4504357 | 4504599 |
| yigX_2 | Complementary strand | 4504723 | 4505169 |
| yjgX_1 | Complementary strand | 4505112 | 4505471 |
| yjgZ | | 4505940 | 4506269 |
| insG | Complementary strand | 4506783 | 4508111 |
| yjhB | | 4508738 | 4609955 |
| yjhC | | 4509967 | 4911085 |
| yjhD | Complementary strand | 4511306 | 4511536 |
| yjhE | | 4511541 | 4511789 |
| insN-2 | | 4511877 | 4512143 |
| insl-3 | Complementary strand | 4512146 | 4513297 |
| insM | Complementary strand | 4513254 | 4513622 |
| insO-2 | | 4513638 | 4514234 |
| yjhW | | 4514231 | 4514473 |
| yjhV | | 4514400 | 4514813 |
| fecE | Complementary strand | 4515370 | 4516137 |
| fecD | Complementary strand | 4516138 | 4517094 |
| fecC | Complementary strand | 4517091 | 4518089 |
| fecB | Complementary strand | 4518086 | 4518988 |
| fecA | Complementary strand | 4519033 | 4521357 |
| fecR | Complementary strand | 4521444 | 4522397 |
| fecl | Complementary strand | 4522394 | 4522915 |
| insA-7 | | 4523207 | 4523482 |
| insB-7_1 | | 4523401 | 4523694 |

**[Table 1-110]**

| Gene name | Coding strand | Start number in SEQ ID NO:1 (stop number when encoded by complementary strand) | Stop number in SEQ ID NO:1 (start number when encoded by complementary strand) |
|---|---|---|---|
| insB-7_2 | | 4523695 | 4523904 |
| yjhU | Complementary strand | 4524018 | 4525004 |
| yjhF | Complementary strand | 4525351 | 4526700 |
| yjhG | Complementary strand | 4526807 | 4528774 |
| yjhH | Complementary strand | 4528785 | 4529690 |
| yjhl | Complementary strand | 4529695 | 4530483 |
| sgcR | Complementary strand | 4530786 | 4531568 |
| sgcE | Complementary strand | 4531585 | 4532217 |
| sgcA | Complementary strand | 4532229 | 4532660 |
| sgcQ | Complementary strand | 4532791 | 4533597 |
| sgcC | Complementary strand | 4533610 | 4534923 |
| sgcB | Complementary strand | 4534935 | 4535213 |
| sgcX | Complementary strand | 4535210 | 4536331 |
| yjhP | Complementary strand | 4537117 | 4537863 |
| yjhQ | Complementary strand | 4537919 | 4538464 |
| yjhX | Complementary strand | 4538476 | 4538733 |
| yjhR | | 4539695 | 4540711 |
| yjhS | Complementary strand | 4541294 | 4542274 |
| yjhT | Complementary strand | 4542339 | 4543445 |
| yjhA | Complementary strand | 4543465 | 4544181 |
| fimB | | 4545637 | 4546239 |
| fimE | | 4546717 | 4547313 |
| fimA | | 4547795 | 4548343 |
| fiml | | 4548408 | 4548947 |
| fimC | | 4548984 | 4549709 |
| fimD | | 4549776 | 4552412 |
| fimF | | 4552422 | 4552952 |
| fimG | | 4552965 | 4553468 |
| fimH | | 4553488 | 4554390 |
| gntP | Complementary strand | 4554633 | 4555976 |
| uxuA | | 4556316 | 4557500 |
| uxuB | | 4557581 | 4559041 |
| uxuR | | 4559256 | 4560029 |
| yjfC | Complementary strand | 4560170 | 4561000 |
| yjiD | | 4561673 | 4562065 |
| yjiE | Complementary strand | 4562058 | 4562969 |
| iadA | Complementary strand | 4563034 | 4564206 |
| yjiG | Complementary strand | 4564219 | 4564680 |
| yjiH | Complementary strand | 4564677 | 4565630 |
| kptA | | 4565610 | 4566164 |

**[Table 1-111]**

| Gene name | Coding strand | Start number in SEQ ID NO:1 (stop number when encoded by complementary strand) | Stop number in SEQ ID NO:1 (start number when encoded by complementary strand) |
|---|---|---|---|
| yjiJ | Complementary strand | 4566177 | 4567355 |
| yjiK | Complementary strand | 4567423 | 4568391 |
| yjiL | Complementary strand | 4568602 | 4569369 |
| yjiM | Complementary strand | 4569379 | 4570530 |
| yjiN | Complementary strand | 4570646 | 4571926 |
| yjiO | Complementary strand | 4571967 | 4573199 |
| yjlP | | 4573678 | 4573989 |
| yjiQ | | 4574038 | 4574598 |
| yjiR | Complementary strand | 4574842 | 4576254 |
| yjiS | | 4576431 | 4576595 |
| yjiT | | 4577094 | 4578611 |
| yjiV | | 4578815 | 4581535 |
| mcrC | Complementary strand | 4581592 | 4582638 |
| mcrB | Complementary strand | 4582638 | 4584017 |
| yjiW | Complementary strand | 4584179 | 4584577 |
| hsdS | Complementary strand | 4584748 | 4586142 |
| hsdM | Complementary strand | 4586139 | 4587728 |
| hsdR | Complementary strand | 4587929 | 4591495 |
| mrr | | 4591629 | 4592543 |
| yjiA | Complementary strand | 4592589 | 4593545 |
| yjiX | Complementary strand | 4593556 | 4593759 |
| yjiY | Complementary strand | 4593809 | 4595959 |
| tsr | | 4596337 | 4597992 |
| yjiZ | Complementary strand | 4598041 | 4599402 |
| yjjM | Complementary strand | 4599617 | 4600531 |
| yjjN | | 4600670 | 4601692 |
| mdoB | Complementary strand | 4601830 | 4604121 |
| yjjA | Complementary strand | 4604375 | 4604869 |
| dnaC | Complementary strand | 4604918 | 4605655 |
| dnaT | Complementary strand | 4605658 | 4606197 |
| yjjB | Complementary strand | 4606304 | 4606630 |
| yjjP | Complementary strand | 4606768 | 4607538 |
| yjjQ | | 4608157 | 4608882 |
| bglJ | | 4608894 | 4609517 |
| fhuF | Complementary strand | 4609555 | 4610343 |
| yjjZ | | 4610484 | 4610720 |
| leuV | Complementary strand | 4610759 | 4610845 |
| leuP | Complementary strand | 4610880 | 4610966 |
| leuQ | Complementary strand | 4610995 | 4611081 |
| rsmC | Complementary strand | 4611349 | 4612380 |

**[Table 1-112]**

| Gene name | Coding strand | Start number in SEQ ID NO:1 (stop number when encoded by complementary strand) | Stop number in SEQ ID NO:1 (start number when encoded by complementary strand) |
|---|---|---|---|
| holD | | 4612483 | 4612896 |
| riml | | 4612865 | 4613311 |
| yjjG | | 4613326 | 4614003 |
| prfC | | 4614094 | 4615683 |
| osmY | | 4616076 | 4616681 |
| ytjA | | 4616790 | 4616969 |
| yjjU | | 4617091 | 4618164 |
| yjjV | | 4618161 | 4618940 |
| yjjW | Complementary strand | 4619360 | 4620223 |
| yjjl | Complementary strand | 4620195 | 4621745 |
| deoC | | 4622003 | 4622782 |
| deoA | | 4622909 | 4624231 |
| deoB | | 4624283 | 4625506 |
| deoD | | 4625563 | 4626282 |
| yjjJ | | 4626449 | 4627780 |
| lplA | Complementary strand | 4627781 | 4628797 |
| ytjB | Complementary strand | 4628825 | 4629469 |
| serB | | 4629575 | 4630543 |
| radA | | 4630592 | 4631974 |
| nadR | | 4631995 | 4633227 |
| yjjK | Complementary strand | 4633535 | 4635202 |
| slt | | 4635413 | 4637350 |
| trpR | | 4637440 | 4637766 |
| yjjX | Complementary strand | 4637913 | 4638425 |
| ytjC | | 4638477 | 4639124 |
| rob | Complementary strand | 4639121 | 4639990 |
| creA | | 4640201 | 4640674 |
| creB | | 4640687 | 4641376 |
| creC | | 4641376 | 4642800 |
| creD | | 4642858 | 4644210 |
| arcA | Complementary strand | 4644270 | 4644986 |
| yjjY | | 4645082 | 4645222 |
| yjtD | | 4645622 | 4646308 |

In addition, (23) an E. *coli* mutant strain having a chromosomal DNA comprising the genes [1] or homologous gene thereof of (1), and not more than 10% of the genes [2] of (1) and the genes [3] below or homologous gene thereof,
[3] chpA gene, uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, ybeM gene, yqcA gene, yqcB gene, yqcC gene, syd gene, yqcD gene, ygdH gene, sdaC gene, sdaB gene, exo gene, fucO gene, fucA gene, fucP gene, fucI gene, fucK gene, fucU gene, fucR gene, ygdE gene, ygdD gene, gcvA gene, ygdI gene, csdA gene, ygdK gene, araD gene, araA gene, araB gene, araC gene, yabI gene, thiQ gene, thiP gene, tbpA gene, sgrR gene, setA gene, yhfZ gene, yhfY gene, yhfX gene, yhfW gene, php gene, yhfU gene, yhfT gene, yhfS gene, frlR gene, frlD gene, frlC gene, frlB gene, frlA gene, yhfL gene, pncA gene, ydjE gene, ydjF gene, ydjG gene, ydjH gene, ydjI gene, ydjJ gene, ydjK gene, ydjL gene, yeaC gene, yeaA gene, ade gene, yicO gene, yicN gene, yicM gene, yicS gene, nlpA gene, yicL gene, setC gene, ygjI gene, ygjJ gene, ygjK gene, fadH gene, ygjM gene, ygjN gene, ygjO gene, ygjP gene, ygjQ gene, ygjR gene, alx gene, sstT gene, ygjV gene, yrhB gene, yrhA gene, yhhZ gene, yhhY gene, yhhX gene, yhhW gene, yeaK gene, yoaI gene, yeaL gene, yeaM gene, yeaN gene, yeaO gene, yoaF gene, yeaP gene, yeaQ gene, yoaG gene, yeaR gene, yeas gene, yeaT gene, yeaU gene, yeaV gene, yeaW gene, yeaX gene, hyfA gene, hyfB gene, hyfC gene, hyfD gene, hyfE gene, hyfF gene, hyfG gene, hyfH gene, hyfI gene, hyfJ gene, hyfR gene, focB gene is also the E. *coli* mutant strain of the present invention.

Further, the E. coli mutant strain of the present invention includes;
(24) The E. *coli* mutant strain obtained via modification of the *E*. *coli* mutant strain according to any one of (1) to (23) above by a method selected from among the following [1] to [5]:
   [1] a method for reducing or removing at least one mechanism to control biosynthesis of the useful substance;
   [2] a method for increasing the expression of at least one enzyme associated with biosynthesis of the useful substance;
   [3] a method for increasing the copy number of at least one enzyme gene associated with biosynthesis of the useful substance;
   [4] a method for attenuating or blocking at least one metabolic pathway which branches from the biosynthetic pathway of the useful substance into metabolites other than the useful substance; or
   [5] a method for selecting a strain that exhibits higher resistance to an analogue of a useful substance than a wild-type *E*. *coli* strain.

The *E*. *coli* mutant strains described in (1) to (23) above appear to lack parts of or entire genes that encode proteins that are not directly involved in biosynthesis of useful substances but indirectly suppress generation of such useful substances. Such *E*. *coli* mutant strains and the mutant strain of (24) above are useful as *E*. *coli* mutant strains for producing useful substances.
The *E*. *coli* mutant strain of the present invention may be any E. *coli* mutant strain, provided that it belongs to *Escherichia coli.* It is preferably a mutant strain of the *E*. *coli* K-12 strain, the *E*. *coli* B strain, or the *E*. *coli* W strain, more preferably a mutant strain of the *E*. *coli* K-12 strain, further preferably a mutant strain of the *E*. *coli* W3110 strain (ATCC27325) or the *E*. *coli* MG1655 strain (ATCC47076), and particularly preferably the *E. coli* W3110 strain (ATCC27325), for example.

### 2. Method for producing the E. coli mutant strain of the present invention

The *E*. *coli* mutant strain of the present invention can be obtained by any method, provided that it allows such mutant strain to be obtained. For example, the following method wherein a given gene or DNA on *E*. *coli* chromosomal DNA is deleted may be employed.

A gene or DNA on *E*. *coli* chromosomal DNA can be deleted via a method utilizing homologous recombination, for example. An example of a general method utilizing homologous recombination is a method involving the use of plasmids for homologous recombination that can be prepared by ligating DNA fragments located at both outer sides of the gene or DNA that is intended to be deleted on *E*. *coli* chromosomal DNA to plasmid DNA having a drug resistance gene that cannot be autonomously replicated in such *E*. *coli.*

After the plasmids for homologous recombination are introduced into an *E*. *coli* cell by a conventional method, transformants into which the plasmids for homologous recombination have been integrated to chromosomal DNA by homologous recombination are selected using the drug resistance as an indicator. The obtained transformants are cultured in a medium that does not contain the aforementioned drug for several hours to 1 day, thereafter the cultures are spread on an agar medium that contains the drug and to an agar medium that does not contain the drug, and strains that do not grow in the former medium but grow in the latter medium are selected. Thus, strains in which the second homologous recombination has taken place on the chromosomal DNA can be obtained. By determining the nucleotide sequence of a region of the chromosomal DNA in which the gene or DNA to be deleted was present, the introduction of deletion of the target gene or DNA on chromosomal DNA can be confirmed.

An example of a method utilizing homologous recombination, and thereby effectively introducing deletion, substitution or addition of nucleotide(s) into multiple genes, is a method involving the use of a linear DNA.
Specifically, a linear DNA that contains DNA fragments existing at both outside regions of a gene or DNA to be deleted on chromosomal DNA is incorporated into a cell to cause homologous recombination between the chromosomal DNA and the introduced linear DNA. This method can be applied to any microorganisms, provided that such *Escherichia coli* effectively incorporate a linear DNA. Such microorganisms include preferably *Escherichia coli* that expresses λ phage-derived recombinant proteins (Red recombination system).

An example of *Escherichia coli* that expresses the λRed recombination system is *Escherichia coli* JM101 possessing pKD46, which is plasmid DNA having the gene of the λ Red recombination system (available from the *E*. *coli* Genetic Stock Center, Yale University, U.S.A.).
Examples of DNAs that can be used for homologous recombination include:
(a) a linear DNA having, at both ends of the drug resistance gene, DNA existing at both outside regions of a chromosomal DNA into which the introduction of deletion, substitution or addition of nucleotide(s) is intended, or DNA homologous thereto;
(b) a linear DNA to which DNA existing at both outside regions of a chromosomal DNA into which the introduction of deletion, substitution or addition of nucleotide(s) is intended, or DNA homologous thereto is directly ligated;
(c) a linear DNA having, at both ends of DNA in which a drug resistance gene and a gene that can be used for negative selection are ligated, DNA existing at both outside regions of chromosomal DNA into which the introduction of deletion, substitution or addition of nucleotide(s) is intended, or DNA homologous thereto; and
(d) a linear DNA according to (a) above, further having a nucleotide sequence which is recognized by yeast-derived Flp recombinase [Proc. Natl. Acad. Sci., U.S.A., 82, 5875, 1985] between a drug resistance gene and DNA existing at both outside regions of a chromosomal DNA or DNA homologous thereto.

Any drug resistance genes can be used, provided that such genes impart drug resistance to a drug to which *E. coli* shows sensitivity. For example, the drug resistance genes include kanamycin resistance genes, chloramphenicol resistance genes, gentamicin resistance genes, spectinomycin resistance genes, tetracycline resistance genes, ampicillin resistance genes, and the like.

The genes that can be used for negative selection are genes that are lethal to *E*. *coli* under given culture conditions, when such genes are expressed in *E*. *coli.* Examples of such genes include *sacB* genes derived from the microorganism which belongs to the genus *Bacillus* [Appl. Environ. Microbiol., 59, 1361-1366, 1993] and *rpsL* genes derived from the microorganism which belongs to the genus *Escherichia* [Genomics, 72, 99-104, 2001].

DNA that exists at both ends of the aforementioned linear DNA which is located at both outside regions of a chromosomal DNA into which introduction of substitution or deletion of nucleotide(s) is intended, or DNA homologous thereto, is oriented on the linear DNA in the same direction as the direction thereof on the chromosomal DNA. The length thereof is preferably about 10 bp to 100 bp, more preferably about 20 bp to 50 bp, and further preferably about 30 bp to 40 bp.

The nucleotide sequence that is recognized by yeast-derived Flp recombinase may be a nucleotide sequence that is recognized by yeast-derived Flp recombinase and has a nucleotide sequence which catalyze homologous recombination, provided that the aforementioned protein recognizes such nucleotide sequence and catalyzes homologous recombination of such nucleotide sequence.
A DNA homologous thereto refers to a DNA which has identity to the extent that homologous recombination takes place between the aforementioned linear DNA and a target region on a chromosomal DNA. Specifically, it refers to DNA having 80% or more, preferably 90% or more, more preferably 95% or more, and further preferably 100% homology.

### Homology of the nucleotide sequences can be determined using the aforementioned programs such as BLAST or FASTA.

The aforementioned linear DNA fragment can be prepared by PCR. Also, DNA containing the linear DNA can be constructed on a plasmid and the linear DNA of interest can then be obtained by restriction enzyme treatment.

Deletion, substitution, or addition of nucleotide(s) can be introduced into chromosomal DNAs of *E. coli* by any of the following methods 1 to 4:
Method 1: a method wherein the linear DNA of (a) or (d) above is introduced into a host microorganism, and a transformant,
into which such linear DNA has been integrated into a chromosomal DNA by homologous recombination, is selected using drug resistance as an indicator;
Method 2: a method to substitute or delete a region on a chromosomal DNA of a microorganism by introducing a DNA wherein DNAs existing at both outside regions of a chromosomal DNA into which the introduction of deletion, substitution or addition of nucleotide(s) is intended, or DNAs homologous thereto are directly ligated into the transformant obtained by the above method 1, and deleting the drug resistance gene integrated into chromosomal DNA by the method;
Method 3:
   [1] introducing the linear DNA of (c) above into a host microorganism and selecting a transformant wherein the linear DNA has been integrated into chromosomal DNA by homologous recombination using drug resistance as an indicator,
   [2] synthesizing a DNA obtained by ligating DNAs homologous to DNAs located at both outside regions of a target region of substitution or deletion on a chromosomal DNA in the same direction as that on the chromosomal DNA and introducing the synthesized product into the transformant obtained in [1] above, and
   [3] culturing transformants subjected to the procedure of [2] above under conditions where the genes that can be used for negative selection are expressed, and selecting strains that can grow in the culture condition as strains in which the drug resistance genes and genes that can be used for negative selection have been deleted from the chromosomal DNA; and
Method 4:
   [1] introducing the linear DNA of (d) above into a host microorganism and selecting a transformant wherein the linear DNA has been integrated into chromosomal DNA by homologous recombination using drug resistance as an indicator, and
   [2] introducing Flp recombinase gene-expression plasmid into the transformant obtained in the above [1] to express the Flp recombinase gene, and obtaining strains sensitive to the drug used in the above[1].

A method of introducing a linear DNA into a host microorganism that is employed in the above methods can be any method as long as the DNA can be introduced into the microorganism. Examples thereof include a method involving the use of calcium ions [Proc. Natl. Acad. Sci., U.S.A., 69, 2110, 1972], the protoplast method [Japanese Published Unexamined Patent Application No. 2483942/88 (1988)], and electroporation [Nucleic Acids Res., 16, 6127, 1988].

By using the DNA comprising at a site around the center thereof any gene to be inserted into a chromosomal DNA as the DNA that is used in method 2 or method 3 [2], drug resistance genes or the like can be deleted, and at the same time, any genes can be introduced into the chromosomal DNA.
According to the methods 2 to 4 above, foreign genes, such as drug resistance genes and genes that can be used for negative selection, do not remain in the chromosomal DNA of the transformant which is finally obtained. With the utilization of such methods, accordingly, the procedures of Method 2, Methods 3[1] to [3] or method 4[1] [2] can be repeated by using the same drug resistance genes and genes that can be used for negative selection. This enables the easy production of *E. coli* having deletion of gene or DNA at two or more different regions on a chromosomal DNA.

The amounts of cells of the *E. coli* mutant strains obtained by such method are greater than those of wild-type *E*. *coli* strains after a given period of culture. This can be confirmed by, for example, culturing the wild-type parental *E*. *coli* strains and the mutant strains using M9 minimal medium that is composed of 1% glucose, 0.6% disodium phosphate, 0.3% potassium dihydrogenphosphate, 0.05% sodium chloride, 0.1% ammonium chloride, 2 mmol/l of magnesium sulfate heptahydrate, 10 mg/l of iron sulfate and 100 µmol/l of calcium chloride, and assaying absorption of culture products at 660 nm at a given frequency, or assaying an ATP content per unit of medium via a conventional technique.

Also, the *E*. *coli* mutant strain of the present invention can be produced by a method wherein a gene or DNA on chromosomal DNA of *E. coli* capable of producing a useful substance is deleted or a method wherein the capacity for producing a useful substance is imparted to *E*. *coli* from which a gene or DNA on chromosomal DNA has been deleted.
*E. coli* capable of producing a useful substance may be any *E*. *coli,* provided that it is capable of producing one or more types of useful substances. Examples of such *E*. *coli* include a strain isolated from nature that is capable of producing a useful substance, and *E*. *coli* to which the ability to produce a desirable useful substance has been artificially imparted by known methods.

Examples of such known methods include:
(a) a method for reducing or removing at least one mechanism for regulating biosynthesis of a useful substance;
(b) a method for increasing the expression level of at least one enzyme associated with biosynthesis of a useful substance;
(c) a method for increasing the copy number of at least one enzyme gene associated with biosynthesis of a useful substance;
(d) a method for attenuating or blocking at least one metabolic pathway which branches from a biosynthetic pathway of a useful substance into metabolites other than the useful substance; and
(e) a method for selecting a strain that exhibits higher resistance to an analogue of a useful substance than a wild-type strain. Such conventional methods can be used alone or in combinations.

When useful substances are amino acids in the specific methods (a) to (e) above, for example, method (a) is described in Agric. Biol. Chem., 43, 105-111, 1979, J. Bacteriol., 110, 761-763, 1972, and Appl. Microbiol. Biotechnol., 39, 318-323, 1993; method (b) is described in Agric. Biol. Chem., 43, 105-111, 1979 and J. Bacteriol., 110, 761-763, 1972; method (c) is described in Appl. Microbiol. Biotechnol., 39, 318-323, 1993 and Agric. Biol. Chem., 39, 371-377, 1987; method (d) is described in Appl. Environ. Microbiol., 38, 181-190, 1979 and Agric. Biol. Chem., 42, 1773-1778, 1978; and method (e) is described in Agric. Biol. Chem., 36, 1675-1684, 1972, Agric. Biol. Chem., 41, 109-116, 1977, Agric. Biol. Chem., 37, 2013-2023, 1973, and Agric. Biol. Chem., 51, 2089-2094, 1987. With reference to these literatures, microorganisms capable of forming and accumulating various amino acids can be produced.

Also, many examples of a method for producing microorganisms capable of forming and accumulating amino acids by any of or two or more of methods (a) to (e) above or a combination thereof are described in Biotechnology 2nd ed., vol. 6, Products of Primary Metabolism (VCH Verlagsgesellschaft mbH, Weinheim, 1996), section 14a or 14b, Advances in Biochemical Engineering/Biotechnology 79, 1-35, 2003, or Amino san Hakko (Amino acid fermentation), Japan Scientific Societies Press, Hiroshi Aida et al. (1986). In addition, many reports have been made concerning a specific method for producing microorganisms capable of forming and accumulating amino acids. Examples thereof include Japanese Published Unexamined Patent Application No. 2003-164297, Agric. Biol. Chem., 39, 153-160, 1975, Agric. Biol. Chem., 39, 1149-1153, 1975, Japanese Published Unexamined Patent Application No. 13599/83(1983), J. Gen. Appl. Microbiol., 4, 272-283, 1958, Japanese Published Unexamined Patent Application No. 94985/88(1988), Agric. Biol. Chem., 37, 2013-2023, 1973, WO 97/15673, Japanese Published Unexamined Patent Application No. 18596/81(1981), Japanese Published Unexamined Patent Application No. 144092/81(1981), and Japanese Published Unexamined Patent Application No. 2003-511086. With reference to these literatures, *E*. *coli* mutants capable of producing one or more kinds of amino acids can be produced. Also, *E. coli* capable of producing other useful substance can be produced by reinforcement of enzymes for biosynthesis of said useful substance, and modification of biosynthesis regulatory mechanism, excretion mechanism and the like in the same way as in the production of *E*. *coli* mutant strain capable of producing an amino acid.

### 3. Method for producing useful substances of the present invention

The present invention relates to a method for producing useful substances characterized by culturing the *E*. *coli* mutant strain of the present invention in a medium so as to form and accumulate such useful substances in the culture and recovering the useful substances from the culture.

Useful substances are not particularly limited, provided that such substances can be produced with the use of the E. coli mutant strain of the present invention. Examples thereof include proteins, amino acids, nucleic acids, vitamins, saccharides, organic acids, and lipids. Examples of proteins include inosine kinase, glutamate 5-kinase (EC 2.7.2.11), glutamate-5-semialdehyde dehydrogenase (EC 1.2.1.41), pyrroline-5-carboxylate reductase (EC 1.5.1.2), and human granulocyte-colony-stimulating factors. An example of peptide is glutathione. Examples of amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophane, L-phenylalanine, L-serine, L-cysteine, L-3-hydroxyproline, and L-4-hydroxyproline. Examples of nucleic acids include adenosine triphosphate, inosine, guanosine, inosinic acid, and guanylic acid. Examples of vitamins include riboflavin, thiamine, and ascorbic acid. Examples of saccharides include xylose and xylitol. Examples of lipids include eicosapentaenoic acid (EPA) and docosahexaenoic acid (DHA).
The method of producing a useful substance of the present invention also includes a method of producing a useful substance using an enzyme reaction requiring ATP, which comprises using the *E*. *coli* mutant strain of the present invention to produce an enzyme protein catalyzing the enzyme reaction and supplying, from the *E*. *coli* mutant strain of the present invention, ATP necessary for maintaining progress of the reaction catalyzed by the enzyme protein.

The *E*. *coli* mutant strain that is used for the production method of the present invention can be cultured in accordance with a conventional method as described below.
A medium for culturing *E*. *coli* mutant strain which is used for the production method of the present invention may be natural or synthetic medium as long as it contains carbon sources, nitrogen sources, inorganic salts, etc. assimilable by the microorganisms and the microorganisms can be efficiently cultured therein.

Any carbon sources assimilable by the microorganisms can be used. As carbon sources, carbohydrates such as glucose, fructose, sucrose, molasses containing any of such substances, starch, and starch hydrolysate; organic acids such as acetic acid and propionic acid; and alcohols such as ethanol and propanol, and the like can be used.
As nitrogen sources, ammonia, ammonium salts of inorganic or organic acids such as ammonium chloride, ammonium sulfate, ammonium acetate, and ammonium phosphate, peptone, meat extract, yeast extract, corn steep liquor, casein hydrolysate, soybean cake and hydrolysate of soybean cake, and various fermentation microorganisms and digests thereof, and the like can be used.

As inorganic salts, monopotassium phosphate, dipotassium phosphate, magnesium phosphate, magnesium sulfate, sodium chloride, iron(I) sulfate, manganese sulfate, copper sulfate, and calcium carbonate, and the like can be used.
Culturing is carried out under aerobic conditions such as shaking culture or submerged aeration agitation culture. Culturing temperature is preferably 15 to 40°C, and culture time is generally 16 hours to 7 days. During culturing, the pH is preferably maintained at 3.0 to 9.0. The pH is adjusted with an inorganic or organic acid, an alkali solution, urea, calcium carbonate, ammonia, or the like.

When useful substances are formed and accumulated extracellularly, precipitates such as cells are removed from the culture after the completion of culturing, and target useful substances can be isolated and purified from the culture via combined use of ion-exchange treatment, concentration, salting out, and other means.
When useful substances are formed and accumulated intracellularly, cells are recovered from the culture after the completion of culturing and then disrupted via adequate means such as mechanical or chemical means. Target useful substances can be isolated and purified from the solution of disrupted cells via combined use of ion-exchange treatment, concentration, salting out, and other means.

### Example 1

### (1) Production method of reduced-genome strain

According to the chromosome processing methods described in JP-A-2004-166576 and WO2006/57341, namely, a method using sacB gene encoding levansucrase derived from Bucillus subtilis, reduced-genome strains S1 - S10 strains defective in the regions containing the genes shown in the following Table 2 on the chromosome of the Escherichia coli W3110 strain were prepared from the Step28 strain (WO2006/57341) defective in the 1026.7 kbp region on the chromosome of W3110red strain (WO2006/57341), which is a strain wherein the recombination system of Escherichia coli K-12 lineage strain W3110 strain has been replaced by a Red recombination system derived from λ phage. According to the method, primer DNAs are designed based on the nucleotide sequences on both ends of the region desired to be defective on the chromosomal DNA and, using these primer DNAs and chromosomal DNA as a template, PCR reaction is performed to amplify DNA fragment, using which any region on the chromosomal DNA can be eliminated.

### (2) Growth test

Then, the growth was compared among the W3110red strain, reduced-genome strain S28 strain (WO2006/57341) and S1 - S10 strains. The gene group wherein step28 strain is defective on the chromosomal DNA is shown in Table 2.

**[Table 2-1]**

| **Strain name** | **Deleted gene** |
|---|---|
| W311Ored | None |
| Step 28 strain | [A] fhuA, fhuC, fhuD, fhuB, yafJ, yafK, yafQ, dinJ, yafL, yafM, fhiA, mbhA, dinB, yafN, yafO, yafP, ykfJ, prfH, yagP, yagQ, yagR, yagS, yagT, yagU, ykgJ, yagV, yagW, yagX, yagY, yagZ, ykgK, ykgL, ykgM(JW5034), ykgM(JW5035), eaeH, ykgA, ykgB, ykgI, ykgC, ykgD, ykgE, ykgF, ykgG, ykgH, betA, betB, betI, betT, yahA, yahB, yahC, yahD ,yahE, yahF, yahG, yahH, yahI, yahJ, yahK, yahL, yahM, yahN, yahO, PrPR, prpB, prpC, prpD, prpE, codB, codA, cynR, cynT, cynS, eynX, lacA, lacY, lacZ, lacI, mhpR, mhpA, mhpB, mhpC, mhpD, mhpF, mhpE, mhpT, yaiL, frmB, frmA, frmR, yaiO, yaiX, yaiF, yaiP, yaiS, tauA, tauB, tauC, tauD, yaiT, yaiU, yaiV, ampH, sbmA, yaiW, yaiY, yaiZ, ddlA, yaiB, phoA, psiF, yaiC, ybbO, tesA, ybbA, ybbP, ylbG, ybbB, ybbS, allA, allR, glxR, ybbV, ybbW, allB, ybbY, glxK, ylbA, allC, allD, fdrA, ylbE_1, ylbE_2, ylbF, ybcF, envY, ybcH, nfrA, nfrB, cusS, cusR, cusC, cusF, cusB, cusA, pheP, ybdG, nfnB, ybdF, ybdJ, ybdK, hakE, ybeL, ybeQ, ybeR, djlB, ybeT, ybeU, djlC, hscG, rihA, gltL, gltK, gltJ, gltI, kdpF, ybfA, ybgA, phr, ybiA, dinG, ybiB, ybiC, ybiJ, ybiI, ybiX, fiu, ybiM, ybiN, ybiO, glnQ, glnP, glnH, yliA, yliB, YliC, YliD, yliE, yliF, yliG, yliH, yliI, yliJ, dacC, deoR, ybjG, ybjC, nfsA, rimK, yhjN, potF, potG, potH, potI, ybjO, rumB, artJ, artM, artQ, artI, artP, ybjP, ybjQ, ybjR, ybjS, ybjT, ltaE, ymcC, ymcD, cspH, cspG, ymcE, gnsA, yccM, torS, torT, torR, torC, torA, torD, cbpM, cbpA, yceE, agp, yccJ, wrbA, ymdF, ycdG, ycdH, ycdI, rarA, ycdK, ycdL, ycdM, ycdC, putA, putP, ycdN_1, ycdN_2, ycdO, ycdB, phoH, ycdP, ycdQ, ycdR, ycdS, ycdT, ymdE, ycdU, elbA, ycgX, ycgE, ycgF, ycgZ, ymgA, ymgB, ymgC, ycgG, ymgF, ycgH_1, ycgH_2, ymgD, ymgG, ymgH, ycgI, ydfH, ydfZ, ydfI, ydfJ, yecT, flhE, flhA, flhB, cheZ, cheY, cheB, cheR, tap, tar, cheW, cheA, motB, motA, flhC, flhD, yecG, otsA, otsB, araH, araG, araF, yecI, yecJ, isrB, yecR, ftn, yecH, tyrP, uvrC, uvrY, yecF, sdiA, yecC, yecS, yedO, fliY, fliZ, fliA, fliC, fliD, fliS, fliT, amyA, yedD, yedE, yedF, yedK, |

**Table 2-2**

| **Strain name** | **Deleted gene** |
|---|---|
| Step 28 strain (continued) | yedL, yedN_2, yedN_1, yedM, intG, fliE, fliF, fliG, fliH, fliI, fliJ, fliK, fliL, fliM, fliN, fliO, fliP, fliQ, fliR, resA, dsrB, yodD, yedA, vsr, dcm, yedJ, yedR, yedS_1, yedS_2, yedS_3, hehA, yedV, yedW, yedX, yedY, yedZ, yodA, wbbL_2, wbbL_1, wbbK, wbbJ, wbbI, wbbH, glf, rfbX, rfbC, rfbA, rfbD, rfbB, yegE, alk4, yegD, yegI, yegJ, yegK, yegL, ryeC, ryeD, mdtA, mcdtB, mdtC, mdtd, baeS, baeR, yegP, yegQ, ryeE, ogrk, yegZ, yegR, yegS, gatR_1, gatR_2, gatD, gatC, gatB, gatA, gatZ, gatY, fbaB, yegT, yegU, yegV, yegW, yegX, molR_1, molR_2, molR_3, yehI, yehk, yehL, yehM, yehP, yehQ, yehR, yehS, yehT, yehU, mlrA, yohO, yehW, yehX, yehY, yehZ, bglX, dld, pbpG, yohC, yeiI, yeiJ, rihB, yeiL, yeiM, yeiN, yeiC, elaB, elaA, elaC, elaD, yfbK, yfbL, yfbM, yfbN, yfbO, yfbP, yfcC, yfcD, yfcE, yfcF, yfcG, folX, yfcH, yfcI, hisP, hisM, hisQ, hisJ, argT, ypdA, ypdB, ypdC, ypdD, ypdE, ypdF, ypdG, ypdH, pinH, ypjB, ypjC, ileY, ygaQ, ygaR, yqaC, yqaD, ygaT, csiR, ygaU, yqaE, ygaV, ygaP, stpA, ygaW, ygaC, ygaM, nrdH, nrdI, nrdE, proV, proW, proX, ygaX, ygaY, ygaZ, ygaH, srlA, srlE, srlB, srlD, gutM, srlR, gutQ, yqeG, yqeH, yqeI, yqeJ, yqek, ygeF, ygeG, ygeH, ygeI, pbl, ygeK, ygeL, ygeM, ygeN, ygeO, ygeP, ygeQ, glyU, ygeR, xdhA, xdhB, xdhC, ygeV, ygeW, ygeX, ygeY, hyuA, yqeA, yqeB, yqeC, ygfJ, ygfK, ssnA, ygfM, xdhD, ygfO, guaD, ygfQ, ygfS, ygfT, ygfU, idi, cmtA, cmtB, yghD, yghE, yghF, yghG, pppA, yghJ, yghK, glcB, glcG, glcF, glcE, glcD, glcC, yghO, yghQ, yghR, yghS, yghT, ygiS, ygiT, ygiU, ygiV, ygiW, qseB, qseC, ygiZ, mdaB, ygiN, gltF, yhcA, yhcD, yhcE, yhcF, yhcG yhcH, nanK, nanE, yhdJ, yhdU, envR, acrE, acrF, yhdV, yhdW, yhdX, yhdY, yijP, yijO, frwD, pflC, pflD, frwB, frwC, ptsA, rhaT, rhaR, rhaS, rhaB, rhaA, rhaD, yiiL, frvA, frvB, frvX, frvR, yiiG, yiiF, yiiE, yiiD, dtd, rbn, yihX, yihW, yihV, yihU, yihT, yihS, yihR, yihQ, yihP, yihO, ornpL, yihN, yihM, yihL, hsrA, rbsR, rbsK, rbsB, rbsC, rbsA, rbsD, trkD, yieN, yieM, asnA, yidB, yidA, yidX, dgoR, dgoK, dgoA, dgoD, dgoT, cbrA, yidR, yidQ, yidE, yidP, glvC, glvB, glvG, ysdC, yidL, yidK, |

**Table 2-3**

| **Strain name** | **Deleted gene** |
|---|---|
| Step 28 (continued) strain | yidJ, yidI, yidH, yidG, yidF, yhiN, yhiM, yhiL, yhiK, yhiJ, yhiI rbbA, yhhJ, chiA, bfd, bifr, gspO, gspM, gspL, gspK, gspJ, gspI, gspH, gspG, gspF, gspE, gspD, gspC, gspA, pioO, yjbE, yjbF, yjbG, yjbH, yjbA, xylE, malG, malF, malE, malK, lamB, malM, yjbI, yjcP, yjcQ, yjcS, alsK, alsE, alsC, alsA, alsB, yjdP, phnP, phnO, phnN, yjcR,phnL, phnK, phnM, phriJ, phnI, phnH, phnG, phnF, phnE(JW4064), phnE(JW4065), phnD, phnC, phnB, phnA, yjdA, yjcZ, yjfI, yjfJ, yjfK, yjfL, yjfM, yjfC, aidB, yjfN, yjfO, yjfP, ulaR, ulaG, ulaA, ulaB, ulaC, ulaD, ulaE, yjhS, yjhT, yjhA, fimB, fimE, fimA, fimI, fimC, fimD, fimF, fimG, fimH, gntP, uxuA, uxuB, uxuR, yjiC, yjiD, yjiE, iadA, yjiG, yjiH, kptA, yjiJ, yjiK, yjiL, yjiM, yjiN, yjiO, yjiP, yjiQ, yjiR, yjiS, yjiT, yjiV, mcrC, mcrB, yjiW, hsdS, hsdM, hsdR, mrr, yjiA, yjiX, yjiY, tsr, yjiZ, yjjM, yjjN, mdoB, yjjA [B] chpA |
| S1 strain | Gene groups deleted in Step 28 strain , uspG, ybdR, rnk, rna, citT, citG, citX, citF, citE, citD, citC, citA, citB, dcuC, crcA, cspE, crcB, ybeH and ybeM |
| S2 strain | Among gene groups deleted in Step 28 strain, gene groups recited in [A] uspG, ybdR, rnk, rna, citT, citG, citX, citF, citE, citD, citC, citA, citB, dcuC, crcA, cspE, crcB, ybeH, ybeM, yqcA, yqcB, yqcC, syd, yqcD, ygdH, sdac, sdaB exo, fuc0, fucA, fucP, fucI, fucK, fucU, fucR, ygdE, ygdD, gcvA, ygdI, csdA, and ygfK |
| S3 strain | Gene groups deleted in S2 strain, araD, araA, araB, araC, yabI, thiQ, thiP, sgrR, and tbpA,setA |
| S4 strain | Gene groups deleted in S3 strain, yhfZ, yhfY, yhfX, yhfW, php, yhfU, yhfT, yhfS, frlR, frlD, frlC, frlB, frlA and yafL |
| S5 strain | Gene groups deleted in S4 strain, pnca, ydjE, ydjF, ydjG, ydjH, ydjI, ydjJ, ydjK, ydjL, yeaC and yeaA |
| S6 strain | Gene groups deleted in S5 strain ade, yicO, yicN, yicM, yicS, nlpA, yicL and setC |

**Table 2-4**

| **Strain name** | **Deleted gene** |
|---|---|
| S7 strain | Gene groups deleted in S6 strain ygjI, ygjJ, ygjK, fadH, ygjM, ygjN, ygjO, ygjP, ygjQ, ygjR, alx, sstT and ygjV |
| S8 strain | Gene groups deleted in S7 sttain yrhB, yrhA, yhhZ, yhhY, yhhX, and yhhW |
| S9 strain | Gene groups deleted in S8 strain yeaK, yoaI, yeaL, yeaM, yeaN, yeaO, yoaF, yeaP, yeaQ, yoaG, yeaR, yeaS, yeaT, yeaU, yeaV, yeaW, and yeaX |
| S10 strain | Gene groups deleted in s9 strain and hyfA, hyfB, hyfC, hyfD, hyfE, hyfF, hyfG, hyfH, hyfI, hyfJ, hyfR, and focB |

Using the strains indicated in Table 2, a growth test was performed. For the growth test, each strain was spread on an LB agar medium (LB medium [Bacto Tryptone (manufactured by Difco) 10 g/l, yeast extract (manufactured by Difco) 10 g/l, sodium chloride 5 g/l] added with Bacto Agar (manufactured by Difco) (15 g/l) and solidified as a plate) containing 30 mg/l kanamycin, and cultured at 37°C overnight to give bacterial cells. A small amount of respective bacterial cells grown on the LB agar medium was seeded on 5 ml of an LB medium containing 30 mg/l kanamycin and cultured at 37°C overnight to give a culture. 300 µl of the culture was seeded on 30 ml of a growth test medium (11.28 g/l 5×M9 premixed salts, 100 mM MOPS buffer, 6% glucose, 1% yeast extract, 2 mM MgSO₄, 0.1 mM CaCl₂, 5 mg/l FeSO₄ pH 6.8, 30 mg/l kanamycin), and cultured with shaking for 24 hr at 30°C. The turbidity of each reduced-genome strain after culture was determined by measuring the absorbance at 660 nm. The turbidity of each reduced-genome strain based on that of the W3110red strain as 100 and the total deletion length of the chromosomal DNA of each reduced-genome strain are shown in Table 3.

[Table 3]

**Table 3**

| **Strain name** | **Total deleted length (kbp)** | **Turbidity at 24th hr (%)** |
|---|---|---|
| W3110red | 0.0 | 100 |
| Step28 strain | 1027.9 | 127 |
| S1 strain | 1046.5 | 133 |
| S2 strain | 1068.6 | 232 |
| S3 strain | 1081. 5 | 221 |
| S4 strain | 1094. 3 | 225 |
| S5 strain | 1104.8 | 235 |
| S6 strain | 1113.6 | 238 |
| S7 strain | 1129.1 | 242 |
| S8 strain | 1135.2 | 240 |
| S9 strain | 1148.4 | 227 |
| S10 strain | 1162.0 | 228 |

### (3) ATP production activity

The ATP production activity of W3110red strain, Step28 strain and S10 strain was each measured by the following method. Each strain was spread on an LB agar medium, and cultured at 30°C overnight. A small amount of the bacterial cells grown on the LB agar medium was scraped and seeded in a 96 well deep well plate containing LB medium (1 ml) containing 3% glucose, and cultured at 30°C for 24 hr. The turbidity after culture was determined by measuring the absorbance at 590 nm. In addition, the ATP production activity per culture medium was measured by the method described in J. Biomol. Screen., 11, 310 (2006). The ATP production activity of Step28 strain and S10 strain per culture medium based on that of the W3110red strain per culture medium as 100 is shown in Table 4. The S10 strain showed a remarkably improved ATP production activity per culture medium.

[Table 4]

**Table 4**

| **Strain name** | **Turbidity at 24th hr (OD590nm)** | **ATP producing activity (%) per culture solution** |
|---|---|---|
| W3110red | 0.79 | 100 |
| Step28 | 1.23 | 133 |
| S10 | 1.98 | 603 |

### Example 2

### Preparation of xylitol producing strain

A strain, wherein the λ Red recombination system retained by the step28 strain and S10 strain was substituted by a wild-type recBCD recombination system, was prepared as follows.
The ygdB gene located near the chromosome where the λRed gene group is present is substituted by a DNA fragment containing chloramphenicol resistance gene and levansucrase gene by the method described in JP-A-2004-166576. The P1 phage obtained from the wild-type strain was transduced into the prepared strain to give a sucrose resistant strain, whereby a strain wherein the vicinity of ygdB gene was substituted by a wild-type chromosome fragment (including recBCD gene group) is obtained. The recBCD strains prepared from the Step28 strain and S10 strain were designated as S28W strain and S10W strain, respectively.

Then, the lacI gene was reintegrated with the S28W strain and S10W strain by the following method. Using the method described in JP-A-2004-166576, a DNA fragment containing chloramphenicol resistance gene and levansucrase gene was inserted into the W3110red strain chromosome while simultaneously deleting the region from the yafW gene to the yahM gene described in Table 1. P1 phage was prepared from said strain, transduced into the S28W strain and S10W strain to give a chloramphenicol resistant strain, whereby lacI revertant, wherein a lacI gene near the marker gene was transduced, was obtained. A marker portion containing chloramphenicol resistance gene and levansucrase gene was removed from the obtained lacI gene revertant by the method described in JP-A-2004-166576. The finally prepared lacI gene revertants derived from S28W strain and S10W strain were designated as S28W_lacI strain and S10W_lacI strain.

Then, using the method described in Appl. Environ. Microbiol., 73, 7657 (2007), a xylitol-producing gene group was introduced into each of the W3110 strain, S28W_lacI strain and S10W_lacI strain to give strains imparted with conversion capacity from xylose to xylitol. Each of the prepared strains was seeded on a medium (30 ml) containing M9 minimal medium added with 6% glucose, 8% xylose, 1% yeast extract, 0.1 mM IPTG, 100 mM MOPS buffer, and cultured with shaking in a 300 ml baffled flask at 30°C, 250 rpm. The xylitol amount in a culture medium at 46 hr culture was measured. As a result, 14.3, 13.5, 20.6 g/l of xylitol was produced in the culture medium by culturing xylitol-producing strains with W3110 strain, S28W_lacI strain and S10W_lacI strain as parental strains, respectively. The productivity of xylitol was the highest in the strain derived the S10 strain.

### Industrial Applicability

Using the *E. coli* mutant strain of the present invention as a host cell, a useful substance can be produced efficiently.

## Claims

1. An *E. coli* mutant strain comprising chromosomal DNA comprising the following genes [1] or homologous genes thereof, not comprising the genes [2] or homologous genes thereof, and being at least 1040 kbp shorter than that of a wild-type *E. coli* strain, provided that when the following genes [1] or homologous genes thereof comprise genes that wild-type *E. coli* strains do not originally contain (hereafter referred to as "NC genes"), the aforementioned *E. coli* mutant strain derived from the wild-type *E. coli* strain does not necessarily have the NC gene.
[1] thrL gene, thrA gene, thrB gene, thrC gene, talB gene, mog gene, htgA gene, dnaK gene, dnaJ gene, mokC gene, hokC gene, sokC gene, nhaA gene, nhaR gene, rpsT gene, ribF gene, ileS gene, lspA gene, fkpB gene, ispH gene, rihC gene, dapB gene, carA gene, carB gene, caiF gene, caiE gene, caiD gene, caiC gene, caiB gene, caiA gene, caiT gene, fixA gene, fixB gene, fixC gene, fixX gene, kefF gene, kefC gene, folA gene, apaH gene, apaG gene, ksgA gene, pdxA gene, surA gene, imp gene, djlA gene, rluA gene, hepA gene, polB gene, leuD gene, leuC gene, leuB gene, leuA gene, leuL gene, leuO gene, ilvI gene, ilvH gene, fruR gene, mraZ gene, mraW gene, ftsL gene, ftsI gene, murE gene, murF gene, mraY gene, murD gene, ftsW gene, murG gene, murC gene, ddlB gene, ftsQ gene, ftsA gene, ftsZ gene, lpxC gene, secM gene, secA gene, mutT gene, coaE gene, guaC gene, hofC gene, hofB gene, ppdD gene, nadC gene, ampD gene, ampE gene, aroP gene, pdhR gene, aceE gene, aceF gene, lpd gene, acnB gene, speD gene, speE gene, cueO gene, gcd gene, hpt gene, can gene, panD gene, panC gene, panB gene, htrE gene, ecpD gene, folK gene, pcnB gene, dksA gene, sfsA gene, ligT gene, hrpB gene, mrcB gene, hemL gene, clcA gene, btuF gene, pfs gene, dgt gene, degP gene, cdaR gene, dapD gene, glnD gene, map gene, tff gene, rpsB gene, tsf gene, pyrH gene, frr gene, dxr gene, ispU gene, cdsA gene, hlpA gene, lpxD gene, fabZ gene, lpxA gene, lpxB gene, rnhB gene, dnaE gene, accA gene, ldcC gene, tilS gene, rof gene, nlpE gene, proS gene, rcsF gene, metQ gene, metI gene, metN gene, gmhB gene, rrsH gene, ileV gene, alaV gene, rrlH gene, rrfH gene, aspU gene, dkgB gene, mltD gene, gloB gene, rnhA gene, dnaQ gene, aspV gene, ivy gene, fadE gene, lpcA gene, pepD gene, gpt gene, frsA gene, crl gene, phoE gene, proB gene, proA gene, thrW gene, hemB gene, proC gene, aroL gene, aroM gene, rdgC gene, mak gene, araJ gene, sbcC gene, sbcD gene, phoB gene, phoR gene, brnQ gene, proY gene, malZ gene, queA gene, tgt gene, secD gene, secF gene, tsx gene, ribD gene, ribE gene, nusB gene, thiL gene, pgpA gene, dxs gene, ispA gene, xseB gene, thiI gene, panE gene, cyoE gene, cyoD gene, cyoC gene, cyoB gene, cyoA gene, ampG gene, bolA gene, tig gene, clpP gene, clpX gene,
lon gene, hupB gene, ppiD gene, cof gene, mdlA gene, mdlB gene, glnK gene, amtB gene, tesB gene, ffs gene, maa gene, hha gene, acrB gene, acrA gene, acrR gene, kefA gene, priC gene, apt gene, dnaX gene, recR gene, htpG gene, adk gene, hemH gene,
aes gene, gsk gene, fsr gene, ushA gene, copA gene, cueR gene, gcl gene, hyi gene, purK gene, purE gene, lpxH gene, ppiB gene, cysS gene, folD gene, sfmA gene, sfmC gene, sfmD gene, sfmH gene, sfmF gene, fimZ gene, argU gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, tatE gene, lipA gene, lipB gene, dacA gene, rlpA gene, mrdB gene, mrdA gene, cobC gene, nadD gene, holA gene, rlpB gene, leuS gene, lnt gene, miaB gene, ubiF gene, glnX gene, glnV gene, metU gene, glnW gene, glnU gene, leuW gene, metT gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, nei gene, abrB gene, gltA gene, sdhC gene, sdhD gene, sdhA gene, sdhB gene, sucA gene, sucB gene, sucC gene, sucD gene, mngR gene, mngA gene, mngB gene, cydA gene, cydB gene, tolQ gene, tolR gene, tolA gene, tolB gene, pal gene, lysT gene, valT gene, lysW gene, valZ gene, lysY gene, lysZ gene, lysQ gene, nadA gene, pnuC gene, zitB gene, aroG gene, gpmA gene, galM gene, galK gene, galT gene, galE gene, modF gene, modE gene, modA gene, modB gene, modC gene, bioA gene, bioB gene, bioF gene, bioC gene, bioD gene, uvrB gene, moaA gene, moaB gene, moaC gene, moaD gene, moaE gene, rhlE gene, dps gene, rhtA gene, ompX gene, rybA gene, mntR gene, fsaA gene, moeB gene, moeA gene, iaaA gene, cmr gene, rybB gene, grxA gene, poxB gene, hcr gene, hcp gene, aqpZ gene, macA gene, macB gene, cspD gene, clps gene, clpA gene, serW gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, ihfB gene, msbA gene, lpxK gene, kdsB gene, smtA gene, mukF gene, mukE gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, serT gene, hyaA gene, hyaB gene, hyaC gene, hyaD gene, hyaE gene, hyaF gene, appC gene, appB gene, appA gene, etp gene, serX gene, csgG gene, csgF gene, csgE gene, csgD gene, csgB gene, csgA gene, csgC gene, mdoC gene, mdoG gene, mdoH gene, msyB gene, mdtG gene, lpxL gene, solA gene, dinI gene, pyrC gene, grxB gene, mdtH gene, rimJ gene, mvim gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, sraB gene, rpmF gene, plsX gene, fabH gene, fabD gene, fabG gene, acpP gene, fabF gene, pabC gene, tmk gene, holB gene, ptsG gene, fhuE gene, hinT gene, nagZ gene, ndh gene, mfd gene, lolC gene, lolD gene, lolE gene, cobB gene, potD gene, potC gene, potB gene, potA gene, pepT gene, phoQ gene, phoP gene, purB gene, hflD gene, trmU gene, minE gene, minD gene, minC gene, hlyE gene, umuD gene, umuC gene, dsbB gene, nhaB gene, fadR gene, dadA gene, dadX gene, cvrA gene, ldcA gene, emtA gene, treA gene, dhaH gene, dhaL gene, dhaK gene, dhaR gene, pth gene, prsA gene, ispE gene, lolB gene, hemA gene, prfA gene, prmC gene, kdsA gene, IdrA gene, rdlA gene, IdrB gene, rdlB gene, IdrC gene, rdlC gene, chaA gene, chaB gene, chaC gene, narL gene, narX gene, narK gene, narG gene, narH gene, narJ gene, narI gene, rttR gene, tpr gene, tyrV gene, tyrT gene, purU gene, rssA gene, rssB gene, galU gene, hns gene, tdk gene, adhE gene, oppA gene, oppB gene,
oppC gene, oppD gene, oppF gene, cls gene, kch gene, tonB gene, ompW gene, trpA gene, trpB gene, trpC gene, trpD gene, trpE gene, trpL gene, rluB gene, btuR gene, sohB gene, topA gene, cysB gene, acnA gene, ribA gene, pgpB gene, pyrF gene, osmB gene, gmr gene, rnb gene, fabI gene, sapF gene, sapD gene,
sapC gene, sapB gene, sapA gene, puuP gene, puuA gene, puuD gene, puuR gene, puuC gene, puuB gene, puuE gene, pspF gene, pspA gene, pspB gene, pspC gene, pspD gene, pspE gene, ompG gene, tyrR gene, tpx gene, mpaA gene, mppA gene, uspE gene,
fnr gene, ogt gene, abgT gene, abgB gene, abgA gene, abgR gene, isrA gene, dbpA gene, uspF gene, ompN gene, micC gene, hslJ gene, ldhA gene, feaR gene, feaB gene, tynA gene, maoC gene, paaA gene, paaB gene, paaC gene, paaD gene, paaE gene, paaF gene, paaG gene, paaH gene, paaI gene, paaJ gene, paaK gene, paaX gene, paaY gene, azoR gene, hrpA gene, aldA gene, gapC_2 gene, gapC_1 gene, cybB gene, hokB gene, mokB gene, sokB gene, trg gene, mdoD gene, rimL gene, tehA gene, tehB gene, ansP gene, nhoA gene, narV gene, narW gene, narY gene, narZ gene, narU gene, fdnG gene, fdnH gene, fdnI gene, adhP gene, sfcA gene, sra gene, bdm gene, osmC gene, ddpF gene, ddpD gene,
ddpC gene, ddpB gene, ddpA gene, ddpX gene, dos gene, gadC gene, gadB gene, pqqL gene, hipA gene, hipB gene, ego gene, lsrC gene, lsrD gene, lsrB gene, lsrF gene, lsrG gene, tam gene, uxaB gene, marC gene, marR gene, marA gene, marB gene, eamA gene, dcp gene, rspB gene, rspA gene, speG gene, dmsD gene, clcB gene, dgsA gene, asr gene, mdtI gene, mdtJ gene, pntB gene, pntA gene, folM gene, rstA gene, rstB gene, tus gene, fumC gene, fumA gene, manA gene, uidC gene, uidB gene, uidA gene, uidR gene, hdhA gene, malI gene, malX gene, malY gene, add gene, blr gene, rsxA gene, rsxB gene, rsxC gene,
rsxD gene, rsxG gene, rsxE gene, nth gene, gst gene, pdxY gene, tyrS gene, pdxH gene, slyB gene, slyA gene, sodC gene, nemA gene, gloA gene, rnt gene, lhr gene, sodB gene, purR gene, cfa gene, ribC gene, mdtK gene, valV gene, valW gene, pykF gene, lpp gene, sufE gene, sufS gene, sufD gene, sufC gene, sufB gene, sufA gene, rydB gene, rprA gene, aroD gene, pps gene, aroH gene, nlpC gene, btuD gene, btuE gene, btuC gene, ihfA gene, pheT gene, pheS gene, pheM gene, rplT gene, rpmI gene, infC gene, thrS gene, arpB_1 gene, arpB_2 gene, pfkB gene,
cedA gene, katE gene, chbG gene, chbF gene, chbR gene, chbA gene, chbC gene, chbB gene, osmE gene, nadE gene, spy gene, astE gene, astB gene, astD gene, astA gene, astC gene, xthA gene, nudG gene, gdhA gene, topB gene, selD gene, sppA gene, ansA gene, gapA gene, mipA gene, rnd gene, fadD gene, pabB gene, sdaA gene, manX gene, manY gene, manZ gene, rrmA gene, cspC gene, kdgR gene, htpX gene, prc gene, proQ gene, pphA gene, ryeA gene, ryeB gene, holE gene, exoX gene, ptrB gene, purT gene, eda gene, edd gene, zwf gene, pykA gene, lpxM gene, znuA gene, znuC gene, znuB gene, ruvB gene, ruvA gene, ruvC gene, nudB gene, aspS gene, torZ gene, torY gene, cutC gene, argS gene, leuZ gene, cysT gene, glyW gene, pgsA gene, dsrA gene, serU gene, asnT gene, shiA gene, amn gene, asnW gene, asnU gene, cbl gene, nac gene, asnV gene, erfK gene, cobT gene, cobs gene, cobU gene, sbmC gene, dacD gene, sbcB gene, hisL gene, hisG gene, hisD gene, hisC gene, hisB gene, hisH gene, hisA gene, hisF gene, hisI gene, cld gene, ugd gene, gnd gene, galF gene, wcaM gene, wcaL gene, wcaK gene, wzxC gene, wcaJ gene, cpsG gene, cpsB gene, wcaI gene, nudD gene, fcl gene,
gmd gene, wcaF gene, wcaE gene, wcaD gene, wcaC gene, wcaB gene, wcaA gene, wzc gene, wzb gene, wza gene, asmA gene, dcd gene, udk gene, thiD gene, thiM gene, mrp gene, metG gene,
dusC gene, cdd gene, sanA gene, mglC gene, mglA gene, mglB gene, galS gene, folE gene, cirA gene, lysP gene, nfo gene, fruA gene, fruK gene, fruB gene, setB gene, spr gene, rtn gene, bcr gene, rsuA gene, rplY gene, proL gene, narP gene, ccmH gene, ccmG gene, ccmF gene, ccmE gene, ccmD gene, ccmC gene, ccmB gene, ccmA gene, napC gene, napB gene, napH gene, napG gene, napA gene, napD gene, napF gene, eco gene, mqo gene,
alkB gene, ada gene, ompC gene, micF gene, rcsD gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, ais gene, arnT gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoC gene, nuoB gene, nuoA gene, lrhA gene, ackA gene, pta gene, ubiX gene, purF gene, cvpA gene, dedD gene, folC gene, accD gene, dedA gene, truA gene, usg gene, pdxB gene, flk gene, fabB gene, trmC gene, mepA gene, aroC gene, prmB gene, sixA gene, fadL gene, vacJ gene, argW gene, dsdC gene, dsdX gene, dsdA gene, emrY gene, emrK gene, evgA gene, evgS gene, oxc gene, frc gene, ddg gene, glk gene, mntH gene, nupC gene, alaX gene, alaW gene, gltX gene, valU gene, valX gene, valY gene, lysV gene, xapR gene, xapB gene, xapA gene, ligA gene, zipA gene, cysZ gene, cysK gene, ptsH gene, ptsI gene, crr gene, pdxK gene, cysM gene, cysA gene, cysW gene, cysU gene, cysP gene, ucpA gene, murP gene, amiA gene, hemF gene, eutL gene, eutC gene, eutB gene, eutA gene, eutH gene, eutG gene, eutJ gene, eutE gene, cchB gene, cchA gene, eutI gene, eutT gene, eutQ gene, eutP gene, maeB gene, talA gene, tktB gene, aegA gene, narQ gene, acrD gene, dapE gene, purC gene, nlpB gene, dapA gene, gcvR gene, bcp gene,
hda gene, uraA gene, upp gene, purM gene, purN gene, ppk gene, ppx gene, guaA gene, guaB gene, xseA gene, der gene, hisS gene, ispG gene, ndk gene, pbpC gene, sseA gene, ryfA gene, sseB gene, pepB gene, fdx gene, hscA gene, hscB gene, iscA gene, iscU gene, iscS gene, iscR gene, suhB gene, csiE gene, hcaT gene, hcaR gene, hcaE gene, hcaF gene, hcaC gene, hcaB gene, hcaD gene, glyA gene, hmp gene, glnB gene, sroF gene, purL gene, tadA gene, acpS gene, pdxJ gene, recO gene, era gene,
rnc gene, lepB gene, lepA gene, rseC gene, rseB gene, rseA gene, rpoE gene, nadB gene, srmB gene, ung gene, trxC gene, pssA gene, kgtP gene, rrfG gene, rrlG gene, gltw gene, rrsG gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, ssrA gene, gabD gene, gabT gene, gabP gene, nrdF gene, mprA gene, emrA gene, emrB gene, luxS gene, sraD gene, gshA gene, argQ gene, argZ gene, argY gene, argV gene, serV gene, csrA gene, alas gene, recX gene, recA gene, mltB gene, norR gene, norV gene, norW gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, pphB gene, rpoS gene, nlpD gene, pcm gene, surE gene, truD gene, ispF gene, ispD gene, ftsB gene, cysC gene, cysN gene, cysD gene, iap gene, cysH gene, cysI gene, cysJ gene, eno gene, pyrG gene, mazG gene, chpR gene, relA gene, rumA gene, barA gene, gudD gene, gudX gene, gudP gene, mltA gene, metZ gene, metW gene, metV gene, amiC gene, argA gene, recD gene, recB gene, ptr gene, recC gene, ppdC gene, ppdB gene, ppdA gene, thyA gene, lgt gene, ptsP gene, nudH gene, mutH gene, tas gene, aas gene, rygA gene, rygB gene, galR gene, lysA gene, lysR gene, araE gene, kduD gene, kduI gene, lysS gene, prfB gene, recJ gene, dsbC gene, xerD gene, fldB gene, bglA gene, gcvP gene, gcvH gene, gcvT gene, visC gene, ubiH gene, pepP gene, zapA gene, ssrS gene, rygC gene, serA gene, rpiA gene, argP gene, argK gene, argO gene, mscS gene, fbaA gene, pgk gene,
epd gene, tktA gene, speB gene, speA gene, metK gene, galP gene, sprT gene, endA gene, gshB gene, ansB gene, mutY gene, mltC gene, nupG gene, speC gene, pheV gene, pitB gene, gss gene, hybG gene, hybF gene, hybE gene, hybD gene, hybC gene, hybB gene, hybA gene, hybO gene, exbD gene, exbB gene, metC gene, dkgA gene, sufI gene, plsC gene, parC gene, parE gene, cpdA gene, nudF gene, tolC gene, zupT gene, ribB gene, glgS gene, rygD gene, rfaE gene, glnE gene, htrG gene, cca gene, bacA gene, folB gene, ttdA gene, ttdB gene, rpsU gene, dnaG gene, rpoD gene, ileX gene, aer gene, ebgR gene, ebgA gene, ebgC gene, uxaA gene, uxaC gene, exuT gene, exuR gene, tdcG gene, tdcF gene, tdcE gene, tdcD gene, tdcC gene, tdcB gene, tdcA gene, tdcR gene, rnpB gene, garK gene, garR gene, garL gene, garP gene, garD gene, sohA gene, agaR gene, kbaZ gene, agaV gene, agaW gene, agaA gene, agaS gene, kbaY gene, agaB gene, agaC gene, agaD gene, agaI gene, mtr gene, deaD gene, nlpI gene, pnp gene, sraG gene, rpsO gene, truB gene, rbfA gene, infB gene, nusA gene, metY gene, argG gene, leuU gene, secG gene, glmM gene, folP gene, ftsH gene, rrmJ gene, greA gene, dacB gene, obgE gene, rpmA gene, rplU gene, ispB gene, sfsB gene, murA gene, kdsD gene, kdsC gene, rpoN gene, ptsN gene, npr gene, mtgA gene, elbB gene, ryhA gene, arcB gene, gltB gene, gltD gene, nanT gene, nanA gene, nanR gene, dcuD gene, sspB gene, sspA gene, rpsI gene, rplM gene, degQ gene, degS gene, mdh gene, argR gene, aaeB gene, aaeA gene, aaeX gene, aaeR gene, tldD gene, rng gene, mreD gene, mreC gene, mreB gene, accB gene, accC gene, panF gene, prmA gene, dusB gene, fis gene, rrfF gene, thrV gene, rrfD gene, rrlD gene, gltv gene, rrsD gene, purH gene, purD gene, zraR gene, zraS gene, zraP gene, hupA gene, nfi gene, hemE gene, nudC gene, rsd gene, thiC gene, thiE gene, thiF gene, thiS gene, thiG gene, thiH gene, htrC gene, rpoC gene, rpoB gene, rplL gene, rplJ gene, rplA gene, rplK gene, nusG gene, secE gene, tufB gene, thrT gene, glyT gene, tyrU gene, thrU gene, coaA gene, birA gene, murB gene, rrfB gene, rrlB gene, gltT gene, rrsB gene, murI gene, btuB gene, trmA gene, fabR gene, sthA gene, oxyR gene, oxyS gene, argH gene, argB gene, argC gene, argE gene, ppc gene, fsaB gene, gldA gene, katG gene, metF gene, metL gene, metB gene, metJ gene, rpmE gene, priA gene, cytR gene, ftsN gene, hslV gene, hslU gene, menA gene, rraA gene, glpf gene, glpK gene, glpX gene, fpr gene, tpiA gene, cdh gene, sbp gene, pfkA gene, fieF gene, cpxP gene, cpxR gene, cpxA gene, kdgT gene, sodA gene, fdhD gene, fdoG gene, fdoH gene, fdoI gene, fdhE gene, bipA gene, glnA gene, glnL gene, glnG gene, hemN gene, csrC gene, spf gene, polA gene, dsbA gene, mobA gene, mobB gene, rrfA gene, rrlA gene, alaT gene, ileT gene, rrsA gene, hemG gene, trkH gene, pepQ gene, fadB gene, fadA gene, fre gene, ubiD gene, rfaH gene, tatD gene, tatC gene, tatB gene, tatA gene, ubiB gene, ubiE gene, rmuC gene, udp gene, metE gene, metR gene, pldB gene, rhtB gene, rhtC gene, recQ gene, pldA gene, rarD gene, corA gene, uvrD gene, xerC gene, dapF gene, cyaY gene, cyaA gene, hemC gene, hemD gene, hemX gene, hemY gene, ryiA gene, aslA gene, aslB gene, proM gene, leuT gene, hisR gene, argX gene, rffM gene, wzyE gene, rffT gene, wzxE gene, rffA gene, rffC gene, rffH gene, rffG gene, rffD gene, rffE gene, wzzE gene, rfe gene, rho gene, rhoL gene, trxA gene, rhlB gene, gpp gene, rep gene, ppiC gene, ilvC gene, ilvY gene, ilvA gene, ilvD gene, ilvE gene, ilvM gene, ilvG_2 gene, ilvG_1 gene, ilvL gene, hdfR gene, trpT gene, aspT gene, rrfC gene, rrlC gene, gltU gene, rrsC gene, asnC gene, mioC gene, oriC gene, gidA gene, gidB gene, atpI gene, atpB gene, atpE gene, atpF gene, atpH gene, atpA gene, atpG gene, atpD gene, atpC gene, glmU gene, glms gene, pstS gene, pstC gene, pstA gene, pstB gene, phoU gene, bglG gene, bglF gene, bglB gene, bglH gene, cbrC gene, mdtL gene, tnaB gene, tnaA gene, tnaC gene, trmE gene, rnpA gene, rpmH gene, dnaA gene, dnaN gene, recF gene, gyrB gene, ibpA gene, ibpB gene, emrD gene, ivbL gene, ilvB gene, ilvN gene, uhpA gene, uhpB gene, uhpC gene, uhpT gene, selC gene, glts gene, recG gene, trmH gene, spoT gene, rpoZ gene, gmk gene, ligB gene, dinD gene, rph gene, pyrE gene, ttk gene, dut gene, dfp gene, rpmB gene, rpmG gene, mutM gene, coaD gene, kdtA gene, rfaQ gene, rfaG gene, rfaP gene, rfaS gene, rfaB gene, rfaI gene, rfaJ gene, rfaY gene, rfaZ gene, rfaK gene, rfaL gene, rfaC gene, rfaF gene, rfaD gene, htrL gene, kbl gene, tdh gene, envC gene, gpmI gene, grxC gene, secB gene, gpsA gene, cysE gene, lldD gene, lldR gene, lldP gene, mtlR gene, mtlD gene, mtlA gene, selA gene, selB gene, aldB gene, sgbE gene, sgbU gene, sgbH gene, lyxK gene, avtA gene, malS gene, bax gene, xylR gene, xylH gene, xylG gene, xylF gene, xylA gene, xylB gene, glyQ gene, glyS gene, hokA gene, cspA gene, tiaE gene, bisC gene, tag gene, eptB gene, proK gene, dppA gene, dppB gene, dppC gene, dppD gene, dppF gene, rdlD gene, ldrD gene, bcsG gene, bcsF gene, bcsE gene, bcsA gene, bcsB gene, bcsZ gene, bcsC gene, dctA gene, kdgK gene, treF gene; gadA gene, gadX gene, gadY gene, gadW gene, mdtF gene, mdtE gene, gadE gene, hdeD gene, hdeA gene, hdeB gene, slp gene, arsC gene, arsB gene, arsR gene, gor gene, prlC gene, uspA gene, pitA gene, nikR gene, nikE gene, nikD gene, nikC gene, nikB gene, nikA gene, acpT gene, dcrB gene, zntA gene, ftsY gene, ftsE gene, ftsX gene, rpoH gene, livJ gene, livK gene, livH gene, livM gene, livG gene, livF gene, ugpB gene, ugpA gene, ugpE gene, ugpC gene, ugpQ gene, ggt gene, gntR gene, gntK gene, gntU gene, asd gene, glgB gene, glgX gene, glgC gene, glgA gene, glgP gene, glpD gene, glpE gene, glpG gene, glpR gene, rtcR gene, rtcB gene, rtcA gene, malT gene, malP gene, malQ gene, gntT gene, gntY gene, gntX gene, bioH gene, feoB gene, feoA gene, greB gene, ompR gene, envZ gene, pck gene, hslO gene, hslR gene, nudE gene, mrcA gene, hofQ gene, aroK gene, aroB gene, damX gene, dam gene, rpe gene, gph gene, trpS gene, cysG gene, nirC gene, nirD gene, nirB gene, tsgA gene, ppiA gene, fic gene, pabA gene, argD gene, crp gene, prkB gene, kefG gene, kefB gene, slyD gene, slyX gene, fkpA gene, rpsL gene, rpsG gene, fusA gene, tufA gene, rpsJ gene, rplC gene, rplD gene, rplW gene, rplB gene, rpsS gene, rplV gene, rpsC gene, rplP gene, rpmC gene, rpsQ gene, rplN gene, rplX gene, rplE gene, rpsN gene, rpsH gene, rplF gene, rplR gene, rpsE gene, rpmD gene, rplO gene, secY gene, rpmJ gene, rpsM gene, rpsK gene, rpsD gene, rpoA gene, rplQ gene, zntR gene, mscL gene, trkA gene, rsmB gene, fmt gene, def gene, smf gene, smg gene, aroE gene, rrsE gene, ileU gene, alaU gene, rrlE gene, rrfE gene, metA gene, aceB gene, aceA gene, aceK gene, arpA gene, iclR gene, metH gene, pepE gene, lysC gene, pgi gene, ubiC gene, ubiA gene, plsB gene, dgkA gene, lexA gene, dinF gene, zur gene, qor gene, dnaB gene, alr gene, tyrB gene, aphA gene, uvrA gene, ssb gene, soxS gene, soxR gene, ryjA gene, actP gene, acs gene, nrfA gene, nrfB gene, nrfC gene, nrfD gene, nrfE gene, nrfF gene, nrfG gene, gltP gene, fdhF gene, rpiR gene, rpiB gene, proP gene, basS gene, basR gene, eptA gene, adiC gene, adiY gene, adiA gene, melR gene, melA gene, melB gene, fumB gene, dcuB gene, dcuR gene, dcuS gene, lysU gene, cadA gene, cadB gene, cadC gene, pheU gene, dipZ gene, cutA gene, dcuA gene, aspA gene, fxsA gene, groS gene, groL gene, efp gene, ecnA gene, ecnB gene, sugE gene, blc gene, ampC gene, frdD gene, frdC gene, frdB gene, frdA gene, poxA gene, psd gene, rsgA gene, orn gene, glyV gene, glyX gene, glyY gene, amiB gene, mutL gene, miaA gene, hfq gene, hflX gene, hflK gene, hflC gene, purA gene, rnr gene, rlmB gene, ulaF gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, treC gene, treB gene, treR gene, mgtA gene, pyrI gene, pyrB gene, pyrL gene, argI gene, valS gene, holC gene, pepA gene, idnR gene, idnT gene, idnO gene, idnD gene, idnK gene, leuX gene, dnaC gene, dnaT gene, bglJ gene, fhuF gene, leuV gene, leuP gene, leuQ gene, rsmC gene, holD gene, rimI gene, prfC gene, osmY gene, deoC gene, deoA gene, deoB gene, deoD gene, IplA gene, serB gene, radA gene, nadR gene, slt gene, trpR gene, rob gene, creA gene, creB gene, creC gene, creD gene, arcA gene
[2]fhuA gene, fhuC gene, fhuD gene, fhuB gene, yafJ gene, yafK gene, yafQ gene, dinJ gene, yafL gene, yafM gene, fhiA gene, mbhA gene, dinB gene, yafN gene, yafO gene, yafP gene, ykfJ gene, prfH gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM(JW5034) gene, ykgM(JW5035) gene, eaeH gene, ykgA gene, ykgB gene, ykgI gene, ykgC gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, yahI gene, yahJ gene, yahK gene, yahL gene, yahM gene, yahN gene, yahO gene, prpR gene, prpB gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, frmB gene, frmA gene, frmR gene, yaiO gene, yaiX gene, yaiF gene, yaiP gene, yaiS gene, tauA gene, tauB gene, tauC gene, tauD gene, yaiT gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, ylbG gene, ybbB gene, ybbS gene, allA gene, allR gene, glxR gene, ybbV gene, ybbW gene, allB gene, ybbY gene, glxK gene, ylbA gene, allC gene, allD gene, fdrA gene, ylbE_1 gene, ylbE_2 gene, ylbF gene, ybcF gene, envY gene, ybcH gene, nfrA gene, nfrB gene, cusS gene, cusR gene, cusC gene, cusF gene, cusB gene, cusA gene, pheP gene, ybdG gene, nfnB gene, ybdF gene, ybdJ gene, ybdK gene, hokE gene, ybeL gene, ybeQ gene, ybeR gene, djlB gene, ybeT gene, ybeU gene, djlC gene, hscC gene, rihA gene, gltL gene, gltK gene, gltJ gene, gltI gene, kdpF gene, ybfA gene, ybgA gene, phr gene, ybiA gene, dinG gene, ybiB gene, ybiC gene, ybiJ gene, ybiI gene, ybiX gene, fiu gene, ybiM gene, ybiN gene, ybiO gene, glnQ gene, glnP gene, glnH gene, yliA gene, yliB gene, yliC gene, yliD gene, yliE gene, yliF gene, yliG gene, yliH gene, yliI gene, yliJ gene, dacC gene, deoR gene, ybjG gene, ybjC gene, nfsA gene, rimK gene, ybjN gene, potF gene, potG gene, potH gene, poti gene, ybjO gene, rumB gene, artJ gene, artM gene, artQ gene, artI gene, artP gene, ybjP gene, ybjQ gene, ybjR gene, ybjS gene, ybjT gene, ltaE gene, ymcC gene, ymcD gene, cspH gene, cspG gene, ymcE gene, gnsA gene, yccM gene, torS gene, torT gene, torR gene, torC gene, torA gene, torD gene, cbpM gene, cbpA gene, yccE gene, agp gene, yccJ gene, wrbA gene, ymdF gene, ycdG gene, ycdH gene, ycdI gene, rarA gene, ycdK gene, ycdL gene, ycdM gene, ycdC gene, putA gene, putP gene, ycdN_1 gene, ycdN_2 gene, ycdO gene, ycdB gene, phoH gene, ycdP gene, ycdQ gene, ycdR gene, ycdS gene, ycdT gene, ymdE gene, ycdU gene, elbA gene, ycgX gene, ycgE gene, ycgF gene, ycgZ gene, ymgA gene, ymgB gene, ymgC gene, ycgG gene, ymgF gene, ycgH_1 gene, ycgH_2 gene, ymgD gene, ymgG gene, ymgH gene, ycgI gene, ydfH gene, ydfZ gene, ydfI gene, ydfJ gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, yecG gene, otsA gene, otsB gene, araH gene, araG gene, araF gene, yecI gene, yecJ gene, isrB gene, yecR gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN_2 gene, yedN_1 gene, yedM gene, intG gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, yodD gene, yedA gene, vsr gene, dcm gene, yedJ gene, yedR gene, yedS_1 gene, yedS_2 gene, yedS_3 gene, hchA gene, yedV gene, yedW gene, yedX gene, yedY gene, yedZ gene, yodA gene, wbbL_2 gene, wbbL_1 gene, wbbK gene, wbbJ gene, wbbI gene, wbbH gene, glf gene, rfbX gene, rfbC gene, rfbA gene, rfbD gene, rfbB gene, yegE gene, alkA gene, yegD gene, yegI gene, yegJ gene, yegK gene, yegL gene, ryeC gene, ryeD gene, mdtA gene, mdtB gene, mdtC gene, mdtD gene, baeS gene, baeR gene, yegP gene, yegQ gene, ryeE gene, ogrK gene, yegZ gene, yegR gene, yegS gene, gatR_1 gene, gatR_2 gene, gatD gene, gatC gene, gatB gene, gatA gene, gatZ gene, gatY gene, fbaB gene, yegT gene, yegU gene, yegV gene, yegW gene, yegX gene, molR_1 gene, molR_2 gene, molR 3 gene, yehI gene, yehK gene, yehL gene, yehM gene, yehP gene, yehQ gene, yehR gene, yehS gene, yehT gene, yehU gene, mlrA gene, yohO gene, yehW gene, yehX gene, yehY gene, yehZ gene, bglX gene, dld gene, pbpG gene, yohC gene, yeiI gene, yeiJ gene, rihB gene, yeiL gene, yeiM gene, yeiN gene, yeiC gene, elaB gene, elaA gene, elaC gene, elaD gene, yfbK gene, yfbL gene, yfbM gene, yfbN gene, yfbO gene, yfbP gene, yfcC gene, yfcD gene, yfcE gene, yfcF gene, yfcG gene, folX gene, yfcH gene, yfcI gene, hisP gene, hisM gene, hisQ gene, hisJ gene, argT gene, ypdA gene, ypdB gene, ypdC gene, ypdD gene, ypdE gene, ypdF gene, ypdG gene, ypdH gene, pinH gene, ypjB gene, ypjC gene, ileY gene, ygaQ gene, ygaR gene, yqaC gene, yqaD gene, ygaT gene, csiR gene, ygaU gene, yqaE gene, ygaV gene, ygaP gene, stpA gene, ygaW gene, ygaC gene, ygaM gene, nrdH gene, nrdI gene, nrdE gene, proV gene, proW gene, proX gene, ygaX gene, ygaY gene, ygaZ gene, ygaH gene, srlA gene, srlE gene, srlB gene, srlD gene, gutM gene, srlR gene, gutQ gene, yqeG gene, yqeH gene, yqeI gene, yqeJ gene, yqeK gene, ygeF gene, ygeG gene, ygeH gene, ygeI gene, pbl gene, ygeK gene, ygeL gene, ygeM gene, ygeN gene, ygeO gene, ygeP gene, ygeQ gene, glyU gene, ygeR gene, xdhA gene, xdhB gene, xdhC gene, ygeV gene, ygeW gene, ygeX gene, ygeY gene, hyuA gene, yqeA gene, yqeB gene, yqeC gene, ygfJ gene, ygfK gene, ssnA gene, ygfM gene, xdhD gene, ygfO gene, guaD gene, ygfQ gene, ygfS gene, ygfT gene, ygfU gene, idi gene, cmtA gene, cmtB gene, yghD gene, yghE gene, yghF gene, yghG gene, pppA gene, yghJ gene, yghK gene, glcB gene, glcG gene, glcF gene, glcE gene, glcD gene, glcC gene, yghO gene, yghQ gene, yghR gene, yghS gene, yghT gene, ygiS gene, ygiT gene, ygiU gene, ygiV gene, ygiW gene, qseB gene, qseC gene, ygiZ gene, mdaB gene, ygiN gene, gltF gene, yhcA gene, yhcD gene, yhcE gene, yhcF gene, yhcG gene, yhcH gene, nanK gene, nanE gene, yhdJ gene, yhdU gene, envR gene, acrE gene, acrF gene, yhdV gene, yhdW gene, yhdX gene, yhdY gene, yijP gene, yijO gene, frwD gene, pflc gene, pflD gene, frwB gene, frwC gene, ptsA gene, rhaT gene, rhaR gene, rhaS gene, rhaB gene, rhaA gene, rhaD gene, yiiL gene, frvA gene, frvB gene, frvX gene, frvR gene, yiiG gene, yiiF gene, yiiE gene, yiiD gene, dtd gene, rbn gene, yihX gene, yihW gene, yihV gene, yihU gene, yihT gene, yihS gene, yihR gene, yihQ gene, yihP gene, yihO gene, ompL gene, yihN gene, yihM gene, yihL gene, hsrA gene, rbsR gene, rbsK gene, rbsB gene, rbsC gene, rbsA gene, rbsD gene, trkD gene, yieN gene, yieM gene, asnA gene, yidB gene, yidA gene, yidX gene, dgoR gene, dgoK gene, dgoA gene, dgoD gene, dgoT gene, cbrA gene, yidR gene, yidQ gene, yidE gene, yidP gene, glvC gene, glvB gene, glvG gene, ysdC gene, yidL gene, yidK gene, yidJ gene, yidI gene, yidH gene, yidG gene, yidF gene, yhiN gene, yhiM gene, yhiL gene, yhiK gene, yhiJ gene, yhiI gene, rbbA gene, yhhJ gene, chiA gene, bfd gene, bfr gene, gspO gene, gspM gene, gspL gene, gspK gene, gspJ gene, gspI gene, gspH gene, gspG gene, gspF gene, gspE gene, gspD gene, gspC gene, gspA gene, pioO gene, yjbE gene, yjbF gene, yjbG gene, yjbH gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, yjdP gene, phnP gene, phnO gene, phnN gene, phnM gene, phnL gene, phnK gene, phnJ gene, phnI gene, phnH gene, phnG gene, phnF gene, phnE(JW4064) gene, phnE(JW4065) gene, phnD gene, phnC gene, phnB gene, phnA gene, yjdA gene, yjcZ gene, yjfI gene, yjfJ gene, yjfK gene, yjfL gene, yjfM gene, yjfC gene, aidB gene, yjfN gene, yjfO gene, yjfP gene, ulaR gene, ulaG gene, ulaA gene, ulaB gene, ulaC gene, ulaD gene, ulaE gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, kptA gene, yjiJ gene, yjiK gene, yjiL gene, yjiM gene, yjiN gene, yjiO gene, yjiP gene, yjiQ gene, yjiR gene, yjiS gene, yjiT gene, yjiV gene, mcrC gene, mcrB gene, yjiW gene, hsdS gene, hsdM gene, hsdR gene, mrr gene, yjiA gene, yjiX gene, yjiY gene, tsr gene, yjiZ gene, yjjM gene, yjjN gene, mdoB gene, yjjA gene.

2. The *E. coli* mutant strain according to claim 1, comprising chromosomal DNA that comprises DNA having the nucleotide sequences as shown in [1] to [83] below of the nucleotide sequence as shown in SEQ ID NO: 1 or DNA equivalent thereto:
[1] a nucleotide sequence of nucleotides 1 to 15298
[2] a nucleotide sequence of nucleotides 16751 to 19620
[3] a nucleotide sequence of nucleotides 20815 to 65857
[4] a nucleotide sequence of nucleotides 78797 to 167400
[5] a nucleotide sequence of nucleotides 173316 to 244300
[6] a nucleotide sequence of nucleotides 253747 to 262299
[7] a nucleotide sequence of nucleotides 387868 to 389474
[8] a nucleotide sequence of nucleotides 404040 to 518365
[9] a nucleotide sequence of nucleotides 533048 to 535840
[10] a nucleotide sequence of nucleotides 550552 to 564276
[11] a nucleotide sequence of nucleotides 608455 to 640668
[12] a nucleotide sequence of nucleotides 659193 to 675439
[13] a nucleotide sequence of nucleotides 689711 to 729156
[14] a nucleotide sequence of nucleotides 739930 to 832888
[15] a nucleotide sequence of nucleotides 848427 to 867974
[16] a nucleotide sequence of nucleotides 883811 to 891334
[17] a nucleotide sequence of nucleotides 909717 to 1049224
[18] a nucleotide sequence of nucleotides 1097358 to 1105534
[19] a nucleotide sequence of nucleotides 1106991 to 1196528
[20] a nucleotide sequence of nucleotides 1225485 to 1295721
[21] a nucleotide sequence of nucleotides 1297023 to 1300822
[22] a nucleotide sequence of nucleotides 1302896 to 1397636
[23] a nucleotide sequence of nucleotides 1399079 to 1413662
[24] a nucleotide sequence of nucleotides 1436899 to 1469664
[25] a nucleotide sequence of nucleotides 1472404 to 1504839
[26] a nucleotide sequence of nucleotides 1506619 to 1528866
[27] a nucleotide sequence of nucleotides 1534766 to 1630050
[28] a nucleotide sequence of nucleotides 1654610 to 1853604
[29] a nucleotide sequence of nucleotides 1864144 to 1875287
[30] a nucleotide sequence of nucleotides 1888500 to 1963662
[31] a nucleotide sequence of nucleotides 1992215 to 1995800
[32] a nucleotide sequence of nucleotides 2027349 to 2032587
[33] a nucleotide sequence of nucleotides 2043516 to 2068287
[34] a nucleotide sequence of nucleotides 2081066 to 2103531
[35] a nucleotide sequence of nucleotides 2115204 to 2145402
[36] a nucleotide sequence of nucleotides 2186995 to 2199807
[37] a nucleotide sequence of nucleotides 2228990 to 2255033
[38] a nucleotide sequence of nucleotides 2262631 to 2292234
[39] a nucleotide sequence of nucleotides 2293834 to 2324498
[40] a nucleotide sequence of nucleotides 2322075 to 2411311
[41] a nucleotide sequence of nucleotides 2413007 to 2422503
[42] a nucleotide sequence of nucleotides 2433238 to 2471990
[43] a nucleotide sequence of nucleotides 2482140 to 2504213
[44] a nucleotide sequence of nucleotides 2513697 to 2519690
[45] a nucleotide sequence of nucleotides 2521089 to 2599856
[46] a nucleotide sequence of nucleotides 2613439 to 2754814
[47] a nucleotide sequence of nucleotides 2788619 to 2794311
[48] a nucleotide sequence of nucleotides 2800029 to 2803445
[49] a nucleotide sequence of nucleotides 2809286 to 2824451
[50] a nucleotide sequence of nucleotides 2829399 to 2909409
[51] a nucleotide sequence of nucleotides 2909710 to 2921193
[52] a nucleotide sequence of nucleotides 2943639 to 2984497
[53] a nucleotide sequence of nucleotides 3032267 to 3076223
[54] a nucleotide sequence of nucleotides 3078056 to 3109245
[55] a nucleotide sequence of nucleotides 3133480 to 3164769
[56] a nucleotide sequence of nucleotides 3172082 to 3184621
[57] a nucleotide sequence of nucleotides 3186056 to 3224889
[58] a nucleotide sequence of nucleotides 3240401 to 3261074
[59] a nucleotide sequence of nucleotides 3262220 to 3361080
[60] a nucleotide sequence of nucleotides 3370892 to 3411501
[61] a nucleotide sequence of nucleotides 3421222 to 3486426
[62] a nucleotide sequence of nucleotides 3496906 to 3536155
[63] a nucleotide sequence of nucleotides 3549687 to 3556477
[64] a nucleotide sequence of nucleotides 3576239 to 3697304
[65] a nucleotide sequence of nucleotides 3709527 to 3742812
[66] a nucleotide sequence of nucleotides 3744293 to 3748980
[67] a nucleotide sequence of nucleotides 3747997 to 3750486
[68] a nucleotide sequence of nucleotides 3751906 to 3762908
[69] a nucleotide sequence of nucleotides 3772817 to 3774086
[70] a nucleotide sequence of nucleotides 3785257 to 3794687
[71] a nucleotide sequence of nucleotides 3803459 to 3868134
[72] a nucleotide sequence of nucleotides 3878460 to 3918087
[73] a nucleotide sequence of nucleotides 3919815 to 3986454
[74] a nucleotide sequence of nucleotides 3988342 to 4003004
[75] a nucleotide sequence of nucleotides 4021347 to 4055371
[76] a nucleotide sequence of nucleotides 4061466 to 4128290
[77] a nucleotide sequence of nucleotides 4141016 to 4170562
[78] a nucleotide sequence of nucleotides 4186909 to 4239493
[79] a nucleotide sequence of nucleotides 4255871 to 4303000
[80] a nucleotide sequence of nucleotides 4316735 to 4318540
[81] a nucleotide sequence of nucleotides 4334946 to 4414758
[82] a nucleotide sequence of nucleotides 4428378 to 4501437
[83] a nucleotide sequence of nucleotides 4604873 to 4646332.

3. The *E. coli* mutant strain according to claim 1 or 2, comprising chromosomal DNA that does not comprise uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, and ybeM gene, or homologous gene thereof.

4. The *E. coli* mutant strain according to any one of claims 1 to 3, comprising chromosomal DNA that does not comprise DNA having a nucleotide sequence of nucleotides 640669 to 659192 in the nucleotide sequence as shown in SEQ ID NO: 1 or DNA equivalent thereto.

5. The *E. coli* mutant strain according to any one of claims 1 to 4, comprising chromosomal DNA that does not comprise yqcA gene, yqcB gene, yqcC gene, syd gene, yqcD gene, ygdH gene, sdaC gene, sdaB gene, exo gene, fucO gene, fucA gene, fucP gene, fuel gene, fucK gene, fucU gene, fucR gene, ygdE gene, ygdD gene, gcvA gene, ygdI gene, csdA gene, and ygdK gene, or homologous gene thereof and comprise chpA gene, or homologous gene thereof.

6. The *E. coli* mutant strain according to any one of claims 1 to 5, comprising chromosomal DNA that does not comprise DNA having a nucleotide sequence of nucleotides 2921194 to 2943638 in the nucleotide sequence as shown in SEQ ID NO: 1 or DNA equivalent thereto.

7. The *E. coli* mutant strain according to any one of claims 1 to 6, comprising chromosomal DNA that does not comprise araD gene, araA gene, araB gene, araC gene, yabI gene, thiQ gene, thiP gene, tbpA gene, sgrR gene, and setA gene, or homologous gene thereof.

8. The *E. coli* mutant strain according to any one of claims 1 to 7, comprising chromosomal DNA that does not comprise DNA having a nucleotide sequences of nucleotides 65858 to 78796 in the nucleotide sequence as shown in SEQ ID NO: 1 or DNA equivalent thereto.

9. The *E. coli* mutant strain according to any one of claims 1 to 8, comprising chromosomal DNA that does not comprise yhfZ gene, yhfY gene, yhfX gene, yhfW gene, php gene, yhfU gene, yhfT gene, yhfS gene, frlR gene, frlD gene, frlc gene, frlB gene, frlA gene, and yhfL gene, or homologous gene thereof.

10. The *E. coli* mutant strain according to any one of claims 1 to 9, comprising chromosomal DNA that does not comprise DNA having a nucleotide sequence of nucleotides 4128291 to 4141015 in the nucleotide sequence as shown in SEQ ID NO: 1 or DNA equivalent thereto.

11. The *E. coli* mutant strain according to any one of claims 1 to 10, comprising chromosomal DNA that does not comprise pncA gene, ydjE gene, ydjF gene, ydjG gene, ydjH gene, ydjI gene, ydjJ gene, ydjK gene, ydjL gene, yeaC gene, and yeaA gene, or homologous gene thereof.

12. The *E. coli* mutant strain according to any one of claims 1 to 11, comprising chromosomal DNA that does not comprise DNA having a nucleotide sequence of nucleotides 1853605 to 1864143 in the nucleotide sequence as shown in SEQ ID NO: 1 or DNA equivalent thereto.

13. The *E. coli* mutant strain according to any one of claims 1 to 12, comprising chromosomal DNA that does not comprise ade gene, yicO gene, yicN gene, yicM gene, yicS gene, nlpA gene, yicL gene, and setC gene, or homologous gene thereof.

14. The *E. coli* mutant strain according to any one of claims 1 to 13, comprising chromosomal DNA that does not comprise DNA having a nucleotide sequence of nucleotides 3794688 to 3803458 in the nucleotide sequence as shown in SEQ ID NO: 1 or DNA equivalent thereto.

15. The *E. coli* mutant strain according to any one of claims 1 to 14, comprising chromosomal DNA that does not comprise ygjI gene, ygjJ gene, ygjK gene, fadH gene, ygjM gene, ygjN gene, ygjO gene, ygjP gene, ygjQ gene, ygjR gene, alx gene, sstT gene, and ygjV gene, or homologous gene thereof.

16. The *E. coli* mutant strain according to any one of claims 1 to 15, comprising chromosomal DNA that does not comprise DNA having a nucleotide sequence of nucleotides 3224890 to 3240400 in the nucleotide sequence as shown in SEQ ID NO: 1 or DNA equivalent thereto.

17. The *E. coli* mutant strain according to any one of claims 1 to 16, comprising chromosomal DNA that does not comprise yrhB gene, yrhA gene, yhhZ gene, yhhY gene, yhhX gene, and yhhW gene, or homologous gene thereof.

18. The *E. coli* mutant strain according to any one of claims 1 to 17, comprising chromosomal DNA that does not comprise DNA having a nucleotide sequence of nucleotides 4055372 to 4061465 in the nucleotide sequence as shown in SEQ ID NO: 1 or DNA equivalent thereto.

19. The *E. coli* mutant strain according to any one of claims 1 to 18, comprising chromosomal DNA that does not comprise yeaK gene, yoaI gene, yeaL gene, yeaM gene, yeaN gene, yeaO gene, yoaF gene, yeaP gene, yeaQ gene, yoaG gene, yeaR gene, yeaS gene, yeaT gene, yeaU gene, yeaV gene, yeaW gene, and yeaX gene, or homologous gene thereof.

20. The *E. coli* mutant strain according to any one of claims 1 to 19, comprising chromosomal DNA that does not comprise DNA having a nucleotide sequence of nucleotides 1875288 to 1888499 in the nucleotide sequence as shown in SEQ ID NO: 1 or DNA equivalent thereto.

21. The *E. coli* mutant strain according to any one of claims 1 to 20, comprising chromosomal DNA that does not comprise hyfA gene, hyfB gene, hyfC gene, hyfD gene, hyfE gene, hyfF gene, hyfG gene, hyfH gene, hyfI gene, hyfJ gene, hyfR gene, and focB gene, or homologous gene thereof.

22. The *E. coli* mutant strain according to any one of claims 1 to 21, comprising chromosomal DNA that does not comprise DNA having a nucleotide sequence of nucleotides 2599857 to 2613438 in the nucleotide sequence as shown in SEQ ID NO: 1 or DNA equivalent thereto.

23. An *E. coli* mutant strain comprising chromosomal DNA comprising the following genes [1] or homologous genes thereof, comprising not more than 10% of the genes [2] and [3] or homologous genes thereof, and being at least 1040 kbp shorter than that of a wild-type *E. coli* strain, provided that when the following genes [1] or homologous genes thereof comprise NC genes, the aforementioned *E. coli* mutant strain derived from the wild-type *E. coli* strain does not necessarily have the NC gene.
[1] thrL gene, thrA gene, thrB gene, thrC gene, talB gene, mog gene, htgA gene, dnaK gene, dnaJ gene, mokC gene, hokC gene, sokC gene, nhaA gene, nhaR gene, rpsT gene, ribF gene, ileS gene, lspA gene, fkpB gene, ispH gene, rihC gene, dapB gene, carA gene, carB gene, caiF gene, caiE gene, caiD gene, caiC gene, caiB gene, caiA gene, caiT gene, fixA gene, fixB gene, fixC gene, fixX gene, kefF gene, kefC gene, folA gene, apaH gene, apaG gene, ksgA gene, pdxA gene, surA gene, imp gene, djlA gene, rluA gene, hepA gene, polB gene, leuD gene, leuC gene, leuB gene, leuA gene, leuL gene, leuO gene, ilvI gene, ilvH gene, fruR gene, mraZ gene, mraW gene, ftsL gene, ftsI gene, murE gene, murF gene, mraY gene, murD gene, ftsW gene, murG gene, murC gene, ddlB gene, ftsQ gene, ftsA gene, ftsZ gene, lpxC gene, secM gene, secA gene, mutT gene, coaE gene, guaC gene, hofC gene, hofB gene, ppdD gene, nadC gene, ampD gene, ampE gene, aroP gene, pdhR gene, aceE gene, aceF gene, lpd gene, acnB gene, speD gene, speE gene, cueO gene, gcd gene, hpt gene, can gene, panD gene, panC gene, panB gene, htrE gene, ecpD gene, folK gene, pcnB gene, dksA gene, sfsA gene, ligT gene, hrpB gene, mrcB gene, hemL gene, clcA gene, btuF gene, pfs gene, dgt gene, degP gene, cdaR gene, dapD gene, glnD gene, map gene, tff gene, rpsB gene, tsf gene, pyrH gene, frr gene, dxr gene, ispU gene, cdsA gene, hlpA gene, lpxD gene, fabZ gene, lpxA gene, lpxB gene, rnhB gene, dnaE gene, accA gene, IdcC gene, tilS gene, rof gene, nlpE gene, proS gene, rcsF gene, metQ gene, metI gene, metN gene, gmhB gene, rrsH gene, ileV gene, alaV gene, rrlH gene, rrfH gene, aspU gene, dkgB gene, mltD gene, gloB gene, rnhA gene, dnaQ gene, aspV gene, ivy gene, fadE gene, IpcA gene, pepD gene, gpt gene, frsA gene, crl gene, phone gene, proB gene, proA gene, thrW gene, hemB gene, proC gene, aroL gene, aroM gene, rdgC gene, mak gene, araJ gene, sbcC gene, sbcD gene, phoB gene, phoR gene, brnQ gene, proY gene, malZ gene, queA gene, tgt gene, secD gene, secF gene, tsx gene, ribD gene, ribE gene, nusB gene, thiL gene, pgpA gene, dxs gene, ispA gene, xseB gene, thiI gene, panE gene, cyoE gene, cyoD gene, cyoC gene, cyoB gene, cyoA gene, ampG gene, bolA gene, tig gene, clpP gene, clpX gene,
Ion gene, hupB gene, ppiD gene, cof gene, mdlA gene, mdlB gene, glnK gene, amtB gene, tesB gene, ffs gene, maa gene, hha gene, acrB gene, acrA gene, acrR gene, kefA gene, priC gene, apt gene, dnaX gene, recR gene, htpG gene, adk gene, hemH gene,
aes gene, gsk gene, fsr gene, ushA gene, copA gene, cueR gene, gcl gene, hyi gene, purK gene, purE gene, lpxH gene, ppiB gene, cysS gene, folD gene, sfmA gene, sfmC gene, sfmD gene, sfmH gene, sfmF gene, fimZ gene, argU gene, entD gene, fepA gene, fes gene, entF gene, fepE gene, fepC gene, fepG gene, fepD gene, fepB gene, entC gene, entE gene, entB gene, entA gene, cstA gene, dsbG gene, ahpC gene, ahpF gene, tatE gene, lipA gene, lipB gene, dacA gene, rlpA gene, mrdB gene, mrdA gene, cobC gene, nadD gene, holA gene, rlpB gene, leuS gene, lnt gene, miaB gene, ubiF gene, glnX gene, glnV gene, metU gene, glnw gene, glnU gene, leuW gene, metT gene, asnB gene, nagD gene, nagC gene, nagA gene, nagB gene, nagE gene, glnS gene, fur gene, fldA gene, seqA gene, pgm gene, potE gene, speF gene, kdpE gene, kdpD gene, kdpC gene, kdpB gene, kdpA gene, nei gene, abrB gene, gltA gene, sdhC gene, sdhD gene, sdhA gene, sdhB gene, sucA gene, sucB gene, sucC gene, sucD gene, mngR gene, mngA gene, mngB gene, cydA gene, cydB gene, tolQ gene, tolR gene, tolA gene, tolB gene, pal gene, lysT gene, valT gene, lysW gene, valZ gene, lysY gene, lysZ gene, lysQ gene, nadA gene, pnuC gene, zitB gene, aroG gene, gpmA gene, galM gene, galK gene, galT gene, galE gene, modF gene, modE gene, modA gene, modB gene, mode gene, bioA gene, bioB gene, bioF gene, bioC gene, bioD gene, uvrB gene, moaA gene, moaB gene, moaC gene, moaD gene, moaE gene, rhlE gene, dps gene, rhtA gene, ompX gene, rybA gene, mntR gene, fsaA gene, moeB gene, moeA gene, iaaA gene, cmr gene, rybB gene, grxA gene, poxB gene, hcr gene, hcp gene, aqpZ gene, macA gene, macB gene, cspD gene, clpS gene, clpA gene, serW gene, infA gene, aat gene, cydC gene, cydD gene, trxB gene, lrp gene, ftsK gene, lolA gene, serS gene, dmsA gene, dmsB gene, dmsC gene, pflA gene, pflB gene, focA gene, serC gene, aroA gene, cmk gene, rpsA gene, ihfB gene, msbA gene, lpxK gene, kdsB gene, smtA gene, mukF gene, mukE gene, mukB gene, aspC gene, ompF gene, asnS gene, pncB gene, pepN gene, ssuB gene, ssuC gene, ssuD gene, ssuA gene, ssuE gene, pyrD gene, uup gene, pqiA gene, pqiB gene, rmf gene, fabA gene, ompA gene, sulA gene, helD gene, mgsA gene, serT gene, hyaA gene, hyaB gene, hyaC gene, hyaD gene, hyaE gene, hyaF gene, appC gene, appB gene, appA gene, etp gene, serX gene, csgG gene, csgF gene, csgE gene, csgD gene, csgB gene, csgA gene, csgC gene, mdoC gene, mdoG gene, mdoH gene, msyB gene, mdtG gene, lpxL gene, solA gene, dinI gene, pyrC gene, grxB gene, mdtH gene, rimJ gene, mvim gene, mviN gene, flgN gene, flgM gene, flgA gene, flgB gene, flgC gene, flgD gene, flgE gene, flgF gene, flgG gene, flgH gene, flgI gene, flgJ gene, flgK gene, flgL gene, rne gene, rluC gene, sraB gene, rpmF gene, plsX gene, fabH gene, fabD gene, fabG gene, acpP gene, fabF gene, pabC gene, tmk gene, holB gene, ptsG gene, fhuE gene, hinT gene, nagZ gene, ndh gene, mfd gene, lolC gene, lolD gene, lolE gene, cobB gene, potD gene, potC gene, potB gene, potA gene, pepT gene, phoQ gene, phoP gene, purB gene, hflD gene, trmU gene, minE gene, minD gene, minC gene, hlyE gene, umuD gene, umuC gene, dsbB gene, nhaB gene, fadR gene, dadA gene, dadX gene, cvrA gene, IdeA gene, emtA gene, treA gene, dhaH gene, dhaL gene, dhaK gene, dhaR gene, pth gene, prsA gene, ispE gene, lolB gene, hemA gene, prfA gene, prmC gene, kdsA gene, IdrA gene, rdlA gene, ldrB gene, rdlB gene, ldrC gene, rdlC gene, chaA gene, chaB gene, chaC gene, narL gene, narX gene, narK gene, narG gene, narH gene, narJ gene, narI gene, rttR gene, tpr gene, tyrV gene, tyrT gene, purU gene, rssA gene, rssB gene, galU gene, hns gene, tdk gene, adhE gene, oppA gene, oppB gene,
oppC gene, oppD gene, oppF gene, cls gene, kch gene, tonB gene, ompW gene, trpA gene, trpB gene, trpC gene, trpD gene, trpE gene, trpL gene, rluB gene, btuR gene, sohB gene, topA gene, cysB gene, acnA gene, ribA gene, pgpB gene, pyrF gene, osmB gene, gmr gene, rnb gene, fabI gene, sapF gene, sapD gene,
sapC gene, sapB gene, sapA gene, puuP gene, puuA gene, puuD gene, puuR gene, puuC gene, puuB gene, puuE gene, pspF gene, pspA gene, pspB gene, pspC gene, pspD gene, pspE gene, ompG gene, tyrR gene, tpx gene, mpaA gene, mppA gene, uspE gene,
fnr gene, ogt gene, abgT gene, abgB gene, abgA gene, abgR gene, isrA gene, dbpA gene, uspF gene, ompN gene, micC gene, hslJ gene, ldhA gene, feaR gene, feaB gene, tynA gene, maoC gene, paaA gene, paaB gene, paaC gene, paaD gene, paaE gene, paaF gene, paaG gene, paaH gene, paaI gene, paaJ gene, paaK gene, paaX gene, paaY gene, azoR gene, hrpA gene, aldA gene, gapC_2 gene, gapC_1 gene, cybB gene, hokB gene, mokB gene, sokB gene, trg gene, mdoD gene, rimL gene, tehA gene, tehB gene, ansP gene, nhoA gene, narY gene, narW gene, narY gene, narZ gene, narU gene, fdnG gene, fdnH gene, fdnI gene, adhP gene, sfcA gene, sra gene, bdm gene, osmC gene, ddpF gene, ddpD gene,
ddpC gene, ddpB gene, ddpA gene, ddpX gene, dos gene, gadC gene, gadB gene, pqqL gene, hipA gene, hipB gene, ego gene, lsrC gene, lsrD gene, lsrB gene, lsrF gene, lsrG gene, tam gene, uxaB gene, marC gene, marR gene, marA gene, marB gene, eamA gene, dcp gene, rspB gene, rspA gene, speG gene, dmsD gene, clcB gene, dgsA gene, asr gene, mdtI gene, mdtJ gene, pntB gene, pntA gene, folM gene, rstA gene, rstB gene, tus gene, fumC gene, fumA gene, manA gene, uidC gene, uidB gene, uidA gene, uidR gene, hdhA gene, mall gene, malX gene, malY gene, add gene, blr gene, rsxA gene, rsxB gene, rsxC gene,
rsxD gene, rsxG gene, rsxE gene, nth gene, gst gene, pdxY gene, tyrS gene, pdxH gene, slyB gene, slyA gene, sodC gene, nemA gene, gloA gene, rnt gene, lhr gene, sodB gene, purR gene, cfa gene, ribC gene, mdtK gene, valV gene, valW gene, pykF gene, lpp gene, sufE gene, sufS gene, sufD gene, sufC gene, sufB gene, sufA gene, rydB gene, rprA gene, aroD gene, pps gene, aroH gene, nlpc gene, btuD gene, btuE gene, btuC gene, ihfA gene, pheT gene, pheS gene, pheM gene, rplT gene, rpmI gene, infC gene, thrS gene, arpB_1 gene, arpB_2 gene, pfkB gene,
cedA gene, katE gene, chbG gene, chbF gene, chbR gene, chbA gene, chbC gene, chbB gene, osmE gene, nadE gene, spy gene, astE gene, astB gene, astD gene, astA gene, astC gene, xthA gene, nudG gene, gdhA gene, topB gene, selD gene, sppA gene, ansA gene, gapA gene, mipA gene, rnd gene, fadD gene, pabB gene, sdaA gene, manX gene, manY gene, manZ gene, rrmA gene, cspC gene, kdgR gene, htpX gene, prc gene, proQ gene, pphA gene, ryeA gene, ryeB gene, holE gene, exoX gene, ptrB gene, purT gene, eda gene, edd gene, zwf gene, pykA gene, IpxM gene, znuA gene, znuC gene, znuB gene, ruvB gene, ruvA gene, ruvC gene, nudB gene, aspS gene, torZ gene, torY gene, cutC gene, argS gene, leuZ gene, cysT gene, glyW gene, pgsA gene, dsrA gene, serU gene, asnT gene, shiA gene, amn gene, asnW gene, asnU gene, cbl gene, nac gene, asnV gene, erfK gene, cobT gene, cobs gene, cobU gene, sbmC gene, dacD gene, sbcB gene, hisL gene, hisG gene, hisD gene, hisC gene, hisB gene, hisH gene, hisA gene, hisF gene, hisI gene, cld gene, ugd gene, gnd gene, galF gene, wcaM gene, wcaL gene, wcaK gene, wzxC gene, wcaJ gene, cpsG gene, cpsB gene, weal gene, nudD gene, fcl gene,
gmd gene, wcaF gene, wcaE gene, wcaD gene, wcaC gene, wcaB gene, wcaA gene, wzc gene, wzb gene, wza gene, asmA gene, dcd gene, udk gene, thiD gene, thiM gene, mrp gene, metG gene,
dusC gene, cdd gene, sanA gene, mglc gene, mglA gene, mglB gene, galS gene, folE gene, cirA gene, lysP gene, nfo gene, fruA gene, fruK gene, fruB gene, setB gene, spr gene, rtn gene, bcr gene, rsuA gene, rplY gene, proL gene, narP gene, ccmH gene, ccmG gene, ccmF gene, ccmE gene, ccmD gene, ccmC gene, ccmB gene, ccmA gene, napC gene, napB gene, napH gene, napG gene, napA gene, napD gene, napF gene, eco gene, mqo gene, alkB gene, ada gene, ompC gene, micF gene, rcsD gene, rcsB gene, rcsC gene, atoS gene, atoC gene, atoD gene, atoA gene, atoE gene, atoB gene, gyrA gene, ubiG gene, nrdA gene, nrdB gene, inaA gene, glpQ gene, glpT gene, glpA gene, glpB gene, glpC gene, ais gene, arnT gene, pmrD gene, menE gene, menC gene, menB gene, menD gene, menF gene, nuoN gene, nuoM gene, nuoL gene, nuoK gene, nuoJ gene, nuoI gene, nuoH gene, nuoG gene, nuoF gene, nuoE gene, nuoC gene, nuoB gene, nuoA gene, lrhA gene, ackA gene, pta gene, ubiX gene, purF gene, cvpA gene, dedD gene, folC gene, accD gene, dedA gene, truA gene, usg gene, pdxB gene, flk gene, fabB gene, trmC gene, mepA gene, aroC gene, prmB gene, sixA gene, fadL gene, vacJ gene, argW gene, dsdC gene, dsdX gene, dsdA gene, emrY gene, emrK gene, evgA gene, evgS gene, oxc gene, frc gene, ddg gene, glk gene, mntH gene, nupC gene, alaX gene, alaW gene, gltX gene, valU gene, valX gene, valY gene, lysV gene, xapR gene, xapB gene, xapA gene, ligA gene, zipA gene, cysZ gene, cysK gene, ptsH gene, ptsI gene, crr gene, pdxK gene, cysM gene, cysA gene, cysW gene, cysU gene, cysP gene, ucpA gene, murP gene, amiA gene, hemF gene, eutL gene, eutC gene, eutB gene, eutA gene, eutH gene, eutG gene, eutJ gene, eutE gene, cchB gene, cchA gene, eutI gene, eutT gene, eutQ gene, eutP gene, maeB gene, talA gene, tktB gene, aegA gene, narQ gene, acrD gene, dapE gene, purC gene, nlpB gene, dapA gene, gcvR gene, bcp gene,
hda gene, uraA gene, upp gene, purM gene, purN gene, ppk gene, ppx gene, guaA gene, guaB gene, xseA gene, der gene, hiss gene, ispG gene, ndk gene, pbpC gene, sseA gene, ryfA gene, sseB gene, pepB gene, fdx gene, hscA gene, hscB gene, iscA gene, iscU gene, iscS gene, iscR gene, suhB gene, csiE gene, hcaT gene, hcaR gene, hcaE gene, hcaF gene, hcaC gene, hcaB gene, hcaD gene, glyA gene, hmp gene, glnB gene, sroF gene, purL gene, tadA gene, acpS gene, pdxJ gene, recO gene, era gene,
rnc gene, lepB gene, lepA gene, rseC gene, rseB gene, rseA gene, rpoE gene, nadB gene, srmB gene, ung gene, trxC gene, pssA gene, kgtP gene, rrfG gene, rrlG gene, gltw gene, rrsG gene, clpB gene, rluD gene, pheL gene, pheA gene, tyrA gene, aroF gene, rplS gene, trmD gene, rimM gene, rpsP gene, ffh gene, grpE gene, recN gene, smpA gene, smpB gene, ssrA gene, gabD gene, gabT gene, gabP gene, nrdF gene, mprA gene, emrA gene, emrB gene, luxS gene, sraD gene, gshA gene, argQ gene, argZ gene, argY gene, argV gene, serV gene, csrA gene, alas gene, recX gene, recA gene, mltB gene, norR gene, norV gene, norW gene, hypF gene, hydN gene, ascG gene, ascF gene, ascB gene, hycI gene, hycH gene, hycG gene, hycF gene, hycE gene, hycD gene, hycC gene, hycB gene, hycA gene, hypA gene, hypB gene, hypC gene, hypD gene, hypE gene, fhlA gene, mutS gene, pphB gene, rpoS gene, nlpD gene, pcm gene, surE gene, truD gene, ispF gene, ispD gene, ftsB gene, cysC gene, cysN gene, cysD gene, iap gene, cysH gene, cysI gene, cysJ gene, eno gene, pyrG gene, mazG gene, chpR gene, relA gene, rumA gene, barA gene, gudD gene, gudX gene, gudP gene, mltA gene, metZ gene, metW gene, metV gene, amiC gene, argA gene, recD gene, recB gene, ptr gene, recC gene, ppdC gene, ppdB gene, ppdA gene, thyA gene, lgt gene, ptsP gene, nudH gene, mutH gene, tas gene, aas gene, rygA gene, rygB gene, galR gene, lysA gene, lysR gene, araE gene, kduD gene, kduI gene, lysS gene, prfB gene, recJ gene, dsbC gene, xerD gene, fldB gene, bglA gene, gcvP gene, gcvH gene, gcvT gene, visC gene, ubiH gene, pepP gene, zapA gene, ssrS gene, rygC gene, serA gene, rpiA gene, argP gene, argK gene, argO gene, mscS gene, fbaA gene, pgk gene, epd gene, tktA gene, speB gene, speA gene, metK gene, galP gene, sprT gene, endA gene, gshB gene, ansB gene, mutY gene, mltC gene, nupG gene, speC gene, pheV gene, pitB gene, gss gene, hybG gene, hybF gene, hybE gene, hybD gene, hybC gene, hybB gene, hybA gene, hybO gene, exbD gene, exbB gene, metC gene, dkgA gene, sufI gene, plsC gene, parC gene, parE gene, cpdA gene, nudF gene, tolC gene, zupT gene, ribB gene, glgS gene, rygD gene, rfaE gene, glnE gene, htrG gene, cca gene, bacA gene, folB gene, ttdA gene, ttdB gene, rpsU gene, dnaG gene, rpoD gene, ileX gene, aer gene, ebgR gene, ebgA gene, ebgC gene, uxaA gene, uxaC gene, exuT gene, exuR gene, tdcG gene, tdcF gene, tdcE gene, tdcD gene, tdcC gene, tdcB gene, tdcA gene, tdcR gene, rnpB gene, garK gene, garR gene, garL gene, garP gene, garD gene, sohA gene, agaR gene, kbaZ gene,
agaV gene, agaW gene, agaA gene, agaS gene, kbaY gene, agaB gene, agaC gene, agaD gene, agaI gene, mtr gene, deaD gene, nlpI gene, pnp gene, sraG gene, rpsO gene, truB gene, rbfA gene, infB gene, nusA gene, metY gene, argG gene, leuU gene, secG gene, glmM gene, folP gene, ftsH gene, rimJ gene, greA gene, dacB gene, obgE gene, rpmA gene, rplU gene, ispB gene, sfsB gene, murA gene, kdsD gene, kdsC gene, rpoN gene, ptsN gene, npr gene, mtgA gene, elbB gene, ryhA gene, arcB gene, gltB gene, gltD gene, nanT gene, nanA gene, nanR gene, dcuD gene, sspB gene, sspA gene, rpsI gene, rplM gene, degQ gene,
degS gene, mdh gene, argR gene, aaeB gene, aaeA gene, aaeX gene, aaeR gene, tldD gene, rng gene, mreD gene, mreC gene, mreB gene, accB gene, accC gene, panF gene, prmA gene, dusB gene, fis gene, rrfF gene, thrV gene, rrfD gene, rrlD gene, gltv gene, rrsD gene, purH gene, purD gene, zraR gene, zras gene, zraP gene, hupA gene, nfi gene, hemE gene, nudC gene, rsd gene, thiC gene, thiE gene, thiF gene, this gene, thiG gene, thiH gene, htrC gene, rpoC gene, rpoB gene, rplL gene, rplJ gene, rplA gene, rplK gene, nusG gene, secE gene, tufB gene, thrT gene, glyT gene, tyrU gene, thrU gene, coaA gene, birA gene, murB gene, rrfB gene, rrlB gene, gltT gene, rrsB gene, murI gene, btuB gene, trmA gene, fabR gene, sthA gene, oxyR gene, oxyS gene, argH gene, argB gene, argC gene, argE gene, ppc gene, fsaB gene, gldA gene, katG gene, metF gene, metL gene, metB gene, metJ gene, rpmE gene, priA gene, cytR gene, ftsN gene, hslV gene, hslU gene, menA gene, rraA gene, glpf gene, glpK gene, glpX gene, fpr gene, tpiA gene, cdh gene, sbp gene, pfkA gene, fieF gene, cpxP gene, cpxR gene, cpxA gene, kdgT gene, sodA gene, fdhD gene, fdoG gene, fdoH gene, fdoI gene, fdhE gene, bipA gene, glnA gene, glnL gene, glnG gene, hemN gene, csrC gene, spf gene, polA gene, dsbA gene, mobA gene, mobB gene, rrfA gene, rrlA gene, alaT gene, ileT gene, rrsA gene, hemG gene, trkH gene, pepQ gene, fadB gene, fadA gene, fre gene, ubiD gene, rfaH gene, tatD gene, tatC gene, tatB gene, tatA gene, ubiB gene, ubiE gene, rmuC gene,
udp gene, metE gene, metR gene, pldB gene, rhtB gene, rhtC gene, recQ gene, pldA gene, rarD gene, corA gene, uvrD gene, xerC gene, dapF gene, cyaY gene, cyaA gene, hemC gene, hemD gene, hemX gene, hemY gene, ryiA gene, aslA gene, aslB gene, proM gene, leuT gene, hisR gene, argX gene, rffM gene, wzyE gene, rffT gene, wzxE gene, rffA gene, rffC gene, rffH gene, rffG gene, rffD gene, rffE gene, wzzE gene, rfe gene, rho gene, rhoL gene, trxA gene, rhlB gene, gpp gene, rep gene, ppiC gene, ilvC gene, ilvY gene, ilvA gene, ilvD gene, ilvE gene, ilvM gene, ilvG_2 gene, ilvG_1 gene, ilvL gene, hdfR gene, trpT
gene, aspT gene, rrfC gene, rrlC gene, gltU gene, rrsC gene, asnC gene, mioC gene, oriC gene, gidA gene, gidB gene, atpI gene, atpB gene, atpE gene, atpF gene, atpH gene, atpA gene, atpG gene, atpD gene, atpC gene, glmU gene, glms gene, psts gene, pstC gene, pstA gene, pstB gene, phoU gene, bglG gene, bglF gene, bglB gene, bglH gene, cbrC gene, mdtL gene, tnaB gene, tnaA gene, tnaC gene, trmE gene, rnpA gene, rpmH gene, dnaA gene, dnaN gene, recF gene, gyrB gene, ibpA gene, ibpB gene, emrD gene, ivbL gene, ilvB gene, ilvN gene, uhpA gene, uhpB gene, uhpC gene, uhpT gene, selC gene, glts gene, recG gene, trmH gene, spoT gene, rpoZ gene, gmk gene, ligB gene, dinD gene, rph gene, pyrE gene, ttk gene, dut gene, dfp gene, rpmB gene, rpmG gene, mutM gene, coaD gene, kdtA gene, rfaQ gene, rfaG gene, rfaP gene, rfaS gene, rfaB gene, rfal gene, rfaJ gene, rfaY gene, rfaZ gene, rfaK gene, rfaL gene, rfaC gene, rfaF gene, rfaD gene, htrL gene, kbl gene, tdh gene, envC gene, gpmI gene, grxC gene, secB gene, gpsA gene, cysE gene, lldD gene, lldR gene, lldP gene, mtlR gene, mtlD gene, mtlA gene, selA gene, selB gene, aldB gene, sgbE gene, sgbU gene, sgbH gene, lyxK gene, avtA gene, malS gene, bax gene, xylR gene, xylH gene, xylG gene, xylF gene, xylA gene, xylB gene, glyQ gene, glyS gene, hokA gene, cspA gene, tiaE gene, bisC gene, tag gene, eptB gene, proK gene, dppA gene, dppB gene, dppC gene, dppD gene, dppF gene, rdlD gene, ldrD gene, bcsG gene, bcsF gene, bcsE gene, bcsA gene, bcsB gene, bcsZ gene, bcsC gene, dctA gene, kdgK gene, treF gene, gadA gene, gadX gene, gadY gene, gadW gene, mdtF gene, mdtE gene, gadE gene, hdeD gene, hdeA gene, hdeB gene, slp gene, arsC gene, arsB gene, arsR gene, gor gene, prlC gene, uspA gene, pitA gene, nikR gene, nikE gene, nikD gene, nikC gene, nikB gene, nikA gene, acpT gene, dcrB gene, zntA gene, ftsY gene, ftsE gene, ftsX gene, rpoH gene, livJ gene, livK gene, livH gene, livM gene, livG gene, livF gene, ugpB gene, ugpA gene, ugpE gene, ugpC gene, ugpQ gene, ggt gene, gntR gene, gntK gene, gntU gene, asd gene, glgB gene, glgX gene, glgC gene, glgA gene, glgP gene, glpD gene, glpE gene, glpG gene, glpR gene, rtcR gene, rtcB gene, rtcA gene, malT gene, malP gene, malQ gene, gntT gene, gntY gene, gntX gene, bioH gene, feoB gene, feoA gene, greB gene, ompR gene, envZ gene, pck gene, hslO gene, hslR gene, nudE gene, mrcA gene, hofQ gene, aroK gene, aroB gene, damX gene, dam gene, rpe gene, gph gene, trpS gene, cysG gene, nirC gene, nirD gene, nirB gene, tsgA gene, ppiA gene, fic gene, pabA gene, argD gene, crp gene, prkB gene, kefG gene, kefB gene, slyD gene, slyX gene, fkpA gene, rpsL gene, rpsG gene, fusA gene, tufA gene, rpsJ gene, rplC gene, rplD gene, rplW gene, rplB gene, rpsS gene, rplV gene, rpsC gene, rplP gene, rpmC gene, rpsQ gene, rplN gene, rplX gene, rplE gene, rpsN gene, rpsH gene, rplF gene, rplR gene, rpsE gene, rpmD gene, rplo gene, secY gene, rpmJ gene, rpsM gene, rpsK gene, rpsD gene, rpoA gene, rplQ gene, zntR gene, mscL gene, trkA gene, rsmB gene, fmt gene, def gene, smf gene, smg gene, aroE gene, rrsE gene, ileU gene, alaU gene, rrlE gene, rrfE gene, metA gene, aceB gene, aceA gene, aceK gene, arpA gene, iclR gene, metH gene, pepE gene, lysC gene, pgi gene, ubiC gene, ubiA gene, plsB gene, dgkA gene, lexA gene, dinF gene, zur gene, qor gene, dnaB gene, alr gene, tyrB gene, aphA gene, uvrA gene, ssb gene, soxS gene, soxR gene, ryjA gene, actP gene, acs gene, nrfA gene, nrfB gene, nrfC gene, nrfD gene, nrfE gene, nrfF gene, nrfG gene, gltP gene, fdhF gene, rpiR gene, rpiB gene, proP gene, basS gene, basR gene, eptA gene, adiC gene, adiY gene, adiA gene, melR gene, melA gene, melB gene, fumB gene, dcuB gene, dcuR gene, dcuS gene, lysU gene, cadA gene, cadB gene, cadC gene, pheU gene, dipZ gene, cutA gene, dcuA gene, aspA gene, fxsA gene, groS gene, groL gene, efp gene, ecnA gene, ecnB gene, sugE gene, blc gene, ampC gene, frdD gene, frdC gene, frdB gene, frdA gene, poxA gene, psd gene, rsgA gene, orn gene, glyV gene, glyX gene, glyY gene, amiB gene, mutL gene, miaA gene, hfq gene, hflX gene, hflK gene, hflC gene, purA gene, rnr gene, rlmB gene, ulaF gene, rpsF gene, priB gene, rpsR gene, rplI gene, fklB gene, cycA gene, cpdB gene, cysQ gene, msrA gene, chpS gene, chpB gene, ppa gene, fbp gene, mpl gene, pmbA gene, cybC gene, nrdG gene, nrdD gene, tree gene, treB gene, treR gene, mgtA gene, pyrI gene, pyrB gene, pyrL gene, argI gene, valS gene, holC gene, pepA gene, idnR gene, idnT gene, idnO gene, idnD gene, idnK gene, leuX gene, dnaC gene, dnaT gene, bglJ gene, fhuF gene, leuV gene, leuP gene, leuQ gene, rsmC gene, holD gene, rimI gene, prfC gene, osmY gene, deoC gene, deoA gene, deoB gene, deoD gene, lplA gene, serB gene, radA gene, nadR gene, slt gene, trpR gene, rob gene, creA gene, creB gene, creC gene, creD gene, arcA gene
[2] fhuA gene, fhuC gene, fhuD gene, fhuB gene, yafJ gene, yafK gene, yafQ gene, dinJ gene, yafL gene, yafM gene, fhiA gene, mbhA gene, dinB gene, yafN gene, yafO gene, yafP gene, ykfJ gene, prfH gene, yagP gene, yagQ gene, yagR gene, yagS gene, yagT gene, yagU gene, ykgJ gene, yagV gene, yagW gene, yagX gene, yagY gene, yagZ gene, ykgK gene, ykgL gene, ykgM(JW5034) gene, ykgM(JW5035) gene, eaeH gene, ykgA gene, ykgB gene, ykgI gene, ykgC gene, ykgD gene, ykgE gene, ykgF gene, ykgG gene, ykgH gene, betA gene, betB gene, betI gene, betT gene, yahA gene, yahB gene, yahC gene, yahD gene, yahE gene, yahF gene, yahG gene, yahH gene, yahI gene, yahJ gene, yahK gene, yahL gene, yahM gene, yahN gene, yahO gene, prpR gene, prpB gene, prpC gene, prpD gene, prpE gene, codB gene, codA gene, cynR gene, cynT gene, cynS gene, cynX gene, lacA gene, lacY gene, lacZ gene, lacI gene, mhpR gene, mhpA gene, mhpB gene, mhpC gene, mhpD gene, mhpF gene, mhpE gene, mhpT gene, yaiL gene, frmB gene, frmA gene, frmR gene, yaiO gene, yaiX gene, yaiF gene, yaiP gene, yaiS gene, tauA gene, tauB gene, tauC gene, tauD gene, yaiT gene, yaiU gene, yaiV gene, ampH gene, sbmA gene, yaiW gene, yaiY gene, yaiZ gene, ddlA gene, yaiB gene, phoA gene, psiF gene, yaiC gene, ybbO gene, tesA gene, ybbA gene, ybbP gene, ylbG gene, ybbB gene, ybbS gene, allA gene, allR gene, glxR gene, ybbV gene, ybbW gene, allB gene, ybbY gene, glxK gene, ylbA gene, allC gene, allD gene, fdrA gene, ylbE_1 gene, ylbE_2 gene, ylbF gene, ybcF gene, envY gene, ybcH gene, nfrA gene, nfrB gene, cusS gene, cusR gene, cusC gene, cusF gene, cusB gene, cusA gene, pheP gene, ybdG gene, nfnB gene, ybdF gene, ybdJ gene, ybdK gene, hokE gene, ybeL gene, ybeQ gene, ybeR gene, djlB gene, ybeT gene, ybeU gene, djlc gene, hscC gene, rihA gene, gltL gene, gltK gene, gltJ gene, gltI gene, kdpF gene, ybfA gene, ybgA gene, phr gene, ybiA gene, dinG gene, ybiB gene, ybiC gene, ybiJ gene, ybiI gene, ybiX gene, fiu gene, ybiM gene, ybiN gene, ybiO gene, glnQ gene, glnP gene, glnH gene, yliA gene, yliB gene, yliC gene, yliD gene, yliE gene, yliF gene, yliG gene, yliH gene, yliI gene, yliJ gene, dacC gene, deoR gene, ybjG gene, ybjC gene, nfsA gene, rimK gene, ybjN gene, potF gene, potG gene, potH gene, potI gene, ybjO gene, rumB gene, artJ gene, artM gene, artQ gene, artI gene, artP gene, ybjP
gene, ybjQ gene, ybjR gene, ybjS gene, ybjT gene, ItaE gene, ymcC gene, ymcD gene, cspH gene, cspG gene, ymcE gene, gnsA gene, yccM gene, torS gene, torT gene, torR gene, torC gene, torA gene, torD gene, cbpM gene, cbpA gene, yccE gene, agp gene, yccJ gene, wrbA gene, ymdF gene, ycdG gene, ycdH gene, ycdI gene, rarA gene, ycdK gene, ycdL gene, ycdm gene, ycdC gene, putA gene, putP gene, ycdN_1 gene, ycdN_2 gene, ycdO gene, ycdB gene, phoH gene, ycdP gene, ycdQ gene, ycdR gene, ycdS gene, ycdT gene, ymdE gene, ycdU gene, elbA gene, ycgX gene, ycgE gene, ycgF gene, ycgZ gene, ymgA gene, ymgB gene,
ymgC gene, ycgG gene, ymgF gene, ycgH_1 gene, ycgH_2 gene, ymgD gene, ymgG gene, ymgH gene, ycgI gene, ydfH gene, ydfZ gene, ydfI gene, ydfJ gene, yecT gene, flhE gene, flhA gene, flhB gene, cheZ gene, cheY gene, cheB gene, cheR gene, tap gene, tar gene, cheW gene, cheA gene, motB gene, motA gene, flhC gene, flhD gene, yecG gene, otsA gene, otsB gene, araH gene, araG gene, araF gene, yecI gene, yecJ gene, isrB gene, yecR gene, ftn gene, yecH gene, tyrP gene, uvrC gene, uvrY gene, yecF gene, sdiA gene, yecC gene, yecS gene, yedO gene, fliY gene, fliZ gene, fliA gene, fliC gene, fliD gene, fliS gene, fliT gene, amyA gene, yedD gene, yedE gene, yedF gene, yedK gene, yedL gene, yedN_2 gene, yedN_1 gene, yedM gene, intG gene, fliE gene, fliF gene, fliG gene, fliH gene, fliI gene, fliJ gene, fliK gene, fliL gene, fliM gene, fliN gene, fliO gene, fliP gene, fliQ gene, fliR gene, rcsA gene, dsrB gene, yodD gene, yedA gene, vsr gene, dcm gene, yedJ gene, yedR gene, yedS_1 gene, yedS_2 gene, yedS_3 gene, hchA gene, yedV gene, yedW gene, yedX gene, yedY gene, yedZ gene, yodA gene, wbbL_2 gene, wbbL_1 gene, wbbK gene, wbbJ gene, wbbI gene, wbbH gene, glf gene, rfbX gene, rfbC gene, rfbA gene, rfbD gene, rfbB gene, yegE gene, alkA gene, yegD gene, yegI gene, yegJ gene, yegK gene, yegL gene, ryeC gene, ryeD gene, mdtA gene, mdtB gene, mdtC gene, mdtD gene, baeS gene, baeR gene, yegP gene, yegQ gene, ryeE gene, ogrK gene, yegZ gene, yegR gene, yegS gene, gatR_1 gene, gatR_2 gene, gatD gene, gatC gene, gatB gene, gatA gene, gatZ gene, gatY gene, fbaB gene, yegT gene, yegU gene, yegV gene, yegW gene, yegX gene, molR_1 gene, molR_2 gene, molR_3 gene, yehI gene, yehK gene, yehL gene, yehM gene, yehP gene, yehQ gene, yehR gene, yehs gene, yehT gene, yehU gene, mlrA gene, yohO gene, yehW gene, yehX gene, yehY gene, yehZ gene, bglX gene, dld gene, pbpG gene, yohC gene, yeiI gene, yeiJ gene, rihB gene, yeiL gene, yeiM gene, yeiN gene, yeiC gene, elaB gene, elaA gene, elaC gene, elaD gene, yfbK gene, yfbL gene, yfbM gene, yfbN gene, yfbO gene, yfbP gene, yfcC gene, yfcD gene, yfcE gene, yfcF gene, yfcG gene, folX gene, yfcH gene, yfcI gene, hisP gene, hisM gene, hisQ gene, hisJ gene, argT gene, ypdA gene, ypdB gene, ypdC gene, ypdD gene, ypdE gene, ypdF gene, ypdG gene, ypdH gene, pinH gene, ypjB gene, ypjC gene, ileY gene, ygaQ gene, ygaR gene, yqaC gene, yqaD gene, ygaT gene, csiR gene, ygaU gene, yqaE gene, ygaV gene, ygaP gene, stpA gene, ygaW gene, ygaC gene, ygaM gene, nrdH gene, nrdI gene, nrdE gene, proV gene, proW gene, proX gene, ygaX gene, ygaY gene, ygaZ gene, ygaH gene, srlA gene, srlE gene, srlB gene, srlD gene, gutM gene, srlR gene, gutQ gene, yqeG gene, yqeH gene, yqeI gene, yqeJ gene, yqeK gene, ygeF gene, ygeG gene, ygeH gene, ygeI gene, pbl gene, ygeK gene, ygeL gene, ygeM gene, ygeN gene, ygeO gene, ygeP gene, ygeQ gene, glyU gene, ygeR gene, xdhA gene, xdhB gene, xdhC gene, ygeV gene, ygeW gene, ygeX gene, ygeY gene, hyuA gene, yqeA gene, yqeB gene, yqeC gene, ygfJ gene, ygfK gene, ssnA gene, ygfM gene, xdhD gene, ygfO gene, guaD gene, ygfQ gene, ygfS gene, ygfT gene, ygfU gene, idi gene, cmtA gene, cmtB gene, yghD gene, yghE gene, yghF gene, yghG gene, pppA gene, yghJ gene, yghK gene, glcB gene, glcG gene, glcF gene, glcE gene, glcD gene, glcC gene, yghO gene, yghQ gene, yghR gene, yghS gene, yghT gene, ygiS gene, ygiT gene, ygiU gene, ygiV gene, ygiW gene, qseB gene, qseC gene, ygiZ gene, mdaB gene, ygiN gene, gltF gene, yhcA gene, yhcD gene, yhcE gene, yhcF gene, yhcG gene, yhcH gene, nanK gene, nanE gene, yhdJ gene, yhdU gene, envR gene, acrE gene, acrF gene, yhdV gene, yhdW gene, yhdX gene, yhdY gene, yijP gene, yijO gene, frwD gene, pflc gene, pflD gene, frwB gene, frwC gene, ptsA gene, rhaT gene, rhaR gene, rhaS gene, rhaB gene, rhaA gene, rhaD gene, yiiL gene, frvA gene, frvB gene, frvX gene, frvR gene, yiiG gene, yiiF gene, yiiE gene, yiiD gene, dtd gene, rbn gene, yihX gene, yihW gene, yihV gene, yihU gene, yihT gene, yihS gene, yihR gene, yihQ gene, yihP gene, yihO gene, ompL gene, yihN gene, yihM gene, yihL gene, hsrA gene, rbsR gene, rbsK gene, rbsB gene, rbsC gene, rbsA gene, rbsD gene, trkD gene, yieN gene, yieM gene, asnA gene, yidB gene, yidA gene, yidX gene, dgoR gene, dgoK gene, dgoA gene, dgoD gene, dgoT gene, cbrA gene, yidR gene, yidQ gene, yidE gene, yidP gene, glvC gene, glvB gene, glvG gene, ysdC gene, yidL gene, yidK gene, yidJ gene, yidI gene, yidH gene, yidG gene, yidF gene, yhiN gene, yhiM gene, yhiL gene, yhiK gene, yhiJ gene, yhiI gene, rbbA gene, yhhJ gene, chiA gene, bfd gene, bfr gene, gspO gene, gspM gene, gspL gene, gspK gene, gspJ gene, gspI gene, gspH gene, gspG gene, gspF gene, gspE gene, gspD gene, gspC gene, gspA gene, pioO gene, yjbE gene, yjbF gene, yjbG gene, yjbH gene, yjbA gene, xylE gene, malG gene, malF gene, malE gene, malK gene, lamB gene, malM gene, yjbI gene, yjcP gene, yjcQ gene, yjcR gene, yjcS gene, alsK gene, alsE gene, alsC gene, alsA gene, alsB gene, yjdP gene, phnP gene, phnO gene, phnN gene, phnM gene, phnL gene, phnK gene, phnJ gene, phnI gene, phnH gene, phnG gene, phnF gene, phnE(JW4064) gene, phnE(JW4065) gene, phnD gene, phnC gene, phnB gene, phnA gene, yjdA gene, yjcZ gene, yjfI gene, yjfJ gene, yjfK gene, yjfL gene, yjfM gene, yjfC gene, aidB gene, yjfN gene, yjfO gene, yjfP gene, ulaR gene, ulaG gene, ulaA gene, ulaB gene, ulaC gene, ulaD gene, ulaE gene, yjhS gene, yjhT gene, yjhA gene, fimB gene, fimE gene, fimA gene, fimI gene, fimC gene, fimD gene, fimF gene, fimG gene, fimH gene, gntP gene, uxuA gene, uxuB gene, uxuR gene, yjiC gene, yjiD gene, yjiE gene, iadA gene, yjiG gene, yjiH gene, kptA gene, yjiJ gene, yjiK gene, yjiL gene, yjiM gene, yjiN gene, yjiO gene, yjiP gene, yjiQ gene, yjiR gene, yjiS gene, yjiT gene, yjiV gene, mcrC gene, mcrB gene, yjiW gene, hsdS gene, hsdM gene, hsdR gene, mrr gene, yjiA gene, yjiX gene, yjiY gene, tsr gene, yjiZ gene, yjjM gene, yjjN gene, mdoB gene, yjjA gene
[3] chpA gene, uspG gene, ybdR gene, rnk gene, rna gene, citT gene, citG gene, citX gene, citF gene, citE gene, citD gene, citC gene, citA gene, citB gene, dcuC gene, crcA gene, cspE gene, crcB gene, ybeH gene, ybeM gene, yqcA gene, yqcB gene, yqcC gene, syd gene, yqcD gene, ygdH gene, sdaC gene, sdaB gene, exo gene, fucO gene, fucA gene, fucP gene, fucI gene, fucK gene, fucU gene, fucR gene, ygdE gene, ygdD gene, gcvA gene, ygdI gene, csdA gene, ygdK gene, araD gene, araA gene, araB gene, araC gene, yabI gene, thiQ gene, thiP gene, tbpA gene, sgrR gene, setA gene, yhfZ gene, yhfY gene, yhfX gene, yhfW gene, php gene, yhfU gene, yhfT gene, yhfS gene, frlR gene, frlD gene, frlc gene, frlB gene, frlA gene, yhfL gene, pncA gene, ydjE gene, ydjF gene, ydjG gene, ydjH gene, ydjI gene, ydjJ gene, ydjK gene, ydjL gene, yeaC gene, yeaA gene, ade gene, yicO gene, yicN gene, yicM gene, yicS gene, nlpA gene, yicL gene, setC gene, ygjI gene, ygjJ gene, ygjK gene, fadH gene, ygjM gene, ygjN gene, ygjO gene, ygjP gene, ygjQ gene, ygjR gene, alx gene, sstT gene, ygjV gene, yrhB gene, yrhA gene, yhhZ gene, yhhY gene, yhhX gene, yhhW gene, yeaK gene, yoaI gene, yeaL gene, yeaM gene, yeaN gene, yeaO gene, yoaF gene, yeaP gene, yeaQ gene, yoaG gene, yeaR gene, yeas gene, yeaT gene, yeaU gene, yeaV gene, yeaW gene, yeaX gene, hyfA gene, hyfB gene, hyfC gene, hyfD gene, hyfE gene, hyfF gene, hyfG gene, hyfH gene, hyfI gene, hyfJ gene, hyfR gene, and focB gene.

24. The *E. coli* mutant strain obtained via modification of the *E. coli* mutant strain according to any one of claims 1 to 23, by a method selected from among the following [1] to [5]:
[1] a method for reducing or removing at least one mechanism for regulating biosynthesis of the useful substance;
[2] a method for increasing the expression of at least one enzyme associated with biosynthesis of the useful substance;
[3] a method for increasing the copy number of at least one enzyme gene associated with biosynthesis of the useful substance;
[4] a method for attenuating or blocking at least one metabolic pathway which branches from the biosynthetic pathway of the useful substance into metabolites other than the useful substance; or
[5] a method for selecting a strain that exhibits higher resistance to an analogue of a useful substance than a wild-type *E. coli* strain.

25. The *E. coli* mutant strain according to any one of claims 1 to 24, wherein the *E*. *coli* strain is the *E. coli* K-12, B, or W strain.

26. The *E. coli* mutant strain according to claim 25, wherein the *E. coli* K-12 strain is the *E. coli* W3110 strain (ATCC27325) or the *E. coli* MG1655 strain (ATCC47076).

27. A method for producing a useful substance comprising culturing the *E. coli* mutant strain according to any one of claims 1 to 26, in a medium so as to generate and accumulate the useful substance in the culture and recovering the useful substance from the culture.

28. The method according to claim 27, wherein the useful substance is selected from the group consisting of proteins, amino acids, nucleic acids, vitamins, saccharides, organic acids, and lipids.
